(19) Europäisches Patentamt
European Patent Office
Office européen des brevets

(11) EP 2 431 743 B1

(12) EUROPEAN PATENT SPECIFICATION

(45) Date of publication and mention of the grant of the patent:
**30.05.2018 Bulletin 2018/22**

(51) Int Cl.:
***G01N 33/574*** *(2006.01)* ***A61K 31/715*** *(2006.01)*

(21) Application number: **11189389.7**

(22) Date of filing: **20.07.2006**

(54) **Cancer specific glycans and use thereof**

Krebsspezifische Glycane und deren Verwendung

Glycanes spécifiques du cancer et leur utilisation

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.07.2005 FI 20055417**

(43) Date of publication of application:
**21.03.2012 Bulletin 2012/12**

(62) Document number(s) of the earlier application(s) in accordance with Art. 76 EPC:
**06764481.5 / 1 910 838**

(73) Proprietor: **Glykos Finland Oy**
**00790 Helsinki (FI)**

(72) Inventors:
- **Satomaa, Tero**
  **00700 Helsinki (FI)**
- **Natunen, Jari**
  **01520 Vantaa (FI)**
- **Heiskanen, Annamari**
  **00370 Helsinki (FI)**
- **Olonen, Anne**
  **15700 Lahti (FI)**
- **Saarinen, Juhani**
  **00370 Helsinki (FI)**
- **Salovuori, Noora**
  **00810 Helsinki (FI)**
- **Helin, Jari**
  **05200 Rajamäki (FI)**

(74) Representative: **Seppo Laine Oy**
**Itämerenkatu 3 B**
**00180 Helsinki (FI)**

(56) References cited:
**WO-A2-00/61636 US-A- 5 902 725**
**US-A1- 2004 147 033**

- **BROCKHAUSEN I: "Pathways of O-glycan biosynthesis in cancer cells", BIOCHIMICA ET BIOPHYSICA ACTA - GENERAL SUBJECTS, ELSEVIER SCIENCE PUBLISHERS, NL, vol. 1473, no. 1, 17 December 1999 (1999-12-17), pages 67-95, XP004276502, ISSN: 0304-4165, DOI: 10.1016/S0304-4165(99)00170-1**
- **MUELLER S ET AL: "Recombinant MUC1 probe authentically reflects cell-specific O-glycosylation profiles of endogenous breast cancer mucin. High density and prevalent core 2-based glycosylation", JOURNAL OF BIOLOGICAL CHEMISTRY, THE AMERICAN SOCIETY OF BIOLOGICAL CHEMISTS, INC, US, vol. 277, no. 29, 19 July 2002 (2002-07-19), pages 26103-26112, XP002264326, ISSN: 0021-9258, DOI: 10.1074/JBC.M202921200**
- **YOUSEFI S ET AL: "INCREASED UDP-GLCNAC GAL-BETA-1-3-GALNAC-R GLCNAC TO GALNAC BETA-1 6-N-ACETYLGLUCOSAMINYLTRANSFERASE ACTIVITY IN METASTATIC MURINE TUMOR CELL LINES CONTROL OF POLYLACTOSAMINE SYNTHESIS", JOURNAL OF BIOLOGICAL CHEMISTRY, vol. 266, no. 3, 1991, pages 1772-1782, XP55017250, ISSN: 0021-9258**
- **BURCHELL JOY ET AL: "An alpha2,3 sialyltransferase (ST3Gal I) is elevated in primary breast carcinomas", GLYCOBIOLOGY, vol. 9, no. 12, December 1999 (1999-12), pages 1307-1311, XP55017255, ISSN: 0959-6658**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 431 743 B1

- **DUBE DANIELLE H ET AL: "Glycans in cancer and inflammation--potential for therapeutics and diagnostics", NATURE REVIEWS. DRUG DISCOVERY, NATURE PUBLISHING GROUP, GB, vol. 4, no. 6, 1 June 2005 (2005-06-01), pages 477-488, XP002522067, ISSN: 1474-1776, DOI: 10.1038/NRD1751**
- **KUMAMOTO K ET AL: "Specific detection of sialyl Lewis X determinant carried on the mucin GlcNAcbeta1-->6GalNAcalpha core structure as a tumor-associated antigen.", BIOCHEMICAL AND BIOPHYSICAL RESEARCH COMMUNICATIONS 18 JUN 1998 LNKD- PUBMED:9642161, vol. 247, no. 2, 18 June 1998 (1998-06-18) , pages 514-517, XP55027328, ISSN: 0006-291X**
- **GUTIÉRREZ GALLEGO RICARDO ET AL: "Enzymatic synthesis of the core-2 sialyl Lewis X O-glycan on the tumor-associated MUC1a' peptide", BIOCHIMIE, MASSON, PARIS, FR, vol. 85, no. 3-4, 1 March 2003 (2003-03-01), pages 275-286, XP002532266, ISSN: 0300-9084, DOI: 10.1016/S0300-9084(03)00050-6**
- **LLOYD K O ET AL: "COMPARISON OF O-LINKED CARBOHYDRATE CHAINS IN MUC-1 MUCIN FROM NORMAL BREAST EPITHELIAL CELL LINES AND BREAST CARCINOMA CELL LINESDEMONSTRATION OF SIMPLER AND FEWER GLYCAN CHAINS IN TUMOR CELLS", JOURNAL OF BIOLOGICAL CHEMISTRY, THE AMERICAN SOCIETY OF BIOLOGICAL CHEMISTS, INC, US, vol. 271, no. 52, 27 December 1996 (1996-12-27), pages 33325-33334, XP000942228, ISSN: 0021-9258, DOI: 10.1074/JBC.271.52.33317**
- **MEICHENIN M ET AL: "Tk, a new colon tumor-associated antigen resulting from altered O-glycosylation1", CANCER RESEARCH, AMERICAN ASSOCIATION FOR CANCER RESEARCH, US, vol. 60, 1 October 2000 (2000-10-01), pages 5499-5507, XP002960527, ISSN: 0008-5472**
- **PAZYNINA ET AL: "Synthesis of N-acetyllactosamine-terminated oligosaccharides - fragments of glycoprotein O-chains", MENDELEEV COMMUNICATIONS, INSTITUTE OF PHYSICS PUBLISHING, BRISTOL, GB, vol. 12, no. 5, 1 January 2002 (2002-01-01), pages 183-184, XP022541516, ISSN: 0959-9436, DOI: 10.1070/MC2002V012N05ABEH001653**
- **BURCHELL J M ET AL: "O-linked glycosylation in the mammary gland: changes that occur during malignancy.", JOURNAL OF MAMMARY GLAND BIOLOGY AND NEOPLASIA JUL 2001 LNKD- PUBMED:11547903, vol. 6, no. 3, July 2001 (2001-07), pages 355-364, XP55027155, ISSN: 1083-3021**
- **ELLIOTT M M ET AL: "Carbohydrate and peptide structure of the alpha- and beta-subunits of human chorionic gonadotropin from normal and aberrant pregnancy and choriocarcinoma.", ENDOCRINE AUG 1997 LNKD- PUBMED:9449027, vol. 7, no. 1, August 1997 (1997-08), pages 15-32, XP55026908, ISSN: 1355-008X**
- **MIZUOCHI T ET AL: "STRUCTURES OF THE ASPARAGINE LINKED SUGAR CHAINS OF HUMAN CHORIONIC GONADOTROPIN PRODUCED IN CHORIO CARCINOMA APPEARANCE OF TRI ANTENNARY SUGAR CHAINS AND UNIQUE BI ANTENNARY SUGAR CHAINS", JOURNAL OF BIOLOGICAL CHEMISTRY, vol. 258, no. 23, 1983, pages 14126-14129, XP55026906, ISSN: 0021-9258**

## Description

FIELD OF THE INVENTION

The present disclosure relates to glycans, which are specifically expressed by certain cancer cells, tumours and other malignant tissues. The present disclosure describes methods to detect cancer specific glycans.

PRIOR ART

Brockhausen et al., 1999 (Biochimica et Biophysica Acta - General Subjects, 1473(1);67-95) disclose pathways of O-glycan biosynthesis in cancer cells. The authors also disclose that cancer diagnosis may be based on the appearance of certain glycosylated epitopes, and therapeutic avenues have been designed to attack cancer cells via their glycans.

Mueller et al., 2002 (Journal of Biological Chemistry, 277(29):26103-26112) disclose a recombinant MUC1 probe authentically reflecting cell-specific O-glycosylation profiles of endogenous breast cancer mucin.

Yousefi et al., 1991 (Journal of Biological Chemistry, 266(3):1772-1782) disclose increased UDP-GlcNAc:Gal beta 1-3GaLNAc-R (GlcNAc to GaLNAc) beta-1, 6-N-acetylglucosaminyltransferase activity in metastatic murine tumor cell lines.

Burchell et al., 1999 (Glycobiology, 9(12):1307-1311) disclose that an alpha2,3 sialyltransferase (ST3Gal I) is elevated in primary breast carcinomas. The authors also disclose that the mucin expressed by breast carcinomas has shorter side-chains, often consisting of sialylated core 1 (Galbeta1-3GalNAc).

Dube et al., 2005 (Nature Reviews. Drug discovery, 4(6); 477-488) disclose that glycans in cancer and inflammation are potential targets for therapeutics and diagnostics.

US 5 902 725 discloses the detection of prostate and other cancers by assaying for cancer-specific antigens having linked oligosaccharides which are at least triantennary.

US 2004/147033 discloses glycan markers for diagnosing and monitoring cancer.

Kumamoto et al., 1998 (Biochemical and Biophysical Research Communications, 247(2):514-517) disclose the specific detection of sialyl Lewis X determinant carried on the mucin GlcNAcbeta1-->6GalNAcalpha core structure as a tumor-associated antigen.

Gutiérrez Gallego et al., 2003 (Biochimie, 85(3-4):275-286) disclose enzymatic synthesis of the core-2 sialyl Lewis X O-glycan on the tumor-associated MUC1a' peptide.

Lloyd et al., 1996 (Journal of Biological Chemistry, 271(52):33325-33334) disclose comparison of O-linked carbohydrate chains in MUC-1 mucin from normal breast epithelial cell lines and breast carcinoma cell lines.

Meichenin et al., 2000 (Cancer Research, 60:5499-5507) disclose Tk, a new colon tumor-associated antigen resulting from altered O-glycosylation.

Pazynina et al., 2002 (Mendeleev Communications, 12(5): 183-184) disclose synthesis of N-acetyllactosamine-terminated oligosaccharides and fragments of glycoprotein O-chains.

Burchell et al., 2001 (Journal of Mammary Gland Biology and Neoplasia, 6(3):355-364) disclos changes of O-linked glycosylation that occur during malignancy in the mammary gland.

Elliot et al., 1997 (Endocrine 7(1):15-32) disclose a carbohydrate and peptide structure of the alpha- and beta-subunits of human chorionic gonadotropin from normal and aberrant pregnancy and choriocarcinoma.

Mizuochi et al., 1983 (Journal of Biological Chemistry 258(23):14126-14129) disclose structures of the asparagine-linked sugar chains of human chorionic gonadotropin produced in choriocarcinoma.

WO 00/61636 discloses the level of aberrantly glycosylated IgGs in serum is correlated to the development of cancer. The authors have found that the ratio of aberrantly glycosylated IgGs versus normal IgGs is significantly increased in cancer patients compared with normal individuals, and that such ratio increases as the cancer progresses.

SUMMARY OF THE INVENTION

The present invention is directed to a method of evaluating the malignancy of a human patient sample comprising the step of detecting the presence or amount of cancer related oligosaccharide sequence in the sample, said oligosaccharide sequence consisting of a neutral O-glycan

Galβ4GlcNAcβ6(Galβ3)GalNAc or Galβ4(Fucα3)GlcNAcβ6(Galβ3)GalNAc.

Preferably, high relative expression rate of said oligosaccharide sequence is indicative of malignant cancer.

The present invention is also directed to a pharmaceutical composition comprising a human antibody specifically against a neutral O-glycan consisting of Galβ4GlcNAcβ6(Galβ3)GalNAc or Galβ4(Fucα3)GlcNAcβ6(Galβ3)GalNAc for use in the treatment of cancer.

Preferably, said antibody in said pharmaceutical composition is used to target a cell killing chemotherapeutics medicine to cancer cells

BRIEF DESCRIPTION OF THE DRAWINGS

**Figure 1.** Example of α-mannosidase digestion schemes of, **A.** healthy lung and lung cancer tumor sample pairs from 7 patients with non-small cell lung adenocarcinoma, and **B.** breast cancer tumor samples from 7 patients with ductale breast adenocarcinoma. The m/z figures in the x-axis correspond to the approximate m/z values of $[M+Na]^+$ adduct ions. The figures in the y-axis correspond to relative glycan signal intensities. The difference between glycan intensities before and after α-mannosidase digestion is equal to the amount of non-reducing terminal α-mannose containing structures.

**Figure 2.** Structural features of Structure group 1 glycans, namely **A.** non-fucosylated glycans Man$\alpha_{0-3}$Manβ4GlcNAcβ4GlcNAc(β-*N*-Asn), and **B.** fucosylated glycans Man$\alpha_{0-4}$Manβ4GlcNAcβ4(Fucα6)GlcNAc(β-*N*-Asn).

**Figure 3.** Example of mass spectrometric analysis of glycans expressed in tumor and healthy control tissues of a non-small cell lung adenocarcinoma patient. **A.** Neutral glycan mass spectrum from the lung cancer tumor, **B.** neutral glycan mass spectrum from healthy lung tissue from the same patient, and **C.** illustration of the differences in spectra A. and B., with regard to the present structure group glycans. The m/z figures in the x-axis correspond to the approximate m/z values of $[M+Na]^+$ adduct ions. The figures in the y-axis correspond to relative glycan signal intensities.

**Figure 4.** Graph presenting the average relative glycan signal intensities in breast cancer tumor - healthy breast tissue sample pairs from 9 patients with ductale breast adenocarcinoma. The m/z figures in the x-axis correspond to the approximate m/z values of $[M+Na]^+$ adduct ions. The figures in the y-axis correspond to relative glycan signal intensities.

**Figure 5.** Graph presenting the average relative glycan signal intensities in breast cancer tumor - healthy breast tissue sample pairs from 7 patients with lobulare breast adenocarcinoma. The m/z figures in the x-axis correspond to the approximate m/z values of $[M+Na]^+$ adduct ions. The figures in the y-axis correspond to relative glycan signal intensities.

**Figure 6.** Example of α-mannosidase digestion schemes of healthy lung and lung cancer tumor sample pairs from 7 patients with non-small cell lung adenocarcinoma. The m/z figures in the x-axis correspond to the approximate m/z values of $[M+Na]^+$ adduct ions. The figures in the y-axis correspond to relative glycan signal intensities. The glycan signal intensities that remain after α-mannosidase digestion are indicative of the presence of α-mannosidase resistant structures.

**Figure 7.** Structural features of Structure group 2 glycans, including **a)** Core 2 type O-glycan structures, and **b)** extended Core 1 type O-glycan structures.

**Figure 8.** Example of mass spectrometric analysis of glycans expressed in tumor and healthy control tissues of a lung cancer patient. **A.** Neutral glycan sample from non-small cell lung adenocarcinoma. **B.** Neutral glycan sample from healthy lung from the same patient. The m/z figures in the x-axis correspond to the approximate m/z values of $[M+Na]^+$ adduct ions. The figures in the y-axis correspond to relative glycan signal intensities.

**Figure 9.** Graph presenting the average glycan signal intensities in breast cancer tumor - healthy breast tissue sample pairs from 9 patients with ductale breast adenocarcinoma. The m/z figures in the x-axis correspond to the approximate m/z values of $[M+Na]^+$ adduct ions. The figures in the y-axis correspond to relative glycan signal intensities.

**Figure 10.** Graph presenting the average glycan signal intensities in breast cancer tumor - healthy breast tissue sample pairs from 7 patients with lobulare breast adenocarcinoma. The m/z figures in the x-axis correspond to the approximate m/z values of $[M+Na]^+$ adduct ions. The figures in the y-axis correspond to relative glycan signal intensities.

**Figure 11.** The structure of the O-glycan fragment Neu5Acα3Galβ4GlcNAcβ6(2-acetamido-3-amino-2,3-dideoxy)hexose, and experiments done to characterize the structure.

**Figure 12.** MALDI-TOF mass spectrometric fragmentation analysis (PSD) of:

**A.** deuteroacetylated m/z 899 $[M+Na]^+$ glycan fragment at m/z 944, and
**B.** deuteroacetylated m/z 608 $[M+Na]^+$ glycan fragment at m/z 653. The m/z figures in the x-axis correspond to the approximate m/z values of either $[M+Na]^+$ or $[M+H]^+$ adduct ions. The figures in the y-axis correspond to relative glycan signal intensities. The nomenclature of the fragment ions is after Domon and Costello (1988).

**Figure 13.** Example MALDI-TOF mass spectra from tumor tissue samples of a non-small cell lung adenocarcinoma patient. **A.** Positive-ion mode mass spectrum of the neutral glycan fraction, **B.** negative-ion mode mass spectrum of the neutral glycan fraction, and **C.** negative-ion mode mass spectrum of the sialylated glycan fraction. The m/z figures in the x-axis correspond to the approximate m/z values of either $[M+Na]^+$ adduct ions (A.) or $[M-H]^-$ ions (B. and C.). The figures in the y-axis correspond to relative glycan signal intensities.

**Figure 14.** Example of MALDI-TOF mass spectrometric analysis of glycans expressed in tumor and healthy control tissues of patients with either malignant ovarian cystadenocarcinoma (**A.** and **C.**) or benign ovarian cystadenoma (**B.** and **D.**). **A.** and **B.** Negative-ion mode mass spectra of the sialylated glycan fractions. **C.** and **D.** Positive-ion mode mass spectra of the neutral glycan fractions. The m/z figures in the x-axis correspond to the approximate m/z values of either $[M-H]^-$ ions (A. and B.) or $[M+Na]^+$ adduct ions (C. and D.). The figures in the y-axis correspond to relative glycan signal intensities.

**Figure 15.** Example of mass spectrometric analysis of glycans expressed in tumor and healthy control tissues of 9 ductale breast cancer patients. The m/z figure in the x-axis corresponds to the $[M+Na]^+$ adduct ion with an approximate m/z value of 899. The figures in the y-axis correspond to relative glycan signal intensities in 1/1000.

**Figure 16.** Example of mass spectrometric analysis of glycans expressed in tumor and healthy control tissues of 7 lobulare breast cancer patients. The m/z figure in the x-axis corresponds to the $[M+Na]^+$ adduct ion with an approximate m/z value of 899. The figures in the y-axis correspond to relative glycan signal intensities in 1/1000.

**Figure 17.** Neutral protein-linked glycan profiles from non-small cell lung adenocarcinoma patients, *x-axis:* approximate mass-to-charge ratio (m/z) of the glycan signals, corresponding to mass of $[M+Na]^+$ ions; *y-axis:* relative abundance of the glycan within the neutral glycan fraction. **Light columns:** healthy lung; **Dark columns:** non-small cell lung adenocarcinoma.

**Figure 18.** MALDI-TOF mass spectrometric analysis of sialylated high-molecular weight protein-linked glycans from **A.** normal ovary tissue, **B.** benign ovarian cystadenoma tumor, and **C.** malignant ovarian cystadenocarcinoma tumor. *x-axis:* mass-to-charge ratio (m/z) of the detected signals at 1500-4000 Da, corresponding to mass of $[M-H]^-$ ions; *y-axis:* relative signal intensity, 0-100 %; *N*: $[M-2H+Na]^-$ ion at +22 Da; K: $[M-2H+K]^-$ ion at +38 Da; *asterisk:* $H_2O$ elimination product at -18 Da; *numbering* of glycan signals refers to **Table 8.**

**Figure 19.** Neutral protein-linked glycan profiles from **A.** normal ovary tissue (1 patient), **B.** benign ovarian cystadenoma tumor (average of 5 patients), and **C.** malignant ovarian cystadenocarcinoma tumor (average of 4 patients). *x-axis:* approximate mass-to-charge ratio (m/z) of the glycan signals, corresponding to mass of $[M+Na]^+$ ions; *y-axis:* relative abundance of the glycan within the neutral glycan fraction. Column code from left to right, **dark columns:** normal ovary; **blank columns:** benign tumor; **light columns:** malignant tumor.

**Figure 20.** MALDI-TOF post-source decay (PSD) mass spectrometric fragmentation analysis of two isomeric glycan structures (**I** and **II**) present in a major sialylated glycan signal of ovarian tumors and normal ovarian tissue. The parent ion corresponds to the sodium adduct ion of permethylated $NeuAc_1Hex_4HexNAc_5dHex_1$. The fragment ions appear as sodium adduct ions, except for m/z 376.5 that is apparently protonated, and they are abbreviated as described by Domon and Costello (1988). The evidence suggests that structure **I** carries a non-reducing terminal HexNAc-HexNAc sequence whereas structure **II** carries a non-reducing terminal NeuAc-HexNAc-HexNAc sequence.

**Figure 21.** Discrimination analysis of neutral protein-linked glycans analysis results of malignant and benign tumors and healthy tissue samples from the same tissues of **A.** breast, **B.** ovary, and **C.** colon. The discrimination is based

on relative abundancies of three neutral protein-linked glycans identified in principal component analysis and an experimental discrimination formula derived from a randomly picked training group of breast cancer patients, as described in the text. The scores resulting from individual samples are plotted on the y-axis.

**Figure 22.** Example of glycan signal analysis of MALDI-TOF mass spectrometric data. **A.** Mass spectrometric raw data showing a window of neutral N-glycan mass spectrum in positive ion mode, **B.** Glycan profile generated from the data in A.

**Figure 23.** Example of glycan signal analysis of MALDI-TOF mass spectrometric data. **A.** Mass spectrometric raw data showing a window of sialylated N-glycan mass spectrum in negative ion mode, **B.** Glycan profile generated from the data in A.

**Figure 24.** Neutral protein-linked glycans of normal lung tissue **(light columns)** and lung cancer tumor **(dark columns).**

**Figure 25.** Neutral and acidic N-glycan profiles of lysosomal protein sample.

**Figure 26.** Reference neutral N-glycan structures for NMR analysis (A-C) in **Table 11.**

**Figure 27.** Reference neutral N-glycan structures for NMR analysis (D-G) in **Table 12.**

**Figure 28.** Reference acidic N-glycan structures for NMR analysis (A-E) in **Table 13.**

**Figure 29.** Neutral protein-linked glycans of normal breast tissue (**light columns**) and ductale type breast carcinoma tumor (**dark columns**) from the same patient.

**Figure 30.** Neutral protein-linked glycans of normal lymph node tissue (**light columns**) and ductale type breast carcinoma lymph node metastasis (**dark columns**) from the same patient.

**Figure 31.** Acidic protein-linked glycans of normal breast tissue (**light columns**) and ductale type breast carcinoma tumor (**dark columns**) from the same patient.

**Figure 32.** Acidic protein-linked glycans of normal lymph node tissue (**light columns**) and ductale type breast carcinoma lymph node metastasis (**dark columns**) from the same patient.

**Figure 33.** Periodate oxidation analysis of cancer derived neutral N-glycans.

**Figure 34.** Periodate oxidation analysis of cancer derived low-mannose type N-glycan.

DETAILED DESCRIPTION OF THE INVENTION

**Analysis of cancer glycomes**

The present disclosure reveals that quantitative analysis of human glycomes is useful for analysis of human cancers. The glycome analysis revealed profiles of glycan expression, which can be used for the analysis of glycomes even without knowledge of exact structures of glycans. The present disclosure is directed to quantitative mass spectromettic profiling of human cancers and analysis of alterations in cancer in comparision with normal corresponding normal tissues. The analysis can be performed based on signals corresponding to glycan structures, these signals were translated to likely monosaccharide compositions and further analysed to reveal structures and correlations between the signals. The disclosure is especially directed to analysis of O-glycan glycomes derived from cancer proteins. The glycans are analysed as neutral and/or acidic signals and glycan mixtures, multiple analysis methods are preferred to obtain maximal amount of data. The disclosure is also directed to methods for analysis of mixture of N-glycans and O-glycans released together.

*LacNAc O-glycans*

O-glycans characteristic for cancer are referred as LacNAc O-glycans which comprise at least one N-acetyllactosamine unit with possible fucosyl-modifications, and which is linked a specific O-glycan core type Galβ3GalNAc

forming characteristic monosaccharide compositions in O-glycan mass spectra.
The preferred structures among the LacNAc O-glycans includes core II structure- type O-glycan structures comprising the branched structure LacNAcβ6(Galβ3)GalNAc. The preferred terminal non-sialyated minimal (group 2) structures also include Galβ4(Fucα3)GlcNAcβ6(Galβ3)GalNAc.

Examples of useful antibody types fro the recognition of neutral core II O-glycans in cancer include antibodies reported to bind Galβ4GlcNAcβ6(Galβ3)GalNAc (Hep27-Mouse monoclonal antibody, Sandee D. et al. (2002) J. Biosci. Bioengin. (1993) 266-273, possible related application JP10084963, TOSOH corp; different antibody JP6046880 Ryuichi Horie. et al), and Galβ4GlcNAcβ6GalNAc (Chung Y-S et al. EP0601859, TOSOH corp). The Sandee publication reports reactivity of the antibody with a human cultivated hepatocellular carcinoma cell line (HCC-S102, not actual cancer), fetal liver , possible undisclosed cancers (relevance to human cancers or to human cultivated cell lines cannot be known) and not to adult liver, the article discusses heaptocellular carcinoma HCC based on the cell line result. Galβ4GlcNAcβ6GalNAcαCer (lipid) binding antibody (FI alpha-75) was suggested for cancers of digestive organ, specifically stomach (Chung Y-S et al.), results show 79% reaction with stomach cancers, when faint labelling is counted, 38 % reactivity with colon cancers and, 57 % for colon cancers in immunohistochemistry. The antibodies were reported to be specific for glycolipids but the present invention reveals that this type of antibodies are also useful for analysis of cancer glycoproteins. The invention furthermore reveals new indication for the antibodies, especially one specific for Galβ4GlcNAcβ6(Galβ3)GalNAc.

Though the prior art implied certain indications usefulness for other indications cannot be known and based on the interest of the company producing the antibody it would appear likely that they would have tested all possible cancers but unfortunately failed with revealing the novel present indications, to which phe present invention is especially directed to.

Examples of useful antibody types fro the recognition of acidic core II O-glycans in cancer include antibody CHO-131 (Walcheck B. et al. Blood (2002) 99, 4063-69) reported to bind specifically core II sLex glycan

NeuNAcα3Galβ4(Fucα3)GlcNAcβ6(Galβ3)GaNAcαSer/Thr-peptide.

*Additional analysis of glycans in cancer involving recognition of carrier proteins*

The disclosure is further directed to methods of analysing any of the glycans from cancer derived proteins, preferably integral (cell bound/transmembrane) cancer tissue or cell released proteins and assigning the glycan structures with specific carrier protein, preferably by specific purification of the protein, e.g. by affinity methods such as immunoprecipitation or by sequencing, preferably by mass spectrometric sequencing, glycopeptides including sequencing and recognizing peptides and thus proteins linked to the proteins.

## The target cancer tissue

The present disclosure is directed to analysis of un-normally transformed tissues, when the transformation is benign and/or malignant cancer type transformation referred as cancer (or tumor). It is realized that benign transformation may be a step towards malignant transformation, and thus the benign cancers are also useful to be analysed and differentiated from normal tissue, which may have also non cancerous or non-transformation related alterations such as swelling or trauma related to physical or e.g. infectious trauma, and it is useful and preferred to differentiate with benign and malignant cancers

Preferably the tissue is human tissue or tissue part such as liquid tissue, cell and/or solid polycellular tumors, and in another alternative preferably a solid human tissue. The solid tissues are preferred for the analysis and/or targeting specific glycan marker structures from the tissues, including intracellulalarily and extracellullarily, preferably cell surface associated, localized markers. The present disclosure is specifically directed to the recognition of cell surface localized and/or mostly cell surface localized marker structures from solid tumor tissues or parts thereof. It is realized that the contacts between cells and this glycomes madiateing these are are affected by presence of cells as solid tumor or as more individual cells. The preferred individual cell type cancers or tumors include preferably blood derived tumors such as leukemias and lymphomas, while solid tumors are preferably includes solid tumors derived from solid tissues suchs gastrointestinal tract tissues, other internal organs such as liver, kidneys, spleen, lungs, gonads and associated organs including preferably ovary, testicle, and prostate. The disclosure further reveals markers from individually or multicellularily presented cancer cells in contrast to solid tumors. The preferred cancer cells to be analyzed includes metastatic cells released from tumors/cancer and blood cell derived cancers, such as leukemias and/or lymphomas. Metastasis from solid tissue tumors forms a separately preferred class of cancer samples with specific characteristics.

The disclosure is furthermore directed to the anlysis of secretions from tumors such as ascites and/or cyst fluids, and cancer secreted materials present in general body fluids such as blood (or its derivatives such as serum nor

plasma), urine, mucous secretions, amnion fluid, lymphatic fluid or spinal fluid, preferably blood, urine or mucous secretions, most preferably blood.

The cancer tissue materials to be analyzed according to the disclosure are in the disclosure also referred as tissue materials or simply as cells, because all tissues comprise cells, however the disclosure is preferably directed to unicellularily and/or multicellularily expressed cancer cells and/or solid tumors as separate preferred characterisitics. The disclosure further reveals normal tissue materials to be compared with the cancer materials. The disclosure is specifically directed to methods according to the disclosure for revealing status of transformed tissue or suspected cancer sample when expression of specific structure of a signal correlated with it is compared to a expression level estimated to correspond to expression in normal tissue or compared with the expression level in an standard sample from the same tissue, preferably a tissue sample from healthy part of the same tissue from the same patient.

The disclosure is directed to analysis of the marker structures and/or glycome profiles from both cancer tissue and corresponding normal tissue of the same patient because part of the glycosylations includes individual changes for example related to rare glycosylation related diseases such as congenital disorders in glycosylation (of glycoproteins/carbohydrates) and/or glycan storage diseases. The disclosure is furthermore directed to method of verifying analysing importance and/or change of a specific structure/structure group or glycan group in glycome in specific cancer and/or a subtype of a cancer optionally with a specific status (e.g. primary cancer, metastase, benign transformation related to a cancer) by methods according to the present disclosure.

The present disclosure is directed to a set of glycan structures which are expressed by human cancers. The presence or increased amount of one or more of these glycans in a patient sample indicates cancerous status of the sample as described below in detail. The glycan structures can be divided to three basic groups:

1) neutral low-mannose type N-glycans having terminal Man, preferentially Manα structure; for example Man$\alpha_{0-3}$Manβ4GlcNAcβ4GlcNAc(β-N-Asn) or Man$\alpha_{0-4}$Manβ4GlcNAcβ4(Fucα6)GlcNAc(β-N-Asn);

2) neutral O-glycans having terminal Galβ4 structure; for example Galβ4[(Fucα3)]$_n$GlcNAcβ6(Galβ3)GalNAc[αSer/Thr]$_p$ wherein p and n are either independently 0 or 1; and

3) sialylated Core 2 type O-glycans having terminal SAα3 structure, for example Neu5Acα3(Galβ4[±Fucα3]GlcNAcβ3)$_{0-2}$Galβ4[±Fucα3]GlcNAcβ(6→GaNAc) and Neu5Acα3(Galβ3[±Fucα4]GlcNAcβ3)$_{0-2}$Galβ4[±Fucβ3]GlcNAcβ(6→GaNAc).

Groups 2 and 3 can be considered together as terminal LacNAc O-glycan group.

The group 1 may be further included in a larger group of terminal man-glycans including high-Man glycans, which also represent cancer specific changes, but in a more modest scale.

The disclosure further reveals structures and structural groups, which presence or alterations of amounts of which are characteristic for cancercers such as an additional group of HexNAc comprising complex N-glycans, which can be further divided to two groups and level of which would give more specific information about the cancer. The HexNAc comprising N-glycans can be divided to two major groups:

Group 4) glycans with terminal (NeuAcα)$_{0-1}$HexNAcβHexNAcβ sequences, preferably terminal LacdiNAc sequences
Group 5) terminal β-linked GlcNAc glycans

Besides the major groups above the present disclosure reveals additional groups such as Group 6) a group of complex N-glycans including fucosylated multiple N-acetyllactosmine N-glycans revealed as extensively altered structures e.g. in analysis of Table 18 and Group 7) including multiple fucosylated (or deoxyhexose, dHex, comprising) structures, e.g. present in samples of pancreatic cancer (Tables 11-13 and corresponding examples) and Group 8) including phosphorylated and/or sulphated glycans, which are also preferred as separate groups and modifications of terminal Man-glycans.

The disclosure revealed furthermore useful additional glycan groups such as: control groups and/or glycan groups with usually modest changes, such as regular complex type glycans or structural groups otherwise characteristic such as blood groups structure glycans, which have carry over in the metastasis samples.

The disclosure revealed that mass spectrometric profiling of glycan group is very effective method for analysing cancer samples as multiple characteristic groups can be analyzed simultaneously.

More detailed analysis of key groups

Group 1 - low mannose glycans

Low-mannose N-glycans are smaller and more rare than the common high-mannose N-glycans ($Man_{5-9}GlcNAc_2$). The low-mannose N-glycans detected in tumor tissues fall into two subgroups: 1) non-fucosylated, with composition $Man_nGlcNAc_2$, where $1 \leq n \leq 4$, and 2) core-fucosylated, with composition $Man_nGlcNAc_2Fuc_1$, where $1 \leq n \leq 5$. The largest of the detected low-mannose structure structures is $Man_5GlcNAc_2Fuc_1$ (m/z 1403 for the sodium adduct ion), which due to biosynthetic reasons most likely includes the structure below (in the figure the glycan is free oligosaccharide and β-anomer; in glycoproteins in tissues the glycan is N-glycan and β-anomer):

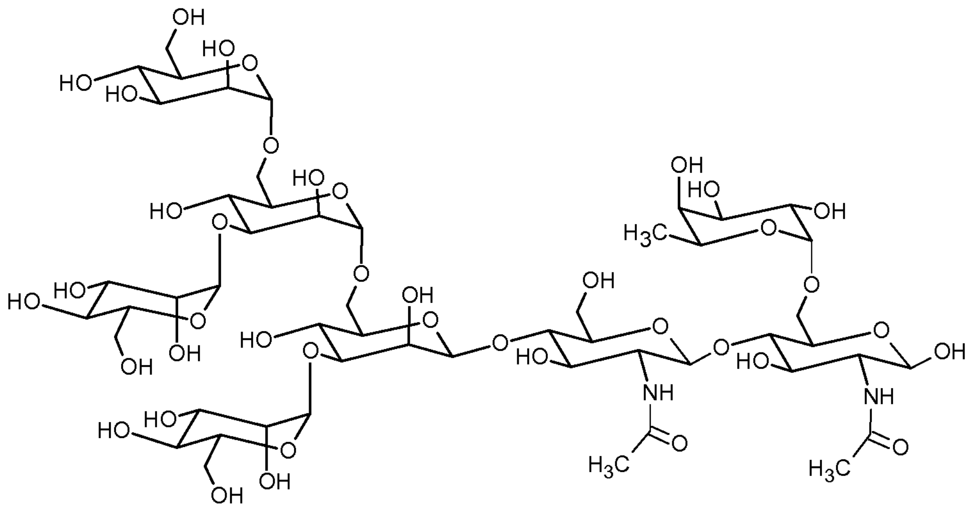

*Preferred general molecular structural features of group 1 glycans*

The low Man glycans described above can be presented in a single Formula:

$$[Man\alpha2]_{n1}[Man\alpha3]_{n2}([Man\alpha2]_{n3}[Man\alpha6)]_{n4})[Man\alpha6]_{n5}([Man\alpha2]_{n6}[Man\alpha2]_{n7}[Man\alpha3]_{n8})Man\beta4GlcNAc\beta4[(Fuc\alpha6)]_{m}GlcNAc[\beta\text{-}N\text{-}Asn]_{p}$$

wherein p, n1, n2, n3, n4, n5, n6, n7, n8, and m are either independently 0 or 1; with the proviso that when n2 is 0, also n1 is 0; when n4 is 0, also n3 is 0; when n5 is 0, also n1, n2, n3, and n4 are 0; when n7 is 0, also n6 is 0; when n8 is 0, also n6 and n7 are 0; the sum of n1, n2, n3, n4, n5, n6, n7, and n8 is less than or equal to (m + 3); and preferably n1, n3, n6, and n7 are 0 when m is 0; [ ] indicates determinant either being present or absent depending on the value of n1, n2, n3, n4, n5, n6, n7, n8, and m; and
( ) indicates a branch in the structure.

Preferred non-fucosylated low-mannose glycans are according to the formula:

$$[Man\alpha3]_{n1}[(Man\alpha6)]_{n2}[Man\alpha6]_{n3}[(Man\alpha3)]_{n4}Man\beta4GlcNAc\beta4GlcNAc[\beta Asn]_{p}$$

wherein p, n1, n2, n3, n4 are either independently 0 or 1,
with the proviso that when n3 is 0, also n1 and n2 are 0, and preferably either n1 or n2 is 0, [ ] indicates determinant either being present or absent
depending on the value of n1, n2, n3, n4,
( ) indicates a branch in the structure.

Preferred fucosylated low-mannose glycans are according to the formula:

$$[Man\alpha3]_{n1}[(Man\alpha6)]_{n2}[Man\alpha6]_{n3}[(Man\alpha3)]_{n4}Man\beta4GlcNAc\beta4(Fuc\alpha6)GlcNAc[\beta Asn]_{p}$$

wherein p, n1, n2, n3, n4 are either independently 0 or 1, with the provisio that when n3 is 0, also n1 and n2 are 0, [ ] indicates determinant either being present or absent depending on the value of n1, n2, n3, n4, ( ) indicates a branch in the structure;

and wherein n1, n2, n3, n4 and m are either independently 0 or 1,
with the provisio that when n3 is 0, also n1 and n2 are 0,
[ ] indicates determinant either being present or absent
depending on the value of n1, n2, n3, n4 and m,
( ) indicates a branch in the structure.

Group 2 - neutral O-glycans

The group 2 represents neutral O-glycans. The major structures represent O-glycans with LacNAc epitope (Galβ4GlcNAc), and fucosylated so-called Lewis x-structure (Galβ4[Fucα3]GlcNAc). The preferred structures include the Core 2 type O-glycans of the figures below. Alternative variants include Galβ4(Fucα3)$_{0-1}$GlcNAcβ3Galβ3GaNAc Core 1 type O-glycan structures (in the figures the glycans are free oligosaccharides and β-anomers; in glycoproteins in tissues the glycans are O-glycans and α-anomers):

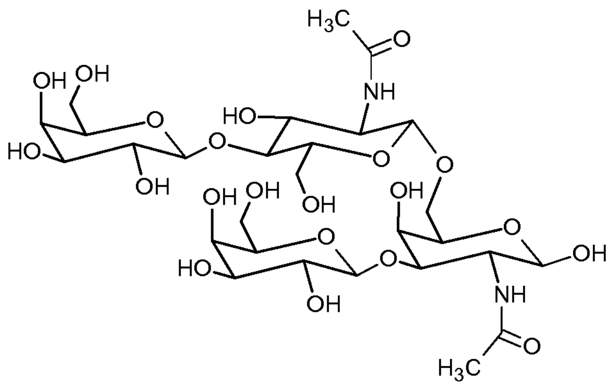

A) Galβ4GlcNAcβ6(Galβ3)GalNAc

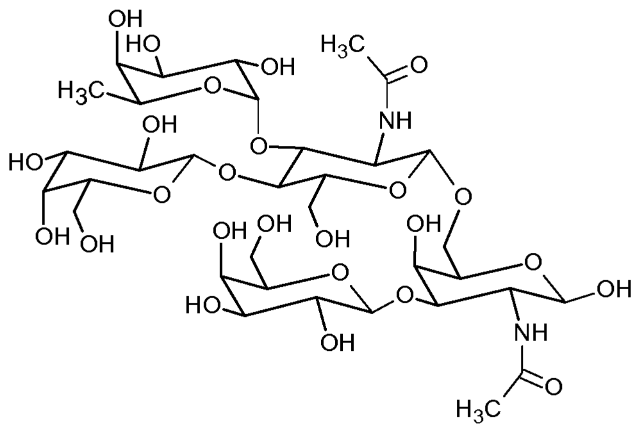

B) Galβ4(Fucα3)GlcNAcβ6(Galβ3)GalNAc

*Preferred molecular structural features of group 2*

Based on the enzymatic digestion data and the release by β-elimination the neutral O-glycans include the following preferred structures

Galβ4[(Fucα3)]$_n$GlcNAcβX[(]$_m$Galβ3[)]$_m$GalNAc[αSer/Thr]$_p$

wherein p, n and m are either independently 0 or 1, [ ] indicates determinant either being present or absent depending on the value of m and n, ( ) indicates a branch in the structure. X is 3, when m is 0; and X is 6 when m is 1.

The most preferred structures are accoding to the formula (when m is 1)

Galβ4[(Fucα3)]$_n$GlcNAcβ6(Galβ3)GalNAc[αSer/Thr]$_p$

wherein
p and n are either independently 0 or 1.

In another alternatuve the the neutral O-glycans are according to the formula

Galβ4[(Fucα3)]$_n$GlcNAcβ3Galβ3GalNAc[αSer/Thr]$_p$

wherein
p and n are either independently 0 or 1.

The Core 2 O-glycan structures are likely produced in Golgi apparatus through Core 2 structure GlcNAcβ6(Galβ3)GalNAcα, the inventors have also analysed larger Core 2 glycans from tissues, as described in the Examples.

Group 3 - sialylated O-glycans

The group 3 represents sialylated O-glycans. The major structure is a Core 2/4 type O-glycan with sialyl-LacNAc, NeuNAcα3Galβ4GlcNAcβ6(R-3)GalNAcαSer/Thr. The R substituent at 3-position of GalNAc is preferentially β1,3-linked Gal (Core 2) or β1,3-linked GlcNAc (Core 4).

*Comparision to O-glycan biosynthesis*

The present structures are different from sialyl-Tn, T and sialyl-T O-glycan structures indicated previously for cancer. Previously sialylation of Core 1 has been considered to prevent Core 2 synthesis in certain cancer models, leading to increased expression of small O-glycan antigens, such as sialyl-Tn. The present disclosure shows opposite result, the increase of Core 2 structures in tumours, especially in malignant vs. benign tumours.

*Molecular structures*

The group 3 structures are a large group of sialylated O-glycans, which have in common the O-glycan core structure GlcNAcβ6(R-3)GalNAc(α-Ser/Thr), where R is a possibly variable structure. In the conditions used for glycan isolation, typical fragment structures are produced from these glycans. The most typical such fragment (at m/z 899 for the sodium adduct ion) is depicted in **Figure 11** together with the experiments done to characterize the structure.

The most preferred structures of group 3 are according to the formula (when m is 1):

NeuNAcαX$_1${Galβ4[(Fucα3)]$_{n1}$GlcNAcβX$_2$}$_m$Galβ4[(Fucα3)]$_{n2}$GlcNAcβ6([NeuNAcαX$_3$]$_{n3}$ Galβ3)GalNAc[αSer/Thr]$_p$

wherein n1, n2, n3 and p are either independently 0 or 1, m is 0, 1 or 2, { } and [ ] indicate determinant either being present or absent depending on the value of m, n1 and n2 ( )
indicates a branch in the structure. When m is 2, either or both of the
Galβ4[(Fucα3)]$_{n1}$GlcNAc -units may be fucosylated
X$_1$, X$_2$, and X$_3$ are 3 or 6, more preferably 3
When m is 2 one X2 may be 3 and the other one 6 in a branched structure on the next Gal residue.

Methods for evaluating the malignancy of patient samples

The present invention is directed to a method of evaluating the malignancy of a patient sample comprising the step of detecting the presence of cancer related oligosaccharide sequences in the sample as defined in the claims.

Combined use of different groups

To increase the effectivity of analysis a mixture of low-Man and LacNAc O-glycans or at least one low-Man and one LacNAc O-glycans is analyzed. Alternatively, neutral glycans of groups 1 and 2 are analyzed.
To increase the specificity of the anlysis one or two structures of Groups 1-3, preferably as preferred above is analyzed with at least one structure (preferably 1, 2 or 3 structures) selected from the groups 4-5, or more preferably selected

from the groups 4-8, preferably so that when when at least two structures are selected, these are selected from different groups.

**Group 1) low-mannose N-glycans with Formula**

[Manα2]$_{n1}$[Manα3]$_{n2}$([Manα2]$_{n3}$[Manα6)]$_{n4}$)[Manα6]$_{n5}$([Manα2]$_{n6}$[Manα2]$_{n7}$ [Manα3]$_{n8}$)Manβ4GlcNAcβ4[(Fucβ6)]$_{m}$GlcNAc[β-N-Asn]$_{p}$

wherein p, n1, n2, n3, n4, n5, n6, n7, n8, and m are either independently 0 or 1; with the proviso that when n2 is 0, also n1 is 0; when n4 is 0, also n3 is 0; when n5 is 0, also n1, n2, n3, and n4 are 0; when n7 is 0, also n6 is 0; when n8 is 0, also n6 and n7 are 0; the sum of n1, n2, n3, n4, n5, n6, n7, and n8 is less than or equal to (m + 3); and preferably n1, n3, n6, and n7 are 0 when m is 0; [ ] indicates determinant either being present or absent depending on the value of n1, n2, n3, n4, n5, n6, n7, n8, and m; and
( ) indicates a branch in the structure.

Preferred structures are described in the Examples, in which association of the structures with cancer was found in major human cancer types.

**Group 2) neutral O-glycans with Formula**

Galβ4[(Fucα3)]$_{n}$GlcNAcβX[(]$_{m}$Galβ3[)]$_{m}$GalNAc[α-O-Ser/Thr]$_{p}$

wherein p, n and m are either independently 0 or 1, [ ] indicates determinant either being present or absent depending on the value of m and n, ( ) indicates a branch in the structure. X is 3, when m is 0; and X is 6 when m is 1; and

Preferred structures are described in the Examples, in which association of the structures with cancer was found in major human cancer types.

**Group 3) sialylated O-glycans with Formula**

SAαX$_{1}${Galβ4[(Fucα3)]$_{n1}$GlcNAcβX$_{2}$}$_{m}$Galβ4[(Fucα3)]$_{n2}$GlcNAcβ6([SAαX$_{3}$]$_{n3}$Galβ3) GalNAc[α-O-Ser/Thr]$_{p}$

wherein n1, n2, n3 and p are either independently 0 or 1; m is 0, 1 or 2; $X_1$ and $X_3$ are independently 3 or 6, however in an alternative both $X_1$ and $X_3$ are 3; $X_2$ is either 3 or 6, however in another alternative $X_2$ is 3; SA is a sialic acid residue, preferentially Neu5Ac or Neu5Gc, however alternatively SA is Neu5Ac; { }and [ ] indicate determinant either being present or absent depending on the value of m, n1 and n2; ( ) indicates a branch in the structure.

Preferred structures are described in the Examples, in which association of the structures with cancer was found in major human cancer types.

<u>Cancer indicating structures and combinations of structures</u>

The inventors found that an increased amount of any one of the abovementioned cancer-related oligosaccharide sequences in said patient sample indicates the cancerous nature of the sample. Furthermore, simultaneous increase of more than one of the abovementioned cancer-related oligosaccharide sequences is highly indicative of cancer, especially when more than one of the abovementioned oligosaccharide sequences, even more preferentially selected from more than one of the abovementioned oligosaccharide sequence groups, is simultaneously expressed in elevated amounts compared to healthy human tissues. The abovementioned glycan structure groups were found to be cancer-associated in all cancer types studied.

It was found that the increased amount of the abovementioned oligosaccharide sequences was indicative of the malignancy of human tumors, though no such increase was found in benign tumors of the colon and the ovaries.. Furthermore, the analysis specificity was increased by combination with analysis of cancer type specific glycan features, as described below..

The present findings are considered medically very interesting. The novel methods to detect and diagnose

cancer described in the present disclosure can be used in the clinical setting e.g. to give tools and data for decisions how to treat human patients. It is realized that ability to detect malignant cancer and to differentiate between benign and malignant tumors is of utmost impostance in terms of efficient clinical decision-making and selection of the correct therapy.

The inventors further discovered that individual differences occur in normal tissue glycosylation and tumor-associated glycosylation changes, and in some patients the cancer-associated glycan changes are more prominent than in others. The present disclosure is further directed to using the methods for selecting patients for most effective therapy options according to their individual glycosylation profiles and the glycosylation profiles expressed in the disease, preferentially in the malignant tumor.

Cancer type specific glycan groups

The inventors also found that changes in the expression of two additional glycan structure groups were indicative of malignant cancer in tumors originating from specific tissues, in addition to the abovementioned oligosaccharide sequences. These indicative glycan structures can be used to detect cancer or to distinguish malignant and benign growth in human patients, either in combination with the abovementioned three glycan groups, or separately.

Both of these structure groups are characterized by a common feature, the presence of a non-reducing terminal β-linked N-acetylhexosamine residue (HexNAcβ). In glycan profiling analyses where monosaccharide compositions can be assigned to analysed glycans, these glycans are indicated among the resulting glycan signals by the formula:

$$n(HexNAc) > n(Hex) \geq 2,$$

wherein n(component) in the amount of the monosaccharide component in a glycan molecular formula. Oligosaccharide sequences that fulfil the formula can be used to distinguish between normal and cancerous tissue materials, and/or benign and malignant tumors. Preferentially, the presence or amount of these oligosaccharide sequences is determined, and optionally compared with the presence or amount of other types of oligosaccharide sequences in the sample and/or specifically chosen oligosaccharide sequences groups. However, these HexNAcβ structures were found to be different in specific human tissues and tumors originating from them, as described below in more detail and described in the Examples.

**Group 4) glycans with terminal (NeuAcα)$_{0-1}$HexNAcβHexNAcβ sequences**

The inventors analyzed protein-linked glycans from patients with ovarian cancer or benign ovarian tumors. The patient samples were from multiple patients with benign ovarian cystadenoma or malignant ovarian cystadenocarcinoma, and a sample from normal ovary. It was found that terminal HexNAcβ structures were present in all the samples in slightly elevated amounts compared to other human tissues, but specifically in benign ovarian tumors these structures were highly increased and were among the major glycan components, as described in the Examples. However, in corresponding malignant tumors, the amounts of terminal HexNAcβ structures were even decreased compared to the normal ovary. These changes were found to be consistent in all the studied samples, indicating that the phenomenon is common in the human ovary and its tumors. Detection of the ovary-specific terminal HexNAcβ oligosaccharide sequence was found to effectively distinguish between benign and malignant tumors of the ovary, and contribute to the separation of the normal and malignant ovary samples.

The inventors characterized the ovary-specific HexNAcβ structures in further detail, as described in the Examples. The results suggested that the observed glycan structures associated with benign ovarian tumors structures include GalNAcPGlcNAcβ and Neu5AcαGalNAcβGlcNAc non-reducing terminal sequences, or non-sialylated and sialylated di-N-acetyllactosediamine (LacdiNAc), occurring mainly in N-glycans. The inventors have previously characterized LacdiNAc structures in human tumors and described novel methods and reagents for the detection and modification of LacdiNAc structures as well as harnessing immune responses against LacdiNAc structures. It is realized that the present indication represents further uses also for previously described methods and reagents, and the present specification is specifically directed to using LacdiNAc-specific reagents and methods for the detection of cancer, preferentially ovarian cancer and especially to distinguish between glycans or glycoconjugates originating from benign and malignant ovarian tumors or normal ovarian tissue.

In an alternative of the present disclosure, ovary-associated terminal HexNAcβ sequences are detected and their presence indicates normal ovary tissue or benign growth of the ovary. However, in an alternative the ovary-associated terminal HexNAcβ sequences are quantitated and their increased amount, compared to other human tissues or normal ovary, indicates presence of normal ovary tissue or benign growth of the ovary. In contrast, malignant tumors of the ovary do not show similar increased amounts of HexNAcβ sequences. In an alternative, ovary-associated oligosaccharide sequences are profiled, and the relative amounts of terminal HexNAcβ sequences are compared to the other oligosaccharide sequences present in the sample. Guidelines for recognition of terminal HexNAcβ sequences and oligosaccharide sequences for comparison are described below. In another alternative, experimental analysis signals corresponding to terminal HexNAcβ oligosaccharide sequences as such, or other cancer-associated oligosaccharide sequences recognized, for example mass spectrometric signals, are used for evaluation of the cancerous status of a sample.

1) Ovary-associated HexNAcβ oligosaccharide sequences

Ovary tissue and tumor samples contain HexNAcβ oligosaccharide sequences as defined by the formula above, more specifically terminal HexNAcβHexNAcβ structures. Typically, HexNAcβHexNAcβ structures include oligosaccharide sequences containing the motifs $Hex_mHexNAc_{m+1}$, $Hex_mHexNAc_{m+3}$, or $Hex_mHexNAc_{m+5}$ in their monosaccharide compositions. Another useful group definition of HexNAcβ oligosaccharide sequences includes glycans that are susceptible to the action of β-hexosaminidase, but not to β-glucosaminidase, as described in the Examples. Typical mass spectrometric signals, monosaccharide compositions, and corresponding oligosaccharide sequences indicative of the cancerous status of a patient sample are further described in the Examples, and the present disclosure is specifically directed to using these signals, monosaccharide compositions, and the corresponding oligosaccharide sequences for the evaluation of the cancerous status of a sample. For practical reasons, the amounts of the HexNAcβHexNAcβ oligosaccharide sequences can be approximated and/or extrapolated from the monosaccharide compositions and experimental evidence from previous analyses of similar tissues, and also these approximations are suitable for effective diagnostic results, as shown in the Examples.

2) Oligosaccharide sequences useful for comparison

In approximate order of increasing specificity, oligosaccharide sequences useful for comparison include total glycans present in the sample, (1) sialylated or (2) neutral glycans, total N-glycans, total complex-type glycans, (1) sialylated or (2) neutral complex-type glycans, total complex-type N-glycans, (1) sialylated or (2) neutral complex-type N-glycans, and normal glycans corresponding to HexNAcβ glycans. In the present list, (1) and (2) indicate glycan groups useful for comparison of (1) sialylated and (2) neutral HexNAcβ oligosaccharide sequences, respectively. The normal glycans in the latter definition have Hex substituted for HexNAc in their monosaccharide compositions, and may be defined for example as oligosaccharide sequences containing the motifs $Hex_{m+1}HexNAc_{m,}$ $Hex_{m+2}HexNAc_{m+1}$, and $Hex_{m+3}HexNAc_{m+2}$ in their monosaccharide compositions, when present in the same sample as HexNAcβ oligosaccharide sequences containing the motifs $Hex_mHexNAc_{m+1}$, $Hex_mHexNAc_{m+3}$, and $Hex_mHexNAc_{m+5}$ in their monosaccharide compositions, respectively. For example, the normal monosaccharide composition motif $Hex_5HexNAc_4$ corresponds to the HexNAcβ composition $Hex_3HexNAc_6$. Another useful group of oligosaccharide sequences for comparison include those that are susceptible to the action of β-glucosaminidase, as described in the Examples.

Practical procedures for comparison of samples and analysis results with regard to HexNAcβ structures and ovarian tissue and tumor patient samples as well as methods for detection and quantitation of oligosaccharide sequences are described in the present disclosure.

**Group 5) terminal β-linked GlcNAc glycans**

In glycan profiling analyses where monosaccharide compositions can be assigned to analysed glycans, these glycans are indicated among the resulting glycan signals by the formula:

$$n(HexNAc) > n(Hex) \geq 2,$$

wherein n(component) in the amount of the monosaccharide component in a glycan molecular formula. Oligosaccharide sequences that fulfil the formula can be used to distinguish between normal and cancerous tissue materials. Preferentially, the presence or amount of these oligosaccharide sequences is determined, and optionally compared with the presence or amount of other types of oligosaccharide sequences in the sample and/or specifically chosen oligosaccharide sequences groups. In general, terminal GlcNAcβ oligosaccharide sequences are susceptible to β-glucosaminidase as well as β-hexosaminidase and other enzymes such as specific β1,4-galactosyltransferase, which together with glycan profiling can be used to distinguish between cancerous and healthy tissue samples. However, as described in the present disclosure, glycosylation is tissue specific and cancer type specific, and information about GlcNAcβ structures can be extrapolated from the reference information of glycosylation described in the present disclosure.

The inventors have previously characterized GlcNAcβ structures in human tumors and described novel methods and reagents for the detection and modification of GlcNAcβ structures as well as harnessing immune responses against GlcNAcβ structures. However, the detection method of GlcNAcβ structures by approximation through the formula above

in glycan profiling of tissue materials, methods for specific comparison with other oligosaccharide sequence groups present in the sample, and especially the combination of data about HexNAcβ oligosaccharide sequences with the other cancer-associated oligosaccharide sequences described in the present disclosure to increase resolution power of the method are novel.

General structures representing oligosaccharide sequences

The cancer related oligosaccharide sequences described herein can be a part of a glycolipid, a part of a glycoprotein, and/or a part of a N-acetyllactosamine chain. The cancer specific oligosaccharide sequences can also be a part of glycolipids, a part of N-linked glycans or O-linked glycans of glycoproteins, free oligosaccharides, or glycans such as glycopeptides. Defects or changes in biosynthetic and/or biodegradative pathways of tumors lead to the synthesis of the cancer related oligosaccharide sequences both on glycolipids and glycoproteins.

The term "oligosaccharide sequence" indicates that the monosaccharide residue/residues in the sequence are part of a larger glycoconjugate, which contains other monosaccharide residues in a chain, which may be branched, or may have natural substituted modifications of oligosaccharide chains. The oligosaccharide chain is normally conjugated to a lipid anchor or to a protein. In an alternative, the oligosaccharide sequences of the present disclosure are non-reducing terminal oligosaccharide sequences, which means here that the oligosaccharide sequences are not linked to other monosaccharide or oligosaccharide structures except optionally from the reducing end of the oligosaccharide sequence. The oligosaccharide sequence when present as conjugate is preferably conjugated from the reducing end of the oligosaccharide sequence, though other linkage positions which are tolerated by the antibody/binding substance binding can also be used. In a more specific alternative the oligosaccharide sequence according to the present disclosure means the corresponding oligosaccharide residue which is not linked by natural glycosidic linkages to other monosaccharide or oligosaccharide structures. The oligosaccharide residue is preferably a free oligosaccharide or a conjugate or derivative from the reducing end of the oligosaccharide residue.

Preferably the tumor specific oligosaccharide sequence is detected by a specific binding substance which can be an aptamer, lectin, peptide, or protein, such as an antibody, a fragment thereof or genetically engineered variants thereof. More preferably the specific binding substance is divalent, oligovalent or polyvalent. Most preferably the binding substance is a lectin or an antibody.

Specific binding combinatorial chemistry libraries can be used to search for the binding molecules. Saccharide binding proteins, antibodies or lectins can be engineered, for example, by phage display methods to produce specific binders for the structures of the disclosure. Labelled bacteria or cells or other polymeric surfaces containing molecules recognizing the structures can be used for the detection. Oligosaccharide sequences can also be released from cancer or tumor cells by endoglycosidase enzymes. Alternatively oligosaccharides can be released by protease enzymes, resulting in glycopeptides. Chemical methods to release oligosaccharides or derivatives thereof include, e.g., ozonolysis of glycolipids and beta-elimination or hydrazinolysis methods to release oligosaccharides from glycoproteins. Alternatively the glycolipid fraction can be isolated. A substance specifically binding to the cancer specific oligosaccharide sequences can also be used for the analysis of the same sequences on cell surfaces. Said sequences can be detected e.g. as glycoconjugates or as released and/or isolated oligosaccharide fractions. The possible methods for the analysis of said sequences in various forms also include NMR spectroscopy, mass spectrometry and glycosidase degradation methods. Preferably at least two analysis methods are used, especially when methods of limited specificity are used.

Analysis of multiple cancer specific structures simultaneously from mass spectrometric profiles

The present disclosure is especially directed to the analysis and/or comparision of several analytical signals, preferably mass spectrometry signals produced from a sample comprising total fraction of oligosaccharides released from a cancer or a tumor sample. A single mass spectrum of an oligosaccharide fraction comprise a profile of glycosylation and multiple peaks indicating the potential presence of the oligosaccharide sequences and potential presence of cancer specific oligosaccharide sequences and altered levels thereof in comparison to normal tissue sample or a benign tumour sample. The profiles are determined preferably by MALDI-TOF mass spectrometry as described in the Examples. The total oligosaccharide fraction corresponds preferably to the total fraction of protein oligosaccharides, preferably comprising at least one cancer or tumor specific oligosaccharide sequence according to the disclosure. In an alternative the total oligosaccharide fraction comprises at least one cancer or tumor specific O-glycosidic and one N-glycosidic oligosaccharide according to the disclosure. The present disclosure is further directed to analysis of the multiple mass spectrometric signals after the total oligosaccharide fraction is released from a cancer or tumor sample is subjected to an enzymatic or a chemical digestion step. The enzymatic digestion is preferably performed by a glycosidase enzyme, preferably selected from the group: galactosidase, sialidase, N-acetylhexosaminidase, N-acetylglucosaminidase, fucosidase, or mannosidase.

The present disclosure is also directed to the use of the tumor specific oligosaccharide sequences or analogs

or derivatives thereof to produce polyclonal or monoclonal antibodies recognizing said structures using following process: 1) producing synthetically or biosynthetically a polyvalent conjugate of an oligosaccharide sequence or analogue or derivative thereof, the polyvalent conjugate being, for instance, according to the following structure: position C1 of the reducing end terminal of an oligosaccharide sequence (OS) comprising the cancer specific sequence described in the present disclosure is linked (-L-) to an oligovalent or a polyvalent carrier (Z), via a spacer group (Y) and optionally via a monosaccharide or oligosaccharide residue (X), forming the following structure

$$[OS\text{-}(X)_n\text{-}L\text{-}Y]_m\text{-}Z$$

wherein integer m has values m > 1 and n is independently 0 or 1; L can be oxygen, nitrogen, sulfur, or a carbon atom; X is preferably lactosyl-, galactosyl-, poly-N-acetyl-lactosaminyl, or part of an O-glycan or an N-glycan oligosaccharide sequence, Y is a spacer group or a terminal conjugate such as a ceramide lipid moiety or a linkage to Z; 2) immunizing an animal or human with polyvalent conjugate together with an immune response activating substance. Preferably the oligosaccharide sequence is polyvalently conjugated to an immune response activating substance and the conjugate is used for immunization alone or together with an additional immune response activating substance. In an alternative the oligosaccharide conjugate is injected or administered mucosally to an antibody-producing organism with an adjuvant molecule or adjuvant molecules. For antibody production the oligosaccharide or analogs or derivatives thereof can be polyvalently conjugated to a protein such as bovine serum albumin, keyhole limpet hemocyanin, a lipopeptide, a peptide, a bacterial toxin, a part of peptidoglycan or immunoactive polysaccharide or to another antibody production activating molecule. The polyvalent conjugates can be injected to an animal with adjuvant molecules to induce antibodies by routine antibody production methods known in the art.

Antibody production or vaccination can also be achieved by analogs or derivatives of the cancer specific oligosaccharide sequences. Simple analogs of the N-acetyl-group containing oligosaccharide sequences include compounds with modified N-acetyl groups, for example, N-alkyls, such as N-propanyl.

It is possible to use the tumor specific oligosaccharide sequences for the purification of antibodies from serum, preferably from human serum. The cancer specific oligosaccharides or derivatives or analogs, such as a close isomer, can also be immobilized for the purification of antibodies from serum, preferably from human serum. The present disclosure is directed to natural human antibodies that bind strongly to the cancer specific oligosaccharide sequences descrribed in the present disclosure.

The cancer specific oligosaccharide sequences can also be used for detection and/or quantitation of the human antibodies binding to the cancer specific oligosaccharide sequences, for example, in enzyme-linked immunosorbent assay (ELISA) or affinity chromatography type assay formats. The detection of human antibodies binding to the cancer specific oligosaccharide sequences is preferably aimed for diagnostics of cancer, development of cancer therapies, especially cancer vaccines against the oligosaccharide sequences described in the present disclosure, and search for blood donors which have high amounts of the antibodies or one type of the antibody.

The production of specific humanized antibodies by gene engineering and biotechnology is also possible: the production of humanized antibodies has been described in US patents Nos. 5,874,060 and 6,025,481, for example. The humanized antibodies are designed to mimic the sequences of human antibodies and therefore they are not rejected by immune system as animal antibodies are, if administered to a human patient.

According to the present disclosure, human antibodies or humanized antibodies against the cancer specific oligosaccharides, or other tolerated substances binding the tumor specific oligosaccharides, are useful to target toxic agents to tumor or to cancer cells. The toxic agent could be, for example, a cell killing chemotherapeutics medicine, such as doxorubicin (Arap et al., 1998), a toxin protein, or a radiochemistry reagent useful for tumor destruction. Such therapies have been demonstrated in the art. The toxic agent may also cause apoptosis or regulate differentiation or potentiate defence reactions against the cancer cells or tumor. The cancer or tumor binding antibodies according to the present disclosure can be also used for targeting prodrugs active against tumor or enzymes or other substances converting prodrugs to active toxic agents which can destroy or inhibit tumor or cancer, for example in so called ADEPT-approaches.

The therapeutic antibodies described above can be used in pharmaceutical compositions for the treatment or prevention of cancer or tumor. It is realized that numerous other agents besides antibodies, antibody fragments, humanized antibodies and the like can be used for therapeutic targeting of cancer or tumors similarily with the diagnostic substances. It is specifically preferred to use non-immunogenic and tolerable substances to target cancer or tumor. The targeting substances binding to the cancer or tumor comprise also specific toxic or cytolytic or cell regulating agents which leads to destruction or inhibition of cancer or tumor. Preferably the non-antibody molecules used for cancer or tumor targeting therapies comprise molecules specifically binding to the cancer or tumor specific oligosaccharide sequences according to the present disclosure are aptamers, lectins, genetically engineered lectins, glycosidases and glycosyltransferase and genetically engineered variants thereof. Labelled bacteria, viruses or cells or other polymeric surfaces containing molecules recognizing the structures can be used for the cancer or tumor targeting therapies. The

cancer or tumor binding non-antibody substances according to the present disclosure can also be used for targeting prodrugs active against cancer or tumor or for targeting enzymes or other substances converting prodrugs to active toxic agents that can destroy or inhibit cancer or tumor.

Use of antibodies for the diagnostics of cancer or tumor and for the targetting of drugs to cancer has been described with other antigens and oligosaccharide structures (US 4,851,511; US 4,904,596; US 5,874,060; US 6,025,481; US 5,795,961; US 4,725, 557; US 5,059,520; US 5,171,667; US 5,173,292; US 6,090,789; US 5,708,163; US 5,902,725 and US 6,203,999). Use of cancer specific oligosaccharides as cancer vaccines has also been demonstrated with other oligosaccharide sequences (US 5,102,663; US 5,660,834; US 5,747,048; US 5,229,289 and US 6,083,929).

Combination of the therapeutic and diagnostic methods

The present disclosure is specifically directed to analysis of abnormal and normal glycosylation structures from human tumors and cancers and use of the analytical information for the production of therapeutic antibodies or cancer vaccines. To achieve effective therapeutic response, it is preferred that the specific cancer type in the patients to be treated expresses cancer-associated glycans according to the present disclosure.

The data in the Examples shows the usefulness of the combination of analysis of the cancer specific structures according to the disclosure, because there are individual variations in glycosylation of tumors and normal tissues. The normal tissue close to tumor may also be partially contaminated by materials secreted by tumor that may be taken to consideration when analyzing the normal tissue data.

The oligosaccharide sequences according to the disclosure can be synthesized, for example, enzymatically by glycosyltransferases, or by transglycosylation catalyzed by a glycosidase enzyme or a transglycosidase enzyme, for review see Ernst et al. (2000). Specificities of the enzymes and their use of co-factors such as nucleotide sugar donors, can be engineered. Specific modified enzymes can be used to obtain more effective synthesis, for example, glycosynthase is modified to achieve transglycosylation but not glycosidase reactions. Organic synthesis of the saccharides and conjugates of the invention or compounds similar to these are known (Ernst et al., 2000). Carbohydrate materials can be isolated from natural sources and be modified chemically or enzymatically into compounds according to the disclosure. Natural oligosaccharides can be isolated from milks of various ruminants and other animals. Transgenic organisms, such as cows or microbes, expressing glycosylating enzymes can be used for the production of saccharides.

It is possible to incorporate an oligosaccharide sequence according to the disclosure, optionally with a carrier, in a pharmaceutical composition, which is suitable for the treatment of cancer or tumor in a patient. Examples of conditions treatable according to the disclosure are cancers in which the tumor expresses one or more of the tumor specific oligosaccharides described in the disclosure. The treatable cancer cases can be discovered by detecting the presence of the tumor specific oligosaccharide sequences in a biological sample taken from a patient. Said sample can be a biopsy or a blood sample.

The pharmaceutical composition according to the invention may also comprise other substances, such as an inert vehicle, or pharmaceutically acceptable carriers, preservatives etc., which are well known to persons skilled in the art.

The substance or pharmaceutical composition according to the invention may be administered in any suitable way. Methods for the administration of therapeutic antibodies or vaccines are well known in the art.

The term "treatment" used herein relates to both treatment in order to cure or alleviate a disease or a condition, and to treatment in order to prevent the development of a disease or a condition. The treatment may be either performed in an acute or in a chronic way.

The term "patient", as used herein, relates to any mammal in need of treatment.

When a cancer specific oligosaccharide or compound specifically recognizing cancer specific oligosaccharides of the disclosure is used for diagnosis or typing, it may be included e.g. in a probe or a test stick, optionally in a test kit. When this probe or test stick is brought into contact with a sample containing antibodies from a cancer patient or cancer cells or tissue of a patient, components of a cancer positive sample will bind the probe or test stick and can be thus removed from the sample and further analyzed.

In the present disclosure the term "tumor" means solid multicellular tumor tissues. Furthermore the term "tumor" means herein premalignant tissue, which is developing to a solid tumor and has tumor specific characteristics. The present disclosure is preferably directed to primary human cancer samples. It is well known that glycosylations in cultivated cancer cells vary and are not in general relevant with regard to cancer. It is also known that transfections, cell culture media and dividing solid tumor to single cells may have daramatic effects for glycosylations. When referring to therapies tumor specific oligosaccharides or oligosaccharide sequences (possibly occasionally referred as cancer specific oligosaccharides/oligosaccharide sequences) are targeted for treatment of all kinds of cancers and tumors. The term cancer includes tumors.

Examples of preferred cancer types includes cancers of larynx, colon cancer, stomach cancer, breast cancer, lung cancer, kidney cancer, pancreas cancer, and ovarian cancer.

Glycolipid and carbohydrate nomenclature is according to recommendations by the IUPAC-IUB Commission

on Biochemical Nomenclature (Carbohydr. Res. 1998, 322:167; Carbohydr. Res. 1997, 297:1; Eur. J. Biochem. 1998, 257:29).

It is assumed that Gal, Glc, Man, GlcNAc, GalNAc, and NeuNAc are of the D-configuration, Fuc of the L-configuration, and that all monosaccharide units are in the pyranose form. Glucosamine is referred as GlcN and galactosamine as GalN. Glycosidic linkages are shown partly in shorter and partly in longer nomenclature, the linkages α3 and α6 of the NeuNAc-residues mean the same as α2-3 and α2-6, respectively, and β1-3, β1-4, and β1-6 can be shortened as β3, β4, and β6, respectively. Lactosamine or N-acetyllactosamine or Galβ3/4GlcNAc means either type one structure residue Galβ3GlcNAc or type two structure residue Galβ1-4GlcNAc, and SA is sialic acid, NeuAc or NeuGc, preferentially Neu5Ac, Lac refers to lactose and Cer is ceramide. Hex is any hexose, preferably Man, Gal, or Glc; HexNAc is any N-acetylhexosamine, preferably GlcNAc or GalNAc; and dHex is preferably Fuc.

The present invention is further illustrated in Examples, which in no way are intended to limit the scope of the invention.

**EXAMPLE 1. Structure analysis of glycans that are expressed in various human cancer types.**

**EXPERIMENTAL PROCEDURES**

*Isolation of glycans from formalin-fixed and paraffin-embedded tissue samples.* Prior to glycan isolation from formalin-fixed and paraffin-embedded samples, the samples were deparaffinised. Glycans were detached from sample glycoproteins by non-reductive β-elimination essentially as described previously (Huang *et al*., 2001) and purified and analyzed essentially as described in **Examples 11 and 12.**

*MALDI-TOF MS.* MALDI-TOF mass spectrometry was performed with a Voyager-DE STR BioSpectrometry Workstation, essentially as described previously (Saarinen *et al*., 1999; Harvey *et al*., 1993).

**1.1 Neutral low-mannose type N-glycans**

*Exoglycosidase digestions.* All exoglycosidase reactions were performed essentially as described previously (Nyman *et al*., 1998; Saarinen *et al*., 1999) and analysed by MALDI-TOF MS. The enzymes and their specific control reactions with characterised oligosaccharides were (R denotes reducing end oligosaccharide sequences in the following examples): β1,4-galactosidase *(Streptococcus pneumoniae,* recombinant, *E. coli;* Calbiochem, USA) digested Galβ1-4GlcNAc-R but not Galβ1-3GlcNAc-R; β-N-acetylglucosaminidase *(Streptococcus pneumoniae,* recombinant, *E. coli;* Calbiochem, USA) digested GlcNAcβ1-6Gal-R in β1,4-galactosidase treated lacto-N-hexaose but not GaNAcβ1-4GlcNAcβ1-3/6Gal-R in a synthetic oligosaccharide; α-mannosidase (Jack bean; Glyko, UK) transformed a mixture of high-mannose N-glycans to the $Man_1GlcNAc_2$ N-glycan core trisaccharide; β-mannosidase (*Helix pomatia;* Calbiochem, USA) digested the β1,4-linked mannose residue from the N-glycan core trisaccharide Manβ4GlcNAcβ4GlcNAc, without affecting the α-linked mannose residues of high-mannose N-glycans. Control digestions were performed in parallel and analysed similarly to the analytical exoglycosidase reactions. Endoglycosidase digestions were performed essentially as described previously (Plummer & Tarentino, 1991), and the reaction products were analyzed by MALDI-TOF MS after purification. The specific N-glycosidase F1 control oligosaccharides and the control reactions were as follows: the enzyme transformed all $Hex_{5-9}HexNAc_2$ oligosaccharides in a sample of high-mannose N-glycans into $Hex_{5-9}HexNAc_1$ oligosaccharides; in contrast, the enzyme was not able to digest a core-fucosylated N-glycan, namely the hexasaccharide Mana6(Manα3)Manβ4GlcNAcβ4(Fucα6)GlcNAc.

**RESULTS**

*Glycan isolation and analysis.* Detached and purified glycans from paraffin-embedded formalin-fixed tissue samples from cancer patients were analysed by MALDI-TOF mass spectrometry after isolation of the neutral glycan fraction. Relative quantification of the glycans were done by comparing relative MALDI-TOF MS signal intensities, which is accurate for the obtained mixtures of purified glycans (Saarinen *et al*., 1999; Harvey *et al*., 1993).

*Specific mannosidase digestion analyses.* The proportions of the non-reducing terminal α-mannose containing glycans were determined by their sensitivity towards hydrolysis with α-mannosidase from Jack beans. After the specific exoglycosidase digestion, the presence of high-mannose and low-mannose type glycans in the original sample can be deduced by their disappearance from the recorded mass spectra, and the simultaneous increase in signal intensities of the expected reaction products at m/z 609 and 755, which correspond to the sodium adduct ions $[Hex_1HexNAc_2+Na]^+$ (calc. m/z 609.21) and $[Hex_1HexNAc_2dHex_1+Na]^+$ (calc. m/z 755.27), respectively. An example of the reaction scheme is presented in Figure 1. The results are summarized in Table 1. According to the digestion results, the majority of the detectable signals in the original samples with proposed compositions $Hex_{4-9}HexNAc_2$ and $Hex_{3-5}HexNAc_2dHex_1$, correspond to glycans that are sensitive to α-mannosidase and contain non-reducing terminal α-mannose residues, whereas

signals with proposed compositions of $Hex_{2-3}HexNAc_2$ and $Hex_2HexNAc_2dHex_1$ most likely partially correspond to other glycan types, although they also contain variable amounts of non-reducing terminal α-mannose residues.

The $Hex_1HexNAc_2dHex_{0-1}$ components were studied with specific β-mannosidase digestion both before and after α-mannosidase digestion. The results are summarized in Table 1. The results indicate that 1) the original components contain variable amounts of non-reducing terminal β-mannose containing oligosaccharides with the compositions $Hex_1HexNAc_2dHex_{0-1}$, and 2) the major α-mannosidase digestion products with the compositions $Hex_1HexNAc_2dHex_{0-1}$ have non-reducing terminal β-mannose residues, as they are susceptible towards digestion with β-mannosidase enzyme.

*N-glycosidase digestion analyses.* The assignment of the majority of the $Hex_{1-9}HexNAc_2$ and $Hex_{1-5}HexNAc_2dHex_1$ glycan components as low-mannose and high-mannose type N-glycans was confirmed by their isolation and digestion analysis by specific endoglycosidase enzymes, namely N-glycosidase F and N-glycosidase F1 from *Chryseobacterium meningosepticum.* The first series of experiments was done with glycan samples isolated from a lung tumor of a patient with non-small cell lung adenocarcinoma. In addition to chemical detachment, the glycans in question could also be isolated by N-glycosidase F digestion, indicating that they are N-glycans. However, all $Hex_{1-9}HexNAc_2$ components, but not any of the $Hex_{1-5}HexNAc_2dHex_1$ components, could be digested with N-glycosidase F1, resulting in transformation of the first glycan group into peaks with masses of one less HexNAc residue with monosaccharide compositions $Hex_{1-9}HexNAc_1$. In combination with the α- and β-mannosidase digestion results, these experiments indicate that all the components are N-glycans that have the chitobiose disaccharide sequence in their reducing end, and that the latter components have a dHex residue linked to the reducing terminal GlcNAc. Furthermore, as the latter components are susceptible to digestion with N-glycosidase F, they must have a reducing terminal sequence dHex-6(GlcNAcβ1-4)GlcNAc.

*Chemical analyses.* In individual samples, the $Hex_{1-9}HexNAc_2$ and $Hex_{1-5}HexNAc_2dHex_1$ components were also studied with periodate oxidation, subsequent reduction with alkaline sodium borohydride, and MALDI-TOF mass spectrometry. Also post-source decay (PSD) MALDI-TOF mass spectrometric analyses were performed to specific components of the structure group. The results from these analyses support the structural features deduced from the experiments described above. Periodate reaction cleaves structures with vicinal hydroxyl groups including non-reducing terminal monosaccharides, and isomeric structures differ from each other in this reaction. The data is in accordance with low-mannose and high-mannose type N-glycans produced by regular biosynthesis.

More specifically, cancer cell N-glycans with the compositions $Hex_2HexNAc_2$ and $Hex_2HexNAc_2dHex_1$, were studied with periodate oxidation, subsequent borohydride reduction, and MALDI-TOF MS. From the $Hex_2HexNAc_2$ component at m/z 771.59 (calc. m/z 771.26 for the ion $[Hex_2HexNAc_2+Na]^+$), two product ions were observed after the reaction at m/z 717.34 (calc. m/z 717.29 for the ion $[Hex_2HexNAc_2-C_2O_2+H_2+Na]$) and 745.34 (calc. m/z 745.29 for the ion $[Hex_2HexNAc_2-C_1O_1+H_2+Na]^+$), which can result from Manα6Manβ4GlcNAcβ4GlcNAc and Manα3Manβ4GlcNAcβ4GlcNAc N-glycan oligosaccharide isomers, respectively. These products occurred in respective relative amounts of approximately 60 % and 40 %, indicating that the original sample contained nearly equal amounts of the both α-mannose linkage isomers. From the $Hex_2HexNAc_2dHex_1$ component at m/z 917.63 (calc. m/z 917.32 for the ion $[Hex_2HexNAc_2dHex_1+Na]^+$), two product ions were observed after the reaction at m/z 835.41 (calc. m/z 835.35 for the ion $[Hex_2HexNAc_2dHex_1-C_3O_3+H_2+Na]^+$) and 863.42 (calc. m/z 863.35 for the ion $[Hex_2HexNAc_2dHex_1-C_2O_2+H_2+Na]^+$), which can result from Manα6Manβ4GlcNAcβ4(Fucα6)GlcNAc and Manα3Manβ4GlcNAcβ4(Fucα6)GlcNAc N-glycan oligosaccharide isomers, respectively. These components occurred in relative amounts of approximately 80% and 20%, respectively, indicating that the original sample contained significantly more of the isomer containing α6-linked mannose, but that the both isomers were present in the sample.

Taken together, all the experiments suggest that the terminal mannose-containing oligosaccharides, which have monosaccharide compositions $Hex_{1-9}HexNAc_2$ and $Hex_{1-5}HexNAc_2dHex_1$, include the structures presented in Figure 2.

### 1.2 Neutral O-glycans

*Exoglycosidase digestions.* All exoglycosidase reactions were performed essentially as described previously (Nyman *et al*., 1998; Saarinen *et al*., 1999) and analysed by MALDI-TOF MS. The enzymes and their specific control reactions with characterised oligosaccharides were (R denotes reducing end oligosaccharide sequences in the following examples): β1,4-galactosidase *(Streptococcus pneumoniae,* recombinant, *E. coli;* Calbiochem, USA) digested Galβ1-4GlcNAc-R but not Galβ1-3GlcNAc-R; α-mannosidase (Jack bean; Glyko, UK) transformed a mixture of high-mannose N-glycans to the $Man_1GlcNAc_2$ N-glycan core trisaccharide; recombinant β1,3-galactosidase (Calbiochem, USA) digested Galβ3GlcNAc-R but not Galβ4GlcNAc-R; α3/4-fucosidase (*Xanthomonas* sp.; Calbiochem, USA) digested Galβ4(Fucα3)GlcNAcβ3Galβ4Glc but not Fucα2Galβ3GlcNAcβ3(Galβ4GlcNAcβ6)Galβ4Glc. Control digestions were performed in parallel and analysed similarly to the analytical exoglycosidase reactions.

## RESULTS

*Glycan isolation and analysis.* Detached and purified glycans from paraffin-embedded formalin-fixed tissue samples from cancer patients were analysed by MALDI-TOF mass spectrometry after isolation of the neutral glycan fraction. Relative quantification of the glycans were done by comparing relative MALDI-TOF MS signal intensities, which is accurate for the obtained mixtures of purified glycans (Saarinen *et al*., 1999; Harvey *et al*., 1993).

*Specific mannosidase digestion analyses.* The proportions of the non-reducing terminal α-mannose containing glycans in the samples were determined by their sensitivity towards hydrolysis with α-mannosidase from Jack beans. After the specific exoglycosidase digestion, the presence of terminal α-mannose residues containing glycans in the original samples could be deduced by their disappearance from the recorded mass spectra, and the simultaneous increase in signal intensities of the expected reaction products at m/z 609 and 755, which correspond to the sodium adduct ions $[Hex_1HexNAc_2+Na]^+$ (calc. m/z 609.21) and $[Hex_1HexNAc_2dHex_1+Na]^+$ (calc. m/z 755.27), respectively. The glycans that resisted digestion with mannosidases, were studied further.

*Specific galactosidase and fucosidase digestion analyses.* The structural features of the mannosidase-resistant glycans in the samples were investigated by digestion with S. *pneumoniae* β1,4-galactosidase, recombinant β1,3-galactosidase, and α3/4-fucosidase. An example of the reaction scheme is presented in Figure 6 and the results are summarized in Table 3. According to the digestion results, a significant proportion of signals in the original samples, with proposed compositions $Hex_2HexNAc_2$ and $Hex_2HexNAc_2dHex_1$, correspond to glycans that contain non-reducing terminal β1,4-linked galactose residues and/or α1,3-fucose residues, respectively.

*N-glycosidase digestion analyses.* The assignment of the majority of the $Hex_{1-9}HexNAc_2$ and $Hex_{1-5}HexNAc_2dHex_1$ glycan components as low-mannose and high-mannose type N-glycans was confirmed by their isolation and digestion analysis by a specific endoglycosidase enzyme, namely N-glycosidase F from *Chryseobacterium meningosepticum.* In combination with the α- and β-mannosidase digestion results, these experiments indicate that the mannosidase-resistant components at m/z 771 and 917, contain glycan species that are not N-glycans.

The presence of a glycan fragment at m/z 608 in the tissue samples corresponds to a structure, in which a $Hex_1HexNAc_1$ unit is linked to the 6-position of an O-glycan core GalNAc fragment. The presence of the m/z 608 peaks in the tissue samples indicates that part of the O-glycan structures may contain the Core 2 O-glycan structure. However, Core 1 O-glycan structures may also be present in the samples. Specific β1,3-galactosidase experiments can be used to reveal the relative proportions of these structures in the samples. Taken together, all the experiments suggest that the mannosidase-resistant oligosaccharides that have monosaccharide compositions $Hex_2HexNAc_2$ and $Hex_2HexNAc_2dHex_1$, include the structures presented in Figure 7.

### 1.3 Sialylated Core 2 type O-glycans

*Exoglycosidase digestions.* All exoglycosidase reactions were performed essentially as described previously (Saarinen *et al*., 1999) and analysed by MALDI-TOF MS. The enzymes and their specific control reactions with characterised oligosaccharides were (R denotes reducing end oligosaccharide sequences in the following examples): β1,4-galactosidase *(Streptococcus pneumoniae,* recombinant, *E. coli;* Calbiochem, USA) digested Galβ4GlcNAc-R but not Galβ3GlcNAc-R; *Arthrobacter ureafaciens* neuraminidase (Calbiochem, USA) digested both Neu5Aca3Galβ4GlcNAc-R and Neu5Aca6Galβ4GlcNAc-R; *Streptococcus pneumoniae* α2,3-sialidase (Calbiochem, USA) digested Neu5Aca3Galβ4GlcNAc-R but not Neu5Acα6Galβ4GlcNAc-R. Control digestions were performed in parallel and analysed similarly to the analytical exoglycosidase reactions.

*Chemical modification reactions.* Mild acid hydrolysis of sialic acid residues was performed with 50 mM trifluoroacetic acid in water at 60 °C for 5 hours. After the reaction, the acid was eliminated by evaporation. Mild periodate oxidation, alkaline reduction with borohydride, mild alkaline hydrolysis for cleavage of carboxylic acid esters, and permethylation for fragmentation analyses were performed essentially as described previously (Ylönen *et al*., 2001). Methylation with iodomethane was performed essentially as described previously (Powell & Harvey, 1996).

## RESULTS

*Glycan isolation and analysis.* Detached and purified glycans from paraffin-embedded formalin-fixed tissue samples from cancer patients were analysed by MALDI-TOF mass spectrometry after isolation of the neutral glycan fraction. Relative quantification of the glycans were done by comparing relative MALDI-TOF MS signal intensities, which is accurate for the obtained mixtures of purified glycans (Papac *et al*., 1996; Harvey, 1993; Saarinen *et al*., 1999).

*Indicative mass spectrometric signals of the structure group.* The indicative mass spectrometric signals of the structure group, in both positive and negative ion mode MALDI-TOF MS, are presented in Table 5. Examples of the glycan antigen signals present in lung cancer tumor samples from a patient with non-small cell lung adenocarcinoma, are presented in Figure 13.

*General features of the structure group.* The indicative glycan signals of the structure group in the non-sialylated glycan fraction, include O-glycan fragments that share in common the presence of an unusual reducing end terminal monosaccharide (2-acetamido-3-amino-2,3-dideoxyhexose, or deoxyamino-HexNAc, in which hexose is either D-galactose, D-gulose, D-allose, or D-glucose), which results from the strong alkaline conditions in the glycan isolation method, as discussed below. The major structure present at m/z 899 in various glycan samples from cancer patients is presented in Figure 11, together with the principal biochemical evidence that supports the proposed structure. The more complex components in the structure group contain 1-2 additional HexHexNAc units and/or 1-2 dHex units, forming either linear or branched structures.

*Nature of the acidic group.* The acidic group of the m/z 899 peak was recognized as N-acetylneuraminic acid, based on the following experiments. Mild acid hydrolysis destroyed the m/z 899 peak without affecting the other peaks in the profile. Simultaneously, this resulted in increase of the signal at m/z 608, which has 291 mass units smaller m/z value, corresponding to a mass difference of an acetylneuraminic acid residue. Mild periodic acid oxidation and subsequent borohydride reduction, resulted in the destroying of the m/z 899 peak. However, the m/z 608 peak together with the neutral glycan peaks in the glycan profile, were not affected by the periodic acid treatment. This corresponds to a cleavage between the C7 and C8 carbons of the glycerol tail, namely removal of 60 mass units ($C_2H_4O_2$) from a neuraminic acid residue, and addition of 2 mass units (due to reduction of the reducing end of the oligosaccharide). The cleavage site was further shown to reside in the acid-labile sialic acid residue, because the neutral fragment at m/z 608 was not affected by mild periodate, but was instead reduced and transformed into m/z 610 during the reaction. Furthermore, both *Arthrobacter ureafaciens* neuraminidase and recombinant *Streptococcus pneumoniae* α2,3-sialidase hydrolyzed the m/z 899 peak from the spectrum, and transformed it into the peak at m/z 608. This suggests that the sialic acid linkage is α2→3, and not either α2→6, α2→8, or α2→9, to the next monosaccharide residue in the sequence. In addition, cleavage of a 291 mass unit fragment was shown to be the major cleavage route of the m/z 899 glycan peak, in post-source decay (PSD) MALDI-TOF mass spectrometric fragmentation experiments (Figure 12). The identification of the sialic acid residue as N-acetylneuraminic acid is based on the strongly alkaline conditions of the glycan isolation procedure. In these conditions, any O-acetyl groups would have been removed from the glycans.

*Oligosaccharide sequence of the m/z 899 glycan.* After removal of the sialic acid residue, the oligosaccharide sequence of the remaining glycan at m/z 608 was studied by specific exoglycosidases. *Streptococcus pneumoniae* β1,4-galactosidase, but not a recombinant β1,3-galactosidase, transformed the m/z 608 peak into a peak at m/z 446, corresponding to the removal of one hexose residue. Together with the known specificity of the enzymes and the general biosynthetic routes of human O-glycan structures (Brockhausen, 1999), this suggests that the major component at m/z 899 in the glycan profiles contains the non-reducing terminal oligosaccharide sequence NeuNAcα2-3Galβ1-4GlcNAcβ-R, where R is the reducing end component of 243 mass units that corresponds to the sodium adduct ion of acetamido-amino-dideoxyhexose $[C_8H_{16}N_2O_5+Na]^+$.

*Nature of the reducing terminal monosaccharide.* The m/z 899 glycan had an intact reducing terminal, as evidenced by the transformation of the peak into a peak at m/z 901 upon reduction with alkaline sodium borohydride. The reducing terminal monosaccharide also contained a free primary amino group, as evidenced by the following experiments. Upon N-acetylation by acetic anhydride, the glycan peak was transformed into a peak at m/z 941, corresponding to an addition of 42 mass units, typical for acetylation. The +42 Da modification was resistant to mild alkaline hydrolysis, which is in accordance with the suggested acetamido linkage. Furthermore, the glycan peaks at m/z 899 and m/z 608 are usually accompanied by peaks with 22 mass units lesser mass in the mass spectra of the present experiments, namely at m/z 877 and m/z 586 respectively, corresponding to proton adduct ions of the same molecule ($[M+H]^+$). In contrast, neither the normal neutral oligosaccharides present in the same glycan profile, nor the acetylated counterparts of the same glycan peaks have the accompanying proton adduct peaks. This suggests that the non-acetylated glycans at m/z 899 and m/z 608 have an unusual basic functional group, which is in accordance with the presence of the suggested free primary amine group. The reducing terminal monosaccharide could also be efficiently methylated by iodomethane in alkaline dimethylsulfoxide. In this reaction, the parent glycan peak at m/z 899 was transformed into a peak at m/z 933 for $[M+C_4H_9]^+$, corresponding to the formation of a quarternary amine group and a molecular ion, and with the sialic acid residue transformed into a methyl ester. A carboxylic acid methyl ester is alkali-labile, and accordingly, upon mild alkali hydrolysis, this group was transformed into a free carboxylic acid. The resulting ion that corresponds to the formation of a quaternary amine strongly suggests for the presence of a primary amine in the original molecule.

The formation of the m/z 899 fragment was further studied by using bovine fetuin as a model glycoprotein. Fetuin contains both Core 1 and Core 2 branched O-glycans with structures NeuNAcα2-3Galβ1-3GalNAc(α-O-Ser/Thr), NeuNAcα2-3Galβ1-3(NeuNAcβ2-6)GalNAc(α-O-Ser/Thr), and NeuNAcα2-3Galβ1-4GlcNAcβ1-6([±NeuNAcα2-3]Galβ1-3)GalNAc(α-O-Ser/Thr), respectively. The non-reductive β-elimation glycan isolation procedure that was used in glycan isolation from cancer patient tissues, produced abundant glycans at m/z 899. This glycan fragment was similar in its biochemical properties to its counterpart in human tissues. The only parent molecules available in the fetuin glycoprotein for the formation of the m/z 899 fragment, are the O-glycans that contain the substructure NeuNAcα2-3Galβ1-4GlcNAcβ1-6(R-3)GalNAc(α-O-Ser/Thr), where R are [±NeuNAcα2-3]Gal(β).

Based on known susceptibility of the 3-position substituent of GalNAc to β-elimination in alkaline conditions, the structure of the m/z 899 glycan peak could be assigned as arising from elimination of the 3-substituent from the O-glycan, and subsequent addition of ammonia into the unsaturated glycan ring, which forms a primary amine functional group into the reducing end monosaccharide that arises from the GalNAc residue. Furthermore, as the fragmentation starts with elimination from the 3-position of GalNAc, the amine modification will reside in the 3-position of the monosaccharide. In conclusion, the evidence suggests that the reducing terminal monosaccharide is 2-acetamido-3-amino-2,3-dideoxyhexose, to which the rest of the glycan sequence is attached at 6-position. The sequence of the major oligosaccharide present at m/z 899 is therefore NeuNAcα2-3Galβ1-4GlcNAcβ1-6(2-acetamido-3-amino-2,3-dideoxy)hexose. As the fragment formation starts from N-acetylgalactosamine, the most likely hexose isomers in the fragment are D-galactose, D-gulose, D-allose, and D-glucose.

*Mass spectrometric fragmentation analyses.* The fragments obtained in post-source decay MALDI-TOF mass spectrometry, from native glycan peaks at m/z 899 and m/z 608, and their acetylated as well as deuteroacetylated forms, showed the presence and sequence of the acetylneuraminic acid, hexose, and N-acetylhexosamine residues in the m/z 899 glycan, thus confirming the structural features described above (Figure 12).

*Analysis of sialylated glycans.* In negative ion mode MALDI-TOF MS of the isolated sialylated glycan fraction of lung tumor and healthy control tissues, more specifically patients with non-small cell lung adenocarcinoma and ovarian cystadenocarcinoma, when tumor samples were compared to the corresponding healthy lung and ovary samples, respectively, sialylated glycan peaks were elevated at m/z 1038, corresponding to $NeuNAc_1Hex_2HexNAc_2$ (calc. m/z 1038.36 for the ion $[M-H]^-$), at m/z 1329, corresponding to $NeuNAc_2Hex_2HexNAc_2$ (calc. m/z 1329.46 for the ion $[M-H]^-$), and at m/z 1475, corresponding to $NeuNAc_2Hex_2HexNAc_2dHex_1$ (calc. m/z 1475.52 for the ion $[M-H]^-$). This indicates that these glycan components are major parent glycans from which originates the m/z 899 glycan peak present in the positive ion mode mass spectra.

In contrast, sialylated glycan peaks were decreased at m/z 673, corresponding to $NeuNAc_1Hex_1HexNAc_1$ (calc. m/z 673.23 for the ion $[M-H]^-$), and at m/z 964, corresponding to $NeuNAc_2Hex_1HexNAc_1$ (calc. m/z 964.33 for the ion $[M-H]^-$), when compared to the larger glycan peaks mentioned above. This indicates that the increase in the amounts of the larger glycans happens in conjunction with the decrease in the amounts of the smaller glycans at m/z 673 and m/z 964. Furthermore, this suggests a change from Core 1 type O-glycans to Core 2 type O-glycans associated with malignant tumor samples.

Furthermore, in the healthy control samples from the lung and the ovary, no detectable peaks were present at m/z at m/z 1081, corresponding to $NeuNAc_1Hex_1HexNAc_3$ (calc. m/z 1081 for the ion $[M-H]^-$), at m/z 1370, corresponding to $NeuNAc_2Hex_1HexNAc_3$ (calc. m/z 1329.46 for the ion $[M-H]^-$), or at m/z 1516, corresponding to $NeuNAc_2Hex_1HexNAc_3dHex_1$ (calc. m/z 1475.52 for the ion $[M-H]^-$). This indicates that the major 3-position substituents of the m/z 899 component present in the positive ion mode mass spectra, may be either a hexose monosaccharide, or a neuraminic acid-hexose disaccharide, in the original sample.

Samples from benign tumors of the ovary, namely benign ovarian cystadenoma, were similar to the healthy ovary sample in respect of their specific glycan structures, indicating that the described changes in the relative amounts of the glycan peaks, reflect a change associated with malignant transformation of cancer, or at least ovarian adenocarcinoma.

**EXAMPLE 2. Expression of glycans in tissue samples of various cancer patients.**

**EXPERIMENTAL PROCEDURES**

*Statistical calculations.* Statistical analyses were performed with the SAS Software (SAS System, version 8.2, SAS Institute Inc., Cary, NC, USA), using SAS/STAT and SAS/BASE modules. All tests were performed as two-sided. The distributions of the experimental data were evaluated as 1) normal and symmetric, 2) only symmetric, or 3) non-symmetric and not normal, and the statistical test used was accordingly chosen as 1) Student's t Test, 2) Wilcoxon Signed Rank Test, or 3) Sign Test. A p value of less than 0.05 was considered statistically significant.

**RESULTS**

**2.1 Neutral low-mannose type N-glycans**

*Neutral low-mannose type N-glycans are more abundant in tumor tissue samples than in healthy control tissue samples from cancer patients.* Formalin-fixed samples, from tumor and surrounding healthy tissue, were obtained from patients with various types of cancer. The studied cancer types included non-small cell lung adenocarcinoma, ductale

breast carcinoma, lobulare breast carcinoma, stomach cancer, colon cancer, kidney cancer, ovarian carcinoma, pancreatic cancer, and cancers of the lymph nodes and the larynx. There were significant differences between the neutral low-mannose type N-glycans isolated from tumor samples and healthy tissue samples (Table 1), more specifically the $Hex_{2-4}HexNAc_2$ and $Hex_{2-5}HexNAc_2dHex_1$ neutral glycans, as described above. Neutral low-mannose type N-glycans were shown to be expressed in statistically significant manner in lung cancer and in two types of breast cancer (Table 2). In the examples below, it must be taken into account that at least m/z 609, 755, 771, and 917, glycan peaks may contain multiple oligosaccharide structures.

### 2.2 Neutral O-glycans

*The neutral O-glycans $Hex_2HexNAc_2dHex_{0-1}$ are more abundant in tumor tissue samples than in healthy control tissue samples from cancer patients.* Formalin-fixed samples, from tumor and surrounding healthy tissue, were obtained from patients with various types of cancer. The studied cancer types included non-small cell lung adenocarcinoma, ductale breast carcinoma, lobulare breast carcinoma, stomach cancer, colon cancer, kidney cancer, ovarian carcinoma, pancreatic cancer, and cancers of the lymph nodes and the larynx. There were significant differences between the neutral O-glycans isolated from tumor samples and healthy tissue samples (Table 3), more specifically the $Hex_2HexNAc_2$ and $Hex_2HexNAc_2dHex_1$ neutral glycans, as described above. O-glycans were shown to be expressed in statistically significant manner in lung cancer and in two types of breast cancer (Table 4). In the examples below, it must be taken into account that the m/z 771 and 917 glycan peaks may contain multiple oligosaccharide structures. However, while the m/z 917 glycan peak contains significant amounts of mannosidase-sensitive glycans, the vast majority of the glycans in the glycan peak at m/z 771, corresponding to $Hex_2HexNAc_2$, are mannosidase-resistant.

### 2.3 m/z 899 series glycans

*The 899 series glycans are more abundant in tumor tissue samples than in healthy control tissue samples from cancer patients.* Formalin-fixed samples, from tumor and surrounding healthy tissue, were obtained from patients with various types of cancer. The studied cancer types included non-small cell lung adenocarcinoma, ductale breast carcinoma, lobulare breast carcinoma, stomach cancer, colon cancer, kidney cancer, ovarian carcinoma, pancreatic cancer, and cancers of the lymph nodes and the larynx. There were significant differences between the m/z 899 series glycans isolated from tumor samples and healthy tissue samples (Figure 13), and the results are summarized in Table 6. The m/z 899 glycan fragment was shown to be expressed in statistically significant manner in comparison to normal tissue in populations of patients, in lung cancer and in two types of breast cancer (Table 7).

## EXAMPLE 3. Expression of glycans in lung cancer patients.

### 3.1 Neutral low-mannose type N-glycans

In a group of lung cancer patients, more specifically non-small cell lung adenocarcinoma, the glycan peaks at m/z 609, 755, 771, 917, 1079, 1095, 1241, and/or 1403 were expressed in significantly elevated amounts in the tissue samples from the tumor (Table 1), when compared to healthy control tissues from the same patients. These glycan peaks correspond to $Hex_1HexNAc_2$, $Hex_1HexNAc_2dHex_1$, $Hex_2HexNAc_2$, $Hex_2HexNAc_2dHex_1$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. An example pair of mass spectra from a lung cancer patient is presented in Figure 3.

### 3.2 Neutral O-glycans

In a group of lung cancer patients, more specifically non-small cell lung adenocarcinoma, the glycan peaks at m/z 771 and 917 were expressed in significantly elevated amounts in the tissue samples from the tumor (Table 3), when compared to healthy control tissues from the same patients. These glycan peaks correspond to $Hex_2HexNAc_2$ and $Hex_2HexNAc_2dHex_1$ glycan epitopes, respectively. This difference was shown to be statistically significant (Table 4). As stated above, the glycan peak at m/z 771 is practically mannosidase-resistant, while the peak m/z 917 consists of multiple structures. An example pair of mass spectra from a lung cancer patient is presented in Figure 8.

### 3.3 m/z 899 series glycans

In a group of lung cancer patients, more specifically non-small cell lung adenocarcinoma, the glycan peaks at m/z 899 and m/z 1045 were expressed in significantly elevated amounts in the tissue samples from the tumor (Table 6 and Figure 13), when compared to healthy control tissues from the same patients. These glycan peaks correspond to

$NeuNAc_1Hex_1HexNAc_1$ and $NeuNAc_1Hex_1HexNAc_1dHex_1$ glycan epitopes linked to the 6-position of the O-glycan core GalNAc residue, respectively, when the GalNAc had been originally substituted to the 3-position in the intact tissue. This difference was shown to be statistically significant (Table 7).

## EXAMPLE 4. Expression of glycans in ductale breast cancer patients.

### 4.1 Neutral low-mannose type N-glycans

In a group of breast cancer patients, more specifically ductale breast carcinoma, the glycan peaks at m/z 609, 771, 917, 933, 1079, 1095, 1241, and/or 1403 were expressed in significantly elevated amounts in the tissue samples from the tumor (Table 1), when compared to healthy control tissues from the same patients. These glycan peaks correspond to $Hex_1HexNAc_2$, $Hex_2HexNAc_2$, $Hex_2HexNAc_2dHex_1$, $Hex_3HexNAc_2$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. This difference was shown to be statistically significant (Table 2). An example pair of mass spectra from a ductale breast cancer patient is presented in Figure 4.

### 4.2 Neutral O-glycans

In a group of breast cancer patients, more specifically ductale breast carcinoma, the glycan peaks at m/z 771 and 917 were expressed in significantly elevated amounts in the tissue samples from the tumor (Table 3), when compared to healthy control tissues from the same patients. These glycan peaks correspond to $Hex_2HexNAc_2$ and $Hex_2HexNAc_2dHex_1$ glycan epitopes, respectively. This difference was shown to be statistically significant (Table 4). As stated above, the glycan peak at m/z 771 is practically mannosidase-resistant, while the peak m/z 917 consists of multiple structures. An example pair of mass spectra from a ductale breast cancer patient is presented in Figure 9.

### 4.3. m/z 899 series glycans

In a group of breast cancer patients, more specifically ductale breast carcinoma, the glycan peak at m/z 899 was expressed in significantly elevated amounts in the tissue samples from the tumor (Table 6), when compared to healthy control tissues from the same patients. This glycan peak corresponds to $NeuNAc_1Hex_1HexNAc_1$ glycan epitope linked to the 6-position of the O-glycan core GalNAc residue, when the GalNAc had been originally substituted to the 3-position in the intact tissue. This difference was shown to be statistically significant (Table 7). An example pair of mass spectra from a ductale breast cancer patient is presented in Figure 15.

## EXAMPLE 5. Expression of glycans in lobulare type breast cancer patients.

### 5.1 Neutral low-mannose type N-glycans

In a group of breast cancer patients, more specifically lobulare breast carcinoma, the glycan peaks at m/z 609, 755, 771, 917, 933, 1079, 1095, 1241, and/or and 1403 were expressed in significantly elevated amounts in the tissue samples from the tumor (Table 1), when compared to healthy control tissues from the same patients. These glycan peaks correspond to $Hex_1HexNAc_2$, $Hex_1HexNAc_2dHex_1$, $Hex_2HexNAc_2$, $Hex_2HexNAc_2dHex_1$, $Hex_3HexNAc_2$, $Hex_3HexNAc_2dHex_1$, $Hex_4HexNAc_2$, $Hex_4HexNAc_2dHex_1$, and $Hex_5HexNAc_2dHex_1$ glycan epitopes, respectively. An example pair of mass spectra from a lobulare breast cancer patient is presented in Figure 5.

### 5.2 Neutral O-glycans

In a group of breast cancer patients, more specifically lobulare breast carcinoma, the glycan peaks at m/z 771 and 917 were expressed in significantly elevated amounts in the tissue samples from the tumor (Table 3), when compared to healthy control tissues from the same patients. These glycan peaks correspond to $Hex_2HexNAc_2$ and $Hex_2HexNAc_2dHex_1$ glycan epitopes, respectively. As stated above, the glycan peak at m/z 771 is practically mannosidase-resistant, while the peak m/z 917 consists of multiple structures. An example pair of mass spectra from a lobulare breast cancer patient is presented in Figure 10.

### 5.3 m/z 899 series glycans

In a group of breast cancer patients, more specifically lobulare breast carcinoma, the glycan peak at m/z 899 was expressed in significantly elevated amounts in the tissue samples from the tumor (Table 6), when compared to healthy control tissues from the same patients. This glycan peak corresponds to $NeuNAc_1Hex_1HexNAc_1$ glycan epitope

linked to the 6-position of the O-glycan core GalNAc residue, when the GalNAc had been originally substituted to the 3-position in the intact tissue. This difference was shown to be statistically significant (Table 7). An example pair of mass spectra from a lobulare breast cancer patient is presented in Figure 16.

**EXAMPLE 6. Expression of m/z 899 series glycans in patients with malignant ovarian tumors.**

In a group of patients with ovarian tumors, more specifically malign ovarian cystadenocarcinoma or benign ovarian cystadenoma, the glycan peaks at m/z 899 and m/z 1045 were expressed in significantly elevated amounts in the tissue samples from the malignant tumors (Table 6 and Figure 14), when compared to either the benign tumors or healthy control tissues from the same patients. These glycan peaks correspond to $NeuNAc_1Hex_1HexNAc_1$ and $NeuNAc_1Hex_1HexNAc_1dHex_1$ glycan epitopes linked to the 6-position of the O-glycan core GalNAc residue, respectively, when the GalNAc had been originally substituted to the 3-position in the intact tissue

**EXAMPLE 7. Expression of glycans in individual patient samples of various cancer types.**

**7.1 Neutral low-mannose type N-glycans**

In addition to the statistically studied larger patient populations in lung and breast cancers, neutral low-mannose type N-glycans were expressed in many cancer types, when compared to healthy control tissues from the same patients. These results are summarized in Table 1.

**7.2 Neutral O-glycans**

The neutral O-glycans at m/z 771 and 917 were expressed in many cancer types, when compared to healthy control tissues from the same patients. These results are summarized in Table 3.

**7.3. m/z 899 series glycans**

The m/z 899 series glycans were expressed in many cancer types, when compared to healthy control tissues from the same patients. These results are summarized in Table 6.

**EXAMPLE 8. Detection of lung tissue and lung tumor-specific glycan structures.**

**EXPERIMENTAL PROCEDURES**

*Tissue samples and glycan isolation.* Archival paraffin-embedded and formalin-fixed tissue samples were from patients with non-small cell adenocarcinoma. After deparaffinisation, protein-linked glycans were detached from tissue sections with non-reductive alkaline β-elimination in concentrated ammonia-ammonium carbonate essentially as described previously (Huang et al., 2001). The isolated glycans were purified and divided into sialylated and non-sialylated glycan fractions as described in the other Examples of the present invention.

*MALDI-TOF mass spectrometry.* MALDI-TOF mass spectrometry was performed with a Voyager-DE STR BioSpectrometry Workstation, essentially as described previously (Saarinen *et al*., 1999; Harvey *et al*., 1993). Relative molar abundancies of both neutral (Naven & Harvey, 1996) and sialylated (Papac *et al*., 1996) glycan components were assigned based on their relative signal intensities.

**RESULTS AND DISCUSSION**

*Occurrence of multiple cancer-associated protein-linked glycans in tissue glycan profiles.* **Figure 17** shows the neutral glycan profiles averaged from multiple lung cancer samples. In the healthy tissue profiles, signals corresponding to neutral O-glycans (m/z 771, 917), sialylated O-glycans (m/z 899), and several signals among the low-mannose N-glycans (m/z 917, 1079, 1095, 1241, and 1403), are indicated as cancer-associated, as discussed in the preceding Examples. Also a profile change in the relative proportions of the signals at m/z 1485, 1647, and 1809 is visible: in the cancer samples the relative glycan abundancies are in the order 1485 > 1647 > 1809, whereas in the healthy samples the signals are approximately of the same abundance. The signals at m/z 1485 and 1647, corresponding to $Hex_3HexNAc_4dHex_1$ and $Hex_4HexNAc_4dHex_1$, contain non-reducing terminal GlcNAcβ residues, as evidenced by *S. pneumoniae* β-glucosaminidase digestion. It is concluded that terminal GlcNAcβ residues are associated with lung cancer and also with the three other cancer-associated glycan groups discussed above. Furthermore, the present method can detect these and other potential cancer-associated glycosylation changes simultaneously, allowing for multiparameter

cancer diagnostics.

**EXAMPLE 9. Detection of ovarian tissue and ovarian tumor-specific glycan structures.**

## EXPERIMENTAL PROCEDURES

*Tissue samples and glycan isolation.* Archival paraffin-embedded and formalin-fixed tissue samples were from patients with malignant ovarian cystadenocarcinoma or benign ovarian cystadenoma. After deparaffinisation, protein-linked glycans were detached from tissue sections with non-reductive alkaline β-elimination in concentrated ammonia-ammonium carbonate essentially as described previously (Huang et al., 2001). The isolated glycans were purified and divided into sialylated and non-sialylated glycan fractions as described in the other Examples of the present invention.

*MALDI-TOF mass spectrometry.* MALDI-TOF mass spectrometry was performed with a Voyager-DE STR BioSpectrometry Workstation, essentially as described previously (Saarinen *et al*., 1999; Harvey *et al*., 1993). Relative molar abundancies of both neutral (Naven & Harvey, 1996) and sialylated (Papac *et al*., 1996) glycan components were assigned based on their relative signal intensities. The mass spectrometric fragmentation analysis was done with the Voyager-DE STR BioSpectrometry Workstation according to manufacturer's instructions. For the fragmentation analysis, sialylated glycans were further purified by gel filtration HPLC and permethylated essentially as described previously (Nyman *et al*., 1998).

*Exoglycosidase digestions.* Digestions with *A. ureafaciens* neuraminidase (Glyko, UK), S. *pneumoniae* β-glucosaminidase (Calbiochem, USA), and Jack bean β-hexosaminidase (C. *ensiformis*; Calbiochem, USA) were performed essentially as described previously (Saarinen et al., 1999). The specificity of the two latter enzymes was controlled with synthetic oligosaccharides with terminal 1) Galβ1-4GlcNAcβ, 2) GlcNAcβ, and 3) GalNAcβ1-4GlcNAcβ epitopes: β-hexosaminidase digested the HexNAc residues in 2) and 3), but not 1), and β-glucosaminidase digested 2) but not 1) or 3).

## RESULTS

*Occurrence of HexNAcβHexNAcβ sequences in sialylated and neutral protein-linked glycans from ovarian tissue samples.* **Figure 18** shows MALDI-TOF mass spectra from normal ovarian tissue (**Fig. 18A**) and ovarian tumors. Especially in benign ovarian tumors (**Fig. 18B**) there occurs high amounts of sialylated glycan structures with more HexNAc than Hex units in their proposed monosaccharide compositions (**Table 8**). Such glycans include glycans No **26, 27, 36,** and **38** (**Table 8**) which are major glycans in the benign tumors. However, only one of these glycans occurs in the malignant tissue and in a significantly reduced amount. In fact, in the sample from normal ovary tissue these glycan signals are more abundant than in the malignant tumour samples. In the neutral glycan fraction, the situation is similar (**Figure 19**). The normal ovary sample and all the benign ovarian tumor samples resemble each other in that they contain more glycans at m/z 1850 and 1891, corresponding to $Hex_4HexNAc_5dHex_1$ and $Hex_3HexNAc_6dHex_1$, respectively.

*Structures of the glycans.* The sialylated glycans were indicated to contain sialic acid residues upon neuraminidase digestion and subsequent analysis by MALDI-TOF mass spectrometry (data not shown). Exoglycosidase digestions of both neutral and sialylated fractions of benign ovarian tumor glycan samples showed that e.g. sialylated glycans 26 and 27, as well as neutral glycans at m/z 1850 and 1891 were resistant to the action of β-glucosaminidase, but upon β-hexosaminidase digestion they lost either two or four HexNAc units. This indicates that the terminal residue in these glycans is not GlcNAcβ but that it may be GalNAcβ which is terminal to another HexNAcβ unit. The possible such structures include LacdiNAc (GalNAcβ4GlcNAcβ) that has been indicated previously in an ovarian glycoprotein. **Figure 20** shows a fragmentation mass spectrometric analysis of permethylated glycan 26 from benign ovarian tumor. The results indicate that glycan 26 contains the structures drawn in the Figure, which include both neutral and sialylated terminal HexNAc-HexNAc sequences. In conclusion, the present results therefore suggest that malignant transformation of the ovary is associated with diminished amounts of HexNAcβHexNAcβ sequences, more specifically including neutral and sialylated LacdiNAc sequences.

*Cancer-associated glycan signals and glycosylation changes.* The present Example shows that multiple glycans and the glycan profiles are different between normal and malignant ovarian tissue, and that benign and malignant tumors differ from each other in multiple glycosylation features (**see Figures 18 and 19**). Significantly, also neutral O-glycan, sialylated O-glycan, and low-mannose N-glycan signals are elevated in all malignant ovarian tumor samples compared to the normal ovary and benign ovarian tumor samples (**Figure 19**), as discussed in the other Examples of the present invention.

**EXAMPLE 10. Discrimination analysis of major cancer types based on mass spectrometric glycan profiling.**

**EXPERIMENTATION AND RESULTS**

Relative abundancy profiles were obtained for the neutral protein-linked glycan fractions of cancer patient tissue samples with ductale and lobulare type breast adenocarcinoma, non-small cell lung adenocarcinoma, colon carcinoma or benign colon tumor, and ovarian cystadenocarcinoma (malignant tumor) or cystadenoma (benign tumor) as described in the other Examples.

*Generation of a discrimination formula.* A principal component and discrimination analysis was done to the results of a randomly picked group of four cancer and four healthy tissue samples from patients with ductale type breast carcinoma. It was found that three glycan signals could resolve the samples into cancer and healthy groups (**Fig. 21A**, 'training group'), and the experimental formula is:

$$5.36 \times I\ (m/z\ 771) + 20.0 \times I\ (m/z\ 899) + 8.13 \times I\ (m/z\ 917) - 60.6,$$

where 'I (glycan signal)' refers to the relative abundance of the glycan signal marked in parenthesis, the three glycan signals correspond to sodium adduct ions of $Hex_2HexNAc_2$, $NeuAc_1Hex_1HexNAc_1$(deoxyamino)HexNAc, and $Hex_2HexNAc_2dHex_1$, respectively, and the resulting 'scores' for each sample are plotted on the y-axis in **Figure 21**. As described in the preceding Examples, these glycan signals more specifically correspond to neutral O-glycans, sialylated Core 2 type O-glycans, and low-mannose N-glycans.

*Testing the discrimination formula.* The obtained experimental discrimination formula was first applied to neutral protein-linked glycan analysis results of a group of ductale type breast carcinoma patients. As seen in Figure xA ('test group'), the formula could correctly discriminate 10 out of 10 samples (100 %). Similarly, the formula was applied to samples from lobulare type breast carcinoma and lung cancer patients, and it discriminated correctly 12 out of 14 (86 %) and 15 out of 17 (88 %) samples from these patients, respectively. When applied to samples of ovarian tumors and healthy ovarian tissue (**Fig. 21B**), the formula correctly discriminated 11 out of 11 samples (100 %) and placed the normal sample into the group of benign tumors. Similarly, when applied to samples of colon tumors and healthy colon tissue (**Fig. 21C**), the formula correctly discriminated 6 out of 6 samples (100 %) and placed the normal samples into the group of benign tumors.

**CONCLUSIONS**

The present results show that a simple experimental discrimination formula derived from a small group of breast cancer patients, applied on the results of neutral protein-linked glycan profiling, could effectively discriminate between cancerous and healthy samples of multiple cancer types. In particular, the same formula could correctly differentiate between malignant and benign tumors in both colon and ovary. In addition, it was shown that benign tumors resemble normal tissues and that malignant tumors differ significantly from both normal tissues and benign tumors with respect to the relative abundancies of glycan signals including neutral O-glycans, sialylated Core 2 type O-glycans, and low-mannose N-glycans. In conclusion, similar discrimination procedures can be used in diagnostics of human tumors and cancer. The present results indicate that there are individual differences in the expression of the cancer-associated glycan structures in both normal tissues between persons and in cancerous tissues between individual tumors. However, this did not effect the detection results of the present method.

Due to the presence of tissue-specific cancer-associated glycosylation revealed in the present invention, such as HexNAcβHexNAcβ glycans in tumors of the ovary (described in the other Examples), it is indicated that tissue-specific discrimination formulas based on the results of the present invention can differentiate between cancer and healthy samples and/or benign tumors even more efficiently than the exemplary formula of the present Example.

**EXAMPLE 11.** Glycan isolation and analysis.

**EXAMPLES OF GLYCAN ISOLATION METHODS**

*Glycan isolation.* N-linked glycans are preferentially detached from cellular glycoproteins by *F. meningosepticum* N-glycosidase F digestion (Calbiochem, USA) essentially as described previously (Nyman *et al*., 1998), after which the released glycans are preferentially purified for analysis by solid-phase extraction methods, including ion exchange separation, and divided into sialylated and non-sialylated fractions. For O-glycan analysis, glycoproteins are preferentially

subjected to reducing alkaline β-elimination essentially as described previously (Nyman *et al*., 1998), after which sialylated and neutral glycan alditol fractions are isolated as described above. Free glycans are preferentially isolated by extracting them from the sample with water.

*Example of a glycan purification method.* Isolated oligosaccharides can be purified from complex biological matrices as follows, for example for MALDI-TOF mass spectrometric analysis. Optionally, contaminations are removed by precipitating glycans with 80-90 % (v/v) aqueous acetone at -20 °C, after which the glycans are extracted from the precipitate with 60 % (v/v) ice-cold methanol. After glycan isolation, the glycan preparate is passed in water through a strong cation-exchange resin, and then through $C_{18}$ silica resin. The glycan preparate can be further purified by subjecting it to chromatography on graphitized carbon material, such as porous graphitized carbon (Davies, 1992). To increase purification efficiency, the column can be washed with aqueous solutions. Neutral glycans can be washed from the column and separated from sialylated glycans by elution with aqueous organic solvent, such as 25 % (v/v) acetonitrile. Sialylated glycans can be eluted from the column by elution with aqueous organic solvent with added acid, such as 0.05 % (v/v) trifluoroacetic acid in 25 % (v/v) acetonitrile, which elutes both neutral and sialylated glycans. A glycan preparation containing sialylated glycans can be further purified by subjecting it to chromatography on microcrystalline cellulose in n-butanol:ethanol:water (10:1:2, v/v) and eluted by aqueous solvent, preferentially 50 % ethanol:water (v/v). Preferentially, glycans isolated from small sample amounts are purified on miniaturized chromatography columns and small elution and handling volumes. An efficient purification method comprises most of the abovementioned purification steps. In an efficient purification sequence, neutral glycan fractions from small samples are purified with methods including carbon chromatography and separate elution of the neutral glycan fraction, and glycan fractions containing sialylated glycans are purified with methods including both carbon chromatography and cellulose chromatography.

*MALDI-TOF mass spectrometry.* MALDI-TOF mass spectrometry is performed with a Voyager-DE STR BioSpectrometry Workstation or a Bruker Ultraflex TOF/TOF instrument, essentially as described previously (Saarinen *et al*., 1999; Harvey *et al*., 1993). Relative molar abundancies of both neutral (Naven & Harvey, 1996) and sialylated (Papac *et al*., 1996) glycan components are assigned based on their relative signal intensities. The mass spectrometric fragmentation analysis is done with the Bruker Ultraflex TOF/TOF instrument according to manufacturer's instructions.

**RESULTS**

*Examples of analysis sensitivity.* Protein-linked and free glycans, including N- and O-glycans, are typically isolated from as little as about 5 x $10^4$ cells in their natual biological matrix and analyzed by MALDI-TOF mass spectrometry.

*Examples of analysis reproducibility and accuracy.* The present glycan analysis methods have been validated for example by subjecting a single biological sample, containing human cells in their natural biological matrix, to analysis by five different laboratory personnel. The results were highly comparable, especially by the terms of detection of individual glycan signals and their relative signal intensities, indicating that the reliability of the present methods in accurately describing glycan profiles of biological samples including cells is excellent. Each glycan isolation and purification phase has been controlled by its reproducibility and found to be very reproducible. The mass spectrometric analysis method has been validated by synthetic oligosaccharide mixtures to reproduce their molar proportions in a manner suitable for analysis of complex glycan mixtures and especially for accurate comparison of glycan profiles from two or more samples. The analysis method has also been successfully transferred from one mass spectrometer to another and found to reproduce the analysis results from complex glycan profiles accurately by means of calibration of the analysis.

*Examples of biological samples and matrices for successful glycan analysis.* The method has been successfully implied on analysis of e.g. blood cells, cell membranes, aldehyde-fixated cells, glycans isolated from glycolipids and glycoproteins, free cellular glycans, and free glycans present in biological matrices such as blood. The experience indicates that the method is especially useful for analysis of oligosaccharide and similar molecule mixtures and their optional and optimal purification into suitable form for analysis.

**EXAMPLE 12.** Glycan profiling.

*Generation of glycan profiles from mass spectrometric data.* **Figure 22A** shows a MALDI-TOF mass spectrum recorded in positive ion mode from a sample of neutral N-glycans. The profile includes multiple signals that interfere with the interpretation of the original sample's glycosylation, including non-glycan signals and multiple signals arising from single glycan signals. According to the present disclosure, the mass spectrometric data is transformed into a glycan profile (**Fig. 22B**), which represents better the original glycan profile of the sample. An exemplary procedure is briefly as follows, and it includes following steps: 1) The mass spectrometric signals are first assigned to proposed monosaccharide compositions e.g. according to **Table 9**. 2) The mass spectrometric signals of ions in the molecular weight are of glycan signals typically show isotopic patterns, which can be calculated based on natural abundancies of the isotopes of the elements in the Earth's crust. The relative signal intensities of mass spectrometric signals near each other can

be overestimated or underestimated, if their isotopic patterns are not taken into account. According to the present method, the isotopic patterns are calculated for glycan signals near each other, and relative intensities of glycan signals corrected based on the calculations. 3) Glycan ions are predominantly present as [M+Na]+ ions in positive ion mode, but also as other adduct ions such as [M+K]+. The proportion of relative signal intensities of [M+Na]+ to [M+K]+ ions is deduced from several signals in the spectrum, and the proportion is used to remove the effect of [M+K]+ adduct ions from the spectrum. 4) Other contaminating mass spectrometric signals not arising from the original glycans in the sample can optionally be removed from the profile, such as known contaminants, products of elimination of water, or in a case of permethylated oligosaccharides, undermethylated glycan signals. 5) The resulting glycan signals in the profile are normalized, for example to 100 %, for allowing comparison between samples.

**Figure 23A** shows a MALDI-TOF mass spectrum recorded in negative ion mode from a sample of neutral N-glycans. The profile includes multiple signals that interfere with the interpretation of the original sample's glycosylation, including non-glycan signals and multiple signals arising from single glycan signals. According to the present disclosure, the mass spectrometric data is transformed into a glycan profile (**Fig. 23B**), which represents better the original glycan profile of the sample. An exemplary procedure is briefly as follows, and it includes following steps: 1) The mass spectrometric signals are first assigned to proposed monosaccharide compositions e.g. according to **Table 10.** 2) The mass spectrometric signals of ions in the molecular weight are of glycan signals typically show isotopic patterns, which can be calculated based on natural abundancies of the isotopes of the elements in the Earth's crust. The relative signal intensities of mass spectrometric signals near each other can be overestimated or underestimated, if their isotopic patterns are not taken into account. According to the present method, the isotopic patterns are calculated for glycan signals near each other, and relative intensities of glycan signals corrected based on the calculations. 3) Glycan ions are predominantly present as [M-H]- ions in negative ion mode, but also as ions such as [M-2H+Na]- or [M-2H+K]-. The proportion of relative signal intensities of e.g. [M-H]- to [M-2H+Na]- and [M-2H+K]- ions is deduced from several signals in the spectrum, and the proportion is used to remove the effect of e.g. these adduct ions from the spectrum. 4) Other contaminating mass spectrometric signals not arising from the original glycans in the sample can optionally be removed from the profile, such as known contaminants or products of elimination of water. 5) The resulting glycan signals in the profile are normalized, for example to 100 %, for allowing comparison between samples.

**EXAMPLE 13.** Glycan structures of Keyhole limpet hemocyanin (KLH) and their modification.

## EXPERIMENTATION AND RESULTS

*Analysis of KLH glycosylation.* Keyhole limpet hemocyanin was from Sigma (*Megathura crenulata*; USA). N-linked and O-linked glycans were released from KLH by N-glycosidase enzyme and alkaline β-elimination, respectively, and the released glycans were purified and analyzed essentially as described in the previous Examples. The detected N-glycan masses, as resolved by MALDI-TOF mass spectrometry, were essentially similar to those described by Kurokawa et al. (2001). However, novel glycan components could be engineered and/or identified on KLH by exoglycosidase digestions with α-mannosidase (Jack beans, C. *ensiformis*, Sigma) and combined β-galactosidase digestion (β1,4-galactosidase from S. *pneumoniae* and recombinant β1,3/6-galactosidase, Calbiochem, USA). More specifically, based on the susceptibility of the $Hex_3HexNAc_2dHex_1$ glycan to β-galactosidase (one hexose removed) and N-glycosidase F (detachment from the glycoprotein) it was found that KLH contains Manα3(Galβ6)Manβ4GlcNAcβ4(Fucα6)GlcNAc that can be converted to Manα3Manβ4GlcNAcβ4(Fucα6)GlcNAc. The latter structure is a novel component in KLH. High-mannose and low-mannose N-glycans were similarly identified based on susceptibility to α-mannosidase. It was concluded that KLH contains at least the following human-type glycans in significant amounts: $(Man\alpha)_3$Manβ4GlcNAcβ4GlcNAc, $(Man\alpha)_4$Manβ4GlcNAcβ4GlcNAc, $(Man\alpha)_5$Manβ4GlcNAcβ4GlcNAc, $(Man\alpha)_6$Manβ4GlcNAcβ4GlcNAc, $(Man\alpha)_1$Manβ4GlcNAcβ4(Fucα6)GlcNAc, $(Man\alpha)_2$Manβ4GlcNAcβ4(Fucα6)GlcNAc, $(Man\alpha)_3$Manβ4GlcNAcβ4(Fucα6)GlcNAc, $(Man\alpha)_4$Manβ4GlcNAcβ4(Fucα6)GlcNAc, and $(Man\alpha)_5$Manβ4GlcNAcβ4(Fucα6)GlcNAc. About 1/5 of the non-fucosylated and 1/3 of the fucosylated glycans with similar masses as the glycans listed above were assigned as containing β1,6-linked galactose residues based on their resistance to the action of α-mannosidase (Kurokawa et al., 2001). The latter are not human-type glycans.

*Production of KLH with human-type low-mannose N-glycans.* β-galactosidase was used to removal of, and the reaction result was characterized by MALDI-TOF mass spectrometry of released glycans, verifying that significant amounts of β-galactose residues, occurring in KLH glycans and capping the human-type low-mannose N-glycans, were removed. Based on the structural knowledge described previously (Kurokawa et al., 2001), it was concluded that a modified spectrum of KLH glycans was produced, containing more low-mannose N-glycans than original KLH, and especially the novel human-type low-mannose glycan Manα3Manβ4GlcNAcβ4(Fucα6)GlcNAc described above. As described in the preceding Examples, the increased glycans present in KLH are associated with malignant tumors in major human cancer types.

**EXAMPLE 14.** Analyses of human tissue material and cell protein-linked glycan structures.

**EXPERIMENTAL PROCEDURES**

Protein-linked glycans were isolated by non-reductive alkaline elimination essentially as described by Huang et al. (2000), or by N-glycosidase digestion to specifically retrieve N-glycans as described in the preceding **Examples.**

**RESULTS AND DISCUSSION**

*Tissue-specific glycosylation analyses and comparison of glycan profiles between tissues.* Human tissue protein-linked glycan profiles were analyzed from lung, breast, kidney, stomach, pancreas, lymph nodes, liver, colon, larynx, ovaries, and blood cells and serum. In addition, cultured human cells were analyzed similarly. **Tables 14** and **15** show neutral and acidic protein-linked glycan signals, respectively, observed in these human tissues and cells together with their classification into glycan structure groups. However, the individual glycan signals in each structure group varied from sample type to sample type, reflecting tissue material and cell type specific glycosylation. Importantly, in analyses of multiple samples, such as 10 samples from an individual human tissue type, glycan group feature proportions remain relatively constant with respect to variation in the occurrence of individual glycan signals.

Furthermore, it was observed that each tissue demonstrated a specific glycan profile that could be distinguished from the other tissues, cells, or blood or serum samples by comparison of glycan profiles according to the methods described in the present disclosure. It was also found out that glycan profile difference could be quantitated by comparing the difference between two glycan profiles, for example according to the Equation (resulting in difference expressed in %):

$$difference = \frac{1}{2}\sum_{i=1}^{n}\left|p_{i,a} - p_{i,b}\right|,$$

wherein *p* is the relative abundance (%) of glycan signal *i* in profile *a* or *b,* and *n* is the total number of glycan signals. For example, the Equation reveals that human lung and ovary tissue protein-linked glycan profiles differ from each other significantly more than human lung and kidney tissue protein-linked glycan profiles differ from each other. Each tissue or cell type could be compared in this manner.

*Comparison of glycosylation features between human tissue materials.* **Table 16** shows how glycan signal structural classification according to the present disclosure was applied to the comparison of quantitative differences in glycan structural features in glycan profiles between human tissue materials. The results show that each sample type was different from each other with respect to the quantitative glycan grouping and classification. Specifically, normal human lung and lung cancer tissues were different from each other both in the neutral glycan and sialylated glycan fractions with respect to the quantitative glycan structure grouping. In particular, lung cancer showed increased amounts of glycan signals classified into terminal HexNAc containing glycans. In analysis of individual glycan signals by β-glucosaminidase digestion, it was found that lung cancer associated glycan signals, such as $Hex_3HexNAc_4dHex_1$, contained terminal β-linked GlcNAc residues, correlating with the classification of these glycan signals into the terminal HexNAc (N>H and/or N=H) glycan groups. Furthermore, the human serum protein-linked glycan profile showed significantly lower amounts of high-mannose and especially low-mannose type N-glycan signals. It is concluded that the glycan grouping profile of human serum is significantly different from the corresponding profiles of solid tissues, and the present methods are suitable for identification of normal and diseased human tissue materials and blood or serum typical glycan profiles from each other.

*Disease- and tissue-specific differences in glycan structure groups.* **Fig. 19** shows a neutral protein-linked glycan profile of human ovary with abnormal growth. As described above, there are clear differences in the overall glycan profiles of **Fig. 19** and other human tissue samples. In analyses of multiple samples of ovarian tissues, it was found that benign abnormal growth of the ovary is especially characterized by increased amounts of glycan signals classified as terminal HexNAc (N>H). In structural analyses by fragmentation mass spectrometry and combined β-hexosaminidase and β-glucosaminidase digestions, the corresponding terminal HexNAc glycan signals were found to include structures with terminal and sialylated β-GalNAc, more specifically terminal and sialylated GalNAcβ4GlcNAcβ (LacdiNAc) structures. According to the glycan structure classification, the protein-linked glycan profiles of normal ovarian tissue also contain increased amounts of terminal HexNAc glycans compared to other human tissues studied in the present disclosure, and normal human ovary preferentially also contains higher amounts of terminal and/or sialylated LacdiNAc structures than other human tissues on average. However, in malignant transformation the proportion of LacdiNAc structures among the protein-linked glycans of the ovary are decreased, and this is also reflected in the glycan grouping classification of malignant ovarian glycan profiles.

The analysis of protein-linked glycan profiles of human tissues revealed also that tissues with abundant epithelial

structures, such as stomach, colon, and pancreas, contain increased amounts of small glycan structures, preferentially mucin-type glycans, and fucosylated glycan structures compared to the other glycan structure groups in structure classification. Similarly as epithelial tissues, mucinous carcinomas were differentiated from other carcinoma types based on analysis of their protein-linked glycan profiles and structure groups according to the methods of the present disclosure.

**EXAMPLE 15.** Proton-NMR analysis of glycan fractions

EXPERIMENTAL NMR PROCEDURES

Glycan material is liberated from biological material by enzymatic or chemical means. To obtain a less complex sample, glycans are fractionated into neutral and acidic glycan fractions by chromatography on a graphitized carbon as described above. A useful purification step prior to NMR analysis is gel filtration high-performance liquid chromatography (HPLC). For glycans of glycoprotein or glycolipid origin, a Superdex Peptide HR10/300 column (Amersham Pharmacia) may be used. For larger glycans, chromatography on a Superdex 75 HR10/300 column may yield superior results. Superdex columns are eluted at a flow rate of 1 ml per minute with water or with 50-200 mM ammonium bicarbonate for the neutral and acidic glycan fractions, respectively, and absorbance at 205-214 nm is recorded. Fractions are collected (typically 0.5 - 1 ml) and dried. Repeated dissolving in water and evaporation may be necessary to remove residual ammonium bicarbonate salts in the fractions. The fractions are subjected to MALDI-TOF mass spectrometry and all fractions containing glycans are pooled.

Prior to NMR analysis, the pooled fractions are dissolved in deuterium oxide and evaporated. With glycan preparations containing about 100 nmol or more material, the sample is finally dissolved in 600 microliters of high-quality deuterium oxide (99.9-99.996%) and transferred to a NMR analysis tube. A roughly equimolar amount of an internal standard, e.g. acetone, is commonly added to the solution. With glycan preparations derived from small tissue specimens or from a small number of cells (5-25 million cells), the sample is preferably evaporated from very high quality deuterium oxide (99.996%) twice or more to eliminate $H_2O$ as efficiently as possible, and then finally dissolved in 99.996% deuterium oxide. These low-material samples are preferebly analyzed by more sensitive NMR techniques. For example, NMR analysis tubes of smaller volumes can be used to obtain higher concentration of glycans. This kind of tubes include e.g. nanotubes (Varian) in which sample is typically dissolved in a volume of 37 microliters. Alternatively, higher sensitivity is achieved by analyzing the sample in a cryo-NMR instrument, which increases the analysis sensitivity through low electronic noise. The latter techniques allow gathering of good quality proton-NMR data from glycan samples containing about 1-5 nmol of glycan material.

ANALYSIS OF NMR DATA

It is realized that numerous studies have shown that proton-NMR data has the ability to indicate the presence of several structural features in the glycan sample. In addition, by careful integration of the spectra, the relative abundancies of these structural features in the glycan sample can be obtained.
For example, the proton bound to monosaccharide carbon-1, i.e. H-1, yields a distinctive signal at the lower field, well separated from the other protons of sugar residues. Most monosaccharide residues e.g. in N-glycans are identified by their H-1 signals (see Tables 4 and 5 for representative examples). In addition, the H-2 signals of mannose residues are indicative of their linkages.
Sialic acids do not possess a H-1, but their H-3 signals (H-3 axial and H-3 equatorial) reside well separated from other protons of sugar residues. Moreover, differently bound sialic acids may be identified by their H-3 signals. For example, the Neu5Ac H-3 signals of Neu5Ac$\alpha$2-3Gal structure are found at 1.797 ppm (axial) and 2.756 ppm (equatorial). On the other hand, the Neu5Ac H-3 signals of Neu5Ac$\alpha$2-6Gal structure are found at 1.719 ppm (axial) and 2.668 ppm (equatorial). By comparing the integrated areas of these signals, the molar ratio of these structural features is obtained.
Other structural reporter signals are commonly known and those familiar with the art use the extensive literature for reference in glycan NMR assignments.

RESULTS OF MALIGNANT TUMOR GLYCAN ANALYSES BY NMR

**Samples:** N-glycan fractions were liberated from pancreas carcinoma samples, purified as described in the preceding Examples, ultimately by gel filtration HPLC, and fractionated into 1) large neutral, 2) small neutral, and 3) acidic N-glycan fractions. These samples were analyzed by cryo-probe 1H-NMR as described above.

**Large neutral N-glycan fraction:** This fraction contained high-mannose N-glycans corresponding to the structural elements of reference structures in **Figure 26.** Correlation with these structures is described in **Table 11,** demonstrating that such structures were the major glycan signals in the glycan fraction.

**Small neutral N-glycan fraction:** This fraction contained low-mannose N-glycans as well as $Man_nGlcNAc_1$

glycans corresponding to the structural elements of reference structures in **Figure 27.** Correlation with these structures is described in **Table 12,** demonstrating that such structures were major glycan signals in the glycan fraction. In particular, the results demonstrated the presence of Manα3(Manα6)Manβ4GlcNAcβ4GlcNAc low-mannose N-glycan, as well as the presence of $(Man\alpha2)_{0-1}$Manα3 N-glycan antenna, and the presence of $(Man\alpha2)_{0-1}$Manα3(Manα6)Manβ4GlcNAc glycans in the glycan fraction.

**Acidic neutral N-glycan fraction:** This fraction contained complex-type N-glycans corresponding to the structural elements of biantennary N-glycan reference structures in **Figure 28**, as well as triantennary N-glycans with Galβ4GlcNAcβ4Manα3 antennae (not shown). Correlation with these structures is described in **Table 13,** demonstrating that such structures were major glycan signals in the glycan fraction. In particular, the results demonstrated the presence of Galβ4GlcNAcβ2Manα3(Galβ4GlcNAcβ2Manα6)Manβ4GlcNAcβ4(±Fucα6)GlcNAc N-glycan core structure as the major structure, elongated with different sialylation as in the antennae of the reference structures. Quantitative analysis of sialic acid linkages by integration of representative signals in the spectrum yielded α2,6- and α2,3-linked sialic acids in relative proportion 2:1 in the analyzed sample.

**NMR References:**

Fu D., Chen L. and O'Neill R.A. (1994) Carbohydr. Res. 261, 173-186

Hard K., Mekking A., Kamerling J.P., Dacremont G.A.A. and Vliegenthart J.F.G. (1991) Glycoconjugate J. 8, 17-28

Hard K., Van Zadelhoff G., Moonen P., Kamerling J.P. and Vliegenthart J.F.G. (1992) Eur. J. Biochem. 209, 895-915

Helin J., Maaheimo H., Seppo A., Keane A. and Renkonen O. (1995) Carbohydr. Res. 266, 191-209


**EXAMPLE 16. Lysosomal organelle-specific N-glycosylation.**

EXPERIMENTAL PROCEDURES

*Lysosomal protein sample* including human myeloperoxidase was chosen to represent lysosomal organelle glycoproteins. The sample was digested with N-glycosidase F to isolate N-glycans, and they were purified for MALDI-TOF mass spectrometric analysis as described in the preceding Examples.

*Alkaline phosphatase digestion* was performed essentially according to manufacturer's instructions. After the digestion glycans were purified for MALDI-TOF mass spectrometric analysis as above.

RESULTS AND DISCUSSION

*Neutral N-glycan profiles.* The neutral N-glycan profile is presented in **Figure 25** (upper panel). The profile is dominated by low-mannose type and high-mannose-type N-glycan signals, comprising 49% and 46% of the total signal intensity, respectively. Especially the high proportion of low-mannose type N-glycans is characteristic to the sample (**Table 17, upper panel**).

*Acidic N-glycan profiles.* The acidic N-glycan profile is presented in **Figure 25** (lower panel). The profile is dominated by three glycan signal groups: 1) sulphated or phosphorylated low-mannose type and high-mannose type N-glycans ($Hex_{3-8}HexNAc_2SP$), 2) fucosylated hybrid-type or monoantennary N-glycans ($NeuAc_1Hex_{3-4}HexNAc_3dHex_1$), and 3) fucosylated complex-type N-glycans ($NeuAc_1Hex_{4-5}HexNAc_4dHex_{1-2}$). Unusual features of the sample are the high proportion of hybrid-type or monoantennary N-glycans (**Table 17, lower panel**), high fucosylation rate of hybrid-type, monoantennary, and complex-type N-glycans, and the high proportion of the characteristic sulphated or phosphorylated low-mannose type and high-mannose type N-glycans.

*Phosphorylated N-glycans.* Major glycan signals with phosphate or sulphate ester (SP) in their monosaccharide compositions were $Hex_5HexNAc_2SP$ (1313), $Hex_6HexNAc_2SP$ (1475), and $Hex_7HexNAc_2SP$ (1637). When the acidic glycan fraction was subjected to alkaline phosphatase digestion, these major signals were specifically digested and disappeared from the acidic glycan spectrum as detected by MALDI-TOF mass spectrometry (data not shown). In contrast, the major glycan signals with sialic acids in their monosaccharide compositions were not digested, including $NeuAc_1Hex_3HexNAc_3dHex_1$ (1549). This indicates that the three original glycan signals corresponded to phosphorylated N-glycans $(PO_3H)Hex_5HexNAc_2$, $(PO_3H)Hex_6HexNAc_2$, and $(PO_3H)Hex_7HexNAc_2$, respectively, wherein $PO_3H$ denotes phosphate ester.

The data further indicated that the present organelle-specific N-glycan profile included phosphorylated low-mannose type and high-mannose type N-glycans $(PO_3H)Hex_3HexNAc_2$ (989), $(PO_3H)Hex_4HexNAc_2$ (1151), $(PO_3H)Hex_5HexNAc_2$ (1313), $(PO_3H)Hex_6HexNAc_2$ (1475), $(PO_3H)Hex_7HexNAc_2$ (1637), and $(PO_3H)Hex_8HexNAc_2$ (1799). In this glycan profile the phosphorylated glycan residues are preferentially mannose residues, more preferentially

α-mannose residues, and most preferentially 6-phospho-α-mannose residues i.e. ($PO_3H$-6Manα).

**EXAMPLE 17. Identification of specific glycosylation signatures from glycan profiles of malignant and normal human tissue samples based on quantitative glycomics.**

EXPERIMENTAL PROCEDURES

Normal lung (Sample I) and malignant lung tumor samples (Sample II) were archival formalin-fixed and paraffin-embedded tissue sections from cancer patients with small cell lung cancer. Protein-linked glycans were isolated from the representative samples by non-reductive β-elimination, purified, and analyzed by MALDI-TOF mass spectrometry as described in the preceding Examples. In the present analysis, the total desialylated protein-linked glycomes from each sample were used.

To analyze the data and to find the major glycan signals associated with either the normal state or the disease, two variables were calculated for the comparison of glycan signals between the two samples:

1. absolute difference $A = (SII - SI)$, and
2. relative difference $R = A / SI$,

wherein *SI* and *SII* are relative abundances of a given glycan signal in Sample I (normal human lung tissue) and Sample II (small cell lung cancer), respectively.

The glycan signals were further classified into structure classes by a one letter code: *a b c d,* wherein *a* is either N (neutral) or S (sialylated); *b* is either L (low-mannose type), M (high-mannose type), H (hybrid-type or monoantennary), C (complex-type), S (soluble), or O (other); c is either - (nothing), F (fucosylated), or E (multifucosylated); and *d* is either - (nothing), T (terminal HexNAc, N>H), or B (terminal HexNAc, N=H); as described in the present disclosure.

RESULTS

To identify protein-linked glycan signals correlating with malignant tumors in total tissue glycomes from cancer patient, major signals specific to either normal lung tissue or malignant small cell lung cancer tumors were selected based on their relative abundances. When *A* and *R* were calculated for the glycan profile datasets of the two samples, and the glycan signals thereafter sorted according to the values of *A* and *R*, the most significant differing glycan signals between the two samples could be identified (**Table 18**). Among the most abundant protein-linked glycan signals in the data, the following three signals had emerged in II (new in Table 18): 1955, 2685, and 2905, corresponding to fucosylated complex-type N-glycans. The absolute differences of these signals were among the ten most large in the data, indicating that they were significant. The signals that experienced the highest relative increase in *R* were: 771 ($R = 2.4$, corresponding to 3.4-fold increase), 1905 ($R = 2.2$, corresponding to 3.2 fold increase), and 1485 ($R = 1.3$, corresponding to 2.3 fold increase). The latter signal corresponded to complex-type N-glycans with terminal HexNAc. Significantly, its +2Hex counterpart 1809 was the most drastically reduced glycan signal in II with A = -8.9 and R =-0.4 (corresponding to 40% decrease in II), indicating a large change in terminal HexNAc expression. Moreover, the data easily shows that the glycan signals 1704, 1866, 1136, and 755 were not present in II.

Further, the obtained results, especially the identified major glycan signals indicative of either Sample II (high A and R) or Sample I (low A and R) were used to compile two alternative algorithms to produce glycan score with which lung cancer sample could be identified from normal lung sample based on the glycan signal values of the quantitative glycome data:

1. glycan score = I(1485) - I(1809),
wherein I(1485) is the relative abundance of glycan signal 1485 and 1(1809) is the relative abundance of glycan signal 1809;
and alternatively:
2. glycan score = 1(1485) / 1(1809)

These glycan score algorithms yield high numerical value when applied to lung cancer sample and low numerical value when applied to normal lung sample.

DISCUSSION

The present identification analysis produced selected glycan signal groups, from where indifferent glycan signals

have been removed and that have reduced noise or background and less observation points, but have the resolving power of the initially obtained glycan profiles. Such selected signal groups and their patterns in different sample types can serve as a signature for the identification of for example **1)** normal human glycosylation, **2)** tissue-specific glycosylation, **3)** disease states affecting tissue glycosylation, **4)** malignant cancer, **5)** malignancy in comparison to benign tumors, and grade of malignancy, or **6)** glycan signals that have individual variation. Moreover, glycan signals can be identified that do not change between samples, including major glycans that can be considered as invariant or housekeeping glycans.

The present data analysis identified potential glycan marker signals for future identification of either the normal lung of the lung tumor glycome profiles. Further, glycan classes that are associated with e.g. disease state in humans can be identified. Specifically, the analysis revealed that within the total complex-type N-glycan structure class in the tissue glycomes, terminal HexNAc (N>H) were typical to small cell lung cancer.

The method also allows identification of major glycans or major changes within glycan structure classes. For example, the proportion of multifucosylated glycans within the total tissue glycome profile was increased in II (1.1%) compared to I (0.3%). The data analysis identified this change predominantly to the appearance of glycan signals 1955 and 2685 in II.

**EXAMPLE 18. Periodate oxidation analysis of N-glycan structures**

EXPERIMENTAL PROCEDURES

Cancer cell derived N-glycans were obtained by N-glycosidase F digestion and purified as described in the preceding Examples. The glycan sample was dissolved in 10 μl of 8 mM sodium metaperiodate prepared in 0.1 M sodium acetate buffer, pH 5.5. The oxidation reaction was allowed to proceed at +4 °C in the dark for two days. The excess of periodate was then destroyed by adding 10 μl of 80 mM ethylene glycol and the mixture was allowed to stand for 6 hours. The reaction mixture was then neutralized by adding 10 μl of 0.1 M aqueous ammonia. The aldehyde groups which were generated in the reaction mixture were then reduced by adding 10 μl of 1.6 M sodium borohydride and allowed to stand overnight at +4 °C.

The carbohydrate material in the reaction mixture was isolated by solid phase extraction on a small column of graphitized carbon and subjected to MALDI-TOF mass spectrometry in 2,5-dihydroxybenzoic acid matrix.

RESULTS

Some of the major signals observed in the spectrum (**Figure 33**) were assigned as follows:

The signal m/z 1831 represents an oxidized-reduced form of $(Man)_9(GlcNAc)_2$ species ($[M+Na]^+$, m/z 1905), containing three terminal Man units. Because periodate oxidizes vicinal hydroxyl groups, the terminal units are oxidized so that they lose the C-3 as formaldehyde, each contributing to a loss of -28 Da in mass. In addition, 2-substituted Man units may get oxidized between C-3 and C-4, yielding two primary alcohol groups (leading to +2 Da mass change each). Typical mammalian $(Man)_9(GlcNAc)_2$ species carry four Manα1,2 residues, and here four 2-substituted Man units were oxidized this way and contribute to a mass increment of +8 Da. The reducing end of the glycan is also reduced, yielding +2 Da increment. No unoxidized $(Man)_9(GlcNAc)_2$ species can be observed in the spectrum.

The signal m/z 1831 is accompanied by a signal at m/z 1861. This signal is assigned as an oxidized-reduced structure where one of the terminal Man units is oxidized only between C2-C3 or C3-C4, but not liberating formaldehyde, thus yielding a structure 30 Da larger than m/z 1831 material.

The signal m/z 1667 represents an oxidized-reduced form of $(Man)_8(GlcNAc)_2$ species ($[M+Na]^+$, m/z 1743), containing three terminal Man units. These terminal units are oxidized so, that they lose the C-3 as formaldehyde, each contributing to a loss of -28 Da. In addition, all three 2-substituted Man units got oxidized between C-3 and C-4, yielding two primary alcohol groups (+2 Da). The m/z 1697 signal is assigned as an oxidized-reduced structure where one of the terminal Man units is oxidized only between C2-C3 or C3-C4, but not liberating formaldehyde, thus yielding a structure 30 Da larger than m/z 1667 material.

Other major signals are derived similarly from the following structures:

m/z 1503 and 1533 from $(Man)_7(GlcNAc)_2$ species (intact m/z 1581);
m/z 1339 and 1369 from $(Man)_6(GlcNAc)_2$ species (intact m/z 1419);
m/z 1175 and 1205 from $(Man)_5(GlcNAc)_2$ species (intact m/z 1257);
m/z 1041 and 1071 from $(Man)_4(GlcNAc)_2$ species (intact m/z 1095).

A part of the mass spectrum shown in **Figure 33** is zoomed in **Figure 34,** showing the mass range of m/z 800-950. A major signal m/z 835 originates from an oxidized-reduced form of m/z 917, a $(Man)_2(Fuc)(GlcNAc)_2$ species.

Here, three formaldehyde units are lost, so the structure is preferably Manα1-6Manβ1-4GlcNAcβ1-4(Fucα-6)GlcNAc. The rationale is that both terminal Man and Fuc are oxidized to release formaldehyde, but also the penultimate 6-substituted Man is oxidized to release formaldehyde. As above, there is a signal +30 Da from m/z 835, i.e. m/z 865. In this species, one of the units mentioned is not completely oxidized to release formaldehyde, but only oxidized between C2-C3 or C3-C4, and reduced to alcohol groups.

The m/z 879 signal is derived from an oxidized-reduced form of m/z 933, a $(Man)_3(GlcNAc)_2$ species. Here, two terminal unsubstituted Man units are oxidized to release formaldehyde, other monosaccharide units are stable to periodate oxidation. Preferred structure corresponding to the original abundant signal is Manα3(Manα6)Manβ4GlcNAcα4GlcNAc.

**EXAMPLE 19.** Human antibody molecules against type II N-acetyllactosamine on Core 2 O-glycan.

EXPERIMENTAL PROCEDURES

Affinity reagent for analyzing antibody molecules recognizing type II N-acetyllactosamine on Core 2 O-glycans was prepared by coupling Galβ4GlcNAcβ6(Galβ3)GalNAcα-O-$(CH_2)_2$-(p-amino)benzyl (IsoSep, Sweden) to N-hydroxysuccinimide activated Sepharose (Amersham Pharmacia, Sweden).

Serum samples were isolated from a person that had had malignant ovarian cancer and recovered from it, as well as control individuals.

Ig antibodies were analyzed by affinity isolating them from human sera, washing, eluting with acid, and detecting Ig subunits by protein detection after SDS-PAGE according to standard procedures.

RESULTS

Ovarian cancer patient samples showed substantial amounts of human Ig antibodies with affinity to the type II N-acetyllactosamine Core 2 O-glycan affinity material, and proteins corresponding to Ig subunits could be detected in SDS-PAGE.

**EXAMPLE 20.** Analysis of large cancer patient sample panel with respect to expression of cancer-associated glycan antigens.

EXPERIMENTAL PROCEDURES

Protein-linked glycans were isolated from formalin-fixed and paraffin-embededed tissue sections and analyzed as describe in the preceding Examples. Following cancer types were analyzed: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer / carcinoma, and lymph node metastases thereof, liver cancer / carcinoma; larynx cancer / carcinoma; pancreas cancer / carcinoma; melanoma and liver metastases thereof; gall bladder cancer / carcinoma, and liver metastases thereof; salivary gland cancer / carcinoma, and skin metastases thereof; and lymph node cancer / carcinoma (lymphoma).

RESULTS

**Low-mannose type N-glycans** were identified based on their indicative mass spectrometric glycan signals for $[M+Na]^+$ ions of $Hex_{1-4}HexNAc_2dHex_{0-1}$, as described in to the present disclosure. In the present analyses the preferred indicative signals of the glycan structure group were m/z 771, 917, 933, 1079, and 1095 due to their abundance in mass spectrometric glycan profiles; 771, 933, and 1095 especially in case of non-fucosylated low-mannose type N-glycans; 917 and 1079 especially in case of detecting fucosylated low-mannose type N-glycans; 933, 1079, and 1095 especially in case of detecting low-mannose type N-glycans when N- and O-glycans were analyzed simultaneously; 1079 and 1095 as preferred sensitive indicators of the group; and/or 1079 as a preferred sensitive single indicator of the group.

Using these criteria, low-mannose type N-glycans were detected to be cancer-associated and expressed in malignant tumors in following cancer types: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer / carcinoma, and lymph node metastases thereof, liver cancer / carcinoma; larynx cancer / carcinoma; pancreas cancer / carcinoma; melanoma and liver metastases thereof; gall bladder cancer / carcinoma, and liver metastases thereof; and lymph node cancer / carcinoma (lymphoma). Further the expression of the glycans was detected in abundant amounts in salivary gland cancer / carcinoma, and skin metastases thereof.

In all these cancer types, both fucosylated and non-fucosylated low-mannose type N-glycans were detected and expression levels were found to be higher in cancer in comparison to normal human tissue samples. In addition, in benign tumors of the ovary and colon, low-mannose type glycan signals were significantly lower than in the corresponding malignant tumors, indicating that low-mannose type glycans are specifically associated with malignancy in human cancers.

**O-glycans** were identified based on their major indicative mass spectrometric glycan signals 771, 917, 899, 1038, and 1329 corresponding to $SA_{0-2}Hex_2HexNAc_2dHex_{0-1}$ glycan compositions, as described in to the present disclosure. In the present analyses the preferred indicative signals of the glycan structure group were m/z 771, 917, and 899 due to their abundance in mass spectrometric glycan profiles; 771 especially in case of non-fucosylated neutral glycans; 917 especially in case of detecting fucosylated neutral glycans; and 899 especially in case of detecting sialylated glycans; 771 and 899 as preferred sensitive indicators of the group; and/or 771 as a preferred sensitive single indicator of the group.

Using these criteria, O-glycans were detected to be cancer-associated and expressed in malignant tumors in following cancer types: lung cancer / carcinoma, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, and carcinoma adenomatosum; kidney cancer / carcinoma, and kidney hypernephroma; gastric cancer / carcinoma; larynx cancer / carcinoma; and pancreas cancer / carcinoma; melanoma and liver metastases thereof; gall bladder cancer / carcinoma, and liver metastases thereof. Further the expression of the glycans was detected in abundant amounts in salivary gland cancer / carcinoma, and skin metastases thereof.

In all these cancer types, neutral O-glycans were the most abundant cancer-associated structures, but both fucosylated and sialylated O-glycans were in all analyses detected to be expressed simultaneously; in most cases also their levels were found to be higher in cancer in comparison to normal human tissue samples. In addition, in benign tumors of the ovary and colon, O-glycan signals were significantly lower than in the corresponding malignant tumors, indicating that O-glycans are specifically associated with malignancy in human cancers.

**Non-reducing terminal HexNAc** glycan expression was detected along with one or both of the above mentioned glycan groups in the following analyzed cancer types: lung cancer, both small cell lung adenocarcinoma and non-small cell lung adenocarcinoma, and lung carcinoma liver metastases; breast cancer; ductale type breast adenocarcinoma and lymph node metastases thereof; lobulare type breast adenocarcinoma and lymph node metastases thereof; ovarian cystadenocarcinoma; colon cancer / carcinoma, carcinoma adenomatosum, and liver metastases thereof; kidney cancer / carcinoma, and kidney hypernephroma; gastric cancer / carcinoma, and lymph node metastases thereof, liver cancer / carcinoma; larynx cancer / carcinoma; pancreas cancer / carcinoma; melanoma and liver metastases thereof; gall bladder cancer / carcinoma, and liver metastases thereof; and lymph node cancer / carcinoma (lymphoma). Further the expression of the glycans was detected in abundant amounts in salivary gland cancer / carcinoma, and skin metastases thereof. The detection was based on their major indicative mass spectrometric glycan signals 1485 and/or 1850 corresponding to $Hex_3HexNAc_4dHex_1$ and $Hex_4HexNAc_5dHex_1$ glycan compositions, respectively, as described in the present disclosure.

**EXAMPLE 21.** Neutral and acidic protein-linked tissue glycan profiles of ductale and lobulare type breast carcinomas.

Protein-linked glycans were isolated from formalin-fixed and paraffin-embededed tissue sections of ductale-type breast cancer and lymph node metastases derived therefrom, and analyzed as described in the preceding Examples.

The results are described as protein-linked glycan profiles of the primary breast cancer tumors and normal brease tissues, as well as metastases and normal lymph node tissues from the same patients in Figures **29, 30, 31, and 32,** respectively. The profiles indicate that major cancer- and metastasis-associated glycan signals and signal groups include low-mannose type glycans and O-glycas, based on the indicative signals for each glycan signal group, respectively.

Further, the acidic glycan profiles were quantitatively analyzed by composition classification into glycan structural features, as described in **Table 20.** Importantly, the classification analysis revealed that especially sulphation and/or phosphorylation and complex fucosylation of acidic glycans were associated with the present tumors as well as the corresponding lymph node metastases in comparison with either of the primary and the secondary normal tissues. Major glycan signals expressing these features were $NeuAc_1Hex_2HexNAc_2SP_1$ and $NeuAc_1Hex_3HexNAc_3SP_2$ wherein SP is either sulphate or phosphate ester, and $NeuAc_1Hex_5HexNAc_4dHex_2$ and $NeuAc_1Hex_5HexNAc_4dHex_3$, respectively.

**EXAMPLE 22.** Metastasis-associated glycans in malignancy and site-specific metastasis to the liver

EXPERIMENTAL PROCEDURES

A series of malignant primary tumors of the lung, colon, skin, and gall bladder, and corresponding liver metastases were analyzed in order to identify glycans associated with malignancy and metastasis formation, especially liver metastases. Protein-linked glycans were isolated from formalin-fixed and paraffin-embedded tissue sections or protein fractions of tumor tissues and analyzed by MALDI-TOF mass spectrometry as described in the preceding Examples.

By comparing the quantitative expression of indicative glycan signals of 1) liver metastases and normal liver tissue, and 2) malignant primary tumor and corresponding normal tissue, malignancy and metastasis-associated glycan signals and glycan structure groups were identified as described in the preceding Examples.

RESULTS AND DISCUSSION

Glycan groups that were associated with liver metastases were 1) low-mannose type glycans, 2) non-reducing terminal HexNAc glycans, especially non-reducing terminal GlcNAc glycans, and 3) neutral and acidic O-glycans, especially neutral and sialylated O-glycans. Preferential glycan signals associated with malignant liver metastases included 933, 1079, and 1095 (1); 1485 (2); and 771, 917, 899, 1038, and 1329 (3), especially 771 and 899 (3); wherein the numbering (1-3) refers to the identified metastasis-associated glycan groups. The glycans were present in the primary tumor in elevated amounts in comparison to corresponding normal tissue, and significantly, further elevated in comparison to normal liver tissue i.e. enriched in the liver metastases. The results indicate that the present glycans identified in metastases are associated with metastasis formation, more specifically liver metastasis formation. Specifically, the present results indicate that low-mannose type, non-reducing terminal GlcNAc, and O-glycans are associated with malignant metastasis formation, more specifically liver metastasis formation; most specifically, low-mannose type and terminal GlcNAc glycans are associated with liver metastases.

**EXAMPLE 23.** Metastasis-associated glycans in malignancy and site-specific metastasis to lymph nodes.

EXPERIMENTAL PROCEDURES

A series of malignant primary tumors of the stomach and breast, and corresponding lymph node metastases were analyzed in order to identify glycans associated with malignancy and metastasis formation, especially lymph node metastases. Protein-linked glycans were isolated from formalin-fixed and paraffin-embedded tissue sections or protein fractions of tumor tissues and analyzed by MALDI-TOF mass spectrometry as described in the preceding Examples.

By comparing the quantitative expression of indicative glycan signals of 1) lymph node metastases and normal lymph node tissue, and 2) malignant primary tumor and corresponding normal tissue, malignancy and metastasis-associated glycan signals and glycan structure groups were identified as described in the preceding Examples.

RESULTS AND DISCUSSION

Glycan groups that were associated with lymph node metastases were 1) low-mannose type glycans and 2) neutral and acidic O-glycans, especially neutral and sialylated O-glycans. Preferential glycan signals associated with malignant liver metastases included 933, 1079, and 1095 (1); and 771, 917, 899, 1038, and 1329 (2), especially 771 and 899 (2); wherein the numbering (1-2) refers to the identified metastasis-associated glycan groups. The glycans were present in the primary tumor in elevated amounts in comparison to corresponding normal tissue, and significantly, further elevated in comparison to normal liver tissue i.e. enriched in the lymph node metastases. The results indicate that the present glycans identified in metastases are associated with metastasis formation, more specifically lymph node metastasis formation. Specifically, the present results indicate that low-mannose type and O-glycans are associated with malignant metastasis formation, more specifically lymph node metastasis formation; most specifically, low-mannose type and sialylated O-glycans are associated with lymph node metastases.

A sample array from different cancer patients with gastric cancer revealed an interesting phenomenon: lymph node metastases of primary gastric cancers transformed the lymph node glycan profiles with glycan signals originating from primary tissue specific glycosylation. For example, in lymph node metastases small O-glycan type signals with either blood group O, A, or B characteristics were detected. These signals in the primary tissue location included in both O, A, and B patients: 1063 (Hex2HexNAc2dHex2) i.e. increased amounts of dHex in glycans with 3 or less HexNAc residues and more clearly in glycans with 2 or less HexNAc residues; specifically in A patients: 1120 (Hex2HexNAc3dHex1) and 1266 (Hex2HexNAc3dHex2) i.e. increased amounts of HexNAc and dHex in glycans with 3 or less Hex residues and more clearly in glycans with 2 or less Hex residues; and specifically in B patients: 714

(Hex2HexNAcldHexl) and/or 1225 (Hex3HexNAc2dHex2) i.e. increased amounts of Hex and dHex in glycans with 3 or less HexNAc residues and more clearly in glycans with 2 or less HexNAc residues. The present results demonstrated that the origin of the primary tumor is reflected in the glycan profile of the metastasis, and that the metastatic cancer cells carry with them abnormal glycan antigens to the site of the metastasis.

**Table 1.** Overexpression data of low-mannose type N-glycans in studied cancer types.

| | m/z | | | | | |
|---|---|---|---|---|---|---|
| **Cancer type** | **917** | **933** | **1079** | **1095** | **1241** | **1403** |
| Non-small cell lung adenocarcinoma | + | - | + | - | + | + |
| Ductale breast adenocarcinoma | + | + | + | + | + | + |
| Lobulare breast adenocarcinoma | + | + | + | - | + | + |
| Ovarian cystadenocarcinoma | + | + | + | - | + | + |
| Colon carcinoma | + | - | + | - | + | - |
| Kidney cancer | - | + | - | - | - | - |
| Gastric cancer | - | + | + | + | - | - |
| Liver cancer | - | + | - | - | - | - |
| Larynx cancer | + | + | + | - | - | - |
| Pancreas cancer | - | + | + | + | - | - |

*CODE:*

| | |
|---|---|
| + | overexpressed in cancer |
| - | no overexpression detected |
| m/z 917 | $Hex_2HexNAc_2dHex_1$ |
| m/z 933 | $Hex_3HexNAc_2$ |
| m/z 1079 | $Hex_3HexNAc_2dHex_1$ |
| m/z 1095 | $Hex_4HexNAc_2$ |
| m/z 1241 | $Hex_4HexNAc_2dHex_1$ |
| m/z 1403 | $Hex_5HexNAc_2dHex_1$ |

**Table 2.** Statistical analysis of low-mannose type N-glycans in breast cancer.

| **DUCTALE BREAST ADENOCARCINOMA** | | | |
|---|---|---|---|
| 9 sample pairs | | | |
| **Approx. m/z** | **Composition** | **P** | **Test** |
| 917 | $Hex_2HexNAc_2dHex_1$ | 0.0039 | Signed Rank |
| 933 | $Hex_3HexNAc_2$ | 0.0031 | Student's t |
| 1079 | $Hex_3HexNAc_2dHex_1$ | 0.0039 | Signed Rank |
| 1241 | $Hex_4HexNAc_2dHex_1$ | 0.0071 | Student's t |

**Table 3.** Overexpression data of neutral O-glycans in studied cancer types.

| | **m/z** | |
|---|---|---|
| **Cancer type** | **771** | **917** |
| Non-small cell lung adenocarcinoma | + | + |

(continued)

| | m/z | |
|---|---|---|
| **Cancer type** | **771** | **917** |
| Ductale breast adenocarcinoma | + | + |
| Lobulare breast adenocarcinoma | + | + |
| Ovarian cystadenocarcinoma | + | + |
| Colon carcinoma | - | + |
| Kidney cancer | - | - |
| Gastric cancer | + | - |
| Liver cancer | - | - |
| Larynx cancer | - | + |
| Pancreas cancer | + | - |

*CODE:*

+ overexpressed in cancer
- no overexpression detected
m/z 771 $Hex_2HexNAc_2$
m/z 917 $Hex_2HexNAc_2dHex_1$

**Table 4.** Statistical analysis of neutral O-glycan overexpression in lung cancer and in breast cancer.

| **DUCTALE BREAST ADENOCARCINOMA** | | | |
|---|---|---|---|
| 9 sample pairs | | | |
| **Approx. m/z** | **Composition** | **P** | **Test** |
| 771 | $Hex_2HexNAc_2$ | 0.0039 | Sign |
| 917 | $Hex_2HexNAc_2dHex_1$ | 0.0078 | Signed Rank |
| | | | |
| **NON-SMALL CELL LUNG ADENOCARCINOMA** | | | |
| 8 sample pairs | | | |
| **Approx. m/z** | **Composition** | **P** | **Test** |
| 771 | $Hex_2HexNAc_2$ | < 0.05 | Student's t |

**Table 5.** The indicative mass spectrometric signals of the glycans.

| A) O-glycan fragments, positive ion mode (isotopic masses). | | | | | |
|---|---|---|---|---|---|
| **monosaccharide composition** | **m/z [M+H]$^+$** | **m/z [M+Na]$^+$** | **m/z [M+K]$^+$** | **m/z [M-H+2Na]$^+$** | **m/z [M-H+Na+K]$^+$** |
| $NeuNAc_1Hex_1HexNAc_1$(deoxyamino) $HexNAc_1$ | 877.34 | 899.32 | 915.30 | 921.31 | 937.28 |
| $NeuNAc_1Hex_1HexNAc_1$(deoxyamino) $HexNAc_1dHex_1$ | 1023.40 | 1045.38 | 1061.36 | 1067.36 | 1083.34 |
| $NeuNAc_1Hex_2HexNAc_2$(deoxyamino) $HexNAc_1$ | | 1264.46 | | 1286.44 | |

(continued)

| A) O-glycan fragments, positive ion mode (isotopic masses). | | | | | |
|---|---|---|---|---|---|
| **monosaccharide composition** | **m/z $[M+H]^+$** | **m/z $[M+Na]^+$** | **m/z $[M+K]^+$** | **m/z $[M-H+2Na]^+$** | **m/z $[M-H+Na+K]^+$** |
| $NeuNAc_1Hex_2HexNAc_2$(deoxyamino) $HexNAc_1dHex_1$ | | 1410.51 | | 1432.50 | |
| $NeuNAc_1Hex_2HexNAc_2$(deoxyamino) $HexNAc_1dHex_2$ | | 1556.57 | | 1578.55 | |
| $NeuNAc_1Hex_3HexNAc_3$(deoxyamino) $HexNAc_1$ | | 1629.59 | | 1651.57 | |
| $NeuNAc_1Hex_3HexNAc_3$(deoxyamino) $HexNAc_1dHex_1$ | | 1775.65 | | 1797.63 | |
| B) O-glycan fragments, negative ion mode (isotopic and average masses) | | | | | |
| **monosaccharide composition** | **m/z $[M-H]^-$** | **average** | | | |
| $NeuNAc_1Hex_1HexNAc_1$(deoxyamino) $HexNAc_1$ | 875.33 | 875.80 | | | |
| $NeuNAc_1Hex_1HexNAc_1$(deoxyamino) $HexNAc_1dHex_1$ | 1021.38 | 1021.94 | | | |
| $NeuNAc_1Hex_2HexNAc_2$(deoxyamino) $HexNAc_1$ | 1240.46 | 1241.14 | | | |
| $NeuNAc_1Hex_2HexNAc_2$(deoxyamino) $HexNAc_1dHex_1$ | 1386.52 | 1387.28 | | | |
| $NeuNAc_1Hex_2HexNAc_2$(deoxyamino) $HexNAc_1dHex_2$ | 1532.57 | 1533.42 | | | |
| $NeuNAc_1Hex_3HexNAc_3$(deoxyamino) $HexNAc_1$ | 1605.59 | 1606.47 | | | |
| $NeuNAc_1Hex_3HexNAc_3$(deoxyamino) $HexNAc_1dHex_1$ | 1751.65 | 1752.61 | | | |
| $NeuNAc_1Hex_3HexNAc_3$(deoxyamino) $HexNAc_1dHex_1$ | 1897.71 | 1898.75 | | | |
| C) Oligosaccharides, negative ion mode (isotopic and average masses). | | | | | |
| **monosaccharide composition** | **m/z $[M-H]^-$** | **average** | | | |
| $NeuNAc_1Hex_2HexNAc_2$ | 1038.36 | 1038.93 | | | |
| $NeuNAc_1Hex_2HexNAc_2dHex_1$ | 1184.42 | 1185.07 | | | |
| $NeuNAc_2Hex_2HexNAc_2$ | 1329.46 | 1330.18 | | | |
| $NeuNAc_1Hex_3HexNAc_3$ | 1403.49 | 1404.26 | | | |
| $NeuNAc_2Hex_2HexNAc_2dHex_1$ | 1475.52 | 1476.32 | | | |
| $NeuNAc_1Hex_3HexNAc_3dHex_1$ | 1549.55 | 1550.40 | | | |
| $NeuNAc_2Hex_3HexNAc_3$ | 1694.59 | 1695.52 | | | |
| $NeuNAc_2Hex_3HexNAc_3dHex_1$ | 1840.65 | 1841.66 | | | |

**Table 6.** Overexpression data of sialylated Core 2 type O-glycans in studied cancer types.

| | m/z |
|---|---|
| **Cancer type** | **899** |
| Non-small cell lung adenocarcinoma | + |
| Ductale breast adenocarcinoma | + |
| Lobulare breast adenocarcinoma | + |
| Ovarian cystadenocarcinoma | + |
| Colon carcinoma | + |
| Kidney cancer | + |
| Gastric cancer | - |
| Liver cancer | - |
| Larynx cancer | - |
| Pancreas cancer | + |

*CODE:*

| | |
|---|---|
| + | overexpressed in cancer |
| - | no overexpression detected |
| m/z 771: | $Hex_2HexNAc_2$ |
| m/z 917: | $Hex_2HexNAc_2dHex_1$ |

**Table 7.** Statistical analysis of sialylated Core 2 type O-glycan overexpression in lung cancer and in two types of breast cancer.

| | | | |
|---|---|---|---|
| **DUCTALE BREAST ADENOCARCINOMA** | | | |
| 9 sample pairs | | | |
| **Approx. m/z** | **Composition** | **P** | **Test** |
| 899 | $NeuAc_1Hex_1HexNAc_1$(deoxyamino) $HexNAc_1$ | 0.0078 | Sign |
| | | | |
| **LOBULARE BREAST ADENOCARCINOMA** | | | |
| 6 sample pairs | | | |
| **Approx. m/z** | **Composition** | **P** | **Test** |
| 899 | $NeuAc_1Hex_1HexNAc_1$(deoxyamino) $HexNAc_1$ | 0.0313 | Signed Rank |
| | | | |
| **NON-SMALL CELL LUNG ADENOCARCINOMA** | | | |
| 8 sample pairs | | | |
| **Approx. m/z** | **Composition** | **P** | **Test** |
| 899 | $NeuAc_1Hex_1HexNAc_1$(deoxyamino) $HexNAc_1$ | < 0.05 | Student's t |

**Table 8.** Proposed monosaccharide compositions for MALDI-TOF mass spectrometric profiling of sialylated protein-linked glycans isolated from **A.** healthy ovarian tissue, **B.** benign ovarian cystadenoma, and **C.** malignant ovarian cystadenocarcinoma in **Figure y1**. Experimental masses (exp. m/z) refer to spectrum **B.**; SO3, sulfate/phosphate.

| No. | calc. m/z | exp. m/z | proposed composition |
|---|---|---|---|
| 1 | 1550.40 | 1550.36 | NeuAc1Hex3HexNAc3dHex1 |
| 2 | 1558.40 | 1558.37 | Hex4HexNAc4SO3 |
| 3 | 1566.40 | 1566.93 | NeuAc1Hex4HexNAc3 |
| 4 | | 1588.17 | unknown |
| 5 | | 1617.27 | unknown |
| 6 | | 1654.70 | unknown |
| 7 | 1712.54 | 1712.13 | NeuAc1Hex4HexNAc3dHex1 |
| 8 | | 1719.82 | unknown |
| 9 | 1728.54 | 1728.33 | NeuAc1Hex5HexNAc3 |
| 10 | 1753.60 | 1753.61 | NeuAc1Hex3HexNAc4dHex1 |
| 11 | 1769.60 | 1769.86 | NeuAc1Hex4HexNAc4 |
| 12 | 1785.59 | 1785.98 | NeuGc1Hex4HexNAc4 |
| 13 | 1816.61 | 1816.45 | NeuAc2Hex5HexNAc2 |
| 14 | 1857.66 | 1857.37 | NeuAc2Hex4HexNAc3 |
| 15 | 1874.68 | 1874.51 | NeuAc1Hex5HexNAc3dHex1 |
| 16 | 1882.69 | 1882.09 | Hex6HexNAc4SO3 |
| 17 | 1915.74 | 1915.68 | NeuAc1Hex4HexNAc4dHex1 |
| 18 | 1931.74 | 1931.73 | NeuAc1Hex5HexNAc4 |
| 19 | 1947.74 | 1947.80 | NeuGc1Hex5HexNAc4 |
| 20 | | 1978.32 | unknown |
| 21 | | 1988.68 | unknown |
| 22 | 2020.83 | 2020.05 | NeuAc1Hex5HexNAc3dHex2 |
| 23 | 2044.85 | 2044.17 | NeuAc2Hex3HexNAc4dHex1 |
| 24 | 2077.88 | 2077.81 | NeuAc1Hex5HexNAc4dHex1 |
| 25 | | 2110.12 | unknown |
| 26 | 2118.93 | 2118.81 | NeuAc1Hex4HexNAc5dHex1 |
| 27 | 2159.98 | 2159.72 | NeuAc1Hex3HexNAc6dHex1 |
| 28 | | 2198.95 | unknown |
| 29 | 2223.00 | 2223.03 | NeuAc2Hex5HexNAc4 |
| 30 | 2281.07 | 2281.08 | NeuAc1Hex5HexNAc5dHex1 |
| 31 | | 2308.09 | unknown |
| 32 | 2328.08 | 2326.60 | NeuAc2Hex3HexNAc6dHex1 |
| 33 | 2352.10 | 2350.72 | NeuAc3Hex45HexNAc4 |
| 34 | 2369.13 | 2369.21 | NeuAc2Hex5HexNAc4dHex1 |
| 35 | 2402.19 | 2401.80 | NeuAc1Hex4HexNAc6dHex1 SO3 |
| 36 | 2410.18 | 2409.32 | NeuAc1Hex4HexNAc5dHex3 |

(continued)

| No. | calc. m/z | exp. m/z | proposed composition |
|---|---|---|---|
| 37 | 2443.21 | 2443.01 | NeuAc1Hex5HexNAc6dHex1 |
| 38 | 2451.24 | 2449.46 | NeuAc2Hex3HexNAc6dHex1 |
| 39 | | 2547.32 | unknown |
| 40 | 2572.32 | 2572.25 | NeuAc2Hex5HexNAc5dHex1 |
| 41 | 2588.32 | 2588.01 | NeuAc2Hex6HexNAc5 |
| 42 | 2734.46 | 2734.46 | NeuAc2Hex6HexNAc5dHex1 |
| 43 | 2775.52 | 2773.43 | NeuAc2Hex5HexNAc6dHex1 |
| 44 | 2808.54 | 2808.40 | NeuAc1Hex7HexNAc6dHex1 |
| 45 | 2816.57 | 2814.30 | NeuAc2Hex4HexNAc7dHex1 |
| 46 | 2879.58 | 2879.54 | NeuAc3Hex6HexNAc5 |
| 47 | 3025.72 | 3025.72 | NeuAc3Hex6HexNAc5dHex1 |
| 48 | 3107.82 | 3104.91 | NeuAc3Hex4HexNAc7dHex1 |
| 49 | 3244.91 | 3245.37 | NeuAc3Hex7HexNAc6 |
| 50 | 3391.05 | 3390.79 | NeuAc3Hex7HexNAc6dHex1 |
| 51 | 3536.17 | 3536.17 | NeuAc4Hex7HexNAc6 |
| 52 | 3682.31 | 3682.59 | NeuAc4Hex7HexNAc6dHex1 |

**Table 9.** Preferred neutral glycan compositions. Calculated mass-to-charge ratios (calc. m/z) refer to the first isotope signal of $[M+Na]^+$ ion.

| Proposed composition | calc. m/z |
|---|---|
| HexHexNAc | 406,13 |
| Hex3 | 527,16 |
| HexHexNAcdHex | 552,19 |
| Hex2HexNAc | 568,19 |
| HexHexNAc2 | 609,21 |
| Hex4 | 689,21 |
| Hex2HexNAcdHex | 714,24 |
| Hex3HexNAc | 730,24 |
| HexHexNAc2dHex | 755,27 |
| Hex2HexNAc2 | 771,26 |
| HexHexNAc3 | 812,29 |
| Hex5 | 851,26 |
| Hex2HexNAcdHex2 | 860,30 |
| Hex4HexNAc | 892,29 |
| HexHexNAc2dHex2 | 901,33 |
| Hex2HexNAc2dHex | 917,32 |
| Hex3HexNAc2 | 933,32 |
| HexHexNAc3dHex | 958,35 |
| Hex2HexNAc3 | 974,34 |
| Hex2HexNAcdHex3 | 1006,36 |
| Hex6 | 1013,32 |
| HexHexNAc4 | 1015,37 |
| Hex3HexNAcdHex2 | 1022,35 |
| Hex5HexNAc | 1054,34 |
| Hex2HexNAc2dHex2 | 1063,38 |
| Hex3HexNAc2dHex | 1079,38 |
| Hex4HexNAc2 | 1095,37 |
| HexHexNAc3dHex2 | 1104,41 |
| Hex2HexNAc3dHex | 1120,40 |
| Hex3HexNAc3 | 1136,40 |
| Hex2HexNAcdHex4 | 1152,42 |
| HexHexNAc4dHex | 1161,43 |
| Hex7 | 1175,37 |
| Hex2HexNAc4 | 1177,42 |
| Hex2HexNAc2dHex3 | 1209,44 |
| Hex6HexNAc | 1216,40 |
| HexHexNAc5 | 1218,45 |
| Hex3HexNAc2dHex2 | 1225,43 |
| Hex4HexNAc2dHex | 1241,43 |
| Hex5HexNAc2 | 1257,42 |
| Hex2HexNAc3dHex2 | 1266,46 |
| Hex3HexNAc3dHex | 1282,45 |
| Hex4HexNAc3 | 1298,45 |
| HexHexNAc4dHex2 | 1307,49 |
| Hex2HexNAc4dHex | 1323,48 |
| Hex8 | 1337,42 |
| Hex3HexNAc4 | 1339,48 |
| Hex2HexNAc2dHex4 | 1355,50 |
| HexHexNAc5dHex | 1364,51 |
| Hex3HexNAc2dHex3 | 1371,49 |
| Hex7HexNAc | 1378,45 |
| Hex4HexNAc2dHex2 | 1387,49 |
| Hex2HexNAc5 | 1380,50 |
| Hex5NexNAc2dHex | 1403,48 |
| Hex2HexNAc3dHex3 | 1412,52 |
| Hex6HexNAc2 | 1419,48 |
| HexHexNAc6 | 1421,53 |
| Hex3HexNAc3dHex2 | 1428,51 |
| Hex4HexNAc3dHex | 1444,51 |
| HexHexNAc4dHex3 | 1453,54 |
| Hex5HexNAc3 | 1460,50 |
| Hex2HexNAc4dHex2 | 1469,54 |
| Hex3HexNAc4dHex | 1485,53 |
| Hex9 | 1499,48 |
| Hex4HexNAc4 | 1501,53 |
| HexHexNAc5dHex2 | 1510,57 |
| Hex3HexNAc2dHex4 | 1517,55 |
| Hex2HexNAc5dHex | 1526,56 |
| Hex4HexNAc2dHex3 | 1533,54 |
| Hex8HexNAc | 1540,50 |
| Hex3HexNAc5 | 1542,56 |
| Hex5HexNAc2dHex2 | 1549,54 |
| Hex6HexNAc2dHex | 1565,53 |
| Hex3HexNAc3dHex3 | 1574,57 |
| Hex7HexNAc2 | 1581,53 |
| Hex2HexNAc6 | 1583,58 |
| Hex4HexNAc3dHex2 | 1590,57 |
| Hex5HexNAc3dHex | 1606,56 |
| Hex2HexNAc4dHex3 | 1615,60 |
| Hex6HexNAc3 | 1622,56 |
| Hex3HexNAc4dHex2 | 1631,59 |
| Hex4HexNAc4dHex | 1647,59 |
| Hex10 | 1661,53 |
| Hex5HexNAc4 | 1663,58 |
| Hex2HexNAc5dHex2 | 1672,62 |
| Hex3HexNAc5dHex | 1688,61 |
| Hex5HexNAc2dHex3 | 1695,60 |
| Hex9HexNAc | 1702,56 |
| Hex4HexNAx5 | 1704,61 |
| Hex6HexNAc2dHex2 | 1711,59 |
| Hex3HexNAc3dHex4 | 1720,63 |
| Hex7HexNAc2dHex | 1727,59 |
| Hex2HexNAc6dHex | 1729,64 |
| Hex4HexNAc3dHex3 | 1736,62 |
| Hex8HexNAc2 | 1743,58 |
| Hex3HexNAc6 | 1745,64 |
| Hex5HexNAc3dHex2 | 1752,62 |

| | |
|---|---|
| Hex6HexNAc3dHex | 1768,61 |
| Hex3HexNAc4dHex3 | 1777,65 |
| Hex7HexNAc3 | 1784,61 |
| Hex4HexNAc4dHex2 | 1793,64 |
| Hex5HexNAc4dHex | 1809,64 |
| Hex2HexNAc5dHex3 | 1818,68 |
| Hex11 | 1823,58 |
| Hex6HexNAc4 | 1825,63 |
| Hex3HexNAc5dHex2 | 1834,67 |
| Hex4HexNAc5dHex | 1850,67 |
| Hex6HexNAc2dHex3 | 1857,65 |
| Hex10HexNAc | 1864,61 |
| Hex5HexNAc5 | 1866,66 |
| Hex7HexNAc2dHex2 | 1873,64 |
| Hex2HexNAc6dHex2 | 1875,70 |
| Hex4HexNAc3dHex4 | 1882,68 |
| Hex8HexNAc2dHex | 1889,64 |
| Hex3HexNAc6dHex | 1891,69 |
| Hex5HexNAc3dHex3 | 1898,68 |
| Hex9HexNAc2 | 1905,63 |
| Hex4HexNAc6 | 1907,69 |
| Hex6HexNAc3dHex2 | 1914,67 |
| Hex3HexNAc4dHex4 | 1923,71 |
| Hex7HexNAc3dHex | 1930,67 |
| Hex2HexNAc7dHex | 1932,72 |
| Hex4HexNAc4dHex3 | 1939,70 |
| Hex8HexNAc3 | 1946,66 |
| Hex5HexNAc4dHex2 | 1955,70 |
| Hex6HexNAc4dHex | 1971,69 |
| Hex3HexNAc5dHex3 | 1980,73 |
| Hex12 | 1985,63 |
| Hex7HexNAc4 | 1987,69 |
| Hex4HexNAc5dHex2 | 1996,72 |
| Hex5HexNAc5dHex | 2012,72 |
| Hex7HexNAc2dHex3 | 2019,70 |
| Hex2HexNAc6dHex3 | 2021,76 |
| Hex11HexNAc | 2026,66 |
| Hex6HexNAc5 | 2028,71 |
| Hex8HexNAc2dHex2 | 2035,70 |
| Hex3HexNAc6dHex2 | 2037,75 |
| Hex5HexNAc3dHex4 | 2044,73 |
| Hex4HexNAc6dHex | 2053,75 |
| Hex6HexNAc3dHex3 | 2060,73 |
| Hex10HexNAc2 | 2067,69 |
| Hex5HexNAc6 | 2069,74 |
| Hex7HexNAc3dHex2 | 2076,72 |
| Hex2HexNAc7dHex2 | 2078,78 |
| Hex4HexNAc4dHex4 | 2085,76 |
| Hex8HexNAc3dHex | 2092,72 |
| Hex3HexNAc7dHex | 2094,77 |
| Hex5HexNAc4dHex3 | 2101,76 |
| Hex9HexNAc3 | 2108,71 |

| | |
|---|---|
| Hex4HexNAc7 | 2110,77 |
| Hex6HexNAc4dHex2 | 2117,75 |
| Hex3HexNAc5dHex4 | 2126,79 |
| Hex7HexNAc4dHex | 2133,75 |
| Hex4HexNAc5dHex3 | 2142,78 |
| Hex13 | 2147,69 |
| Hex8HexNAc4 | 2149,74 |
| Hex5HexNAc5dHex2 | 2158,78 |
| Hex6HexNAc5dHex | 2174,77 |
| Hex8HexNAc2dHex3 | 2181,76 |
| Hex3HexNAc6dHex3 | 2183,81 |
| Hex12HexNac | 2188,71 |
| Hex7HexNAc5 | 2190,77 |
| Hex4HexNAc6dHex2 | 2199,80 |
| Hex5HexNAc6dHex | 2215,80 |
| Hex7HexNAc3dHex3 | 2222,78 |
| Hex2HexNAc7dHex3 | 2224,84 |
| Hex11HexNAc2 | 2229,74 |
| Hex6HexNAc6 | 2231,79 |
| Hex8HexNAc3dHex2 | 2238,78 |
| Hex3HexNAc7dHex2 | 2240,83 |
| Hex5HexNAc4dHex4 | 2247,81 |
| Hex4HexNAc7dHex | 2256,83 |
| Hex6HexNAc4dHex3 | 2263,81 |
| Hex5HexNAc7 | 2272,82 |
| Hex7HexNAc4dHex2 | 2279,80 |
| Hex4HexNAc5dHex4 | 2288,84 |
| Hex5HexNAc5dHex3 | 2304,84 |
| Hex14 | 2309,74 |
| Hex9HexNAc4 | 2311,79 |
| Hex6HexNAc5dHex2 | 2320,83 |
| Hex7HexNAc5dHex | 2336,82 |
| Hex4HexNAc6dHex3 | 2345,86 |
| Hex8HexNAc5 | 2352,82 |
| Hex5HexNAc6dHex2 | 2361,86 |
| Hex6HexNAc6dHex | 2377,85 |
| Hex8HexNAc3dHex3 | 2384,83 |
| Hex3HexNAc7dHex3 | 2386,89 |
| Hex12HexNac2 | 2391,79 |
| Hex7HexNAc6 | 2393,85 |
| Hex4HexNAc7dHex2 | 2402,88 |
| Hex6HexNAc4dHex4 | 2409,87 |
| Hex5HexNAc7dHex | 2418,88 |
| Hex7HexNAc4dHex3 | 2425,86 |
| Hex6HexNAc7 | 2434,87 |
| Hex5HexNAc5dHex4 | 2450,89 |
| Hex6HexNAc5dHex3 | 2466,89 |
| Hex15 | 2471,79 |
| Hex7HexNAc5dHex2 | 2482,88 |
| Hex8HexNAc5dHex | 2498,88 |
| Hex5HexNAc6dHex3 | 2507,91 |
| Hex6HexNAc6dHex2 | 2523,91 |

| | |
|---|---|
| Hex7HexNAc6dHex | 2539,90 |
| Hex4HexNAc7dHex3 | 2548,94 |
| Hex13HexNAc2 | 2553,85 |
| Hex8HexNAc6 | 2555,90 |
| Hex5HexNAc7dHex2 | 2564,94 |
| Hex6HexNAc7dHex | 2580,93 |
| Hex6HexNAc5dHex4 | 2612,95 |
| Hex7HexNAc5dHex3 | 2628,94 |
| Hex16 | 2633,85 |
| Hex8HexNAc5dHex2 | 2644,94 |
| Hex6HexNAc6dHex3 | 2669,97 |
| Hex7HexNAc6dHex2 | 2685,96 |
| Hex5HexNAc7dHex3 | 2710,99 |
| Hex14HexNAc2 | 2715,90 |
| Hex6HexNAc7dHex2 | 2726,99 |
| Hex7HexNAc7dHex | 2742,98 |
| Hex8HexNAc7 | 2758,98 |
| Hex7Hexnac5dHex4 | 2775,00 |
| Hex8HexNAc5dHex3 | 2790,99 |
| Hex17 | 2795,90 |
| Hex7HexNAc6dHex3 | 2832,02 |
| Hex16HexNAc | 2836,92 |
| Hex9HexNAc6dHex | 2864,01 |
| Hex6HexNAc7dHex3 | 2873,05 |

| | |
|---|---|
| Hex15HexNAc2 | 2877,95 |
| Hex8HexNAc7dHex | 2905,04 |
| Hex8Hexnac5dHex4 | 2937,05 |
| Hex18 | 2957,95 |
| Hex7HexNAc6dHex4 | 2978,08 |
| Hex17HexNAc | 2998,98 |
| Hex8HexNAc7dHex2 | 3051,09 |
| Hex9HexNAc8 | 3124,11 |
| Hex8HexNAc6dHex4 | 3140,13 |
| Hex8HexNAc7dHex3 | 3197,15 |
| Hex9HexNAc8dHex / Hex7HexNAc6dHex6 | 3270,17 |
| Hex9HexNAc6dHex4 | 3302,18 |
| Hex8HexNAc7dHex4 | 3343,21 |
| Hex9HexNAc8dHex2 | 3416,23 |
| Hex10HexNAc6dHex4 | 3464,24 |
| Hex10HexNAc9 | 3489,24 |
| Hex9HexNAc8dHex3 | 3562,28 |
| Hex11HexNAc6dHex4 | 3626,29 |
| Hex10HexNAc9dHex | 3635,30 |
| Hex9HexNAc8dHex4 | 3708,34 |
| Hex10HexNAc9dHex2 / Hex8HexNAc7dHex7 | 3781,36 |
| Hex9HexNAc8dHex5 / Hex7HexNAc6dHex10 | 3854,40 |

**Table 10.** Preferred acidic glycan compositions. Calculated mass-to-charge ratios (calc. m/z) refer to the first isotope signal of $[M\text{–}H]^-$ ion.

| Proposed composition | calc. m/z |
|---|---|
| NeuAcHexHexNAc | 673,23 |
| NeuAcHexHexNAcdHex | 819,29 |
| NeuAcHex2HexNAc | 835,28 |
| NeuAcHexHexNAc2 | 876,31 |
| NeuAc2HexHexNAc | 964,33 |
| NeuAcHexHexNAcdHex2 | 965,35 |
| NeuAcHex2HexNAcdHex | 981,34 |
| Hex3HexNAc2SP | 989,28 |
| NeuAcHex3HexNAc | 997,34 |
| NeuAcHexHexNAc2dHex | 1022,37 |
| NeuAcHex2HexNAc2 | 1038,36 |
| NeuAcHexHexNAc3 | 1079,39 |
| NeuAc2HexHexNAcdHex | 1110,38 |
| NeuAc2Hex2HexNAc | 1126,38 |
| NeuAcHex2HexNAcdHex2 | 1127,40 |
| NeuAcHex3HexNAcdHex | 1143,39 |
| Hex4HexNAc2SP | 1151,33 |
| NeuAcHex4HexNAc | 1159,39 |
| NeuAc2HexHexNAc2 | 1167,41 |
| NeuAcHexHexNAc2dHex2 | 1168,43 |
| NeuAcHex2HexNAc2dHex | 1184,42 |
| Hex3HexNAc3SP | 1192,36 |
| NeuAcHex3HexNAc2 / NeuGcHex2HexNAc2dHex | 1200,42 |
| NeuGcHex3HexNAc2 | 1216,41 |
| NeuAcHexHexNAc3dHex | 1225,45 |
| NeuAcHex2HexNAc3 | 1241,44 |
| NeuAc2Hex2HexNAcdHex | 1272,44 |
| NeuAcHexHexNAc4 | 1282,47 |
| NeuAc2Hex3HexNAc | 1288,43 |
| NeuAcHex4HexNAcdHex | 1305,45 |
| NeuAc2HexHexNAc2dHex | 1313,46 |
| NeuAcHex5HexNAc / NeuAcHex2HexNAc3SP | 1321,44 / 1321,40 |
| NeuAc2Hex2HexNAc2 / NeuGcNeuAcHexHexNAc2dHex | 1329,46 |
| NeuAcHex2HexNAc2dHex2 | 1330,48 |
| Hex3HexNAc3dHexSP | 1338,41 |
| NeuAcHex3HexNAc2dHex | 1346,47 |
| Hex4HexNAc3SP | 1354,41 |
| NeuAcHex4HexNAc2 | 1362,47 |
| NeuAc2HexHexNAc3 | 1370,48 |
| NeuAcHex2HexNAc3dHex | 1387,50 |
| NeuAcHex3HexNAc3 | 1403,49 |
| NeuGcHex3HexNAc3 | 1419,49 |
| NeuAcHexHexNAc4dHex | 1428,53 |
| NeuAc2Hex3HexNAcdHex | 1434,49 |
| NeuAcHex2HexNAc4 | 1444,52 |
| NeuAcHex3HexNAc3Ac | 1445,51 |
| NeuAc2Hex4HexNAc | 1450,48 |
| Hex5HexNAc2dHexSP | 1459,44 |
| NeuAc2Hex2HexNAc2dHex | 1475,52 |
| NeuAcHex6HexNAc / NeuAcHex3HexNAc3SP | 1483,49 / 1483,45 |
| NeuAc2Hex3HexNAc2 | 1491,51 |
| NeuAcHex3HexNAc2dHex2 | 1492,53 |
| Hex4HexNAc3dHexSP | 1500,47 |
| NeuAcHex4HexNAc2dHex | 1508,53 |
| NeuAc2HexHexNAc3dHex / Hex5HexNAc3SP | 1516,54 / 1516,46 |
| NeuAcHex5HexNAc2 | 1524,52 |
| NeuAc2Hex2HexNAc3 | 1532,54 |
| NeuAcHex2HexNAc3dHex2 | 1533,56 |
| NeuAcHex3HexNAc3dHex | 1549,55 |
| NeuAc2Hex2HexNAc2dHexSP | 1555,47 |
| Hex4HexNAc4SP | 1557,49 |
| NeuAcHex3HexNAc3(SP)2 | 1563,41 |
| NeuAcHex4HexNAc3 | 1565,55 |
| NeuAc2HexHexNAc4 | 1573,56 |
| NeuGcHex4HexNAc3 | 1581,54 |
| NeuAcHex2HexNac4dHex | 1590,58 |
| NeuAc2Hex4HexNAcdHex | 1596,54 |
| NeuAcHex3HexNAc4 | 1606,57 |
| NeuAc2Hex2HexNAc2dHex2 / Hex6HexNAc2dHexSP | 1621,57 / 1621,49 |
| NeuAc2Hex3HexNAc2dHex | 1637,57 |
| NeuAcHex4HexNAc3SP | 1645,50 |
| NeuAcHex2HexNAc5 | 1647,60 |
| NeuAcHex4HexNAc2dHex2 | 1654,58 |
| Hex5HexNAc3dHexSP | 1662,52 |
| NeuAcHex5HexNAc2dHex | 1670,58 |
| NeuAc2Hex2HexNAc3dHex | 1678,60 |
| NeuAcHex2HexNAc3dHex3 | 1679,62 |
| NeuAcHex6HexNAc2 | 1686,57 |
| NeuAc2Hex3HexNAc3 | 1694,59 |
| Hex4HexNAc4dHexSP | 1703,55 |
| NeuAcHex3HexNAc3dHex(SP)2 | 1709,47 |
| NeuGcNeuAcHex3HexNAc3 | 1710,59 |
| NeuAcHex4HexNAc3dHex | 1711,61 |
| Hex5HexNAc4SP | 1719,54 |
| NeuAcHex4HexNAc3(SP)2 | 1725,46 |
| Hex4HexNAc3dHex2(SP)2 / NeuGc2Hex3HexNAc3 | 1726,48 / 1726,58 |
| NeuAcHex5HexNAc3 / NeuGcHex4HexNAc3dHex | 1727,60 |
| NeuAc2Hex2HexNAc4 | 1735,62 |
| NeuAcHex2HexNAc4dHex2 | 1736,64 |
| NeuGcHex5HexNAc3 | 1743,60 |
| NeuAcHex3HexNAc4dHex | 1752,63 |
| NeuAc2Hex2HexNAc3dHexSP | 1758,55 |
| NeuAcHex3HexNAc4(SP)2 / NeuAcHex6HexNAc2SP | 1766,49 / 1766,53 |
| Hex6HexNAc2dHex2SP / Hex3HexNAc4dHex2(SP)2 / NeuAc2Hex2HexNAc2dHex3 | 1767,55 / 1767,51 |
| NeuAcHex4HexNAc4 | 1768,63 |

| | |
|---|---|
| NeuAc2Hex6HexNAc / NeuAc2Hex3HexNAc3SP | 1774,59 / 1774,55 |
| Hex7HexNAc2dHexSP | 1783,55 |
| NeuGcHex4HexNac4 | 1784,62 |
| NeuAcHex4HexNAc3dHexSP | 1791,56 |
| NeuAcHex2HexNAc5dHex | 1793,66 |
| NeuAc2Hex4HexNAc2dHex / Hex5HexNAc4(SP)2 | 1799,62 |
| NeuAcHex3HexNac5 | 1809,65 |
| NeuAc2Hex5HexNAc2 / NeuAc2Hex2HexNAc4SP | 1815,62 |
| NeuAcHex5HexNAc2dHex2 / NeuAcHex2HexNAc4dHex2SP | 1816,64 |
| Hex6NexNAc3dHexSP | 1824,57 |
| NeuGcHex3HexNAc5 | 1825,65 |
| NeuAcHex6HexNAc2dHex | 1832,63 |
| NeuAc2Hex3HexNAc3dHex | 1840,65 |
| NeuAcHex3HexNAc3dHex3 | 1841,67 |
| NeuAc2Hex4HexNAc3 | 1856,64 |
| NeuAcHex4HexNAc3dHex2 | 1857,66 |
| Hex5HexNAc4dHexSP | 1865,60 |
| NeuAcHex4HexNAc3dHex(SP)2 | 1871,52 |
| NeuAcHex5HexNAc3dHex / NeuGcHex4HexNAc3dHex2 | 1873,66 |
| Hex6HexNAc4SP | 1881,65 |
| NeuAcHex5HexNAC3(SP)2 | 1887,51 |
| NeuAcHex6HexNAc3 | 1889,65 |
| NeuAcHex3HexNAc4dHex2 | 1898,69 |
| Hex4HexNAc5dHexSP | 1906,63 |
| NeuAcHex6HexNAc2dHexSP / NeuAcHex3HexNAc4dHex(SP)2 | 1912,59 |
| NeuAcHex4HexNAc4dHex | 1914,68 |
| NeuAc2Hex3HexNAc3dHexSP | 1920,60 |
| Hex5HexNAc5SP | 1922,62 |
| NeuAcHex4HexNAc4(SP)2 | 1928,54 |
| NeuAcHex5HexNAc4 | 1930,68 |
| NeuGcHex5HexNAc4 | 1946,67 |
| NeuAcHex5HexNAc3dHexSP | 1953,62 |
| NeuAcHex3HexNAc5dHex | 1955,71 |
| NeuAc2Hex5HexNAc2dHex / Hex6HexNAc4(SP)2 | 1961,67 / 1961,55 |
| NeuAcHex4HexNAc5 | 1971,71 |
| NeuAcHex5HexNAc4Ac | 1972,69 |
| NeuAcHex6HexNAc2dHex2 / NeuAcHex3HexNAc4dHex2SP | 1978,69 / 1978,65 |
| NeuAc2Hex4HexNAc3dHex / Hex8HexNAc3SP | 2002,70 / 2002,62 |
| NeuAcHex4HexNAc3dHex3 | 2003,72 |
| NeuAcHex5HexNAc4SP | 2010,64 |
| Hex5HexNAc4dHex2SP | 2011,66 |
| NeuAc2Hex5HexNAc3 / NeuGcNeuAcHex4HexNAc3dHex | 2018,70 |
| NeuAcHex5HexNAc3dHex2 | 2019,72 |
| NeuGcHex5HexNAc4SP | 2026,63 |
| Hex6HexNAc4dHexSP | 2027,65 |
| NeuAcHex6HexNAc3dHex | 2035,71 |
| NeuAc2Hex3HexNAc4dHex / Hex7HexNAc4SP | 2043,73 / 2043,65 |
| NeuAcHex7HexNAc3 | 2051,71 |

| | |
|---|---|
| Hex4HexNAc5dHex2SP | 2052,68 |
| NeuAc2Hex4HexNAc4 | 2059,72 |
| NeuAcHex4HexNAc4dHex2 | 2060,74 |
| Hex5HexNAc5dHexSP | 2068,68 |
| NeuAcHex4HexNAc4dHex(SP)2 | 2074,60 |
| NeuAcHex5HexNAc4dHex | 2076,74 |
| NeuAc2Hex4HexNAc3dHexSP | 2082,66 |
| NeuGc2Hex4HexNAc4 | 2091,71 |
| NeuAcHex6HexNAc4 / NeuGcHex5HexNAc4dHex | 2092,73 |
| NeuAc2Hex5HexNAc3SP / NeuGcNeuAcHex4HexNAc3dHexSP | 2098,65 |
| NeuAcHex5HexNAc3dHex2SP / NeuGcHex4HexNAc3dHex3SP | 2099,67 |
| NeuAc2Hex3HexNAc5 | 2100,75 |
| NeuAcHex3HexNAc5dHex2 / NeuAc2Hex4HexNAc4Ac | 2101,77 / 2101,73 |
| NeuAcHex6HexNAc3dHexSP | 2115,67 |
| NeuAcHex4HexNAc5dHex | 2117,76 |
| Hex7HexNAc3dHex2SP / NeuAc2Hex3HexNAc3dHex3 | 2132,68 / 2132,76 |
| NeuAcHex5HexNAc5 | 2133,76 |
| Hex8HexNAc3dHexSP / NeuAc2Hex4HexNAc3dHex2 | 2148,68 |
| NeuAcHex8Hexnac2dHex / NeuAcHex5HexNAc4dHexSP | 2156,74 / 2156,69 |
| Hex5HexNAC4dHex3SP | 2157,71 |
| NeuAc2Hex5HexNAc3dHex | 2164,75 |
| NeuAcHex5HexNAc3dHex3 | 2165,77 |
| NeuAcHex9HexNAc2 / NeuAcHex6HexNAc4SP / NeuGcHex5HexNAc4dHexSP | 2172,73 / 2172,69 |
| NeuAcHex4Hexnac6 | 2174,79 |
| NeuAc2Hex6HexNAc3 / NeuGc2Hex4HexNAc3dHex2 | 2180,75 |
| NeuAcHex6HexNAc3dHex2 | 2181,77 |
| NeuAc3Hex3HexNAc4 / NeuGcHex6HexNAc4SP / NeuAc2NeuGcHex2HexNAc4dHex | 2188,76 / 2188,68 |
| NeuAc2Hex3HexNAc4dHex2 / Hex7HexNAc4dHexSP | 2189,79 / 2189,70 |
| NeuAcHex3HexNAc4dHex4 | 2190,81 |
| NeuGcNeuAcHex6HexNAc3 / NeuGc2Hex5HexNAc3dHex | 2196,74 |
| Hex4HexNAc5dHex3SP | 2198,74 |
| NeuAc2Hex4HexNAc4dHex | 2205,78 |
| NeuAcHex4HexNAc4dHex3 | 2206,80 |
| NeuAc2Hex4HexNAc4(SP)2 | 2219,64 |
| NeuAc2Hex5HexNAc4 | 2221,78 |
| NeuAcHex5HexNAc4dHex2 | 2222,80 |
| Hex6HexNAc5dHexSP | 2230,73 |
| NeuGcNeuAcHex5HexNAc4 | 2237,77 |
| NeuAcHex6HexNAc4dHex / NeuGcHex5HexNAc4dHex2 | 2238,79 |
| NeuAc2Hex3HexNAc5dHex | 2246,81 |
| NeuAcHex3HexNAc5dHex3 | 2247,83 |
| NeuGc2Hex5Hexnac4 | 2253,76 |
| NeuAcHex7HexNAc4 / NeuGcHex6HexNAc4dHex | 2254,79 |
| NeuAc2Hex4HexNAc5 | 2262,80 |

| | |
|---|---|
| NeuAcHex4HexNAc5dHex2 / NeuAc2Hex5HexNAc4Ac | 2263,82 / 2263,79 |
| NeuAcHex5HexNAc5dHex | 2279,82 |
| NeuAc2Hex4HexNAc4dHexSP | 2285,74 |
| NeuAcHex4HexNAc4dHex3SP | 2286,76 |
| NeuAcHex8HexNAc3SP / NeuAc3Hex4HexNAc3dHex | 2293,72 / 2293,80 |
| NeuAc2Hex4HexNAc3dHex3 | 2294,82 |
| NeuAcHex6HexNAc5 | 2295,81 |
| NeuAc2Hex5HexNAc4SP | 2301,73 |
| NeuAcHex5HexNAc4dHex2SP | 2302,75 |
| NeuAc2Hex5HexNAc4Ac2 | 2305,80 |
| NeuAc2Hex5HexNAc3dHex2 / NeuGcNeuAcHex4HexNAc3dHex3 | 2310,81 |
| NeuAcHex5HexNAc3dHex4 / NeuGcHex6HexNAc5 | 2311,83 |
| NeuAcHex6HexNAc4dHexSP | 2318,75 |
| Hex6HexNAc4dHex3SP / NeuGcNeuAcHex3HexNAc6 | 2319,77 |
| NeuAcHex4HexNAc6dHex | 2320,84 |
| NeuAcHex5HexNAc5dHexAc | 2321,83 |
| NeuAc2Hex6HexNAc3dHex | 2326,81 |
| NeuAcHex6HexNAc3dHex3 | 2327,83 |
| NeuAcHex7HexNAc4SP / NeuGcHex6HexNAc4dHexSP / NeuAcHex10HexNAc2 | 2334,74 / 2334,79 |
| NeuAcHex5HexNAc6 | 2336,84 |
| NeuAc3Hex4HexNac4 | 2350,82 |
| NeuAc2Hex4HexNAc4dHex2 / Hex8HexNAc4dHexSP | 2351,84 / 2351,76 |
| NeuGcNeuAc2Hex4HexNAc4 | 2366,81 |
| NeuAc2Hex5HexNAc4dHex | 2367,83 |
| NeuAcHex5HexNAc4dHex3 | 2368,85 |
| NeuAcHex5HexNAc4dHex2(SP)2 | 2382,71 |
| NeuAc2Hex6HexNAc4 / NeuGcNeuAcHex5HexNAc4dHex | 2383,83 |
| NeuAcHex6HexNAc4dHex2 / NeuGcHex5HexNAc4dHex3 | 2384,85 |
| NeuAc3Hex5HexNAc3SP / NeuAc2Hex5HexNAc4Ac4 | 2389,75 / 2389,82 |
| NeuAc2Hex5HexNAc3dHex2SP | 2390,77 |
| NeuAcHex5HexNAc3dHex4SP / NeuAc3Hex3HexNAc5 | 2391,79 / 2391,84 |
| NeuAc2Hex3HexNAc5dHex2 | 2392,86 |
| NeuAcHex3HexNAc5dHex4 | 2393,89 |
| NeuGc2Hex5HexNAc4dHex | 2399,82 |
| Hex4HexNAc6dHex3SP | 2401,82 |
| NeuAc2Hex6HexNAc3dHexSP | 2406,76 |
| NeuAc2Hex4HexNAc5dHex | 2408,86 |
| NeuAcHex4HexNAc5dHex3 / NeuAc2Hex5HexNAc4dHexAc | 2409,88 / 2409,84 |
| NeuAc2Hex5HexNAc5 | 2424,85 |
| NeuAcHex5HexNAc5dHex2 | 2425,87 |
| NeuAcHex8HexNAc3dHexSP / NeuAc3Hex4HexNAc3dHex2 | 2439,77 |
| NeuAcHex6HexNAc5dHex | 2441,87 |
| NeuAc2Hex8HexNAc2dHex / NeuAc2Hex5HexNAc4dHexSP | 2447,83 / 2447,79 |
| NeuAcHex8HexNAc2dHex3 / NeuAcHex5HexNAc4dHex3SP | 2448,85 / 2448,81 |
| NeuAcHex3HexNAc6dHex3 | 2450,91 |
| NeuAc2Hex5HexNAc4dHexAc2 | 2451,85 |
| NeuAc2Hex5HexNAc3dHex3 | 2456,87 |
| NeuAcHex7HexNAc5 | 2457,86 |
| NeuAcHex5HexNAc5dHex2Ac | 2467,89 |
| NeuAc2Hex6HexNAc3dHex2 | 2472,86 |
| NeuAcHex6HexNAc3dHex4 / NeuGcHex7HexNAc5 | 2473,88 |
| NeuAcHex5HexNAc6dHex | 2482,90 |
| NeuAcHex6HexNAc5Ac | 2483,88 |
| NeuAc2Hex7HexNAc3dHex | 2488,86 |
| NeuAcHex7HexNAc3dHex3 | 2489,88 |
| NeuAcHex6HexNAc6 / NeuGcHex5hexNAc6dHex | 2498,89 |
| NeuAc3Hex5HexNAc4 | 2512,87 |
| NeuAc2Hex5HexNAc4dHex2 | 2513,89 |
| NeuAcHex5HexNAc4dHex4 | 2514,91 |
| NeuAcHex6HexNAc5dHexSP / NeuAcHex9HexNAc3dHex / NeuAc3Hex2HexNAc5dHex2 | 2521,83 / 2521,87 |
| Hex6HexNAc5dHex3SP | 2522,85 |
| NeuGcNeuAc2Hex5HexNAc4 | 2528,87 |
| NeuAc2Hex6HexNAc4dHex / NeuGcNeuAcHex5HexNAc4dHex2 | 2529,89 |
| NeuAcHex6HexNAc4dHex3 | 2530,91 |
| NeuAc3Hex3HexNAc5dHex / NeuGcHex6HexNAc5dHexSP / NeuAcHex7HexNAc5SP | 2537,90 / 2537,82 |
| NeuAc2Hex3HexNAc5dHex3 | 2538,92 |
| NeuAcHex5HexNAc7 / NeuAcHex3HexNAc5dHex5 | 2539,92 |
| NeuGc2NeuAcHex5HexNAc4 | 2544,86 |
| NeuGc2Hex5Hexnac4dHex2 / NeuGcNeuAcHex6HexNAc4dHex | 2545,88 |
| NeuAc3Hex4HexNAc5 | 2553,90 |
| NeuAc2Hex4HexNAc5dHex2 | 2554,92 |
| NeuAcHex4HexNAc5dHex4 | 2555,94 |
| NeuGc3Hex5HexNAc4 | 2560,86 |
| NeuAc2Hex5HexNAc5dHex | 2570,91 |
| NeuAcHex5HexNAc5dHex3 | 2571,93 |
| NeuAc2Hex6HexNAc5 | 2586,91 |
| NeuAcHex6HexNAc5dHex2 | 2587,93 |
| Hex7HexNAc6dHexSP | 2595,86 |
| NeuGcNeuAcHex6HexNAc5 | 2602,90 |
| NeuAcHex7HexNAc5dHex / NeuGcHex6HexNAc5dHex2 | 2603,92 / 603,92 |
| NeuGc2Hex6HexNac5 | 2618,90 |
| NeuAcHex8HexNAc5 / NeuGcHex7HexNAc5dHex | 2619,92 |
| NeuAc2Hex5HexNAc6 | 2627,93 |
| NeuAcHex5HexNAc6dHex2 | 2628,95 |
| NeuGcHex8HexNAc5 / NeuAcHex4HexNAc5dHex4SP | 2635,91 / 2635,89 |
| NeuAcHex6HexNAc6dHex | 2644,95 |
| NeuAc2Hex5HexNAc5dHexSP | 2650,87 |
| NeuAc2Hex5HexNAc4dHex3 | 2659,95 |
| NeuAcHex7HexNAc6 | 2660,94 |
| NeuGcNeuAc2Hex5HexNAc4dHex NeuAc3Hex6HexNAc4 | 2674,92 |

| | |
|---|---|
| NeuGcHex6HexNAc5dHexSP / NeuAcHex7HexNAc5dHexSP | 2683,88 |
| NeuAcHex5HexNAc7dHex | 2685,98 |
| NeuAc2Hex7HexNAc4dHex | 2691,94 |
| NeuAcHex7HexNAc4dHex3 | 2692,96 |
| NeuAc2Hex4HexNAc5dHex2(SP)2 | 2714,83 |
| NeuAcHex4HexNAc5dHex4(SP)2 / NeuAc3Hex5HexNAc5 | 2715,85 / 2715,95 |
| NeuAc2Hex5HexNAc5dHex2 | 2716,97 |
| NeuAcHex5HexNAc5dHex4 | 2717,99 |
| NeuAc2Hex6HexNAc5dHex | 2732,97 |
| NeuAcHex6HexNAc5dHex3 | 2733,99 |
| NeuAcHex6HexNAc5dHex2(SP)2 | 2747,84 |
| NeuGcNeuAcHex6HexNAc5dHex | 2748,96 |
| NeuAc3Hex4HexNAc6 | 2756,98 |
| NeuAc2Hex4HexNAc6dHex2 | 2758,00 |
| NeuAcHex4HexNAc6dHex4 | 2759,02 |
| NeuAc3Hex6HexNAc3dHex2 | 2763,96 |
| NeuAc2Hex6HexNAc3dHex4 / NeuGc2Hex6HexNAc5dHex / NeuGcHex7HexNAc5 | 2764,98 / 2764,96 |
| NeuAcHex8HexNAc5dHex | 2765,98 |
| NeuAc2Hex5HexNAc6dHex | 2773,99 |
| NeuAcHex5HexNAc6dHex3 | 2775,01 |
| NeuGc2Hex7HexNAc5 | 2780,95 |
| NeuGcHex8HexNAc5dHex / NeuAcHex9HexNac5 | 2781,97 |
| NeuAc2Hex6HexNAc6 | 2789,99 |
| NeuAcHex6HexNAc6dHex2 | 2791,01 |
| NeuAc4Hex5HexNAc4 | 2803,97 |
| NeuAc3Hex5HexNAc4dHex2 / NeuAcHex6HexNAc6dHex(SP)2 | 2804,99 / 2804,86 |
| Hex6HexNAc6dHex3SP2 | 2805,88 |
| NeuAc2Hex5HexNAc4dHex4 | 2806,01 |
| NeuAcHex7Hexnac6dHex | 2807,00 |
| NeuAc2Hex6HexNAc5dHexSP | 2812,92 |
| NeuAcHex6HexNAc5dHex3SP | 2813,94 |
| NeuGcNeuAc3Hex5HexNAc4 | 2819,96 |
| NeuAc3Hex6HexNAc4dHex / NeuGcNeuAc2Hex5HexNAc4dHex2 | 2820,98 |
| NeuAc2Hex6HexNAc4dHex3 | 2822,00 |
| NeuAcHex8HexNAc6 | 2823,00 |
| NeuGc2NeuAc2Hex5HexNAc4 | 2835,96 |
| NeuGc2NeuAcHex5HexNAc4dHex2 | 2836,98 |
| NeuAc3Hex6HexNAc5 | 2878,00 |
| NeuAc2Hex6HexNAc5dHex2 | 2879,02 |
| NeuAcHex6HexNAc5dHex4 | 288,04 |
| NeuAcHex7HexNAc6dHexSP / NeuAcHex10HexNAc4dHex | 2886,96 / 2887,00 |
| NeuGcNeuAc2Hex6HexNAc5 | 2894,00 |
| NeuAc2Hex7HexNAc5dHex / NeuGcNeuAcHex6HexNAc5dHex2 | 2895,02 |
| NeuAc3Hex6HexNAc4dHexSP / NeuGcNeuAc2Hex5HexNAc4dHex2SP | 2900,94 |
| NeuGc2NeuAcHex6HexNAc5 | 2909,99 |
| NeuGc2Hex6HexNAc5dHex2 | 2911,01 |
| NeuAc3Hex5HexNAc6 | 2919,03 |
| NeuAc2Hex5HexNAc6dHex2 | 2920,05 |
| NeuAcHex5HexNAc6dHex4 | 2921,07 |
| NeuGc3Hex6HexNAc5 | 2925,99 |
| NeuGcNeuAc2Hex5HexNAc6 | 2935,02 |
| NeuAc2Hex6HexNAc6dHex / NeuGcNeuAcHex5HexNAc6dHex2 | 2936,04 |
| NeuAcHex6HexNAc6dHex3 | 2937,07 |
| NeuGc2NeuAcHex5HexNAc6 / NeuAc3Hex5HexNAc4dHex3 | 2951,02 / 2951,04 |
| NeuAc2Hex7HexNAc6 | 2952,04 |
| NeuAcHex7HexNAc6dHex2 | 2953,06 |
| NeuAc2Hex6HexNAc5dHex2SP | 2958,98 |
| NeuAcHex6HexNAc5dHex4SP | 2960,00 |
| NeuAc2Hex4HexNAc7dHex2 | 2961,08 |
| NeuAcHex4HexNAc7dHex4 | 2962,10 |
| NeuAcHex6HexNAc7dHex2 | 2994,09 |
| NeuAcHex7HexNAc7dHex | 3010,08 |
| NeuAc3Hex6HexNAc5dHex | 3024,06 |
| NeuAc2Hex6HexNAc5dHex3 | 3025,08 |
| NeuAcHex8HexNAc7 | 3026,08 |
| NeuAc3Hex5HexNAc6dHex | 3065,09 |
| NeuAc2Hex5HexNAc6dHex3 | 3066,11 |
| NeuAcHex7HexNAc8 | 3067,10 |
| NeuAc3Hex6HexNAc6 | 3081,08 |
| NeuAc2Hex6HexNAc6dHex2 | 3082,10 |
| NeuAc2Hex7HexNAc6dHex | 3098,10 |
| NeuAcHex7HexNAc6dHex3 | 3099,12 |
| NeuAc3Hex6HexNAc5dHexSP | 3104,02 |
| NeuAc2Hex6HexNAc5dHex3SP | 3105,04 |
| NeuAcHex8HexNAc7SP / NeuAc3Hex4HexNAc7dHex | 3106,03 / 3106,11 |
| Hex8HexNAc7dHex2SP / NeuAc2Hex4HexNAc7dHex3 | 3107,05 / 3107,13 |
| NeuAcHex7HexNAc7dHex2 | 3156,14 |
| NeuAc3Hex6HexNAc5dHex2 | 3170,12 |
| NeuAc2Hex6HexNAc5dHex4 | 3171,14 |
| NeuAcHex8HexNAc7dHex | 3172,13 |
| NeuAc2Hex7HexNAc6dHexSP | 3178,05 |
| NeuAc3Hex6HexNAc6dHex | 3227,14 |
| NeuAc2Hex6HexNAc6dHex3 | 3228,16 |
| NeuAcHex8HexNAc8 | 3229,16 |
| NeuAc3Hex7HexNAc6 | 3243,13 |
| NeuAc2Hex7HexNAc6dHex2 | 3244,16 |
| NeuAcHex7HexNAc6dHex4 | 3245,18 |
| NeuAc2Hex8HexNAc6dHex / NeuGcNeuAcHex7HexNAc6dHex2 | 3260,15 |
| NeuAcHex8HexNAc6dHex3 / NeuGcHex7HexNAc6dHex4 | 3261,17 |
| NeuAc3Hex7HexNAc5dHexSP / NeuGcNeuAc2Hex6HexNAc5dHex2SP | 3266,07 |
| NeuAc3Hex5HexNAc7dHex / NeuGcHex8HexNAc7dHexSP | 3268,17 / 3268,09 |
| NeuAc2Hex5HexNAc7dHex3 | 3269,19 |
| NeuAcHex7HexNAc9 | 3270,18 |
| NeuGc2Hex7HexNAc6dHex2 | 3276,15 |
| NeuAc4Hex4HexNAc5dHex2(SP)2 | 3297,02 |
| NeuAc3Hex4HexNAc5dHex4(SP)2 | 3298,04 |
| NeuAc2Hex7HexNAc7dHex | 3301,18 |
| NeuAcHex7HexNAc7dHex3 | 3302,20 |

| | |
|---|---|
| NeuAc3Hex6HexNAc5dHex3 | 3316,18 |
| NeuAc2Hex8HexNAc7 | 3317,17 |
| NeuAcHex8HexNAc7dHex2 | 3318,19 |
| NeuAc3Hex7HexNAc6dHex | 3389,19 |
| NeuAc2Hex7HexNAc6dHex3 | 3390,21 |
| NeuAcHex7HexNAc6dHex5 / NeuAcHex9HexNAc8 | 3391,23 |
| NeuAc3Hex5HexNAc7dHex2 | 3414,22 |
| NeuAc2Hex5HexNAc7dHex4 | 3415,24 |
| NeuAcHex7HexNAc9dHex | 3416,24 |
| NeuAc3Hex6HexNAc7dHex | 3430,22 |
| NeuAc2Hex6HexNAc7dHex3 | 3431,24 |
| NeuAcHex8HexNAc9 | 3432,24 |
| NeuAc2Hex8Hexnac7dHex | 3463,23 |
| NeuAcHex8HexNAc7dHex3 | 3464,25 |
| NeuAc3Hex7HexNAc6dHexSP | 3469,15 |
| NeuAc2Hex7HexNAc6dHex3SP | 3470,17 |
| NeuAc3Hex5HexNAc8dHex | 3471,25 |
| NeuAc2Hex5HexNAc8dHex3 | 3472,27 |
| NeuAcHex7HexNAc10 | 3473,26 |
| NeuAc4Hex7HexNAc6 | 3534,23 |
| NeuAc3Hex7HexNAc6dHex2 | 3535,25 |
| NeuAc2Hex7HexNAc6dHex4 | 3536,27 |
| NeuAcHex9HexNAc8dHex | 3537,27 |
| NeuAc4Hex5HexNAc7dHex | 3559,26 |
| NeuAc3Hex5HexNAc7dHex3 | 3560,28 |
| NeuAc2Hex7HexNAc9 | 3561,28 |
| NeuAcHex7HexNAc9dHex2 | 3562,30 |
| NeuAc3Hex7HexNac7dHex | 3592,27 |
| NeuAc2Hex7HexNAc7dHex3 | 3593,29 |
| NeuAcHex9HexNAc9 | 3594,29 |
| NeuAc3Hex8HexNAc7 | 3608,27 |
| NeuAc2Hex8HexNac7dHex2 | 3609,29 |
| NeuAcHex8HexNac7dHex4 | 3610,31 |
| NeuAc3Hex5HexNAc8dHex2 | 3617,30 |
| NeuAc2Hex5HexNAc8dHex4 | 3618,32 |
| NeuAcHex7HexNAc10dHex | 3619,32 |
| NeuAc3Hex6HexNAc8dHex | 3633,30 |
| NeuAc4Hex7HexNAc6dHex | 3680,29 |
| NeuAc3Hex7HexNAc6dHex3 | 3681,31 |
| NeuAc2Hex9HexNAc8 | 3682,30 |
| NeuAcHex9HexNAc8dHex2 | 3683,32 |
| NeuAc4Hex6HexNAc7dHex | 3721,31 |
| NeuAc3Hex6HexNAc7dHex3 | 3722,34 |
| NeuAc2Hex8HexNAc9 | 3723,33 |
| NeuAcHex8HexNAc9dHex2 | 3724,35 |
| NeuAc3Hex7HexNac7dHex2 | 3738,33 |
| NeuAc2Hex7HexNAc7dHex4 | 3739,35 |
| NeuAcHex9HexNAc9dHex | 3740,35 |
| NeuAc3Hex8HexNAc7dHex | 3754,33 |
| NeuAc2Hex8HexNAc7dHex3 | 3755,35 |
| NeuAcHex10HexNAc9 / NeuAcHex8HexNAc7dHex5 | 3756,34 |
| NeuAc4Hex6HexNAc8 | 3778,34 |
| NeuAc3Hex6HexNAc8dHex2 | 3779,36 |
| NeuAc2Hex6HexNAc8dHex4 | 3780,38 |
| NeuAcHex8HexNAc10dHex | 3781,37 |
| NeuAc4Hex7HexNAc6dHex2 | 3826,35 |
| NeuAc3Hex7Hexnac6dHex4 | 3827,37 |
| NeuAc2Hex9HexNAc8dHex | 3828,36 |
| NeuAcHex9HexNAc8dHex3 | 3829,38 |
| NeuAc4Hex8HexNAc7 | 3899,36 |
| NeuAc3Hex8HexNAc7dHex2 | 3900,38 |
| NeuAc2Hex8HexNAc7dHex4 | 3901,40 |
| NeuAcHex10HexNAc9dHex | 3902,40 |
| NeuAc4Hex6HexNAc8dHex | 3924,39 |
| NeuAc3Hex6HexNAc8dHex3 | 3925,41 |
| NeuAc2Hex8HexNAc10 | 3926,41 |
| NeuAcHex8HexNAc10dHex2 | 3927,43 |
| NeuAc3Hex9HexNAc8 | 3973,40 |
| NeuAc2Hex9HexNAc8dHex2 | 3974,42 |
| NeuAcHex9HexNAc8dHex4 | 3975,44 |
| NeuAc4Hex8HexNAc7dHex | 4045,42 |
| NeuAc3Hex8HexNAc7dHex3 | 4046,44 |
| NeuAc2Hex10HexNAc9 / NeuAc2Hex8HexNAc7dHex5 | 4047,44 |
| NeuAcHex10HexNAc9dHex2 | 4048,46 |
| NeuAc3Hex9HexNAc8dHex | 4119,46 |
| NeuAc2Hex9HexNAc8dHex3 | 4120,48 |
| NeuAcHex11HexNAc10 / NeuAcHex9HexNAc8dHex5 | 4121,47 |
| NeuAc2Hex10HexNAc9dHex2 | 4339,55 |
| NeuAcHex10HexNAc9dHex4 | 4340,57 |
| NeuAc2Hex10HexNAc9dHex3 | 4485,61 |

**Table 11.** NMR analysis of large neutral N-glycan fraction from pancreatic cancer sample. Reference glycans A-C are as in **Figure 26.**

| | | | ppm | | | |
|---|---|---|---|---|---|---|
| **Residue** | **Linkage** | **Proton** | **A** | **B** | **C** | **Sample** |
| D-GlcNAc | | H-1α | 5.188 | 5.191 | 5.187 | 5.187 |
| | | H-1β | 4.695 | 4.690 | 4.693 | 4.693 |
| | | NAc | 2.038<br>2.036 | 2.042 | 2.037 | 2.036 |
| β-D-GlcNAc | 4 | H-1 | 4.601<br>4.592 | 4.596 | 4.586 | 4.952 |
| | | NAc | 2.064<br>2.063 | 2.072 | 2.063 | 2.062 |
| β-D-Man | 4,4 | H-1 | 4.780 | 4.775 | 4.771 | under HDO |
| | | H-2 | 4.250 | 4.238 | 4.234 | 4.232/4.253 |
| α-D-Man | 6,4,4 | H-1 | 4.870 | 4.869 | 4.870 | 4.870 |
| | | H-2 | 4.145 | 4.149 | 4.149 | 4.146 |
| α-D-Man | 6,6,4,4 | H-1 | 4.907 | 5.153 | 5.151 | 4.907/5.148 |
| | | H-2 | 3.984 | 4.025 | 4.021 | 3.984 |
| α-D-Man | 2,6,6,4,4 | H-1 | - | 5.047 | 5.042 | 5.041 |
| | | H-2 | - | 4.074 | 4.069 | 4.067 |
| α-D-Man | 3,6,4,4 | H-1 | 5.092 | 5.414 | 5.085 | 5.090/5.407 |
| | | H-2 | 4.065 | 4.108 | 4.069 | 4.067/4.108 |
| α-D-Man | 2,3,6,4,4 | H-1 | - | 5.047 | - | 5.041 |
| | | H-2 | - | 4.074 | - | 4.067 |
| α-D-Man | 3,4,4 | H-1 | 5.097 | 5.343 | 5.341 | 5.090/5.345 |
| | | H-2 | 4.076 | 4.108 | 4.099 | 4.067/4.108 |
| α-D-Man | 2,3,4,4 | H-1 | - | 5.317 | 5.309 | 5.308 |
| | | H-2 | - | 4.108 | 4.099 | 4.108 |
| α-D-Man | 2,2,3,4,4 | H-1 | - | 5.047 | 5.042 | 5.041 |
| | | H-2 | - | 4.074 | 4.069 | 4.067 |

**Table 12.** NMR analysis of small N-glycan fraction from pancreatic cancer sample. Reference glycans D-G are as in **Figure 27.**

| | | | ppm | |
|---|---|---|---|---|
| **Residue** | **Linkage** | **Proton** | **D** | **Sample** |
| D-GlcNAc | | H-1α | 5.188 | 5.188 |
| | | H-1β | 4.696 | under HDO |
| | | NAc | 2.038 | 2.037 |
| β-D-GlcNAc | 4 | H-1α | 4.612 | 4.606 |
| | | H-1β | 4.603 | |
| | | NAc | 2.078 | 2.078 |
| β-D-Man | 4,4 | H-1 | 4.780 | under HDO |
| | | H-2 | 4.254 | 4.250/4.231 |
| α-D-Man | 6,4,4 | H-1 | 4.915 | 4.915 |
| | | H-2 | 3.974 | 3.970 |
| α-D-Man | 3,4,4 | H-1 | 5.101 | 5.100/5.350 |
| | | H-2 | 4.067 | 4.067/4.103/4 .082 |

(continued)

| Residue | Linkage | Proton | E | F | G | Sample |
|---|---|---|---|---|---|---|
| β-D-GlcNAc | | H-1α | 5.213 | 5.212 | 5.212 | 5.210 |
| | | H-1β | 4.722 | 4.721 | 4.72 | under HDO |
| | | Nacα | 2.057 | 2.057 | 2.057 | 2.056 |
| | | Nacβ | 2.054 | 2.054 | 2.054 | |
| β-D-Man | 4 | H-1(α) | 4.791 | 4.781 | 4.780 | under HDO |
| | | H-1 (β) | 4.783 | 4.774 | 4.773 | |
| | | H-2(α) | 4.265 | 4.246 | 4.244 | 4.250 |
| | | H-2(β) | 4.254 | 4.238 | 4.235 | 4.231 |
| α-D-Man | 6,4 | H-1 | 4.917 | 4.918 | 4.917 | 4.915 |
| | | H-2 | 3.973 | 3.977 | 3.98 | 3.970 |
| α-D-Man | 3,4 | H-1 | 5.104 | 5.352 | 5.345 | 5.100/5.350 |
| | | H-2 | 4.070 | 4.105 | 4.080 | 4.067/4.103/4.082 |
| α-D-Man | 2,3,4 | H-1 | - | 5.051 | 5.303 | 5.046/5.304 |
| | | H-2 | - | 4.069 | 4.105 | 4.067/4.101 |
| α-D-Man | 2,2,3,4 | H-1 | - | - | 5.043 | 5.046 |
| | | H-2 | - | - | 4.064 | 4.067 |

**Table 13.** NMR analysis of acidic N-glycan fraction from pancreatic cancer sample. Reference glycans A-E are as in **Figure 28.**

| | | | ppm | | | | | |
|---|---|---|---|---|---|---|---|---|
| **Residue** | **Linkage** | **Proton** | **A** | **B** | **C** | **D** | **E** | **Sample** |
| D-GlcNAc | | H-1α | 5.189 | 5.188 | 5.188 | 5.189 | 5.181 | 5.188 |
| | | H-1β | 4.694 | n.a. | n.a. | 4.695 | n.a. | 4.692 |
| | | NAc | 2.038 | 2.038 | 2.038 | 2.038 | 2.039 | 2.036 |
| α-L-Fuc | 6 | H-1α | - | - | - | - | 4.892 | 4.894 |
| | | H-1β | - | - | - | - | 4.900 | |
| | | H-5α | - | - | - | - | 4.10 | |
| | | H-5b | - | - | - | - | n.a. | |
| | | CH3α | - | - | - | - | 1.211 | 1.209 |
| | | CH3β | - | - | - | - | 1.223 | 1.219 |
| β-D-GlcNAc | 4 | H-1α | 4.613 | 4.614 | 4.612 | 4.614 | 4.663 | |
| | | H-1β | 4.604 | 4.606 | 4.604 | 4.606 | n.a. | 4.604 |
| | | NAc(α/β) | 2.084 | 2.081 | 2.081 | 2.081 | 2.096<br>2.093 | 2.082/2.094 |
| β-D-Man | 4,4 | H-1 | n.a. | n.a. | n.a. | n.a. | n.a. | n.a. |
| | | H-2 | 4.258 | 4.250 | 4.246 | 4.253 | 4.248 | 4.258 |
| α-D-Man | 6,4,4 | H-1 | 4.948 | 4.930 | 4.928 | 4.930 | 4.922 | 4.948 |
| | | H-2 | 4.117 | 4.112 | 4.11 | 4.112 | 4.11 | 4.115 |
| β-D-GlcpNAc | 2,6,4,4 | H-1 | 4.604 | 4.582 | 4.581 | 4.582 | 4.573 | 4.604 |
| | | Nac | 2.066 | 2.047 | 2.047 | 2.047 | 2.043 | 2.066/2.047 |
| β-D-Gal | 4,2,6,4,4 | H-1 | 4.447 | 4.473 | 4.473 | 4.473 | 4.550 | 4.444/4.47 |
| | | H-3 | n.a. | n.a. | n.a. | n.a. | 4.119 | 4.115 |
| α-D-Neup5Ac | 3,4,2,6,4,4 | H-3a | - | - | - | - | 1.800 | 1.800 |
| | | H-3e | - | - | - | - | 2.758 | 2.758 |
| | | NAc | - | - | - | - | 2.031 | 2.029 |
| α-D-Neup5Ac | 6,4,2,6,4,4 | H-3a | 1.719 | - | - | - | - | 1.722 |

(continued)

| | | | ppm | | | | | |
|---|---|---|---|---|---|---|---|---|
| Residue | Linkage | Proton | A | B | C | D | E | Sample |
| | | H-3e | 2.673 | - | - | - | - | 2.668 |
| | | NAc | 2.029 | - | - | - | - | 2.029 |
| α-D-Man | 3,4,4 | H-1 | 5.133 | 5.123 | 5.118 | 5.135 | 5.116 | 5.132/5.11 |
| | | H-2 | 4.197 | 4.195 | 4.190 | 4.196 | 4.189 | 4.197 |
| β-D-GlcpNAc | 2,3,4,4 | H-1 | 4.604 | 4.606 | 4.573 | 4.606 | 4.573 | 4.604/4.570 |
| | | NAc | 2.070 | 2.043 | 2.047 | 2.069 | 2.048 | 2.069/2.047 |
| β-D-Galp | 4,2,3,4,4 | H-1 | 4.443 | - | 4.545 | 4.445 | 4.544 | 4.444 |
| | | H-3 | n.a. | - | 4.113 | n.a. | 4.113 | 4.115 |
| α-D-Neup5Ac | 6,4,2,3,4,4 | H-3a | 1.719 | - | - | 1.719 | - | 1.722 |
| | | H-3e | 2.667 | - | - | 2.668 | - | 2.668 |
| | | NAc | 2.029 | - | - | 2.030 | - | 2.029 |
| α-D-Neup5Ac | 3,4,2,3,4,4 | H-3a | - | 1.783 | 1.797 | - | 1.797 | 1.800 |
| | | H-3e | - | 2.759 | 2.756 | - | 2.758 | 2.758 |
| | | NAc | - | 2.030 | 2.030 | - | 2.031 | 2.029 |

**Table 14.**

| **$Hex_{5-9}HexNAc_2$** (including high-mannose type N-glycans) **Proposed composition** | **m/z** | **Human tissue** | **Human cell line** |
|---|---|---|---|
| Hex5HexNAc2 | 1257 | + | + |
| Hex6HexNAc2 | 1419 | + | + |
| Hex7HexNAc2 | 1581 | + | + |
| Hex8HexNAc2 | 1743 | + | + |
| Hex9HexNAc2 | 1905 | + | + |

| **$Hex_{1-4}HexNAc_2dHex_{0-1}$** (including low-mannose type N-glycans) **Proposed composition** | **m/z** | **Human tissue** | **Human cell line** |
|---|---|---|---|
| HexHexNAc2 | 609 | + | |
| HexHexNAc2dHex | 755 | + | |
| Hex2HexNAc2 | 771 | + | + |
| Hex2HexNAc2dHex | 917 | + | + |
| Hex3HexNAc2 | 933 | + | + |
| Hex3HexNAc2dHex | 1079 | + | + |
| Hex4HexNAc2 | 1095 | + | + |
| Hex4HexNAc2dHex | 1241 | + | + |

| **$Hex_{10-12}HexNAc_2$** (including glucosylated high-mannose type N-glycans) **Proposed composition** | **m/z** | **Human cells** | **Human cell line** |
|---|---|---|---|
| Hex10HexNAc2 | 2067 | + | + |
| Hex11HexNAc2 | 2229 | + | |
| Hex12HexNAc2 | 2391 | + | |

| **$Hex_{5-9}HexNAc_2dHex_1$** (including fucosylated high-mannose type N-glycans) **Proposed composition** | **m/z** | **Human tissue** | **Human cell line** |
|---|---|---|---|
| Hex5HexNAc2dHex | 1403 | + | + |
| Hex6HexNAc2dHex | 1565 | + | + |

| **HexNAc=3 and Hex≥2** (including hybrid-type and monoantennary N-glycans) **Proposed composition** | **m/z** | **Human cells** | **Human cell line** |
|---|---|---|---|
| Hex2HexNAc3 | 974 | + | |
| Hex2HexNAc3dHex | 1120 | + | |
| Hex3HexNAc3 | 1136 | + | + |
| Hex2HexNAc3dHex2 | 1266 | + | |
| Hex3HexNAc3dHex | 1282 | + | + |
| Hex4HexNAc3 | 1298 | + | + |
| Hex3HexNAc3dHex2 | 1428 | + | |
| Hex4HexNAc3dHex | 1444 | + | + |
| Hex5HexNAc3 | 1460 | + | + |
| Hex4HexNAc3dHex2 | 1590 | + | + |
| Hex5HexNAc3dHex | 1606 | + | + |
| Hex6HexNAc3 | 1622 | + | + |
| Hex5HexNAc3dHex2 | 1752 | + | + |
| Hex6HexNAc3dHex | 1768 | + | + |
| Hex7HexNAc3 | 1784 | + | + |
| Hex7HexNAc3dHex | 1930 | + | + |
| Hex8HexNAc3 | 1946 | + | |

| **HexNAc≥4 and Hex≥3** (including complex-type N-glycans) **Proposed composition** | **m/z** | **Human cells** | **Human cell line** |
|---|---|---|---|
| Hex3HexNAc4 | 1339 | + | + |
| Hex3HexNAc4dHex | 1485 | + | + |
| Hex4HexNAc4 | 1501 | + | + |
| Hex3HexNAc5 | 1542 | + | + |
| Hex4HexNAc4dHex | 1647 | + | + |
| Hex5HexNAc4 | 1663 | + | + |
| Hex3HexNAc5dHex | 1688 | + | + |
| Hex4HexNAx5 | 1704 | + | + |
| Hex4HexNAc4dHex2 | 1793 | + | + |
| Hex5HexNAc4dHex | 1809 | + | + |
| Hex6HexNAc4 | 1825 | + | + |
| Hex4HexNAc5dHex | 1850 | + | + |
| Hex5HexNAc5 | 1866 | + | |
| Hex3HexNAc6dHex | 1891 | + | + |
| Hex5HexNAc4dHex2 | 1955 | + | + |
| Hex6HexNAc4dHex | 1971 | + | + |
| Hex7HexNAc4 | 1987 | + | + |
| Hex4HexNAc5dHex2 | 1996 | + | + |
| Hex5HexNAc5dHex | 2012 | + | |
| Hex6HexNAc5 | 2028 | + | + |
| Hex5HexNAc4dHex3 | 2101 | + | + |
| Hex6HexNAc4dHex2 | 2117 | + | |
| Hex7HexNAc4dHex | 2133 | + | + |
| Hex4HexNAc5dHex3 | 2142 | + | |
| Hex8HexNAc4 | 2149 | + | + |
| Hex5HexNAc5dHex2 | 2158 | + | |
| Hex6HexNAc5dHex | 2174 | + | + |
| Hex7HexNAc5 | 2190 | + | + |
| Hex5HexNAc6dHex | 2215 | + | + |
| Hex6HexNAc6 | 2231 | + | |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc5dHex | 2336 | + | |
| Hex8HexNAc5 | 2352 | + | + |
| Hex7HexNAc6 | 2393 | + | + |
| Hex7HexNAc4dHex3 | 2425 | + | |
| Hex6HexNAc5dHex3 | 2466 | + | |
| Hex8HexNAc5dHex | 2498 | + | |
| Hex7HexNAc6dHex | 2539 | + | + |
| Hex6HexNAc5dHex4 | 2612 | + | + |
| Hex8HexNAc7 | 2758 | + | |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| **$Hex_{1-9}HexNAc_1$** (including soluble glycans) **Proposed composition** | **m/z** | **Human cells** | **Human cell line** |
|---|---|---|---|
| Hex2HexNAc | 568 | + | |
| Hex3HexNAc | 730 | + | + |
| Hex4HexNAc | 892 | + | |
| Hex5HexNAc | 1054 | + | + |
| Hex6HexNAc | 1216 | + | + |
| Hex7HexNAc | 1378 | + | + |
| Hex8HexNAc | 1540 | + | + |
| Hex9HexNAc | 1702 | + | |

| **HexNAc≥3 and dHex≥1** (including fucosylated hybrid/monoant. N-glycans) **Proposed composition** | **m/z** | **Human cells** | **Human cell line** |
|---|---|---|---|
| Hex2HexNAc3dHex | 1120 | + | |
| Hex2HexNAc3dHex2 | 1266 | + | |
| Hex3HexNAc3dHex | 1282 | + | + |
| Hex3HexNAc3dHex2 | 1428 | + | |
| Hex4HexNAc3dHex | 1444 | + | + |
| Hex4HexNAc3dHex2 | 1590 | + | + |
| Hex5HexNAc3dHex | 1606 | + | + |
| Hex5HexNAc3dHex2 | 1752 | + | + |
| Hex6HexNAc3dHex | 1768 | + | + |
| Hex7HexNAc3dHex | 1930 | + | + |
| Hex3HexNAc4dHex | 1485 | + | + |
| Hex4HexNAc4dHex | 1647 | + | + |
| Hex3HexNAc5dHex | 1688 | + | + |
| Hex4HexNAc4dHex2 | 1793 | + | + |
| Hex5HexNAc4dHex | 1809 | + | + |
| Hex4HexNAc5dHex | 1850 | + | + |
| Hex3HexNAc6dHex | 1891 | + | + |
| Hex5HexNAc4dHex2 | 1955 | + | + |
| Hex6HexNAc4dHex | 1971 | + | + |
| Hex4HexNAc5dHex2 | 1996 | + | + |
| Hex5HexNAc5dHex | 2012 | + | |
| Hex5HexNAc4dHex3 | 2101 | + | + |
| Hex6HexNAc4dHex2 | 2117 | + | |
| Hex7HexNAc4dHex | 2133 | + | + |
| Hex4HexNAc5dHex3 | 2142 | + | |
| Hex5HexNAc5dHex2 | 2158 | + | |
| Hex6HexNAc5dHex | 2174 | + | + |
| Hex5HexNAc6dHex | 2215 | + | + |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc5dHex | 2336 | + | |
| Hex7HexNAc4dHex3 | 2425 | + | |
| Hex6HexNAc5dHex3 | 2466 | + | |
| Hex8HexNAc5dHex | 2498 | + | |
| Hex7HexNAc6dHex | 2539 | + | + |
| Hex6HexNAc5dHex4 | 2612 | + | + |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| **HexNAc=Hex≥5** (terminal HexNAc, N=H) **Proposed composition** | **m/z** | **Human cells** | **Human cell line** |
|---|---|---|---|
| Hex5HexNAc5 | 1866 | + | |
| Hex5HexNAc5dHex | 2012 | + | |
| Hex5HexNAc5dHex2 | 2158 | + | |
| Hex6HexNAc6 | 2231 | + | |

| **HexNAc≥3 and dHex≥2** (including multifucosylated hybrid/monoant. N-glycans) **Proposed composition** | **m/z** | **Human cells** | **Human cell line** |
|---|---|---|---|
| Hex2HexNAc3dHex2 | 1266 | + | |
| Hex3HexNAc3dHex2 | 1428 | + | |
| Hex4HexNAc3dHex2 | 1590 | + | + |
| Hex5HexNAc3dHex2 | 1752 | + | + |
| Hex4HexNAc4dHex2 | 1793 | + | + |
| Hex5HexNAc4dHex2 | 1955 | + | + |
| Hex4HexNAc5dHex2 | 1996 | + | + |
| Hex5HexNAc4dHex3 | 2101 | + | + |
| Hex6HexNAc4dHex2 | 2117 | + | |
| Hex4HexNAc5dHex3 | 2142 | + | |
| Hex5HexNAc5dHex2 | 2158 | + | |
| Hex5HexNAc4dHex4 | 2247 | + | + |
| Hex7HexNAc4dHex2 | 2279 | + | |
| Hex5HexNAc5dHex3 | 2304 | + | + |
| Hex6HexNAc5dHex2 | 2320 | + | + |
| Hex7HexNAc4dHex3 | 2425 | + | |
| Hex6HexNAc5dHex3 | 2466 | + | |
| Hex6HexNAc5dHex4 | 2612 | + | + |
| Hex7Hexnac5dHex4 | 2775 | + | + |
| Hex8HexNAc5dHex4 | 2937 | + | + |
| Hex8HexNAc6dHex4 | 3140 | + | + |
| Hex9HexNAc6dHex4 | 3302 | + | + |
| Hex10HexNAc6dHex4 | 3464 | + | + |
| Hex11HexNAc6dHex4 | 3626 | + | + |

| **HexNAc>Hex≥2** (terminal HexNAc, N>H) **Proposed composition** | **m/z** | **Human cells** | **Human cell line** |
|---|---|---|---|
| Hex2HexNAc3 | 974 | + | |
| Hex2HexNAc3dHex | 1120 | + | |
| Hex2HexNAc3dHex2 | 1266 | + | |
| Hex3HexNAc4 | 1339 | + | + |
| Hex3HexNAc4dHex | 1485 | + | + |
| Hex3HexNAc5 | 1542 | + | + |
| Hex3HexNAc5dHex | 1688 | + | + |
| Hex4HexNAx5 | 1704 | + | + |
| Hex4HexNAc5dHex | 1850 | + | + |
| Hex3HexNAc6dHex | 1891 | + | + |
| Hex4HexNAc5dHex2 | 1996 | + | + |
| Hex4HexNAc5dHex3 | 2142 | + | |
| Hex5HexNAc6dHex | 2215 | + | + |

**Table 15.**

| **HexNAc=3 and Hex≥2** (including hybrid-type and monoantennary N-glycans) Proposed composition | m/z | Human cells | Human cell line |
|---|---|---|---|
| Hex3HexNAc3SP | 1192 | + | + |
| Hex3HexNAc3dHexSP | 1338 | + | + |
| Hex4HexNAc3SP | 1354 | + | + |
| NeuAcHex3HexNAc3 | 1403 | + | + |
| NeuGcHex3HexNAc3 | 1419 | + | |
| Hex4HexNAc3dHexSP | 1500 | + | + |
| Hex5HexNAc3SP | 1516 | + | + |
| NeuAcHex3HexNAc3dHex | 1549 | + | + |
| NeuAcHex3HexNAc3SP2 | 1563 | + | |
| NeuAcHex4HexNAc3 | 1565 | + | + |
| NeuGcHex4HexNAc3 | 1581 | + | + |
| Hex4HexNAc3dHex2SP | 1646 | + | |
| Hex5HexNAc3dHexSP | 1662 | + | + |
| Hex6HexNAc3SP and/or NeuAc2Hex2HexNAc3dHex | 1678 | + | + |
| NeuAc2Hex3HexNAc3 | 1694 | + | + |
| NeuAcHex3HexNAc3dHexSP2 | 1709 | + | |
| NeuAcHex4HexNAc3dHex | 1711 | + | + |
| NeuAcHex5HexNAc3 and/or NeuGcHex4HexNAc3dHex | 1727 | + | + |
| NeuGcHex5HexNAc3 | 1743 | + | + |
| NeuAcHex4HexNAc3dHexSP | 1791 | + | |
| Hex5HexNAc3dHex2SP | 1808 | + | |
| Hex6NexNAc3dHexSP | 1824 | + | + |
| NeuAc2Hex3HexNAc3dHex | 1840 | + | + |
| NeuAc2Hex4HexNAc3 | 1856 | + | |
| NeuAcHex4HexNAc3dHex2 | 1857 | + | |
| NeuAcHex5HexNAc3dHex and/or NeuGcHex4HexNAc3dHex2 | 1873 | + | + |
| NeuAcHex5HexNAc3SP2 | 1887 | + | |
| NeuAcHex6HexNAc3 | 1889 | + | + |
| Hex8HexNAc3SP and/or NeuAc2Hex4HexNAc3dHex | 2002 | + | + |
| NeuAcHex4HexNAc3dHex3 | 2003 | + | |
| NeuAc2Hex5HexNAc3 and/or NeuGcNeuAcHex4HexNAc3dHex | 2018 | + | + |
| NeuAcHex5HexNAc3dHex2 | 2019 | + | + |
| NeuGcNeuAcHex5HexNAc3 and/or NeuGc2Hex4HexNAc3dHex | 2034 | + | |
| NeuAcHex6HexNAc3dHex | 2035 | + | + |
| NeuGc2Hex5HexNAc3 | 2050 | + | |
| NeuAcHex7HexNAc3 | 2051 | + | + |
| NeuAc2Hex4HexNAc3dHexSP and/or Hex8HexNAc3SP2 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAc2Hex5HexNAc3dHex and/or Hex6HexNAc5SP2 | 2164 | + | + |
| NeuAcHex5HexNAc3dHex3 | 2165 | + | + |
| NeuAcHex8HexNAc3SP and/or NeuAc3Hex4HexNAc3dHex | 2293 | + | |
| NeuAc2Hex5HexNAc3dHex2 and/or NeuGcNeuAcHex4HexNAc3dHex3 | 2310 | + | |
| NeuAc3Hex5HexNAc3SP | 2389 | + | |
| NeuAc2Hex5HexNAc3dHex2SP | 2390 | + | + |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAcHex9HexNAc3dHex | 2521 | + | |

| **HexNAc≥4 and Hex≥3** (including complex-type N-glycans) Proposed composition | m/z | Human cells | Human cell line |
|---|---|---|---|
| Hex4HexNAc4SP | 1557 | + | + |
| NeuAcHex3HexNAc4 | 1606 | + | |
| Hex4HexNAc4SP2 | 1637 | + | |
| Hex4HexNAc4dHexSP | 1703 | + | + |
| Hex4HexNAc4SP3 and/or Hex7HexNAc2SP2 | 1717 | + | |
| Hex5HexNAc4SP | 1719 | + | + |
| NeuAcHex4HexNAc4 | 1768 | + | + |
| NeuGcHex4HexNac4 | 1784 | + | |
| Hex5HexNAc4SP2 and/or Hex8HexNAc2SP | 1799 | + | |
| NeuAcHex3HexNac5 | 1809 | + | |
| NeuGcHex3HexNAc5 | 1825 | + | + |
| Hex5HexNAc4dHexSP | 1865 | + | + |
| Hex6HexNAc4SP | 1881 | + | + |
| Hex4HexNAc5dHexSP | 1906 | + | |
| NeuAcHex4HexNAc4dHex | 1914 | + | + |
| NeuAcHex4HexNAc4SP2 | 1928 | + | |
| NeuAcHex5HexNAc4 | 1930 | + | + |
| NeuGcHex5HexNAc4 | 1946 | + | + |
| NeuAcHex4HexNAc5 | 1971 | + | |
| NeuAcHex5HexNAc4Ac | 1972 | + | |
| Hex5HexNAc5SP2 | 2002 | + | |
| NeuAcHex5HexNAc4SP | 2010 | + | + |
| Hex5HexNAc4dHex2SP | 2011 | + | |
| NeuGcHex5HexNAc4SP | 2026 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| NeuAcHex4HexNAc5SP | 2051 | + | |
| Hex4HexNAc5dHex2SP | 2052 | + | |
| NeuAc2Hex4HexNAc4 | 2059 | + | |
| NeuAcHex4HexNAc4dHex2 | 2060 | + | + |
| NeuAcHex4HexNAc4dHexSP2 | 2074 | + | |
| NeuAcHex5HexNAc4dHex | 2076 | + | + |
| NeuAcHex6HexNAc4 and/or NeuGcHex5HexNAc4dHex | 2092 | + | + |
| NeuAcHex3HexNAc5dHex2 and/or NeuAc2Hex4HexNAc4Ac | 2101 | + | |
| NeuGcHex6HexNAc4 | 2108 | + | |
| NeuAcHex4HexNAc5dHex | 2117 | + | |
| Hex4HexNAc5dHex2SP2 | 2132 | + | |
| NeuAcHex5HexNAc5 | 2133 | + | + |
| NeuAc2Hex4HexNAc4SP | 2139 | + | |
| NeuAcHex5HexNAc4dHexSP | 2156 | + | + |
| Hex5HexNAc4dHex3SP | 2157 | + | |
| Hex6HexNAc5SP2 | 2164 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAcHex4HexNAc6 | 2174 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc4dHex | 2205 | + | |
| NeuAc2Hex4HexNAc4SP2 | 2219 | + | |
| NeuAc2Hex5HexNAc4 | 2221 | + | + |
| NeuAcHex5HexNAc4dHex2 | 2222 | + | + |
| Hex6HexNAc5dHexSP | 2230 | + | |
| NeuGcNeuAcHex5HexNAc4 | 2237 | + | |
| NeuAcHex6HexNAc4dHex and/or NeuGcHex5HexNAc4dHex2 | 2238 | + | + |
| NeuAc2Hex3HexNAc5dHex and/or | 2246 | + | |

| | | | |
|---|---|---|---|
| Hex7HexNAc5SP | | | |
| NeuGc2Hex5HexNAc4 | 2253 | + | |
| NeuAcHex7HexNAc4 and/or NeuGcHex6HexNAc4dHex | 2254 | + | + |
| NeuAc2Hex4HexNAc5 | 2262 | + | |
| NeuAcHex4HexNAc5dHex2 and/or NeuAc2Hex5HexNAc4Ac | 2263 | + | |
| Hex5HexNAc6dHexSP | 2271 | + | |
| NeuAcHex5HexNAc5dHex | 2279 | + | + |
| NeuAc2Hex4HexNAc4dHexSP and/or Hex11HexNAc2SP | 2285 | + | |
| NeuAcHex6HexNAc5 | 2295 | + | + |
| NeuAc2Hex5HexNAc4SP | 2301 | + | |
| NeuAcHex5HexNAc4dHex2SP | 2302 | + | |
| NeuAc2Hex5HexNAc4Ac2 | 2305 | + | |
| NeuAcHex6HexNAc4dHexSP | 2318 | + | + |
| Hex6HexNAc4dHex3SP and/or NeuGcNeuAcHex3HexNAc6 | 2319 | + | |
| NeuAcHex4HexNAc6dHex | 2320 | + | |
| NeuAcHex5HexNAc5dHexAc | 2321 | + | |
| Hex7HexNAc4dHex2SP and/or Hex4HexNAc6dHex2SP2 | 2335 | + | |
| NeuAcHex5HexNAc6 | 2336 | + | + |
| NeuAc3Hex4HexNac4 | 2350 | + | |
| NeuAc2Hex4HexNAc4dHexSP | 2365 | + | |
| NeuAc2Hex5HexNAc4dHex | 2367 | + | + |
| NeuAcHex5HexNAc4dHex3 | 2368 | + | + |
| NeuAc2Hex6HexNAc4 and/or NeuGcNeuAcHex5HexNAc4dHex | 2383 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuGc2Hex5HexNAc4dHex | 2399 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAc2Hex5HexNAc5 | 2424 | + | |
| NeuAcHex5HexNAc5dHex2 | 2425 | + | + |
| NeuAcHex6HexNAc5dHex | 2441 | + | + |
| NeuAc2Hex5HexNAc4dHexSP | 2447 | + | + |
| NeuAcHex5HexNAc4dHex3SP | 2448 | + | |
| NeuAcHex7HexNAc5 and/or NeuGcHex6HexNAc5dHex | 2457 | + | + |
| NeuGcHex7HexNAc5 | 2473 | + | |
| NeuAcHex5HexNAc6dHex | 2482 | + | |
| NeuAcHex4HexNAc5dHex3SP | 2489 | + | |
| Hex6HexNAc7SP | 2490 | + | |
| NeuAc3Hex5HexNAc4 | 2512 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuGcNeuAc2Hex5HexNAc4 | 2528 | + | |
| NeuAc2Hex6HexNAc4dHex and/or NeuGcNeuAcHex5HexNAc4dHex2 | 2529 | + | |
| NeuGc2NeuAcHex5HexNAc4 | 2544 | + | |
| NeuGc2Hex5HexNAc4dHex2 and/or NeuGcNeuAcHex6HexNAc4dHex | 2545 | + | |
| NeuGc3Hex5HexNAc4 | 2560 | + | |
| NeuGc2Hex6HexNAc4dHex | 2561 | + | |
| NeuAc2Hex5HexNAc5dHex | 2570 | + | + |
| NeuAcHex5HexNAc5dHex3 | 2571 | + | |
| NeuAc2Hex6HexNAc5 | 2586 | + | + |
| NeuAcHex6HexNAc5dHex2 | 2587 | + | + |
| Hex7HexNAc6dHexSP | 2595 | + | |
| NeuGcNeuAcHex6HexNAc5 | 2602 | + | |
| NeuAcHex7HexNAc5dHex and/or NeuGcHex6HexNAc5dHex2 | 2603 | + | + |
| NeuAcHex8HexNAc5 and/or NeuGcHex7HexNAc5dHex | 2619 | + | |
| NeuAc2Hex5HexNAc6 | 2627 | + | |
| NeuGcHex8HexNAc5 and/or NeuAcHex4HexNAc5dHex4SP | 2635 | + | |
| NeuAcHex6HexNAc6dHex | 2644 | + | + |
| NeuAc2Hex5HexNAc4dHex3 | 2659 | + | |
| NeuAcHex7HexNAc6 | 2660 | + | + |
| NeuGcNeuAc2Hex5HexNAc4dHex and/or NeuAc3Hex6HexNAc4 | 2674 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAc2Hex6HexNAc5dHex | 2732 | + | + |
| NeuAcHex6HexNAc5dHex3 | 2733 | + | + |
| NeuGcNeuAcHex6HexNAc5dHex | 2748 | + | |
| NeuAcHex8HexNAc5dHex | 2765 | + | + |
| NeuGcHex8HexNAc5dHex and/or NeuAcHex9HexNAc5 | 2781 | + | |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex7HexNAc6dHex | 2807 | + | + |
| NeuAc2Hex6HexNAc5dHexSP | 2812 | + | |
| NeuAcHex6HexNAc5dHex3SP | 2813 | + | |
| NeuGcNeuAc3Hex5HexNAc4 | 2819 | + | |
| NeuAc3Hex6HexNAc4dHex and/or NeuGcNeuAc2Hex5HexNAc4dHex2 | 2820 | + | |
| NeuAc3Hex6HexNAc5 | 2878 | + | + |
| NeuAc2Hex6HexNAc5dHex2 | 2879 | + | + |
| NeuAcHex6HexNAc5dHex4 | 2880 | + | + |
| NeuGcNeuAc2Hex6HexNAc5 | 2894 | + | |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuGc3Hex6HexNAc5 | 2925 | + | |
| NeuGcNeuAc2Hex5HexNAc6 | 2935 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAc2Hex7HexNAc6 | 2952 | + | |
| NeuAcHex7HexNAc6dHex2 | 2953 | + | + |
| Hex8HexNAc7dHexSP | 2961 | + | |
| NeuAc2Hex4HexNAc7dHex2 | 2961 | + | |
| NeuAcHex7HexNAc7dHex | 3010 | + | |
| NeuAc3Hex6HexNAc5dHex | 3024 | + | + |
| NeuAc2Hex6HexNAc5dHex3 | 3025 | + | + |
| NeuAcHex8HexNAc7 | 3026 | + | |
| NeuGc3Hex6HexNAc5dHex and/or NeuGc2NeuAcHex7HexNAc5 | 3072 | + | |
| NeuAc3Hex6HexNAc6 | 3081 | + | + |
| NeuAc2Hex6HexNAc6dHex2 | 3082 | + | |
| NeuAc2Hex7HexNAc6dHex | 3098 | + | + |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAcHex8HexNAc7dHex | 3172 | + | |
| NeuAc3Hex6HexNAc6dHex | 3227 | + | |
| NeuAc2Hex6HexNAc6dHex3 | 3228 | + | |
| NeuAc3Hex7HexNAc6 | 3243 | + | |
| NeuAc2Hex7HexNAc6dHex2 | 3244 | + | + |
| NeuAcHex7HexNAc6dHex4 | 3245 | + | + |
| NeuAc2Hex7HexNAc7dHex | 3301 | + | |
| NeuAcHex7HexNAc7dHex3 | 3302 | + | |
| NeuAc2Hex8HexNAc7 | 3317 | + | |
| NeuAcHex8HexNAc7dHex2 | 3318 | + | |
| NeuAc3Hex7HexNAc6dHex | 3389 | + | + |
| NeuAc2Hex7HexNAc6dHex3 | 3390 | + | + |
| NeuAcHex7HexNAc6dHex5 and/or NeuAcHex9HexNAc8 | 3391 | + | |

| | | | |
|---|---|---|---|
| NeuAc2Hex8HexNAc7dHex | 3463 | + | |
| NeuAcHex8HexNAc7dHex3 | 3464 | + | |
| NeuAc2Hex7HexNAc6dHex4 | 3536 | + | |
| NeuAcHex9HexNAc8dHex | 3537 | + | |
| NeuAc3Hex8HexNAc7 | 3608 | + | |
| NeuAc2Hex8HexNac7dHex2 | 3609 | + | |
| NeuAcHex8HexNac7dHex4 | 3610 | + | |
| NeuAc4Hex7HexNAc6dHex | 3680 | + | |
| NeuAc3Hex7HexNAc6dHex3 | 3681 | + | |
| NeuAc2Hex9HexNAc8 | 3682 | + | |
| NeuAcHex9HexNAc8dHex2 | 3683 | + | |
| NeuAc3Hex8HexNAc7dHex | 3754 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc4Hex6HexNAc8 | 3778 | + | |
| NeuAc3Hex7HexNAc6dHex4 | 3827 | + | |
| NeuAc2Hex9HexNAc8dHex | 3828 | + | |
| NeuAcHex9HexNAc8dHex3 | 3829 | + | |
| NeuAc2Hex8HexNAc7dHex4 | 3901 | + | |
| NeuAc2Hex9HexNAc8dHex2 | 3974 | + | |
| NeuAcHex9HexNAc8dHex4 | 3975 | + | |
| NeuAc4Hex8HexNAc7dHex | 4045 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc3Hex9HexNAc8dHex | 4119 | + | |
| NeuAc2Hex9HexNAc8dHex3 | 4120 | + | |

| **HexNAc≥3 and dHex≥1** (including fucosylated N-glycans) **Proposed composition** | **m/z** | **Human cells** | **Human cell line** |
|---|---|---|---|
| Hex3HexNAc3dHexSP | 1338 | + | + |
| Hex4HexNAc3dHexSP | 1500 | + | + |
| NeuAcHex3HexNAc3dHex | 1549 | + | + |
| Hex4HexNAc3dHex2SP | 1646 | + | |
| Hex5HexNAc3dHexSP | 1662 | + | + |
| Hex6HexNAc3SP and/or NeuAc2Hex2HexNAc3dHex | 1678 | + | + |
| NeuAcHex3HexNAc3dHexSP2 | 1709 | + | |
| NeuAcHex4HexNAc3dHex | 1711 | + | + |
| NeuAcHex5HexNAc3 and/or NeuGcHex4HexNAc3dHex | 1727 | + | + |
| NeuAcHex4HexNAc3dHexSP | 1791 | + | |
| Hex5HexNAc3dHex2SP | 1808 | + | |
| Hex6NexNAc3dHexSP | 1824 | + | + |
| NeuAc2Hex3HexNAc3dHex | 1840 | + | + |
| NeuAcHex4HexNAc3dHex2 | 1857 | + | |
| NeuAcHex5HexNAc3dHex and/or NeuGcHex4HexNAc3dHex2 | 1873 | + | + |
| Hex8HexNAc3SP and/or NeuAc2Hex4HexNAc3dHex | 2002 | + | + |
| NeuAcHex4HexNAc3dHex3 | 2003 | + | |
| NeuAc2Hex5HexNAc3 and/or NeuGcNeuAcHex4HexNAc3dHex | 2018 | + | + |
| NeuAcHex5HexNAc3dHex2 | 2019 | + | + |
| NeuGcNeuAcHex5HexNAc3 and/or NeuGc2Hex4HexNAc3dHex | 2034 | + | |
| NeuAcHex6HexNAc3dHex | 2035 | + | + |
| NeuAc2Hex4HexNAc3dHexSP and/or Hex8HexNAc3SP2 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAc2Hex5HexNAc3dHex and/or Hex6HexNAc5SP2 | 2164 | + | + |
| NeuAcHex5HexNAc3dHex3 | 2165 | + | + |
| NeuAcHex8HexNAc3SP and/or NeuAc3Hex4HexNAc3dHex | 2293 | + | |
| NeuAc2Hex5HexNAc3dHex2 and/or NeuGcNeuAcHex4HexNAc3dHex3 | 2310 | + | |
| NeuAc2Hex5HexNAc3dHex2SP | 2390 | + | + |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAcHex9HexNAc3dHex | 2521 | + | |
| Hex4HexNAc4dHexSP | 1703 | + | + |
| Hex5HexNAc4dHexSP | 1865 | + | + |
| Hex4HexNAc5dHexSP | 1906 | + | |
| NeuAcHex4HexNAc4dHex | 1914 | + | + |
| Hex5HexNAc4dHex2SP | 2011 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| Hex4HexNAc5dHex2SP | 2052 | + | |
| NeuAcHex4HexNAc4dHex2 | 2060 | + | + |
| NeuAcHex4HexNAc4dHexSP2 | 2074 | + | |
| NeuAcHex5HexNAc4dHex | 2076 | + | + |
| NeuAcHex6HexNAc4 and/or NeuGcHex5HexNAc4dHex | 2092 | + | + |
| NeuAcHex3HexNAc5dHex2 and/or NeuAc2Hex4HexNAc4Ac | 2101 | + | |
| NeuAcHex4HexNAc5dHex | 2117 | + | |
| Hex4HexNAc5dHex2SP2 | 2132 | + | |
| NeuAcHex5HexNAc4dHexSP | 2156 | + | + |
| Hex5HexNAc4dHex3SP | 2157 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAc3Hex3HexNAc4 and/or NeuGcHex6HexNAc4SP and/or NeuAc2NeuGcHex2HexNAc4dHex | 2188 | + | |

| | | | |
|---|---|---|---|
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc4dHex | 2205 | + | |
| NeuAcHex5HexNAc4dHex2 | 2222 | + | + |
| Hex6HexNAc5dHexSP | 2230 | + | |
| NeuAcHex6HexNAc4dHex and/or NeuGcHex5HexNAc4dHex2 | 2238 | + | + |
| NeuAc2Hex3HexNAc5dHex and/or Hex7HexNAc5SP | 2246 | + | |
| NeuAcHex7HexNAc4 and/or NeuGcHex6HexNAc4dHex | 2254 | + | + |
| NeuAcHex4HexNAc5dHex2 and/or NeuAc2Hex5HexNAc4Ac | 2263 | + | |
| Hex5HexNAc6dHexSP | 2271 | + | |
| NeuAcHex5HexNAc5dHex | 2279 | + | + |
| NeuAc2Hex4HexNAc4dHexSP and/or Hex11HexNAc2SP | 2285 | + | |
| NeuAcHex5HexNAc4dHex2SP | 2302 | + | |
| NeuAcHex6HexNAc4dHexSP | 2318 | + | + |
| Hex6HexNAc4dHex3SP and/or NeuGcNeuAcHex3HexNAc6 | 2319 | + | |
| NeuAcHex4HexNAc6dHex | 2320 | + | |
| NeuAcHex5HexNAc5dHexAc | 2321 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAc2Hex5HexNAc4dHex | 2367 | + | + |
| NeuAcHex5HexNAc4dHex3 | 2368 | + | + |
| NeuAc2Hex6HexNAc4 and/or NeuGcNeuAcHex5HexNAc4dHex | 2383 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuGc2Hex5HexNAc4dHex | 2399 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAcHex5HexNAc5dHex2 | 2425 | + | + |
| NeuAcHex6HexNAc5dHex | 2441 | + | + |
| NeuAc2Hex5HexNAc4dHexSP | 2447 | + | + |
| NeuAcHex5HexNAc4dHex3SP | 2448 | + | |
| NeuAcHex7HexNAc5 and/or NeuGcHex6HexNAc5dHex | 2457 | + | + |
| NeuAcHex5HexNAc6dHex | 2482 | + | |
| NeuAcHex4HexNAc5dHex3SP | 2489 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuAc2Hex6HexNAc4dHex and/or NeuGcNeuAcHex5HexNAc4dHex2 | 2529 | + | |
| NeuGc2Hex5HexNAc4dHex2 and/or NeuGcNeuAcHex6HexNAc4dHex | 2545 | + | |
| NeuGc2Hex6HexNAc4dHex | 2561 | + | |
| NeuAc2Hex5HexNAc5dHex | 2570 | + | + |
| NeuAcHex5HexNAc5dHex3 | 2571 | + | |
| NeuAcHex6HexNAc5dHex2 | 2587 | + | + |
| Hex7HexNAc6dHexSP | 2595 | + | |
| NeuAcHex7HexNAc5dHex and/or NeuGcHex6HexNAc5dHex2 | 2603 | + | + |
| NeuAcHex8HexNAc5 and/or NeuGcHex7HexNAc5dHex | 2619 | + | |
| NeuGcHex8HexNAc5 and/or NeuAcHex4HexNAc5dHex4SP | 2635 | + | |
| NeuAcHex6HexNAc6dHex | 2644 | + | + |
| NeuAc2Hex5HexNAc4dHex3 | 2659 | + | |
| NeuGcNeuAc2Hex5HexNAc4dHex | 2674 | + | |
| and/or NeuAc3Hex6HexNAc4 | | | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAc2Hex6HexNAc5dHex | 2732 | + | + |
| NeuAcHex6HexNAc5dHex3 | 2733 | + | + |
| NeuGcNeuAcHex6HexNAc5dHex | 2748 | + | |
| NeuAcHex8HexNAc5dHex | 2765 | + | + |
| NeuGcHex8HexNAc5dHex and/or NeuAcHex9HexNAc5 | 2781 | + | |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex7HexNAc6dHex | 2807 | + | + |
| NeuAc2Hex6HexNAc5dHexSP | 2812 | + | |
| NeuAcHex6HexNAc5dHex3SP | 2813 | + | |
| NeuAc3Hex6HexNAc4dHex and/or NeuGcNeuAc2Hex5HexNAc4dHex2 | 2820 | + | |
| NeuAc2Hex6HexNAc5dHex2 | 2879 | + | + |
| NeuAcHex6HexNAc5dHex4 | 2880 | + | + |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAcHex7HexNAc6dHex2 | 2953 | + | + |
| Hex8HexNAc7dHexSP | 2961 | + | |
| NeuAc2Hex4HexNAc7dHex2 | 2961 | + | |
| NeuAcHex7HexNAc7dHex | 3010 | + | |
| NeuAc3Hex6HexNAc5dHex | 3024 | + | + |
| NeuAc2Hex6HexNAc5dHex3 | 3025 | + | + |
| NeuGc3Hex6HexNAc5dHex and/or NeuGc2NeuAcHex7HexNAc5 | 3072 | + | |
| NeuAc2Hex6HexNAc6dHex2 | 3082 | + | |
| NeuAc2Hex7HexNAc6dHex | 3098 | + | + |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAcHex8HexNAc7dHex | 3172 | + | |
| NeuAc3Hex6HexNAc6dHex | 3227 | + | |
| NeuAc2Hex6HexNAc6dHex3 | 3228 | + | |
| NeuAc2Hex7HexNAc6dHex2 | 3244 | + | + |
| NeuAcHex7HexNAc6dHex4 | 3245 | + | + |
| NeuAc2Hex7HexNAc7dHex | 3301 | + | |
| NeuAcHex7HexNAc7dHex3 | 3302 | + | |
| NeuAcHex8HexNAc7dHex2 | 3318 | + | |
| NeuAc3Hex7HexNAc6dHex | 3389 | + | + |
| NeuAc2Hex7HexNAc6dHex3 | 3390 | + | + |
| NeuAcHex7HexNAc6dHex5 and/or NeuAcHex9HexNAc8 | 3391 | + | |
| NeuAc2Hex8HexNAc7dHex | 3463 | + | |
| NeuAcHex8HexNAc7dHex3 | 3464 | + | |
| NeuAc2Hex7HexNAc6dHex4 | 3536 | + | |
| NeuAcHex9HexNAc8dHex | 3537 | + | |
| NeuAc2Hex8HexNac7dHex2 | 3609 | + | |
| NeuAcHex8HexNac7dHex4 | 3610 | + | |
| NeuAc4Hex7HexNAc6dHex | 3680 | + | |
| NeuAc3Hex7HexNAc6dHex3 | 3681 | + | |
| NeuAcHex9HexNAc8dHex2 | 3683 | + | |
| NeuAc3Hex8HexNAc7dHex | 3754 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc3Hex7HexNAc6dHex4 | 3827 | + | |
| NeuAc2Hex9HexNAc8dHex | 3828 | + | |
| NeuAcHex9HexNAc8dHex3 | 3829 | + | |
| NeuAc2Hex8HexNAc7dHex4 | 3901 | + | |

| | | | |
|---|---|---|---|
| NeuAc2Hex9HexNAc8dHex2 | 3974 | + | |
| NeuAcHex9HexNAc8dHex4 | 3975 | + | |
| NeuAc4Hex8HexNAc7dHex | 4045 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc3Hex9HexNAc8dHex | 4119 | + | |
| NeuAc2Hex9HexNAc8dHex3 | 4120 | + | |

| **HexNAc≥3 and dHex≥2** (including multifucosylated N-glycans) **Proposed composition** | **m/z** | **Human cells** | **Human cell line** |
|---|---|---|---|
| Hex4HexNAc3dHex2SP | 1646 | + | |
| NeuAcHex3HexNAc3dHexSP2 | 1709 | + | |
| Hex5HexNAc3dHex2SP | 1808 | + | |
| NeuAcHex4HexNAc3dHex2 | 1857 | + | |
| NeuAcHex5HexNAc3dHex and/or NeuGcHex4HexNAc3dHex2 | 1873 | + | + |
| NeuAcHex4HexNAc3dHex3 | 2003 | + | |
| NeuAcHex5HexNAc3dHex2 | 2019 | + | + |
| Hex8HexNAc3dHexSP and/or NeuAc2Hex4HexNAc3dHex2 | 2148 | + | |
| NeuAcHex5HexNAc3dHex3 | 2165 | + | + |
| NeuAc2Hex5HexNAc3dHex2 and/or NeuGcNeuAcHex4HexNAc3dHex3 | 2310 | + | |
| NeuAc2Hex5HexNAc3dHex2SP | 2390 | + | + |
| NeuAcHex8HexNAc3dHexSP and/or NeuAc3Hex4HexNAc3dHex2 | 2439 | + | |
| Hex5HexNAc4dHex2SP | 2011 | + | |
| Hex4HexNAc5dHex2SP | 2052 | + | |
| NeuAcHex4HexNAc4dHex2 | 2060 | + | + |
| NeuAcHex3HexNAc5dHex2 and/or NeuAc2Hex4HexNAc4Ac | 2101 | + | |
| Hex4HexNAc5dHex2SP2 | 2132 | + | |
| Hex5HexNAc4dHex3SP | 2157 | + | |
| NeuAcHex6HexNAc4SP and/or NeuGcHex5HexNAc4dHexSP | 2172 | + | + |
| Hex6HexNAc4dHex2SP and/or Hex3HexNAc6dHex2SP2 | 2173 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAcHex5HexNAc4dHex2 | 2222 | + | + |
| NeuAcHex6HexNAc4dHex and/or NeuGcHex5HexNAc4dHex2 | 2238 | + | + |
| NeuAcHex4HexNAc5dHex2 and/or NeuAc2Hex5HexNAc4Ac | 2263 | + | |
| NeuAc2Hex4HexNAc4dHexSP and/or Hex11HexNAc2SP | 2285 | + | |
| NeuAcHex5HexNAc4dHex2SP | 2302 | + | |
| Hex6HexNAc4dHex3SP and/or NeuGcNeuAcHex3HexNAc6 | 2319 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAcHex5HexNAc4dHex3 | 2368 | + | + |
| NeuAcHex6HexNAc4dHex2 and/or NeuGcHex5HexNAc4dHex3 | 2384 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAcHex4HexNAc5dHex3 and/or NeuAc2Hex5HexNAc4dHexAc | 2409 | + | |
| NeuAcHex5HexNAc5dHex2 | 2425 | + | + |
| NeuAcHex5HexNAc4dHex3SP | 2448 | + | |
| NeuAcHex4HexNAc5dHex3SP | 2489 | + | |
| NeuAc2Hex5HexNAc4dHex2 | 2513 | + | + |
| NeuAcHex5HexNAc4dHex4 | 2514 | + | |
| NeuAcHex6HexNAc5dHexSP and/or NeuAc3Hex2HexNAc5dHex2 | 2521 | + | |
| Hex6HexNAc5dHex3SP | 2522 | + | |
| NeuAc2Hex6HexNAc4dHex and/or NeuGcNeuAcHex5HexNAc4dHex2 | 2529 | + | |
| NeuGc2Hex5HexNAc4dHex2 and/or NeuGcNeuAcHex6HexNAc4dHex | 2545 | + | |
| NeuAcHex5HexNAc5dHex3 | 2571 | + | |
| NeuAcHex6HexNAc5dHex2 | 2587 | + | + |
| NeuAcHex7HexNAc5dHex and/or NeuGcHex6HexNAc5dHex2 | 2603 | + | + |
| NeuGcHex8HexNAc5 and/or NeuAcHex4HexNAc5dHex4SP | 2635 | + | |
| NeuAc2Hex5HexNAc4dHex3 | 2659 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAcHex6HexNAc5dHex3 | 2733 | + | + |
| NeuAcHex6HexNAc6dHex2 | 2791 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAcHex6HexNAc5dHex3SP | 2813 | + | |
| NeuAc3Hex6HexNAc4dHex and/or NeuGcNeuAc2Hex5HexNAc4dHex2 | 2820 | + | |
| NeuAc2Hex6HexNAc5dHex2 | 2879 | + | + |
| NeuAcHex6HexNAc5dHex4 | 2880 | + | + |
| NeuAc2Hex7HexNAc5dHex and/or NeuGcNeuAcHex6HexNAc5dHex2 | 2895 | + | + |
| NeuAc3Hex6HexNAc4dHexSP and/or NeuGcNeuAc2Hex5HexNAc4dHex2SP | 2900 | + | |
| NeuGc2Hex6HexNAc5dHex2 | 2911 | + | |
| NeuAc2Hex5HexNAc6dHex2 | 2920 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuGc2NeuAcHex5HexNAc6 and/or NeuAc3Hex5HexNAc4dHex3 | 2951 | + | |
| NeuAcHex7HexNAc6dHex2 | 2953 | + | + |
| NeuAc2Hex4HexNAc7dHex2 | 2961 | + | |
| NeuAc2Hex6HexNAc5dHex3 | 3025 | + | + |
| NeuAc2Hex6HexNAc6dHex2 | 3082 | + | |
| NeuAcHex7HexNAc6dHex3 | 3099 | + | + |
| NeuAc3Hex6HexNAc5dHexSP | 3104 | + | |
| NeuAc2Hex6HexNAc5dHex3SP | 3105 | + | |
| NeuAc3Hex6HexNAc5dHex2 | 3170 | + | |
| NeuAc2Hex6HexNAc5dHex4 | 3171 | + | |
| NeuAc2Hex6HexNAc6dHex3 | 3228 | + | |
| NeuAc2Hex7HexNAc6dHex2 | 3244 | + | + |
| NeuAcHex7HexNAc6dHex4 | 3245 | + | + |
| NeuAcHex7HexNAc7dHex3 | 3302 | + | |
| NeuAcHex8HexNAc7dHex2 | 3318 | + | |
| NeuAc2Hex7HexNAc6dHex3 | 3390 | + | + |
| NeuAcHex7HexNAc6dHex5 and/or NeuAcHex9HexNAc8 | 3391 | + | |
| NeuAcHex8HexNAc7dHex3 | 3464 | + | |
| NeuAc2Hex7HexNAc6dHex4 | 3536 | + | |
| NeuAc2Hex8HexNac7dHex2 | 3609 | + | |
| NeuAcHex8HexNac7dHex4 | 3610 | + | |
| NeuAc3Hex7HexNAc6dHex3 | 3681 | + | |
| NeuAcHex9HexNAc8dHex2 | 3683 | + | |
| NeuAc2Hex8HexNAc7dHex3 | 3755 | + | |
| NeuAcHex10HexNAc9 and/or NeuAcHex8HexNAc7dHex5 | 3756 | + | |
| NeuAc3Hex7HexNAc6dHex4 | 3827 | + | |
| NeuAcHex9HexNAc8dHex3 | 3829 | + | |
| NeuAc2Hex8HexNAc7dHex4 | 3901 | + | |
| NeuAc2Hex9HexNAc8dHex2 | 3974 | + | |
| NeuAcHex9HexNAc8dHex4 | 3975 | + | |
| NeuAc3Hex8HexNAc7dHex3 | 4046 | + | |
| NeuAc2Hex10HexNAc9 and/or NeuAc2Hex8HexNAc7dHex5 | 4047 | + | |
| NeuAc2Hex9HexNAc8dHex3 | 4120 | + | |

| **HexNAc>Hex≥3** (terminal HexNAc, N>H) Proposed composition | m/z | Human cells | Human cell line |
|---|---|---|---|
| Hex6HexNAc3SP and/or NeuAc2Hex2HexNAc3dHex | 1678 | + | + |
| NeuAcHex3HexNAc4 | 1606 | + | |
| NeuAcHex3HexNac5 | 1809 | + | |
| NeuGcHex3HexNAc5 | 1825 | + | + |
| Hex4HexNAc5dHexSP | 1906 | + | |
| NeuAcHex4HexNAc5 | 1971 | + | |
| Hex7HexNAc4SP and/or Hex4HexNAc6SP2 and/or NeuAc2Hex3HexNAc4dHex | 2043 | + | |
| NeuAcHex4HexNAc5SP | 2051 | + | |
| Hex4HexNAc5dHex2SP | 2052 | + | |
| NeuAcHex3HexNAc5dHex2 and/or NeuAc2Hex4HexNAc4Ac | 2101 | + | |
| NeuAcHex4HexNAc5dHex | 2117 | + | |
| Hex4HexNAc5dHex2SP2 | 2132 | + | |
| Hex6HexNAc4dHex2SP and/or Hex3HexNAc6dHex2SP2 | 2173 | + | |
| NeuAcHex4HexNAc6 | 2174 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| NeuAcHex3HexNAc4dHex4 | 2190 | + | + |
| Hex4HexNAc5dHex3SP | 2198 | + | + |
| NeuAc2Hex4HexNAc5 | 2262 | + | |
| NeuAcHex4HexNAc5dHex2 and/or NeuAc2Hex5HexNAc4Ac | 2263 | + | |
| Hex5HexNAc6dHexSP | 2271 | + | |
| NeuAcHex4HexNAc6dHex | 2320 | + | |
| Hex7HexNAc4dHex2SP and/or Hex4HexNAc6dHex2SP2 | 2335 | + | |
| NeuAcHex5HexNAc6 | 2336 | + | + |
| NeuAc2Hex3HexNAc5dHex2 and/or Hex7HexNAc5dHexSP | 2392 | + | + |
| NeuAcHex3HexNAc5dHex4 | 2393 | + | |
| NeuAcHex4HexNAc6dHexSP and/or NeuGcHex6HexNAc4dHex2 and/or NeuAcHex7HexNAc4dHex | 2400 | + | |
| Hex4HexNAc6dHex3SP | 2401 | + | |
| NeuAc2Hex4HexNAc5dHex | 2408 | + | |
| NeuAcHex4HexNAc5dHex3 and/or NeuAc2Hex5HexNAc4dHexAc | 2409 | + | |
| NeuAcHex5HexNAc6dHex | 2482 | + | |
| NeuAcHex4HexNAc5dHex3SP | 2489 | + | |
| Hex6HexNAc7SP | 2490 | + | |
| NeuGc3Hex5HexNAc4 | 2560 | + | |
| NeuAc2Hex5HexNAc6 | 2627 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | + |
| NeuAc2Hex5HexNAc6dHex2 | 2920 | + | |
| NeuGcNeuAc2Hex5HexNAc6 | 2935 | + | |
| NeuAc2Hex4HexNAc7dHex2 | 2961 | + | |
| NeuAc4Hex6HexNAc8 | 3778 | + | |

| **HexNAc=Hex≥5** (terminal HexNAc, N=H) Proposed composition | m/z | Human cells | Human cell line |
|---|---|---|---|
| Hex5HexNAc5SP2 | 2002 | + | |
| NeuAcHex5HexNAc5 | 2133 | + | + |
| NeuAcHex5HexNAc5dHex | 2279 | + | + |
| NeuAcHex5HexNAc5dHexAc | 2321 | + | |
| NeuAc2Hex5HexNAc5 | 2424 | + | |
| NeuAcHex5HexNAc5dHex2 | 2425 | + | + |
| NeuAc2Hex5HexNAc5dHex | 2570 | + | + |
| NeuAcHex5HexNAc5dHex3 | 2571 | + | |
| NeuAcHex6HexNAc6dHex | 2644 | + | + |
| NeuAcHex4HexNAc5dHex4SP2 and/or NeuAc3Hex5HexNAc5 | 2715 | + | + |
| NeuAc2Hex5HexNAc5dHex2 | 2716 | + | |
| NeuAcHex6HexNAc6dHex2 | 2791 | + | |
| Hex6HexNAc6dHex3SP2 | 2805 | + | |
| NeuAc2Hex6HexNAc6dHex and/or NeuGcNeuAcHex5HexNAc6dHex2 | 2936 | + | |
| NeuAcHex6HexNAc6dHex3 | 2937 | + | |
| NeuAcHex7HexNAc7dHex | 3010 | + | |
| NeuAc3Hex6HexNAc6 | 3081 | + | + |
| NeuAc2Hex6HexNAc6dHex2 | 3082 | + | |
| NeuAc3Hex6HexNAc6dHex | 3227 | + | |
| NeuAc2Hex6HexNAc6dHex3 | 3228 | + | |
| NeuAc2Hex7HexNAc7dHex | 3301 | + | |
| NeuAcHex7HexNAc7dHex3 | 3302 | + | |

| SP≥1 (including sulphated and/or phosphorylated glycans) Proposed composition | m/z | Human cells | Human cell line |
|---|---|---|---|
| Hex3HexNAc2SP | 989 | + | |
| Hex3HexNAc2dHexSP | 1135 | + | |
| Hex4HexNAc2SP | 1151 | + | |
| Hex3HexNAc3SP | 1192 | + | + |
| Hex5HexNAc2SP | 1313 | + | |
| Hex3HexNAc3dHexSP | 1338 | + | + |
| Hex4HexNAc3SP | 1354 | + | + |
| Hex5HexNAc2dHexSP | 1459 | + | + |
| Hex6HexNAc2SP | 1475 | + | |
| Hex4HexNAc3dHexSP | 1500 | + | + |
| Hex5HexNAc3SP | 1516 | + | |
| Hex6HexNAc2SP2 | 1555 | + | |
| Hex4HexNAc4SP | 1557 | + | + |
| NeuAcHex3HexNAc3SP2 | 1563 | + | |
| Hex6HexNAc2dHexSP | 1621 | + | + |
| Hex4HexNAc4SP2 and/or Hex7HexNAc2SP | 1637 | + | |
| Hex4HexNAc3dHex2SP | 1646 | + | |
| Hex5HexNAc3dHexSP | 1662 | + | + |
| Hex6HexNAc3SP | 1678 | + | |
| Hex4HexNAc4dHexSP | 1703 | + | + |
| NeuAcHex3HexNAc3dHexSP2 | 1709 | + | |
| Hex4HexNAc4SP3 and/or Hex7HexNAc2SP2 | 1717 | + | |
| Hex5HexNAc4SP | 1719 | + | + |
| Hex7HexNAc2dHexSP | 1783 | + | |
| NeuAcHex4HexNAc3dHexSP | 1791 | + | |
| Hex5HexNAc4SP2 and/or Hex8HexNAc2SP | 1799 | + | |
| Hex5HexNAc3dHex2SP | 1808 | + | |
| NeuAc2Hex5HexNAc2 and/or NeuAc2Hex2HexNAc4SP | 1815 | + | |
| Hex6NexNAc3dHexSP | 1824 | + | + |
| Hex5HexNAc4dHexSP | 1865 | + | + |
| Hex6HexNAc4SP | 1881 | + | + |
| Hex4HexNAc5dHexSP | 1906 | + | |
| NeuAcHex6HexNAc2dHexSP and/or NeuAcHex3HexNAc4dHexSP2 | 1912 | + | |
| NeuAcHex4HexNAc4SP2 | 1928 | + | |
| Hex8HexNAc3SP and/or Hex5HexNAc5SP2 and/or NeuAc2Hex4HexNAc3dHex | 2002 | + | + |
| NeuAcHex5HexNAc4SP | 2010 | + | + |
| Hex5HexNAc4dHex2SP | 2011 | + | |
| NeuGcHex5HexNAc4SP | 2026 | + | |
| Hex6HexNAc4dHexSP | 2027 | + | + |
| Hex7HexNAc4SP and/or Hex4HexNAc6SP2 and/or NeuAc2Hex3HexNAc4dHex | 2043 | + | |
| NeuAcHex7HexNAc3 and/or NeuAcHex4HexNAc5SP | 2051 | + | + |
| Hex4HexNAc5dHex2SP | 2052 | + | |
| NeuAcHex4HexNAc4dHexSP2 | 2074 | + | |
| NeuAc2Hex4HexNAc3dHexSP and/or Hex8HexNAc3SP2 and/or Hex5HexNAc5SP3 | 2082 | + | |
| NeuAcHex6HexNAc3dHexSP | 2115 | + | |
| Hex8HexNAc3dHexSP and/or NeuAc2Hex4HexNAc3dHex2 | 2148 | + | |
| NeuAcHex5HexNAc4dHexSP and/or NeuAcHex8HexNAc2dHex | 2156 | + | + |
| Hex5HexNAc4dHex3SP | 2157 | + | |
| NeuAc2Hex5HexNAc3dHex and/or Hex6HexNAc5SP2 | 2164 | + | + |
| NeuAcHex6HexNAc4SP and/or NeuGcHex5HexNAc4dHexSP and/or NeuAcHex9HexNAc2 | 2172 | + | + |
| Hex6HexNAc4dHex2SP and/or Hex3HexNAc6dHex2SP2 | 2173 | + | |
| NeuAc3Hex3HexNAc4 and/or NeuGcHex6HexNAc4SP and/or NeuAc2NeuGcHex2HexNAc4dHex | 2188 | + | |
| NeuAc2Hex3HexNAc4dHex2 and/or Hex7HexNAc4dHexSP and/or Hex4HexNAc6dHexSP2 | 2189 | + | + |
| Hex4HexNAc5dHex3SP | 2198 | + | + |
| NeuAc2Hex4HexNAc4SP2 | 2219 | + | |
| Hex6HexNAc5dHexSP | 2230 | + | |
| NeuAc2Hex3HexNAc5dHex and/or Hex7HexNAc5SP | 2246 | + | |
| NeuAc2Hex4HexNAc4dHexSP and/or Hex11HexNAc2SP | 2285 | + | |
| NeuAcHex8HexNAc3SP and/or NeuAc3Hex4HexNAc3dHex | 2293 | + | |
| NeuAc2Hex5HexNAc4SP | 2301 | + | |
| NeuAcHex5HexNAc4dHex2SP | 2302 | + | |
| NeuAcHex6HexNAc4dHexSP | 2318 | + | + |
| Hex6HexNAc4dHex3SP | 2319 | + | |
| Hex7HexNAc4dHex2SP and/or Hex4HexNAc6dHex2SP2 | 2335 | + | |
| NeuAc2Hex4HexNAc4dHexSP | 2365 | + | |
| NeuAc3Hex5HexNAc3SP and/or NeuAc2Hex5HexNAc4Ac4 | 2389 | + | |
| NeuAc2Hex5HexNAc3dHex2SP | 2390 | + | + |
| NeuAc2Hex3HexNAc5dHex2 and/or Hex7HexNAc5dHexSP | 2392 | + | + |
| NeuAcHex4HexNAc6dHexSP and/or NeuGcHex6HexNAc4dHex2 and/or NeuAcHex7HexNAc4dHex | 2400 | + | |
| NeuAc2Hex6HexNAc3dHexSP | 2406 | + | |
| NeuAcHex8HexNAc3dHexSP and/or NeuAc3Hex4HexNAc3dHex2 | 2439 | + | |
| NeuAc2Hex5HexNAc4dHexSP and/or NeuAc2Hex8HexNAc2dHex and/or Hex12HexNAc2SP | 2447 | + | + |
| NeuAcHex5HexNAc4dHex3SP and/or NeuAcHex8HexNAc2dHex3 | 2448 | + | |
| NeuAcHex7HexNAc3dHex3 and/or NeuAcHex4HexNAc5dHex3SP | 2489 | + | |
| Hex6HexNAc7SP | 2490 | + | |
| NeuAcHex6HexNAc5dHexSP and/or NeuAcHex9HexNAc3dHex and/or NeuAc3Hex2HexNAc5dHex2 | 2521 | + | |
| Hex6HexNAc5dHex3SP | 2522 | + | |
| Hex7HexNAc6dHexSP | 2595 | + | |
| NeuGcHex8HexNAc5 and/or NeuAcHex4HexNAc5dHex4SP | 2635 | + | |
| NeuAc2Hex5HexNAc5dHexSP | 2650 | + | |
| Hex7HexNAc7SP | 2652 | + | |
| Hex6HexNAc5dHex4SP | 2668 | + | |
| NeuGcHex6HexNAc5dHexSP and/or NeuAcHex7HexNAc5dHexSP | 2683 | + | |
| NeuAc2Hex4HexNAc5dHex2SP2 | 2714 | + | |
| NeuAcHex4HexNAc5dHex4SP2 and/or NeuAc3Hex5HexNAc5 | 2715 | + | |
| Hex6HexNAc6dHex3SP | 2725 | + | |
| Hex7HexNAc6dHex2SP | 2741 | + | |
| NeuAcHex6HexNAc5dHex2SP2 | 2747 | + | |
| NeuAc2Hex4HexNAc6dHex2 and/or Hex8HexNAc6dHexSP | 2757 | + | |
| Hex7HexNAc7dHexSP | 2798 | + | |
| NeuAc3Hex5HexNAc4dHex2 and/or NeuAcHex6HexNAc6dHexSP2 | 2804 | + | |
| Hex6HexNAc6dHex3SP2 | 2805 | + | |
| NeuAc2Hex6HexNAc5dHexSP | 2812 | + | |
| NeuAcHex6HexNAc5dHex3SP | 2813 | + | |
| Hex8HexNAc7SP | 2814 | + | |
| Hex6HexNAc6dHex4SP | 2871 | + | |
| NeuAcHex7HexNAc6dHexSP and/or NeuAcHex10HexNAc4dHex | 2887 | + | |
| Hex7HexNAc6dHex3SP | 2887 | + | |
| NeuAc3Hex6HexNAc4dHexSP and/or NeuGcNeuAc2Hex5HexNAc4dHex2SP | 2900 | + | |
| NeuAc3Hex4HexNAc6dHex and/or NeuAcHex8HexNAc6SP | 2903 | + | |
| Hex7HexNAc7dHex2SP | 2945 | + | |
| NeuAc2Hex6HexNAc5dHex2SP | 2958 | + | |
| NeuAcHex6HexNAc5dHex4SP | 2960 | + | |

| | | | |
|---|---|---|---|
| Hex8HexNAc7dHexSP | 2961 | + | |
| Hex8HexNAc8SP | 3018 | + | |
| Hex7HexNAc6dHex4SP | 3034 | + | |
| Hex7HexNAc7dHex3SP | 3091 | + | |
| NeuAc3Hex6HexNAc5dHexSP | 3104 | + | |
| NeuAc2Hex6HexNAc5dHex3SP | 3105 | + | |
| NeuAcHex8HexNAc7SP and/or NeuAc3Hex4HexNAc7dHex | 3106 | + | |
| Hex8HexNAc7dHex2SP and/or NeuAc2Hex4HexNAc7dHex3 | 3107 | + | |
| NeuAc2Hex7HexNAc6dHexSP | 3178 | + | |
| Hex7HexNAc7dHex4SP | 3237 | + | |
| NeuAc3Hex7HexNAc5dHexSP and/or NeuGcNeuAc2Hex6HexNAc5dHex2SP | 3266 | + | |
| NeuAc3Hex5HexNAc7dHex and/or NeuGcHex8HexNAc7dHexSP | 3268 | + | |
| NeuAc4Hex4HexNAc5dHex2SP2 | 3297 | + | |
| NeuAc3Hex4HexNAc5dHex4SP2 | 3298 | + | |
| Hex8HexNAc8dHex3SP and/or NeuAc2Hex4HexNAc8dHex4 | 3456 | + | |
| NeuAc3Hex7HexNAc6dHexSP | 3469 | + | |
| NeuAc2Hex7HexNAc6dHex3SP | 3470 | + | |

**Table 16.** Structural classification of neutral glycan fraction glycan signals isolated from **normal human** lung tissue (1. column), human **lung cancer** tissue (2. column), normal **human serum** (5. column), and a cultured **human cell line** (6. column). Acidic glycan fraction glycans analyzed as neutral desialylated glycan signals together with the corresponding neutral glycan fraction are similarly classified from the same human tissue samples (3. and 4. column, **total normal** and **total cancer**).

| **Structural features of Neutral N-glycans** | | % | | | | | |
|---|---|---|---|---|---|---|---|
| structural feature | proposed composition | normal lung | lung cancer | total normal | total cancer | human serum | human cell line |
| $Hex_{5-9}HexNAc_2$ | high-mannose | 47,0 | 46,0 | 17,8 | 22,3 | 25,7 | 53,7 |
| $Hex_{1-4}HexNAc_2dHex_{0-1}$ | low-mannose | 28,0 | 19,5 | 15,5 | 24,4 | 0,7 | 8,5 |
| $Hex_{10-12}HexNAc_2$ | high-mannose / Glc | 0,0 | 0,0 | 0,0 | 0,0 | 0,0 | 1,9 |
| $Hex_{5-6}HexNAc_2dHex_1$ | low-mannose + Fuc | 0,7 | 0,0 | 0,3 | 0,2 | 0,0 | 1,0 |
| $n_{HexNAc} = 3$ ja $n_{Hex} \geq 2$ | hybrid / monoantennary | 7,9 | 8,7 | 8,4 | 7,1 | 6,6 | 7,3 |
| $n_{HexNAc} \geq 4$ ja $n_{Hex} \geq 2$ | complex type | 15,8 | 24,4 | 57,8 | 46,0 | 66,2 | 9,3 |
| $Hex_{1-9}HexNAc$ | soluble | 0,7 | 0,5 | 0,0 | 0,0 | 0,8 | 11,3 |
| other | - | 0,0 | 0,9 | 0,2 | 0,0 | 0,0 | 6,9 |
| $n_{dHex} \geq 1$ | fucosylation | 19,4 | 33,6 | 42,8 | 34,6 | 50,5 | 13,9 |
| $n_{dHex} \geq 2$ | α2/3/4- Fuc | 0,0 | 0,8 | 0,3 | 1,1 | 0,0 | 1,3 |
| $n_{HexNAc} > n_{Hex} \geq 2$ | terminal HexNAc | 3,9 | 17,8 | 3,8 | 7,1 | 21,8 | 4,2 |
| $n_{HexNAc} = n_{Hex} \geq 3$ | terminal HexNAc | 6,9 | 8,2 | 8,2 | 5,0 | 31,4 | 1,9 |

**Table 17. N-glycan structural classification of lysosomal protein sample.**

| **Neutral N-glycan structural features:** | | |
|---|---|---|
| Glycan feature | Proposed structure | Proportion, % |
| $Hex_{5-10}HexNAc_2$ | High-mannose type / $Glc_1$ | 46 |
| $Hex_{1-4}HexNAc_2dHex_{0-1}$ | Low-mannose type | 49 |
| $n_{HexNAc} = 3$ ja $n_{Hex} \geq 2$ | Hybrid-type / Monoantennary | 2 |
| $n_{HexNAc} \geq 4$ ja $n_{Hex} \geq 2$ | Complex-type | 0,6 |
| Other | - | < 3 |
| $n_{dHex} \geq 1$ | Fucosylation | 29 |
| $n_{dHex} \geq 2$ | $\alpha$2/3/4-linked Fuc | 0,8 |
| $n_{HexNAc} > n_{Hex} \geq 2$ | Terminal HexNAc (N>H) | 0,2 |
| $n_{HexNAc} = n_{Hex} \geq 5$ | Terminal HexNAc (N=H) | - |
| **Acidic N-glycan structural features:** | | |
| Glycan feature | Proposed structure | Proportion, % |
| $n_{HexNAc} = 3$ ja $n_{Hex} \geq 3$ | Hybrid-type / Monoantennary | 46 |
| $n_{HexNAc} \geq 4$ ja $n_{Hex} \geq 3$ | Complex-type | 37 |
| muut | - | 17 |
| $n_{dHex} \geq 1$ | Fucosylation | 80 |
| $n_{dHex} \geq 2$ | $\alpha$2/3/4-linked Fuc | 10 |
| $n_{HexNAc} > n_{Hex} \geq 2$ | Terminal HexNAc (N>H) | 0,1 |
| $n_{HexNAc} = n_{Hex} \geq 5$ | Terminal HexNAc (N=H) | 0,4 |
| + 80 Da | Sulphate or phosphate ester | 17 |

**Table 18. Identification of disease-specific glycosylation by quantitative glycome analysis.**

| Composition | m/z | Class | I | II | m/z | Class | Abs. differ. | m/z | Class | Rel. differ. |
|---|---|---|---|---|---|---|---|---|---|---|
| Hex1HexNAc2 | 609 | NL | 0,00 | 0,00 | 771 | NL | 12,8 | 1955 | NCE | new |
| Hex2HexNAc1dHex1 | 714 | NOF | 0,00 | 0,00 | 1485 | NCFT | 3,5 | 2685 | NCE | new |
| Hex3HexNAc1 | 730 | NS | 0,00 | 0,00 | 1743 | NM | 2,1 | 2905 | NCF | new |
| Hex1HexNAc2dHex1 | 755 | NLF | 2,47 | 0,00 | 1905 | NM | 1,8 | 771 | NL | 2,4 |
| Hex2HexNAc2 | 771 | NL | 5,44 | 18,25 | 1419 | NM | 1,4 | 1905 | NM | 2,2 |
| Hex2HexNAc2dHex1 | 917 | NLF | 1,81 | 2,61 | 917 | NLF | 0,8 | 1485 | NCFT | 1,3 |
| Hex3HexNAc2 | 933 | NL | 2,47 | 1,12 | 1581 | NM | 0,5 | 2394 | NC | 1,3 |
| Hex2HexNAc3 | 974 | NH-T | 0,00 | 0,00 | 1955 | NCE | 0,4 | 1743 | NM | 1,2 |
| Hex2HexNAc2dHex2 | 1063 | NOE | 0,00 | 0,00 | 2685 | NCE | 0,4 | 917 | NLF | 0,4 |
| Hex3HexNAc2dHex1 | 1079 | NLF | 1,81 | 1,12 | 2905 | NCF | 0,4 | 1419 | NM | 0,4 |
| Hex4HexNAc2 | 1095 | NL | 1,48 | 1,30 | 2539 | NCF | 0,3 | 2539 | NCF | 0,4 |
| Hex2HexNAc3dHex1 | 1120 | NHFT | 0,00 | 0,00 | 2394 | NC | 0,2 | 1581 | NM | 0,2 |
| Hex3HexNAc3 | 1136 | NH | 0,82 | 0,00 | 2175 | NCF | 0,2 | 1282 | NHF | 0,1 |
| Hex2HexNAc2dHex3 | 1209 | NOE | 0,00 | 0,00 | 1622 | NH | 0,2 | 2012 | NCFB | 0,1 |
| Hex3HexNAc2dHex2 | 1225 | NOE | 0,00 | 0,00 | 1282 | NHF | 0,1 | 1622 | NH | 0,1 |
| Hex4HexNAc2dHex1 | 1241 | NLF | 0,00 | 0,00 | 2012 | NCFB | 0,1 | 1339 | NH-T | 0,1 |
| Hex5HexNAc2 | 1257 | NM | 8,90 | 7,64 | 1339 | NH-T | 0,0 | 2320 | NCE | 0,1 |
| Hex2HexNAc3dHex2 | 1266 | NHET | 0,00 | 0,00 | 2320 | NCE | 0,0 | 2175 | NCF | 0,0 |
| Hex3HexNAc3dHex1 | 1282 | NHF | 0,82 | 0,93 | 609 | NL | 0,0 | 609 | NL | 0,0 |
| Hex4HexNAc3 | 1298 | NH | 1,48 | 1,12 | 714 | NOF | 0,0 | 714 | NOF | 0,0 |
| Hex3HexNAc4 | 1339 | NH-T | 0,33 | 0,37 | 730 | NS | 0,0 | 730 | NS | 0,0 |
| Hex5HexNAc2dHex1 | 1403 | NMF | 0,33 | 0,19 | 974 | NH-T | 0,0 | 974 | NH-T | 0,0 |
| Hex6HexNAc2 | 1419 | NM | 3,95 | 5,40 | 1063 | NOE | 0,0 | 1063 | NOE | 0,0 |
| Hex3HexNAc3dHex2 | 1428 | NHE | 0,00 | 0,00 | 1120 | NHFT | 0,0 | 1120 | NHFT | 0,0 |
| Hex4HexNAc3dHex1 | 1444 | NHF | 1,65 | 1,30 | 1209 | NOE | 0,0 | 1209 | NOE | 0,0 |
| Hex5HexNAc3 | 1460 | NH | 2,47 | 2,42 | 1225 | NOE | 0,0 | 1225 | NOE | 0,0 |
| Hex3HexNAc4dHex1 | 1485 | NCFT | 2,64 | 6,15 | 1241 | NLF | 0,0 | 1241 | NLF | 0,0 |
| Hex4HexNAc4 | 1501 | NC | 1,32 | 0,93 | 1266 | NHET | 0,0 | 1266 | NHET | 0,0 |
| Hex3HexNAc5 | 1542 | NC-T | 0,00 | 0,00 | 1428 | NHE | 0,0 | 1428 | NHE | 0,0 |
| Hex7HexNAc2 | 1581 | NM | 2,31 | 2,79 | 1542 | NC-T | 0,0 | 1542 | NC-T | 0,0 |
| Hex6HexNAc3 | 1622 | NH | 1,15 | 1,30 | 1688 | NCFT | 0,0 | 1688 | NCFT | 0,0 |
| Hex4HexNAc4dHex1 | 1647 | NCF | 3,95 | 2,23 | 2028 | NC | 0,0 | 2028 | NC | 0,0 |
| Hex5HexNAc4 | 1663 | NC | 17,63 | 13,97 | 1460 | NH | -0,1 | 1460 | NH | 0,0 |
| Hex3HexNAc5dHex1 | 1688 | NCFT | 0,00 | 0,00 | 1850 | NCFT | -0,1 | 1095 | NL | -0,1 |
| Hex4HexNAc5 | 1704 | NC-T | 0,16 | 0,00 | 1403 | NMF | -0,1 | 1257 | NM | -0,1 |
| Hex8HexNAc2 | 1743 | NM | 1,81 | 3,91 | 1704 | NC-T | -0,2 | 1850 | NCFT | -0,2 |
| Hex5HexNAc4dHex1 | 1809 | NCF | 20,59 | 11,73 | 1095 | NL | -0,2 | 1663 | NC | -0,2 |
| Hex6HexNAc4 | 1825 | NC | 2,47 | 0,56 | 1444 | NHF | -0,3 | 1444 | NHF | -0,2 |
| Hex4HexNAc5dHex1 | 1850 | NCFT | 0,66 | 0,56 | 1298 | NH | -0,4 | 1298 | NH | -0,2 |
| Hex5HexNAc5 | 1866 | NC-B | 0,49 | 0,00 | 1501 | NC | -0,4 | 1501 | NC | -0,3 |
| Hex9HexNAc2 | 1905 | NM | 0,82 | 2,61 | 1866 | NC-B | -0,5 | 1079 | NLF | -0,4 |
| Hex5HexNAc4dHex2 | 1955 | NCE | 0,00 | 0,37 | 1079 | NLF | -0,7 | 1809 | NCF | -0,4 |
| Hex5HexNAc5dHex1 | 2012 | NCFB | 0,82 | 0,93 | 1136 | NH | -0,8 | 1647 | NCF | -0,4 |
| Hex6HexNAc5 | 2028 | NC | 1,32 | 1,30 | 1257 | NM | -1,3 | 1403 | NMF | -0,4 |
| Hex6HexNAc5dHex1 | 2175 | NCF | 4,12 | 4,28 | 933 | NL | -1,4 | 933 | NL | -0,5 |
| Hex6HexNAc5dHex2 | 2320 | NCE | 0,33 | 0,37 | 1647 | NCF | -1,7 | 1825 | NC | -0,8 |
| Hex7HexNAc6 | 2394 | NC | 0,16 | 0,37 | 1825 | NC | -1,9 | 1704 | NC-T | gone |
| Hex7HexNAc6dHex1 | 2539 | NCF | 0,82 | 1,12 | 755 | NLF | -2,5 | 1866 | NC-B | gone |
| Hex7HexNAc6dHex2 | 2685 | NCE | 0,00 | 0,37 | 1663 | NC | -3,7 | 1136 | NH | gone |
| Hex8HexNAc7dHex1 | 2905 | NCF | 0,00 | 0,37 | 1809 | NCF | -8,9 | 755 | NLF | gone |

**Table 19. Detected tissue material N-linked and soluble glycome compositions.**

| **Neutral N-glycan structural features:** | | |
|---|---|---|
| Glycan feature | Proposed structure | Proportion, % |
| $Hex_{5-10}HexNAc_2$ | High-mannose type / $Glc_1$ | 10-60 |
| $Hex_{1-4}HexNAc_2dHex_{0-1}$ | Low-mannose type | 0-50 |
| $n_{HexNAc} = 3$ ja $n_{Hex} \geq 2$ | Hybrid-type / Monoantennary | 5-20 |
| $n_{HexNAc} \geq 4$ ja $n_{Hex} \geq 2$ | Complex-type | 5-75 |
| $Hex_{1-9}HexNAc_1$ | Soluble | 0-10 |
| $n_{dHex} \geq 1$ | Fucosylation | 10-80 |
| $n_{dHex} \geq 2$ | $\alpha$2/3/4-linked Fuc | 0-40 |
| $n_{HexNAc} > n_{Hex} \geq 2$ | Terminal HexNAc (N>H) | 1-30 |
| $n_{HexNAc} = n_{Hex} \geq 5$ | Terminal HexNAc (N=H) | 1-40 |
| **Acidic N-glycan structural features:** | | all |
| Glycan feature | Proposed structure | Proportion, % |
| $n_{HexNAc} = 3$ ja $n_{Hex} \geq 3$ | Hybrid-type / Monoantennary | 5-60 |
| $n_{HexNAc} \geq 4$ ja $n_{Hex} \geq 3$ | Complex-type | 40-95 |
| $n_{dHex} \geq 1$ | Fucosylation | 20-90 |
| $n_{dHex} \geq 2$ | $\alpha$2/3/4-linked Fuc | 0-50 |
| $n_{HexNAc} > n_{Hex} \geq 2$ | Terminal HexNAc (N>H) | 0-40 |
| $n_{HexNAc} = n_{Hex} \geq 5$ | Terminal HexNAc (N=H) | 0-40 |
| + 80 Da | Sulphate or phosphate ester | 0-25 |

**Table 20. Breast carcinoma acidic protein-linked glycan structural classification.**

| **Acidic N-glycan classification:**<br>Ductale type breast carcinoma, normal and tumor tissues<br>Lymph node metastasis, normal and metastatic tissues | | Proportion of acidic glycans, % | | | |
|---|---|---|---|---|---|
| Composition | Classification | **normal** | **cancer** | **LNN** | **LNM** |
| $n_{dHex} \geq 1$ | Fucosylated | 61 | 48 | 61 | 68 |
| $n_{dHex} \geq 2$ | $\alpha$2/3/4-Fuc (complex fucosylation) | 1 | 8 | 6 | 7 |
| +42 Da | Acetylated | 0.3 | 0.2 | 1 | 2 |
| +80 Da | Sulfated or phosphorylated | - | 2 | 1 | 4 |

## REFERENCES

Arap, W., et al. (1998) Science 279(5349):377-80
Brockhausen, I. (1999) Biochim. Biophys. Acta 1473(1):67-95
Davies, et al. (1992) J. Chromatogr. 609(1-2): 125-31
Domon, B. & Costello, C.E. (1988) Glycoconj. J. 5:397-409
Dube, D.H. & Bertozzi, C.R. (2005) Nat. Rev. Drug Discov. 4(6):477-88
Ernst, B., Hart, G.W., and Sinaÿ, P. (eds.) (2000) Carbohydrates in chemistry and biology, ISBN 3-527-29511-9, Weiley-VHC, Weinheim.

Fernandez, L.E., et al. (2003) Expert Rev. Vaccines 2(6) :817-23
Harvey, D.J., et al. (1993) Rapid Commun. Mass Spectrom. 7(7):614-9
Holmberg, L.A. & Sandmeier, B.M. (2001) Expert Opin. Biol. Ther. 1(5) :881-91
Huang, Y., et al. (2001) Anal. Chem. 73(24):6063-9
Kurokawa, T., et al. (2002) Eur. J. Biochem. 269:5459-73
Naven, T.J. & Harvey, D.J. (1996) Rapid Commun. Mass Spectrom. 10(11): 1361-6
Nyman, T.A., et al. (1998) Eur. J. Biochem. 253(2):485-93
Papac, D.I., et al. (1996) Anal. Chem. 68(18):3215-23
Packer, N.H., et al. (1998) Glycoconj. J. 15(8):737-47
Plummer, T.H. Jr. & Tarentino, A.L. (1991) Glycobiology 1(3):257-63
Powell, A.K. & Harvey, D.J. (1996) Rapid Commun. Mass Spectrom. 10(9):1027-32
Saarinen, J., et al. (1999) Eur. J. Biochem. 259(3):829-40
Verostek et al. (2000) Anal. Biochem. 278(2): 111-22
Ylönen, A., et al. (2001) Glycobiology 11(7):523-31

## Claims

1. A method of evaluating the malignancy of a human patient sample comprising the step of detecting the presence or amount of cancer related oligosaccharide sequence in the sample, said oligosaccharide sequence consisting of a neutral O-glycan

   Galβ4GlcNAcβ6(Galβ3)GalNAc or Galβ4(Fucα3)GlcNAcβ6(Galβ3)GalNAc.

2. The method according to claim 1 wherein high relative expression rate of said oligosaccharide sequence is indicative of malignant cancer.

3. A pharmaceutical composition comprising a human antibody specifically against a neutral O-glycan consisting of Galβ4GlcNAcβ6(Galβ3)GalNAc or Galβ4(Fucα3)GlcNAcβ6(Galβ3)GalNAc for use in the treatment of cancer.

4. A pharmaceutical composition for use according to claim 3, wherein said antibody is used to target a cell killing chemotherapeutics medicine to cancer cells.

## Patentansprüche

1. Verfahren zum Bewerten der Malignität einer Patientenprobe eines Menschen, umfassend den Schritt des Nachweisens der Anwesenheit oder des Grades von Krebs bezüglich einer Oligosaccharidsequenz in der Probe, wobei die Oligosaccharidsequenz aus einem neutralen O-Glycan Galβ4GlcNAcβ6(Galβ3)GalNAc oder Galβ4(Fucα3)GlcNAcβ6(Galβ3)GalNAc besteht.

2. Verfahren nach Anspruch 1, wobei eine hohe relative Expressionsrate der Oligosaccharidsequenz indikativ für malignen Krebs ist.

3. Pharmazeutische Zusammensetzung, umfassend einen menschlichen Antikörper spezifisch gegen ein neutrales O-Glycan, bestehend aus Galβ4GlcNAcβ6(Galβ3)GalNAc oder Galβ4(Fucα3)GlcNAcβ6(Galβ3)GalNAc zur Verwendung in der Behandlung von Krebs.

4. Pharmazeutische Zusammensetzung zur Verwendung nach Anspruch 3, wobei der Antikörper zum gezielten Einsatz einer zelltötenden Chemotherapeutikamedizin auf Krebszellen verwendet wird.

## Revendications

1. Procédé d'évaluation de la malignité d'un échantillon de patient humain comprenant l'étape consistant à détecter la présence ou la quantité d'une séquence oligosaccharidique liée à un cancer dans l'échantillon, ladite séquence oligosaccharidique étant constituée d'un O-glycane neutre

Galβ4GlcNAcβ6(Galβ3)GalNAc ou Galβ4(Fucα3)GlcNAcβ6(Galβ3)GalNAc.

**2.** Procédé selon la revendication 1, dans lequel un taux d'expression relatif élevé de ladite séquence oligosaccharidique est le signe d'un cancer malin.

**3.** Composition pharmaceutique comprenant un anticorps humain dirigé spécifiquement contre un O-glycane neutre constitué par Galβ4GlcNAcβ6(Galβ3)GalNAc ou Galβ4(Fucα3)GlcNAcβ6(Galβ3)GalNAc pour son utilisation dans le traitement d'un cancer.

**4.** Composition pharmaceutique pour son utilisation selon la revendication 3, dans laquelle ledit anticorps est utilisé pour cibler un médicament chimiothérapeutique destructeur de cellules sur des cellules cancéreuses.

**Figure 1.**

A.

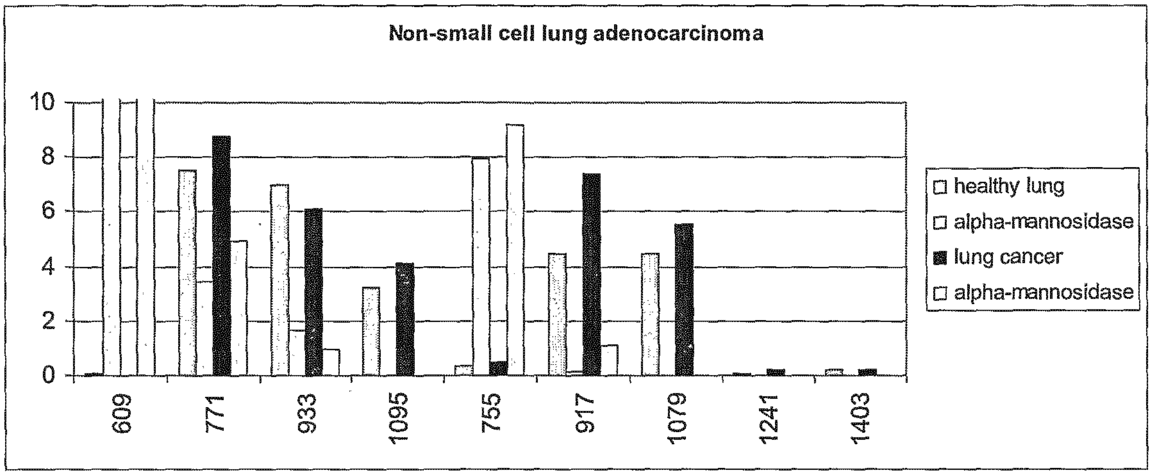
Non-small cell lung adenocarcinoma
10
8
6
4
2
0
609
771
933
1095
755
917
1079
1241
1403
healthy lung
alpha-mannosidase
lung cancer
alpha-mannosidase

B.

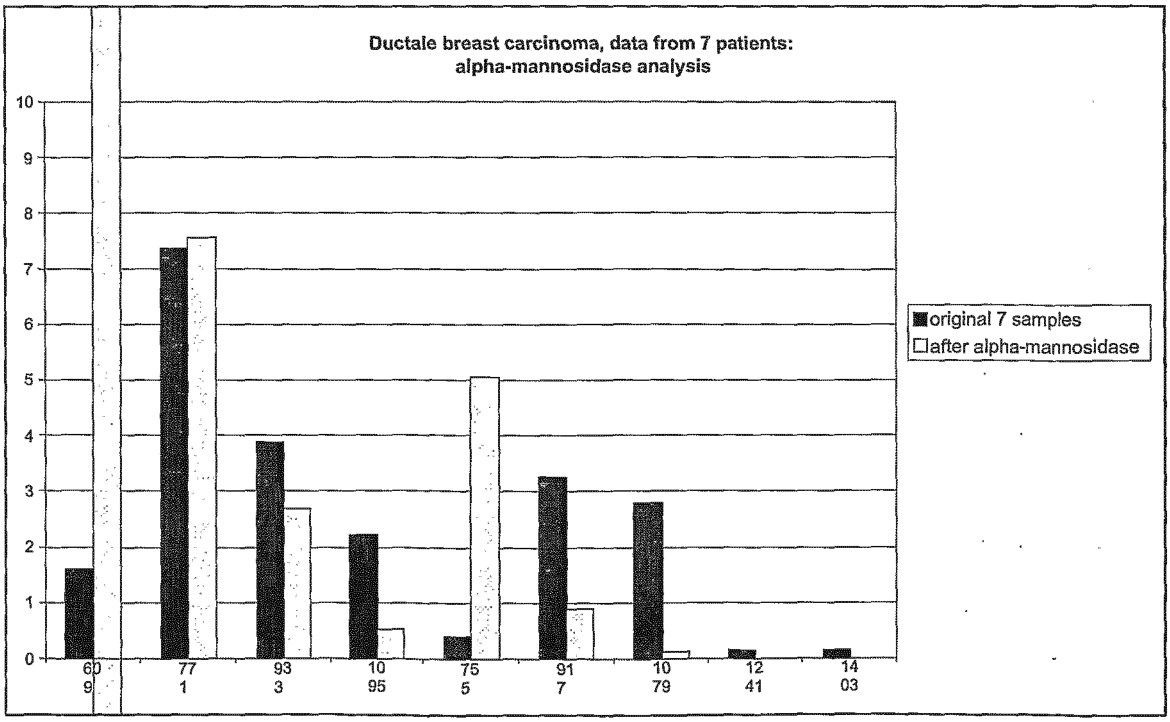
Ductale breast carcinoma, data from 7 patients:
alpha-mannosidase analysis
10
9
8
7
6
5
4
3
2
1
0
60 9
77 1
93 3
10 95
75 5
91 7
10 79
12 41
14 03
original 7 samples
after alpha-mannosidase

**Figure 2.**

A.

General structure:

Man$\alpha_{(0\text{-}3)}$Man$\beta$4GlcNAc$\beta$4GlcNAc($\beta$-*N*-Asn)

Structures of N-glycan biosynthesis products:

0 – 3 Manα residues

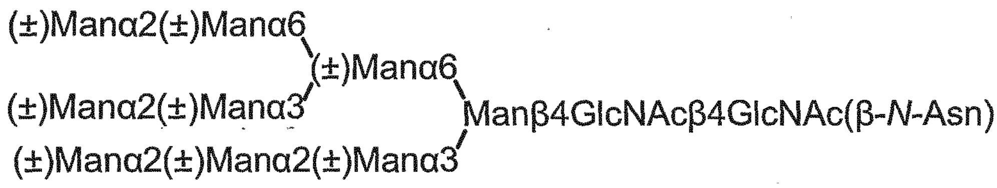

B.

General structure:

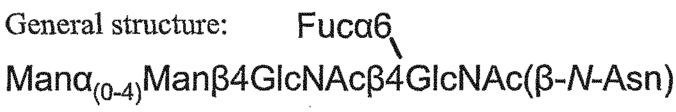

Structures of N-glycan biosynthesis products:

0 – 4 Manα residues

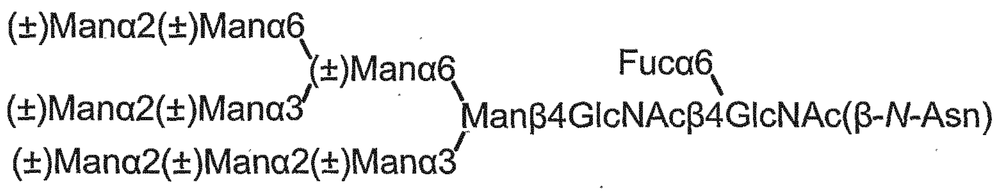

**Figure 3.**

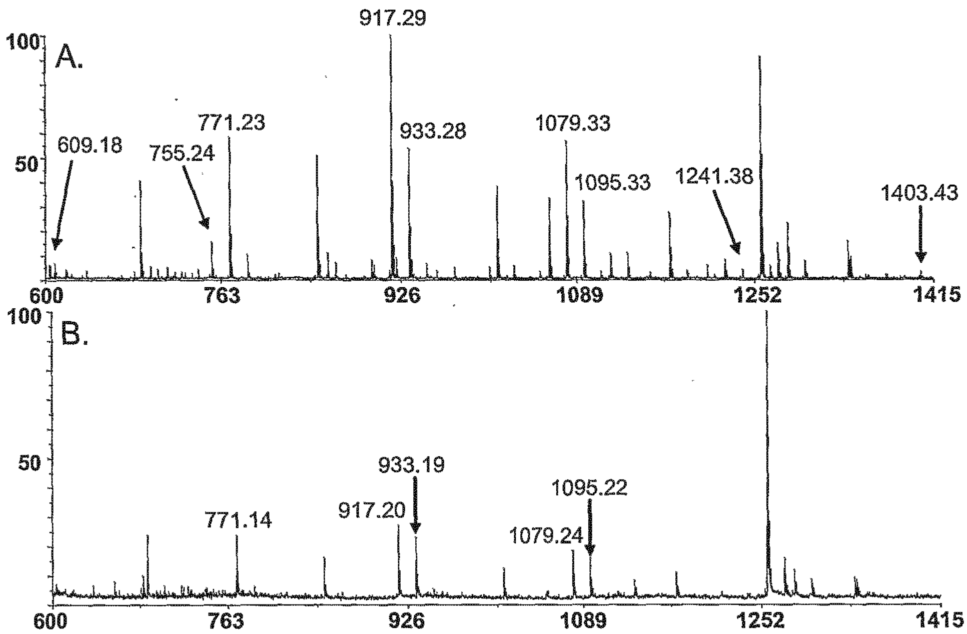
A.
917.29
771.23
933.28
1079.33
609.18
755.24
1095.33
1241.38
1403.43
100
50
600
763
926
1089
1252
1415
B.
933.19
1095.22
771.14
917.20
1079.24
100
50
600
763
926
1089
1252
1415

C.

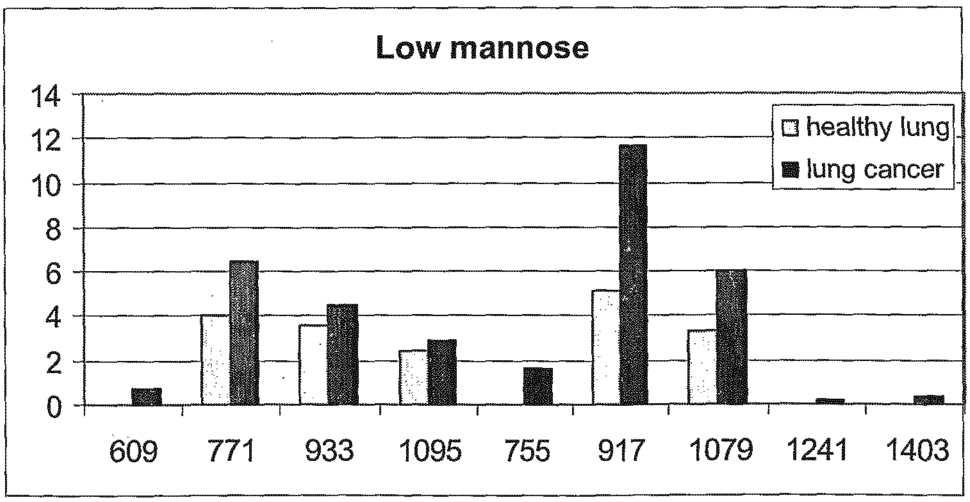
Low mannose
14
12
10
8
6
4
2
0
healthy lung
lung cancer
609
771
933
1095
755
917
1079
1241
1403

Figure 4.

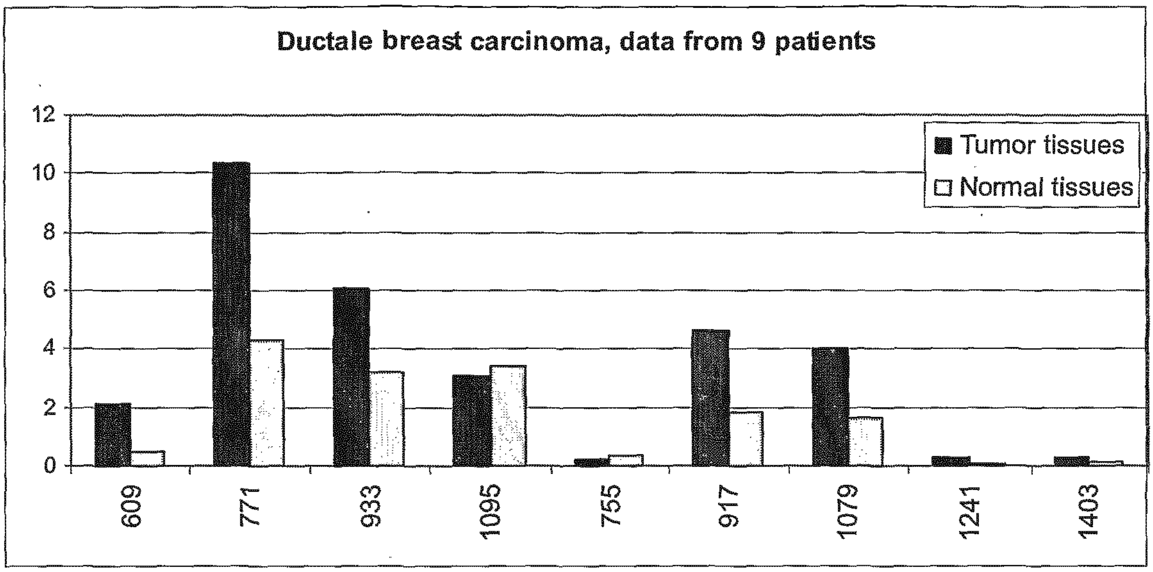
Ductale breast carcinoma, data from 9 patients
12
10
8
6
4
2
0
Tumor tissues
Normal tissues
609
771
933
1095
755
917
1079
1241
1403

**Figure 5.**

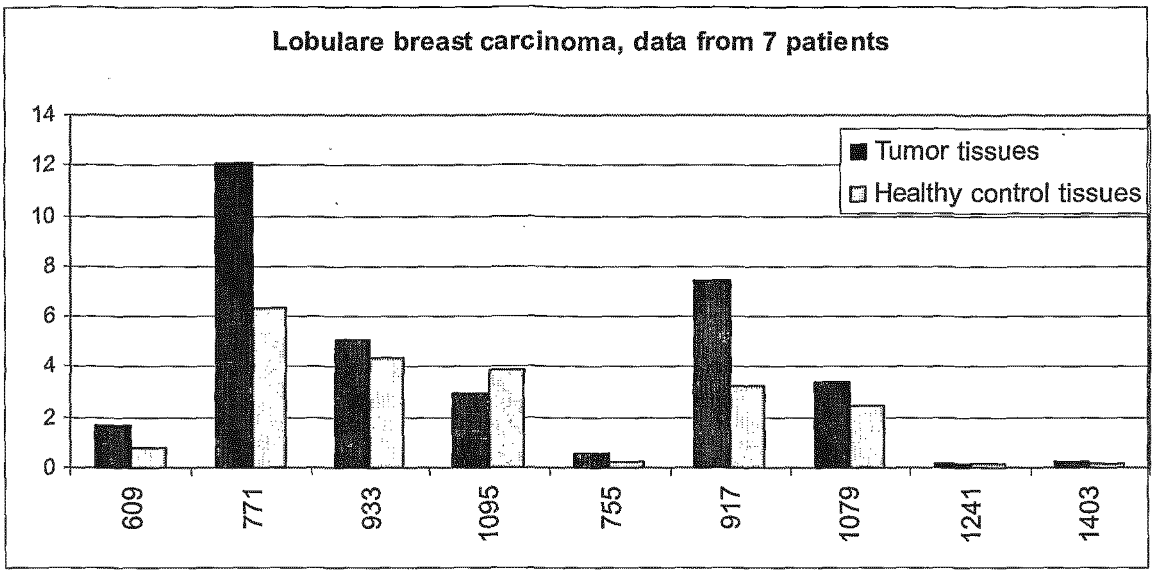
Lobulare breast carcinoma, data from 7 patients
14
12
10
8
6
4
2
0
Tumor tissues
Healthy control tissues
609
771
933
1095
755
917
1079
1241
1403

**Figure 6.**

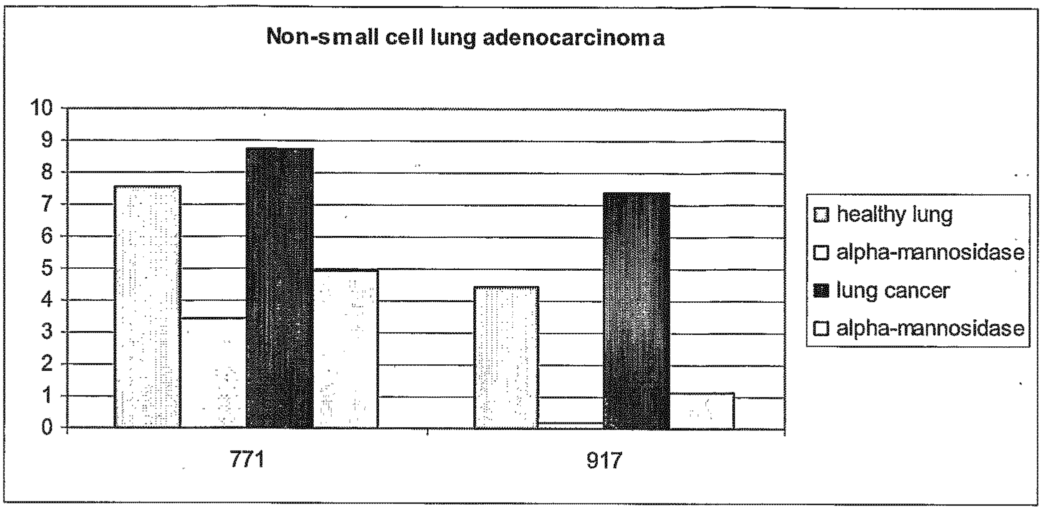
Non-small cell lung adenocarcinoma
10
9
8
7
6
5
4
3
2
1
0
771
917
healthy lung
alpha-mannosidase
lung cancer
alpha-mannosidase

Figure 7.

a)

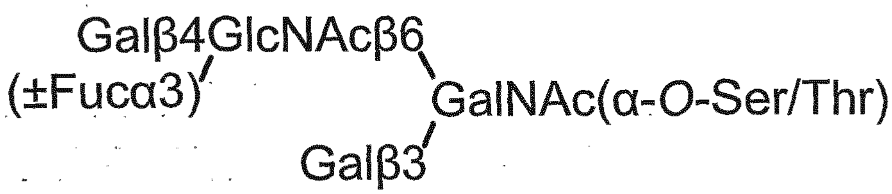

b)

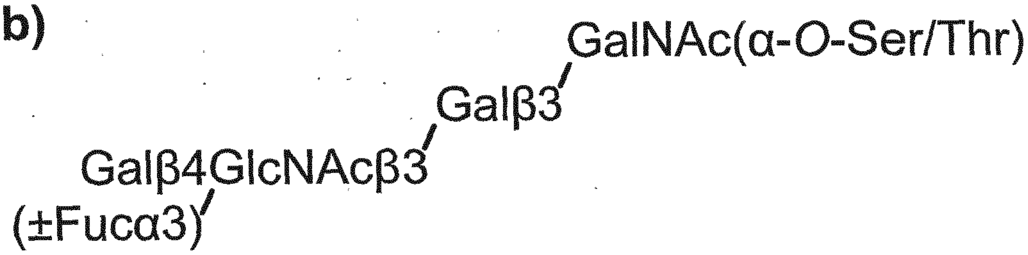

Figure 8.

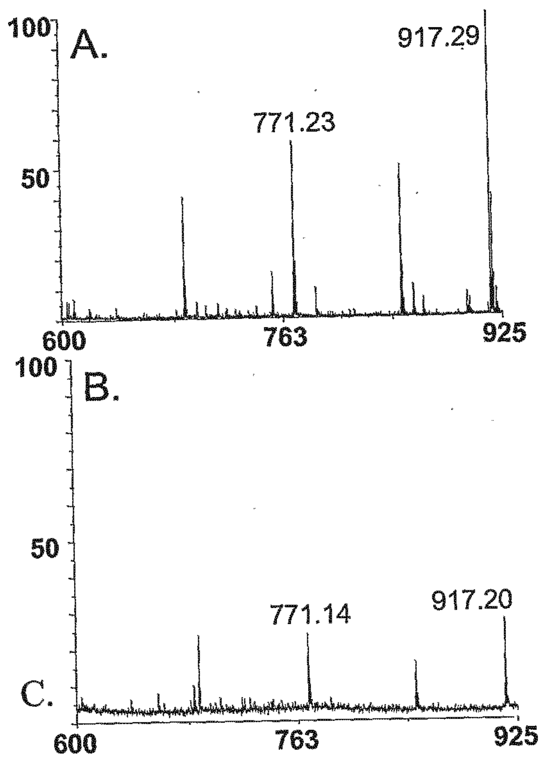

100
A.
917.29
771.23
50
600
763
925
100
B.
50
771.14
917.20
C.
600
763
925

Figure 9.

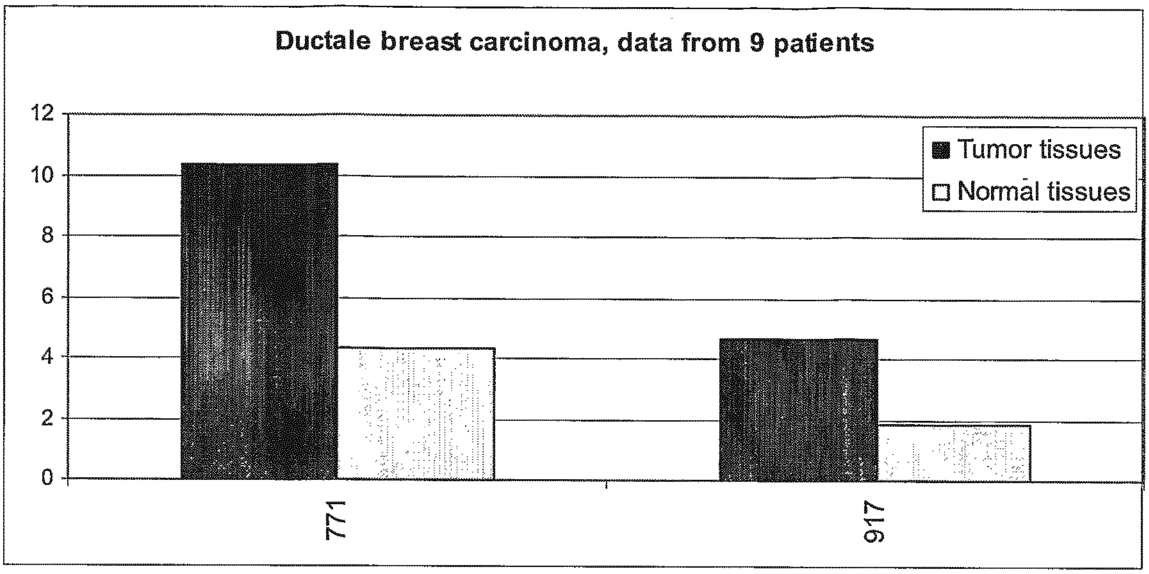
Ductale breast carcinoma, data from 9 patients
12
10
8
6
4
2
0
Tumor tissues
Normal tissues
771
917

Figure 10.

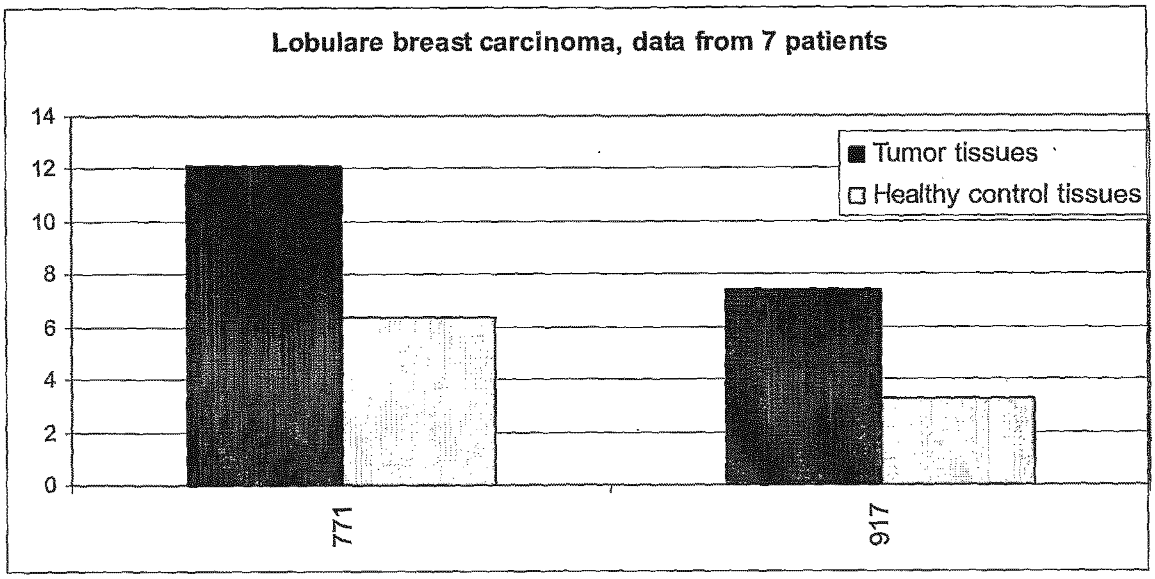
Lobulare breast carcinoma, data from 7 patients
14
12
10
8
6
4
2
0
Tumor tissues
Healthy control tissues
771
917

**Figure 11.**

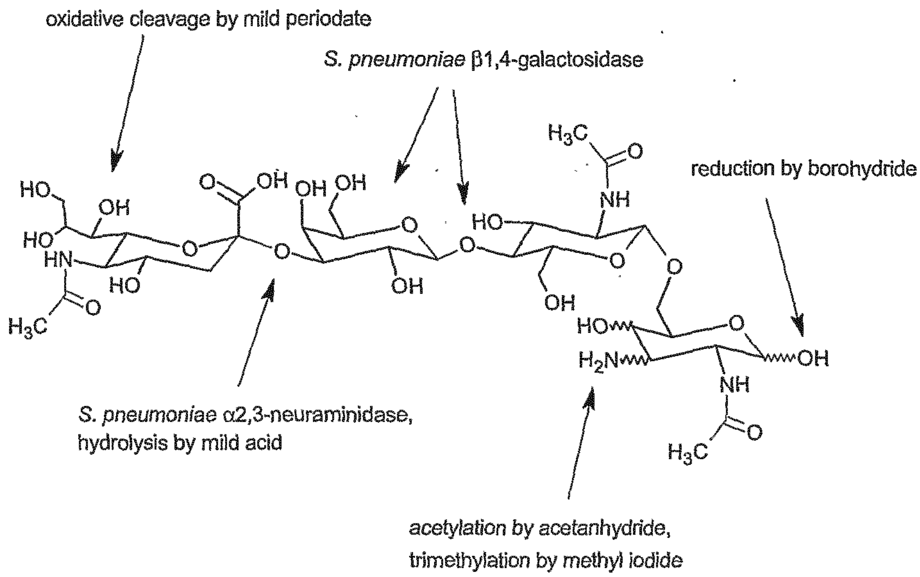
oxidative cleavage by mild periodate
*S. pneumoniae* β1,4-galactosidase
reduction by borohydride
*S. pneumoniae* α2,3-neuraminidase,
hydrolysis by mild acid
acetylation by acetanhydride,
trimethylation by methyl iodide

Figure 12.

A.

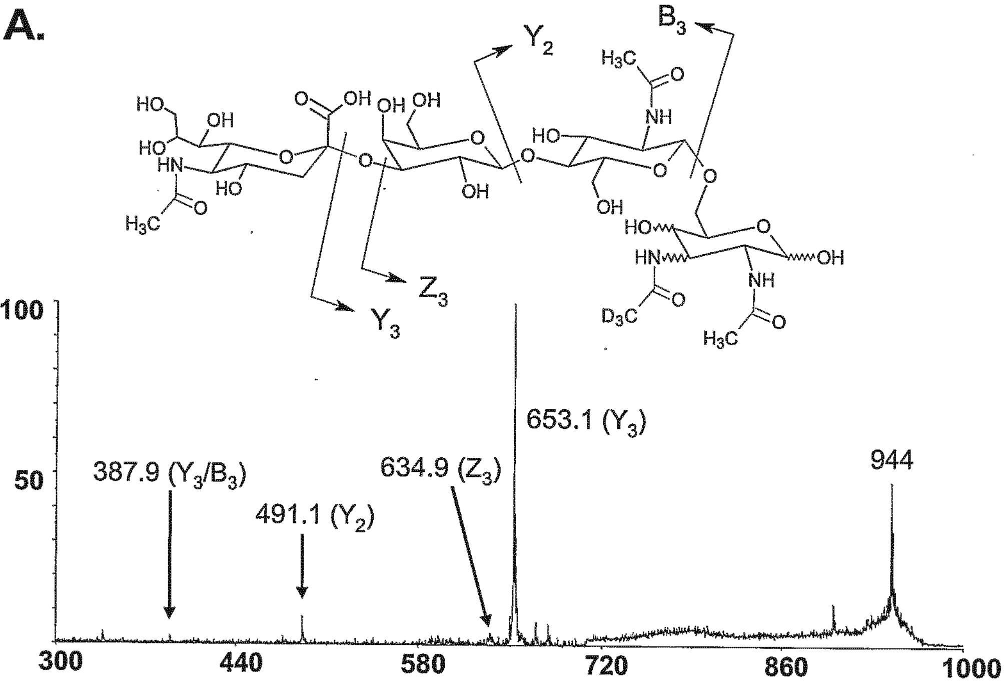
653.1 ($Y_3$)
944
387.9 ($Y_3$/$B_3$)
634.9 ($Z_3$)
491.1 ($Y_2$)
100
50
300
440
580
720
860
1000

B.

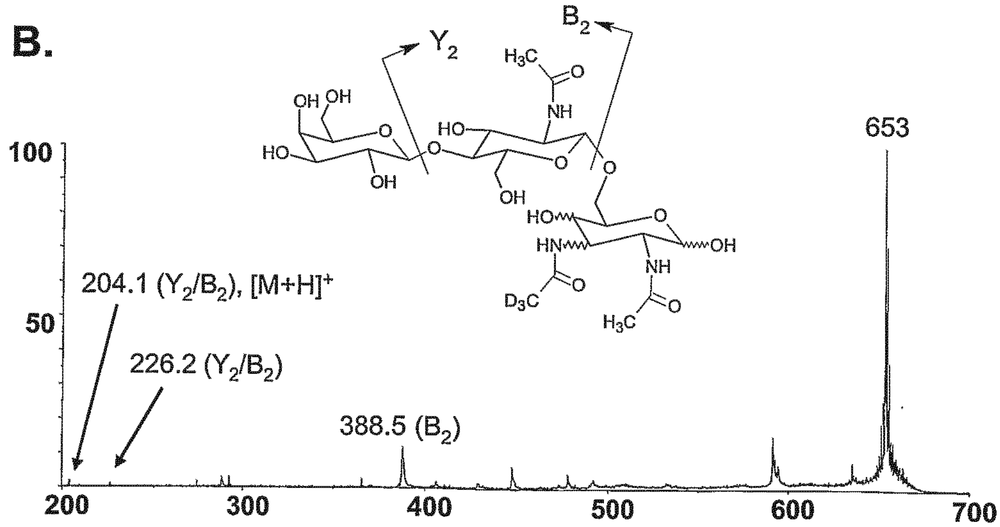
653
204.1 ($Y_2$/$B_2$), $[M+H]^+$
226.2 ($Y_2$/$B_2$)
388.5 ($B_2$)
100
50
200
300
400
500
600
700

**Figure 13.**

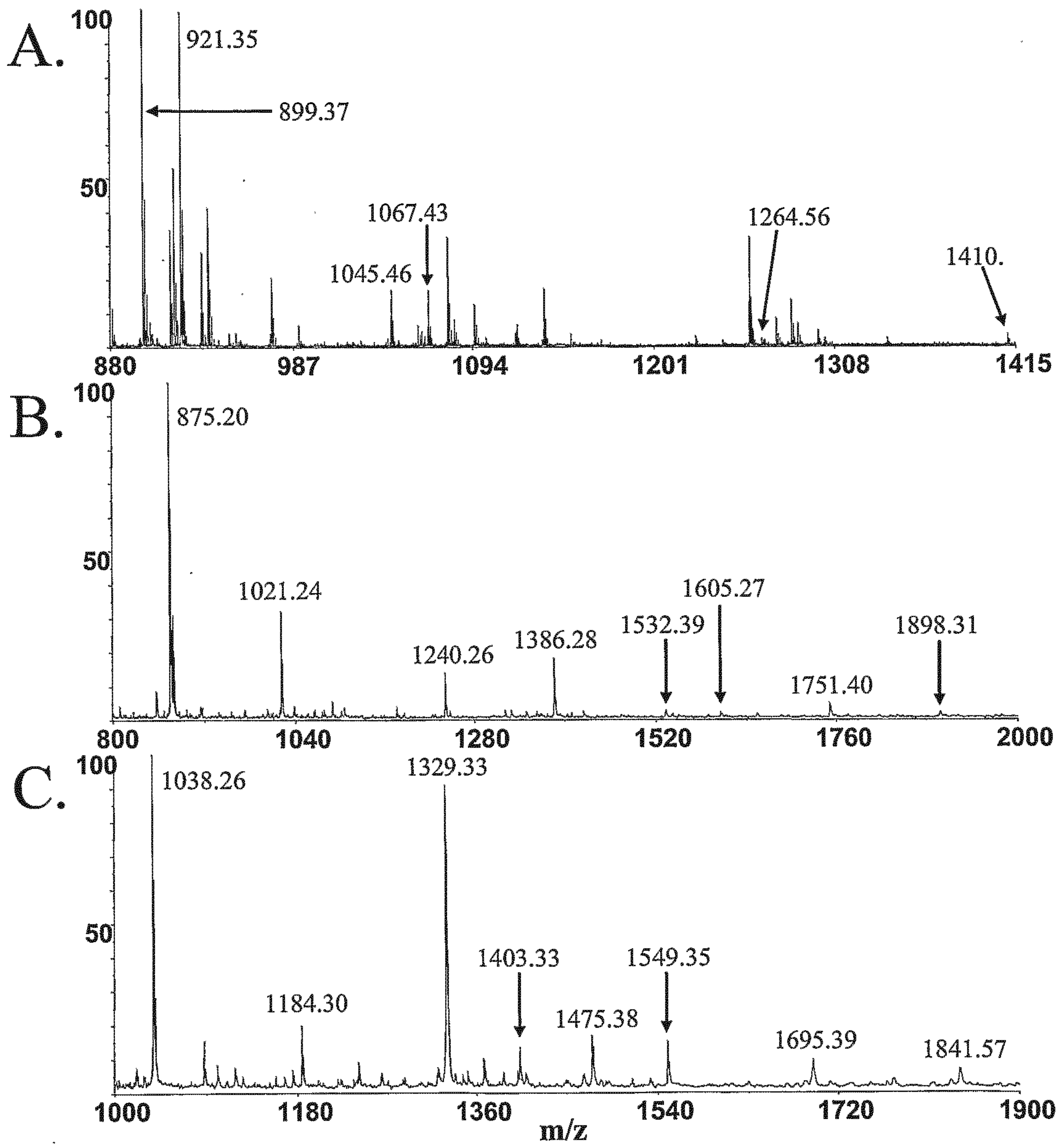
A.
100
50
921.35
899.37
1067.43
1045.46
1264.56
1410.
880
987
1094
1201
1308
1415
B.
100
50
875.20
1021.24
1240.26
1386.28
1532.39
1605.27
1751.40
1898.31
800
1040
1280
1520
1760
2000
C.
100
50
1038.26
1329.33
1184.30
1403.33
1475.38
1549.35
1695.39
1841.57
1000
1180
1360
1540
1720
1900
m/z

**Figure 14.**

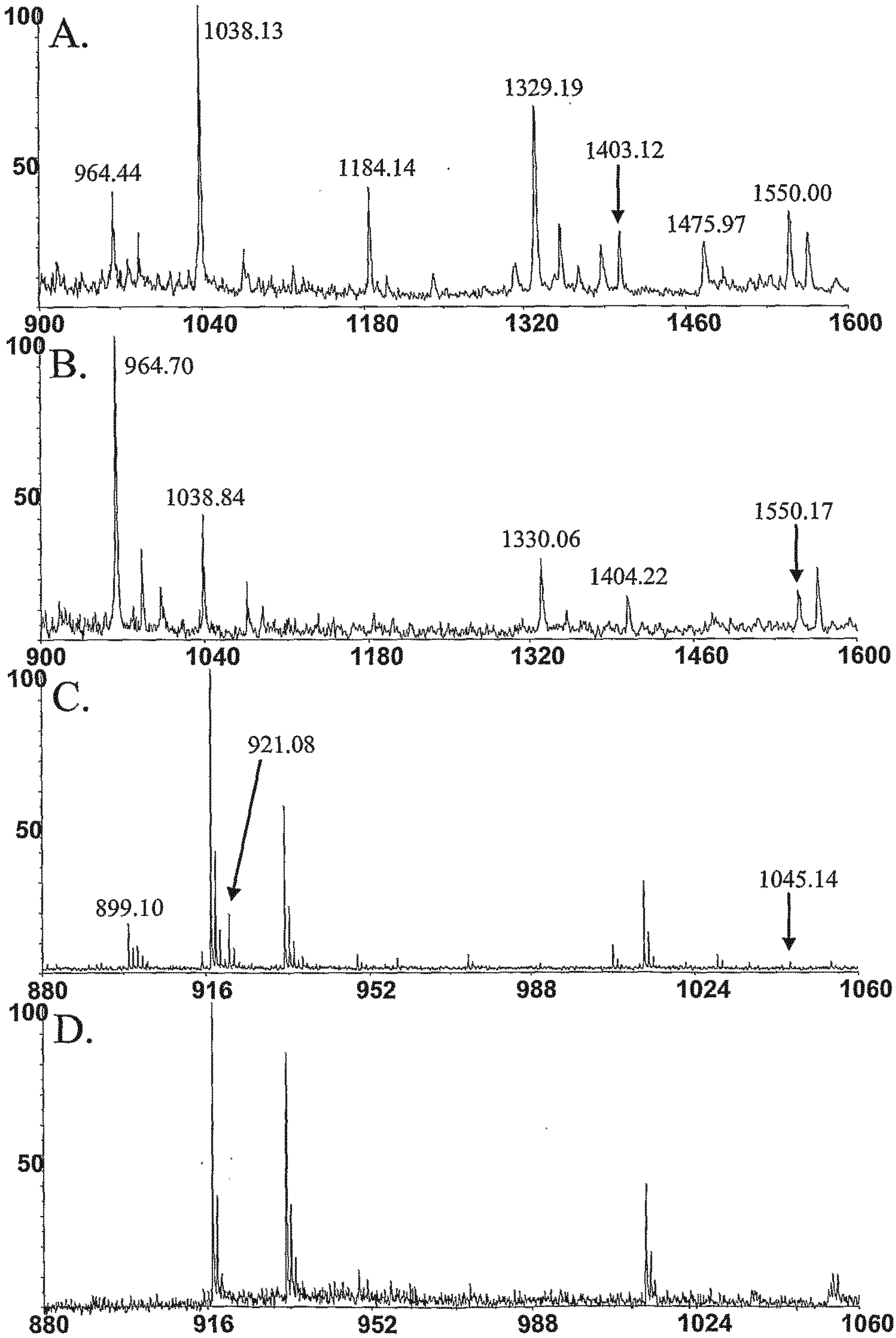
A.
100
50
1038.13
964.44
1184.14
1329.19
1403.12
1475.97
1550.00
900
1040
1180
1320
1460
1600
B.
100
50
964.70
1038.84
1330.06
1404.22
1550.17
900
1040
1180
1320
1460
1600
C.
100
50
921.08
899.10
1045.14
880
916
952
988
1024
1060
D.
100
50
880
916
952
988
1024
1060

Figure 15.

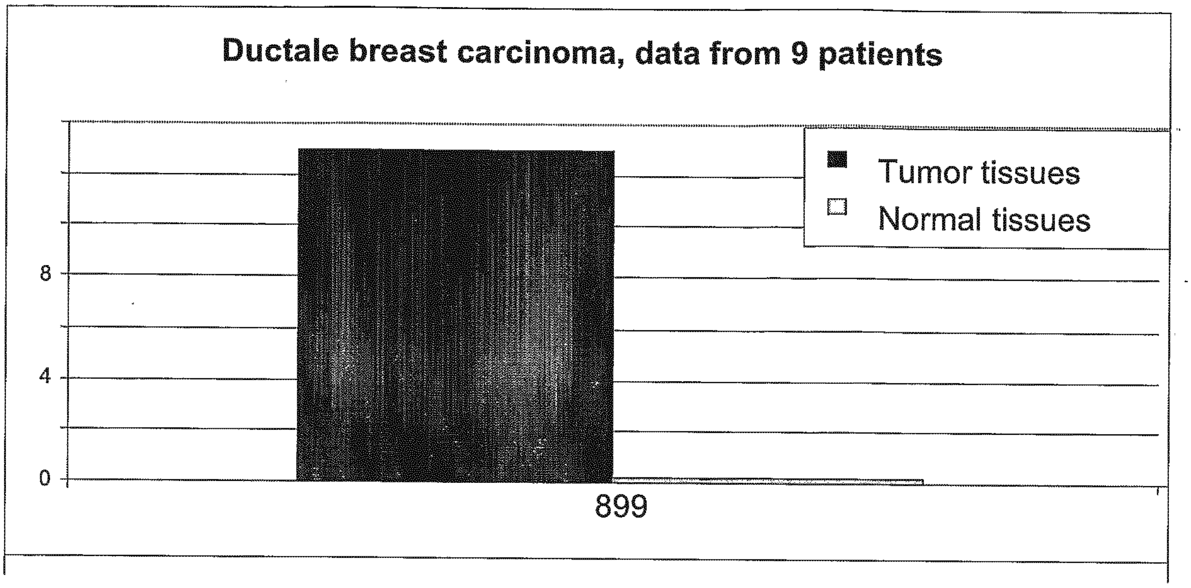
Ductale breast carcinoma, data from 9 patients
Tumor tissues
Normal tissues
8
4
0
899

Figure 16.

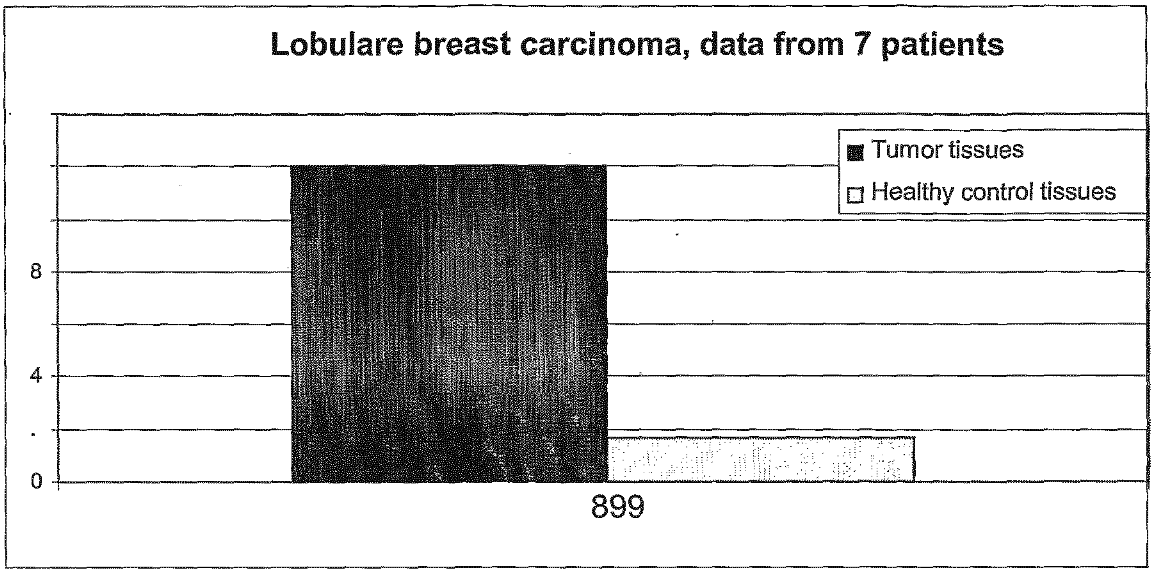
Lobulare breast carcinoma, data from 7 patients
Tumor tissues
Healthy control tissues
8
4
0
899

**Figure 17.**

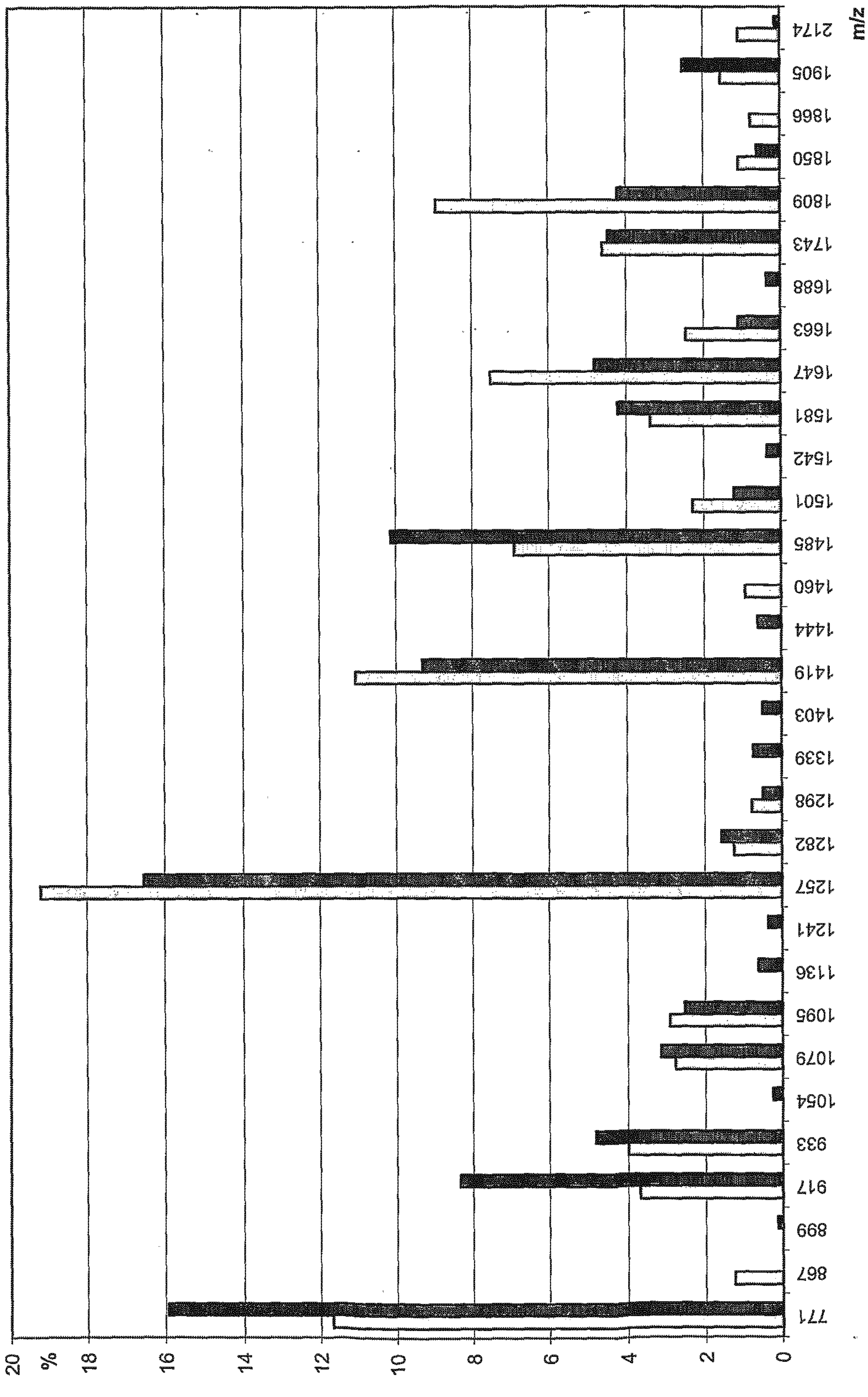
m/z
771
867
899
917
933
1054
1079
1095
1136
1241
1257
1282
1298
1339
1403
1419
1444
1460
1485
1501
1542
1581
1647
1663
1688
1743
1809
1850
1866
1905
2174
%
0
2
4
6
8
10
12
14
16
18
20

**Figure 18.**

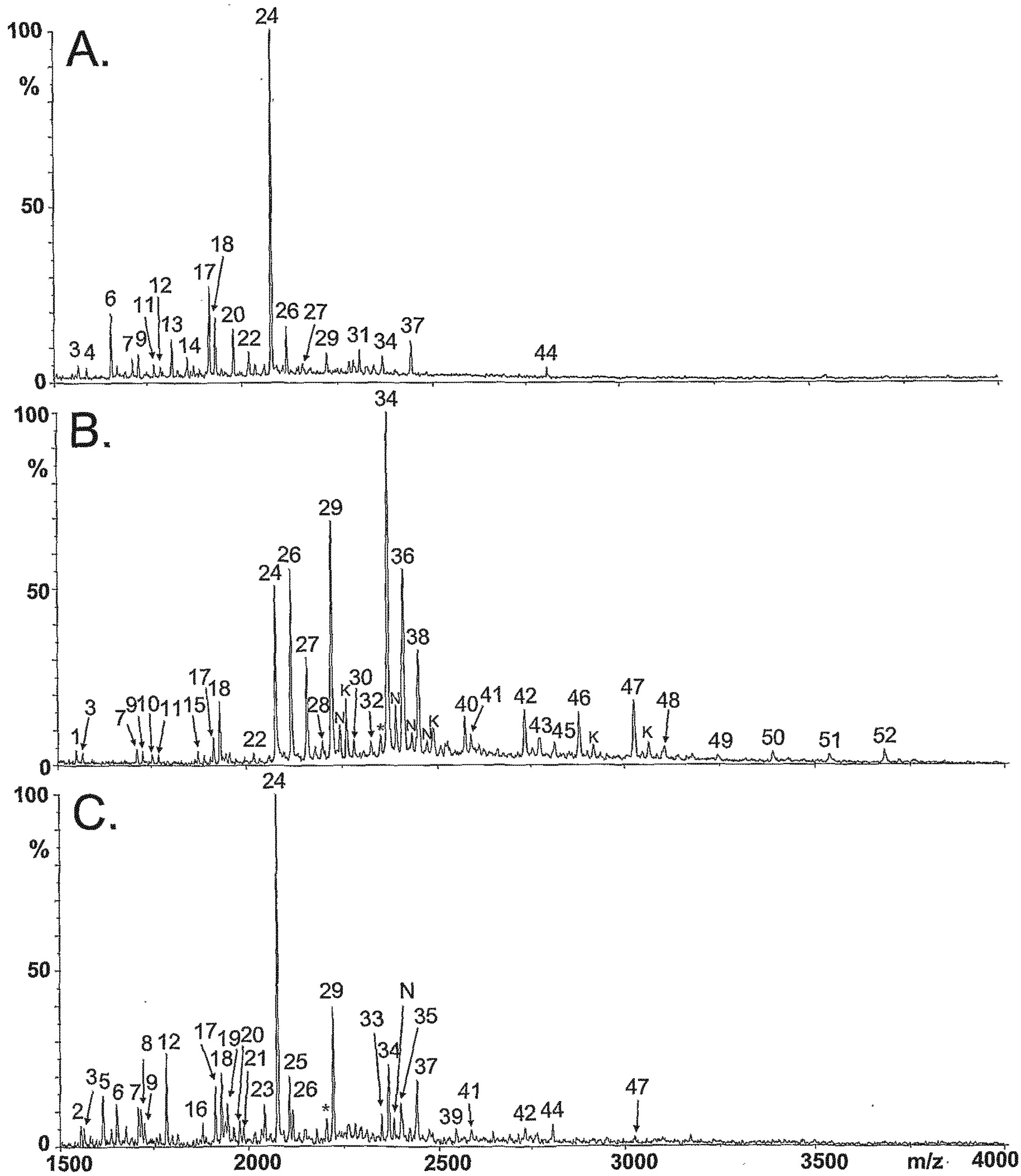

**Figure 19.**

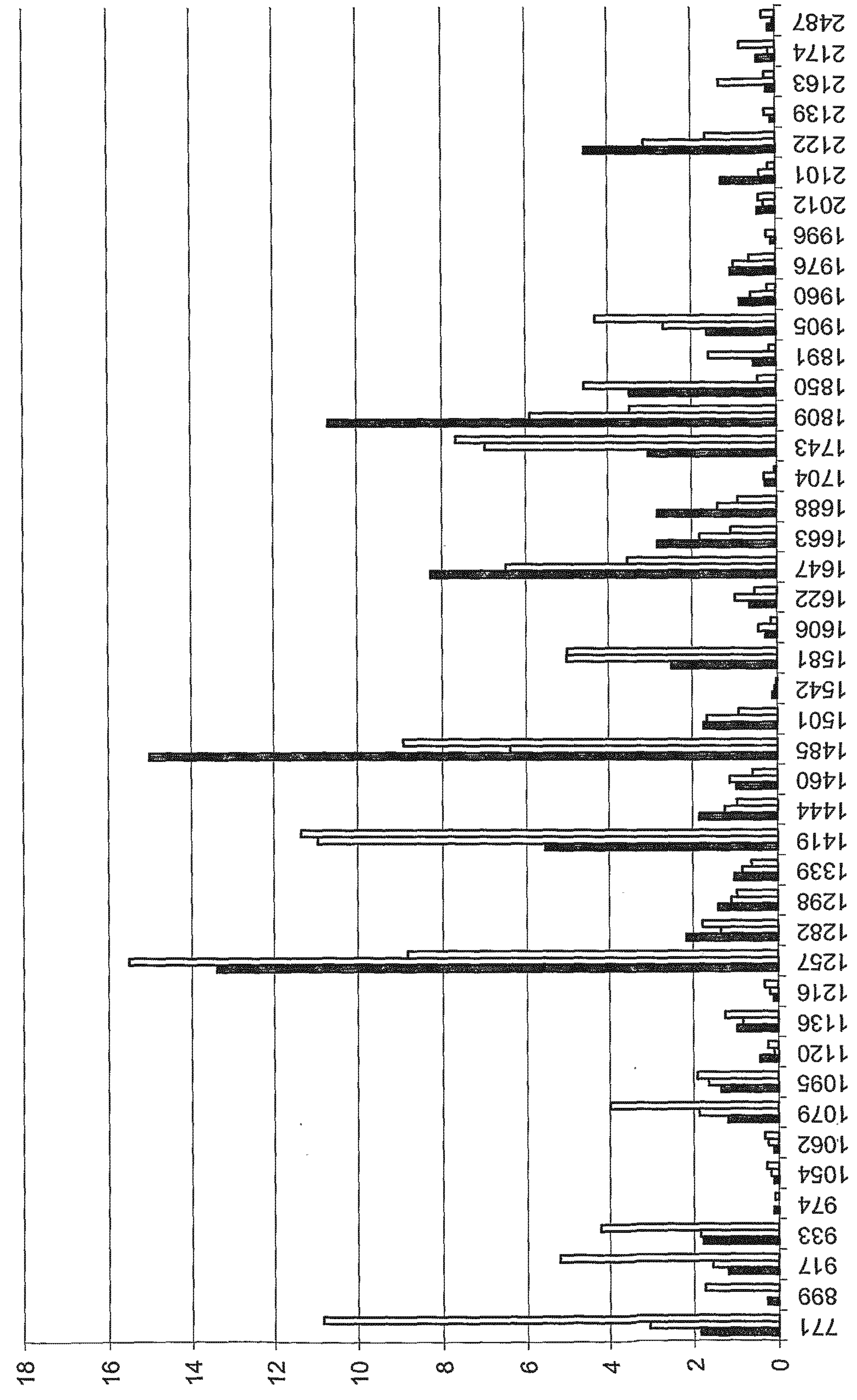
771
899
917
933
974
1054
1062
1079
1095
1120
1136
1216
1257
1282
1298
1339
1419
1444
1460
1485
1501
1542
1581
1606
1622
1647
1663
1688
1704
1743
1809
1850
1891
1905
1960
1976
1996
2012
2101
2122
2139
2163
2174
2487
0
2
4
6
8
10
12
14
16
18

**Figure 20.**

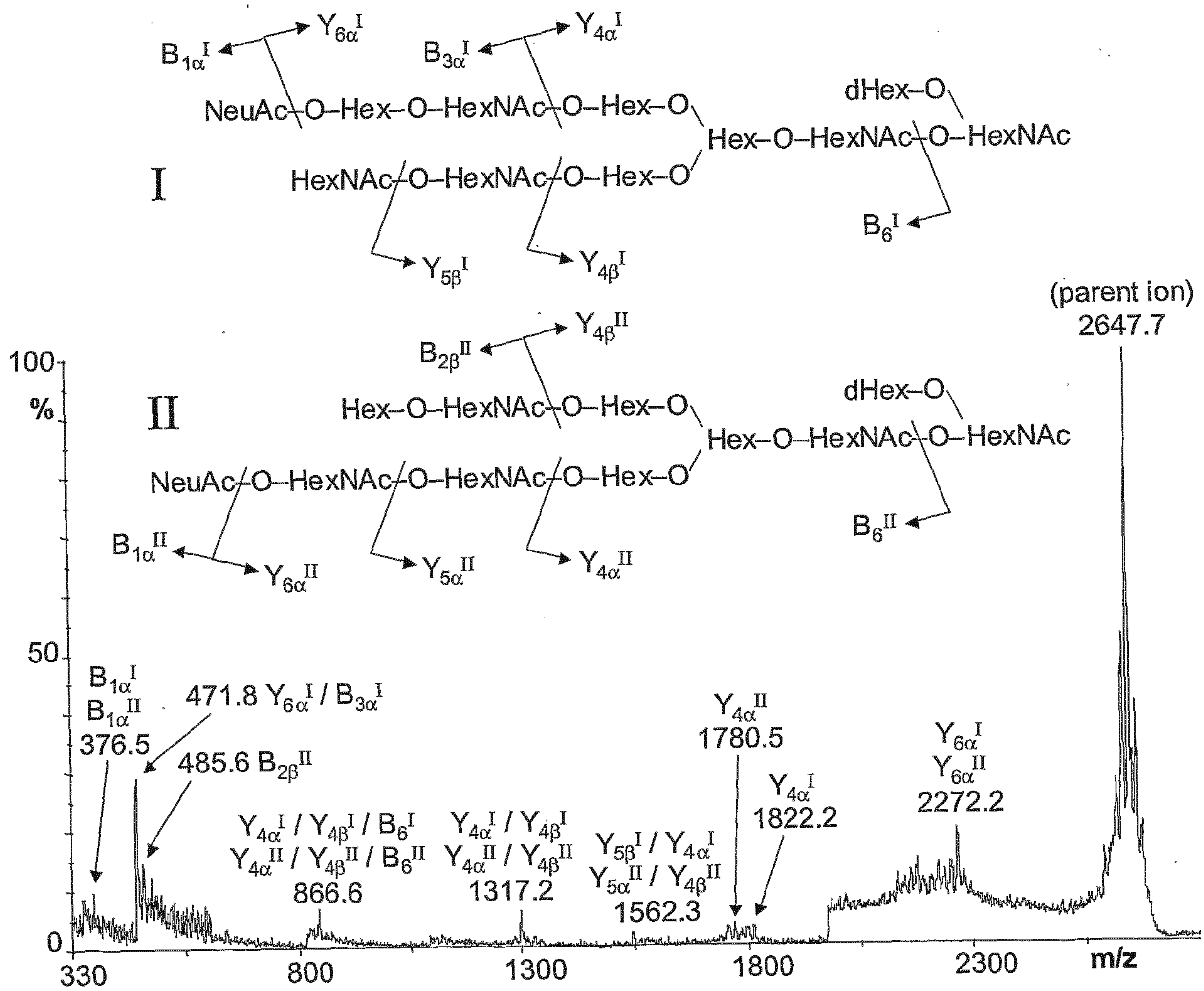
(parent ion)
2647.7
100
%
50
0
$B_{1\alpha}^{I}$
$B_{1\alpha}^{II}$
376.5
471.8 $Y_{6\alpha}^{I}$ / $B_{3\alpha}^{I}$
485.6 $B_{2\beta}^{II}$
$Y_{4\alpha}^{I}$ / $Y_{4\beta}^{I}$ / $B_{6}^{I}$
$Y_{4\alpha}^{II}$ / $Y_{4\beta}^{II}$ / $B_{6}^{II}$
866.6
$Y_{4\alpha}^{I}$ / $Y_{4\beta}^{I}$
$Y_{4\alpha}^{II}$ / $Y_{4\beta}^{II}$
1317.2
$Y_{5\beta}^{I}$ / $Y_{4\alpha}^{I}$
$Y_{5\alpha}^{II}$ / $Y_{4\beta}^{II}$
1562.3
$Y_{4\alpha}^{II}$
1780.5
$Y_{4\alpha}^{I}$
1822.2
$Y_{6\alpha}^{I}$
$Y_{6\alpha}^{II}$
2272.2
330
800
1300
1800
2300
m/z

Figure 21.

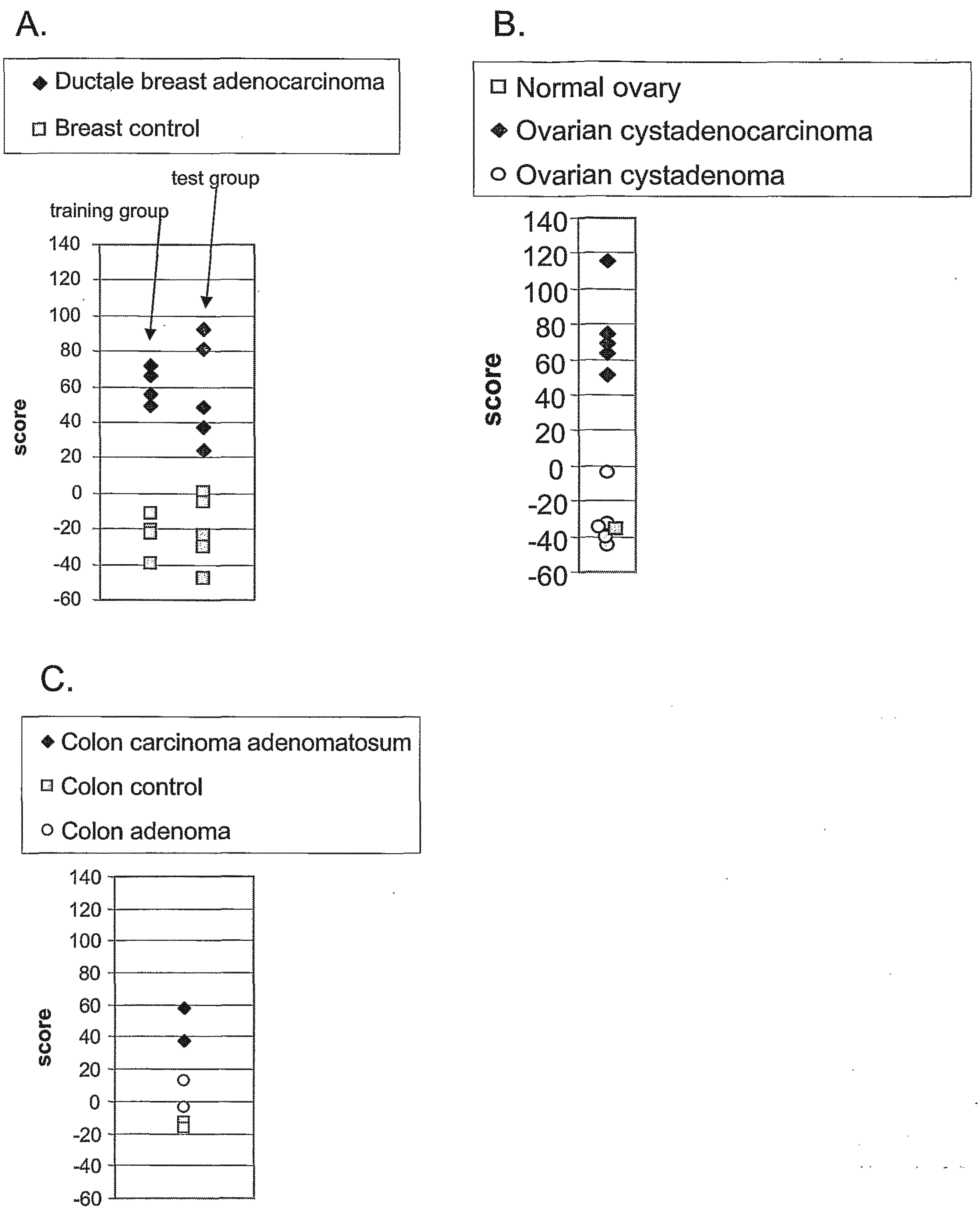
A.
Ductale breast adenocarcinoma
Breast control
test group
training group
score
140
120
100
80
60
40
20
0
-20
-40
-60
B.
Normal ovary
Ovarian cystadenocarcinoma
Ovarian cystadenoma
score
140
120
100
80
60
40
20
0
-20
-40
-60
C.
Colon carcinoma adenomatosum
Colon control
Colon adenoma
score
140
120
100
80
60
40
20
0
-20
-40
-60

**Figure 22.**

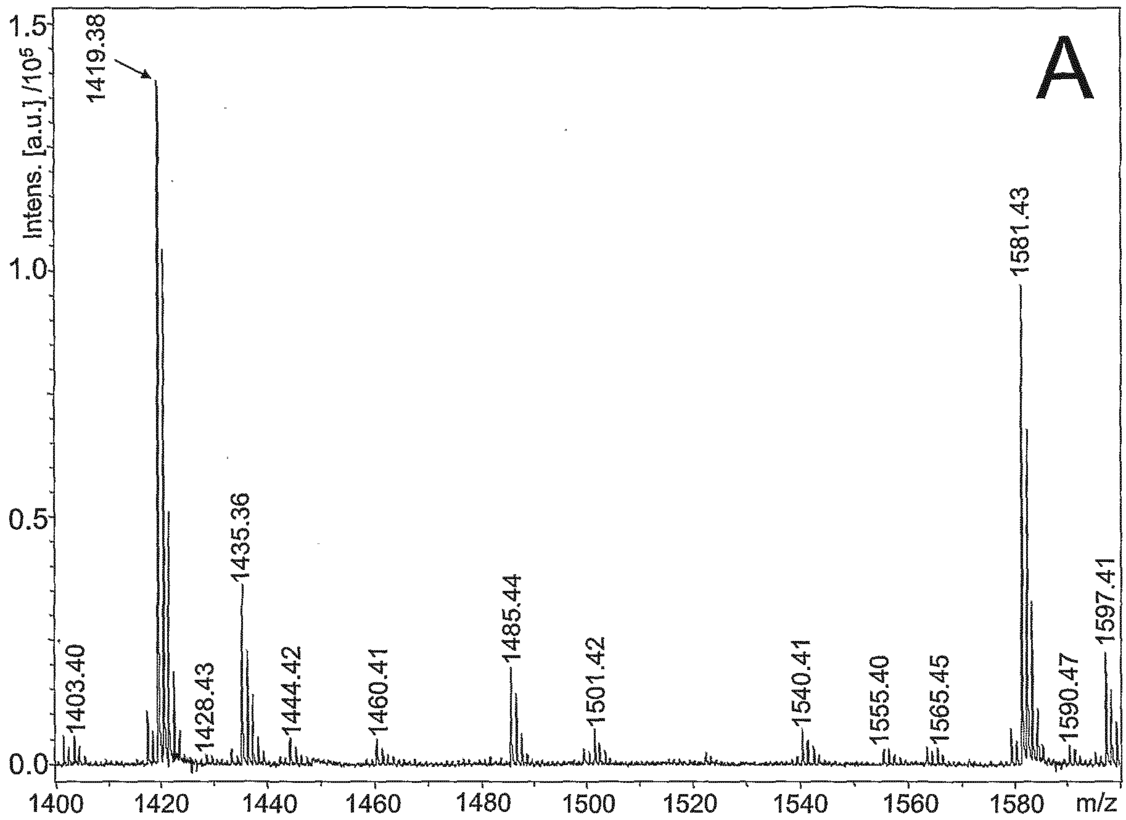
A
Intens. [a.u.] /10^5
1.5
1.0
0.5
0.0
1419.38
1403.40
1428.43
1435.36
1444.42
1460.41
1485.44
1501.42
1540.41
1555.40
1565.45
1581.43
1590.47
1597.41
1400
1420
1440
1460
1480
1500
1520
1540
1560
1580
m/z

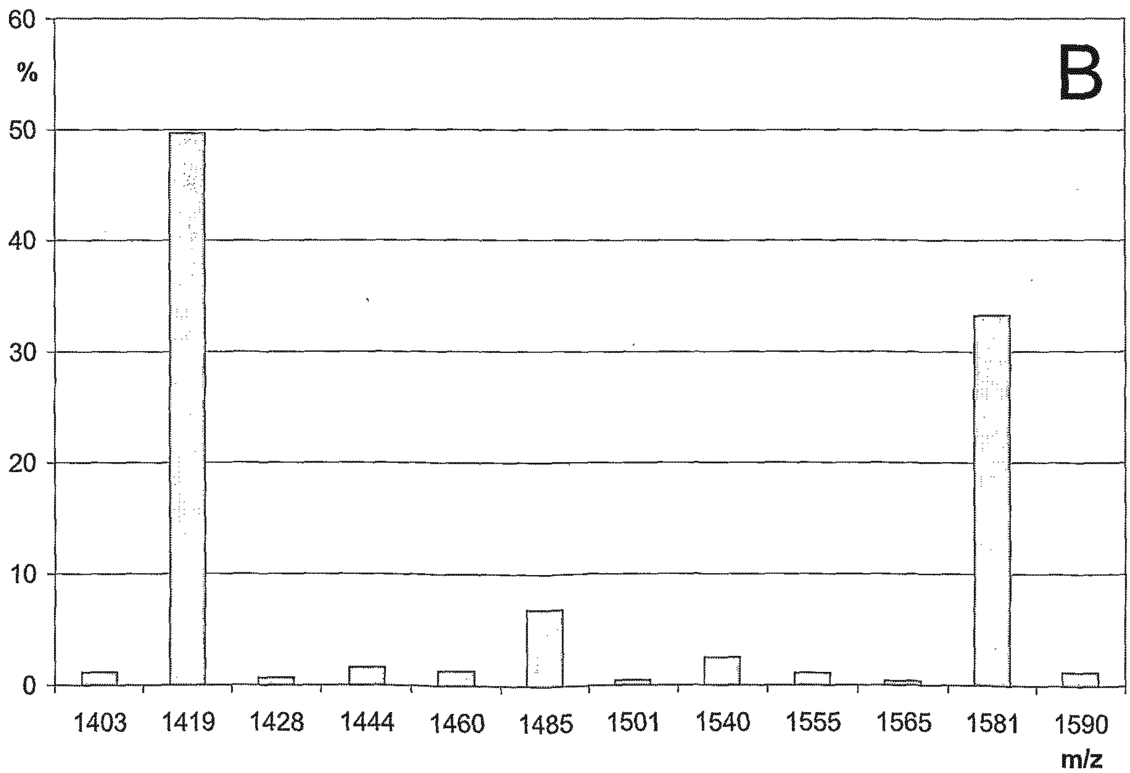
B
60
%
50
40
30
20
10
0
1403
1419
1428
1444
1460
1485
1501
1540
1555
1565
1581
1590
m/z

Figure 23.

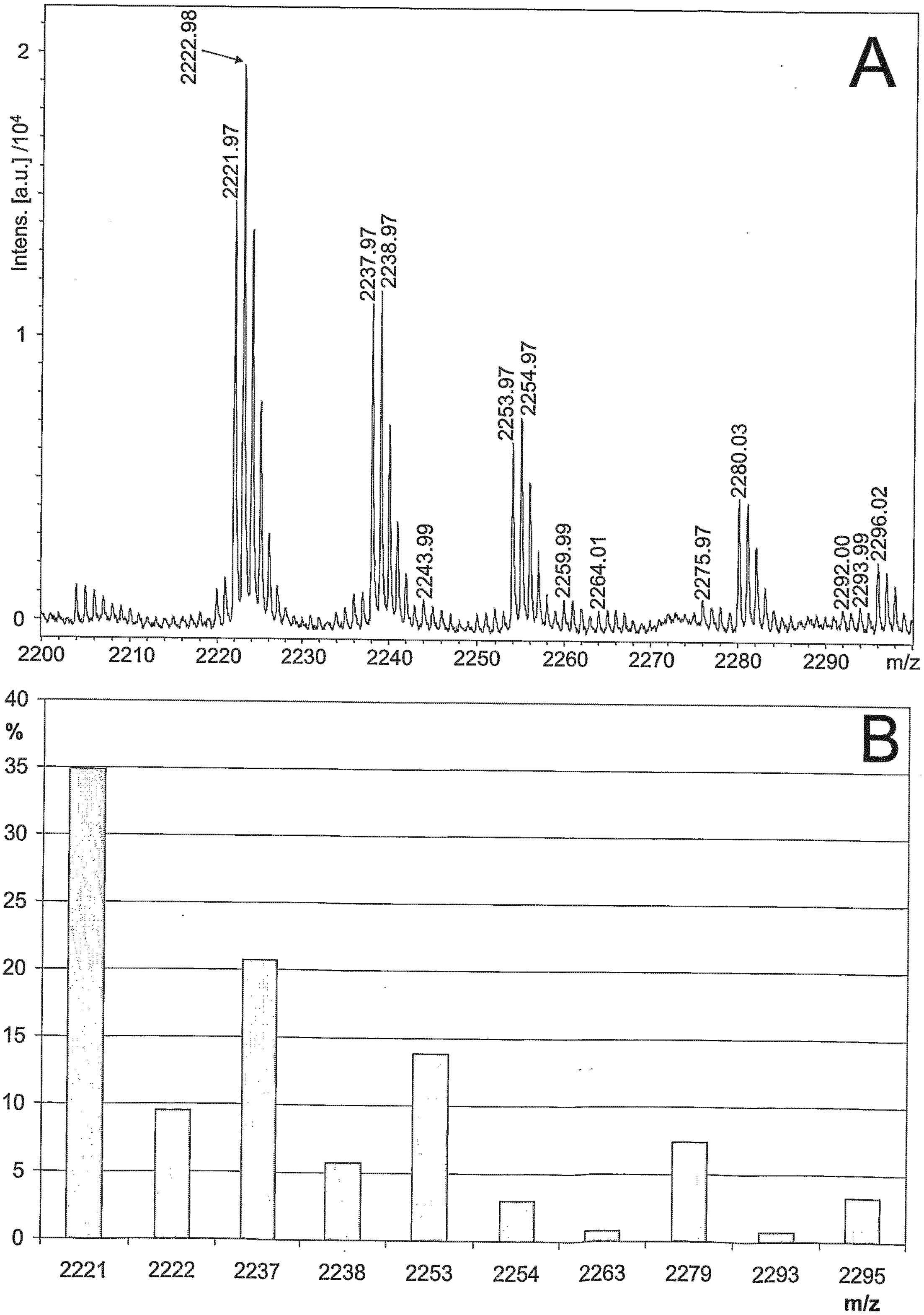
A
Intens. [a.u.] /10^4
2
1
0
2222.98
2221.97
2237.97
2238.97
2243.99
2253.97
2254.97
2259.99
2264.01
2275.97
2280.03
2292.00
2293.99
2296.02
2200
2210
2220
2230
2240
2250
2260
2270
2280
2290
m/z
B
%
40
35
30
25
20
15
10
5
0
2221
2222
2237
2238
2253
2254
2263
2279
2293
2295
m/z

Figure 24.

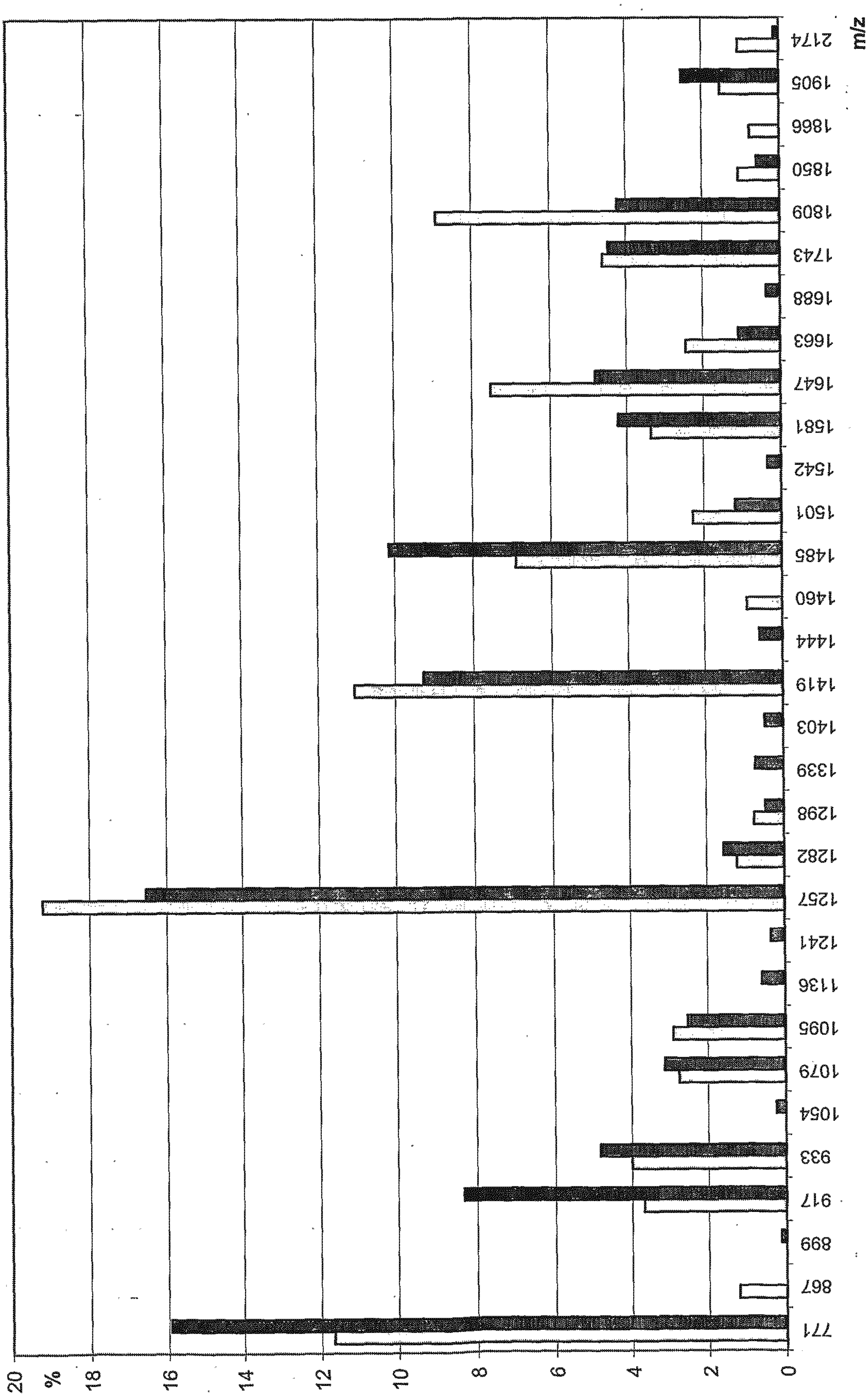
m/z
771
867
899
917
933
1054
1079
1095
1136
1241
1257
1282
1298
1339
1403
1419
1444
1460
1485
1501
1542
1581
1647
1663
1688
1743
1809
1850
1866
1905
2174
%
0
2
4
6
8
10
12
14
16
18
20

**Figure 25.**

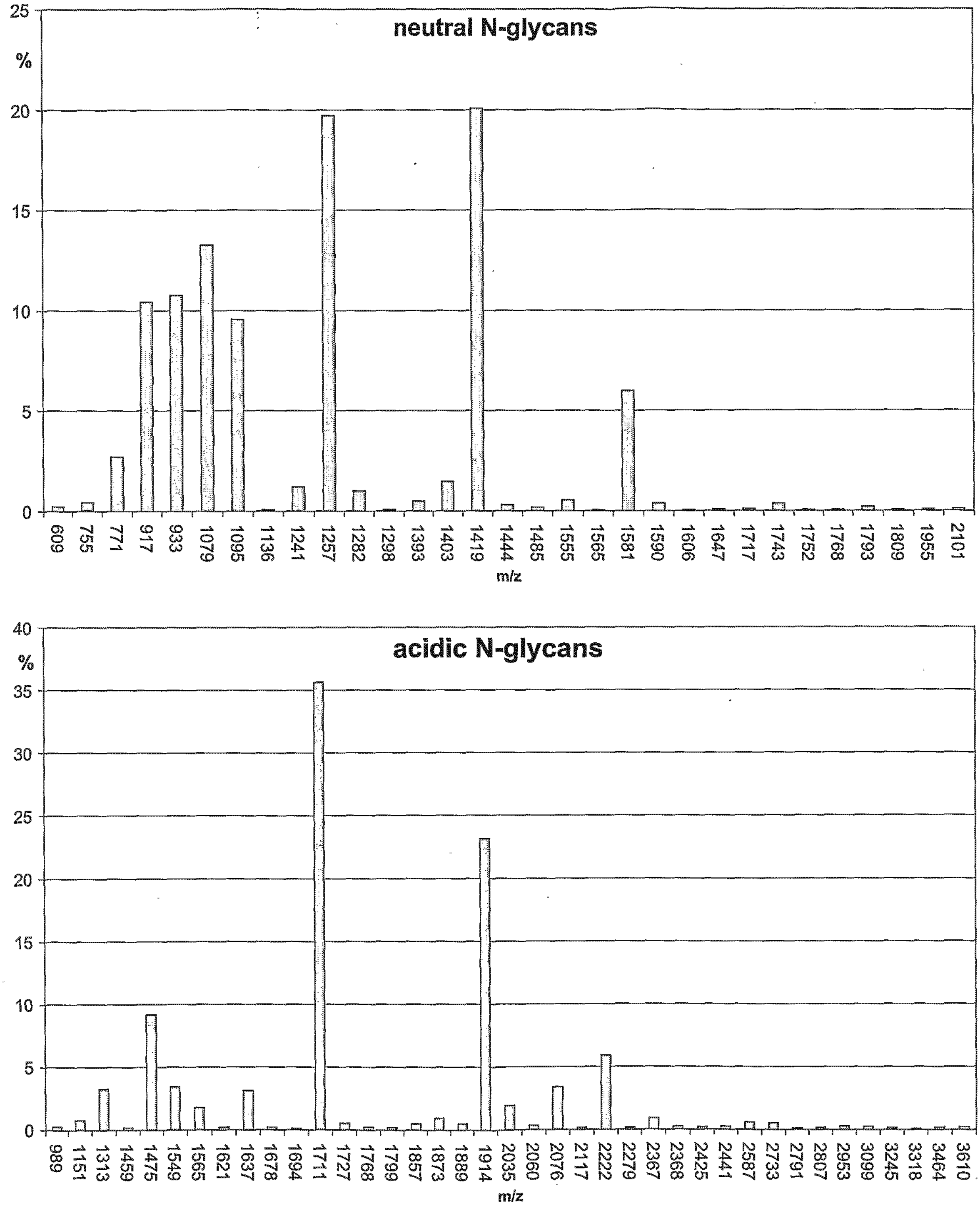
neutral N-glycans
%
25
20
15
10
5
0
609 755 771 917 933 1079 1095 1136 1241 1257 1282 1298 1393 1403 1419 1444 1485 1555 1565 1581 1590 1606 1647 1717 1743 1752 1768 1793 1809 1955 2101
m/z
acidic N-glycans
%
40
35
30
25
20
15
10
5
0
989 1151 1313 1459 1475 1549 1565 1621 1637 1678 1694 1711 1727 1768 1799 1857 1873 1889 1914 2035 2060 2076 2117 2222 2279 2367 2368 2425 2441 2587 2733 2791 2807 2953 3099 3245 3318 3464 3610
m/z

**Figure 26.**

**A**

```
                          a-D-Manp-(1-6)+
                                        |
                                  a-D-Manp-(1-6)+
                                        |        |
                          a-D-Manp-(1-3)+   b-D-Manp-(1-4)-b-D-GlcpNAc-(1-4)-D-GlcNAc
                                                 |
                                  a-D-Manp-(1-3)+
```

**B**

```
       a-D-Manp-(1-2)-a-D-Manp-(1-6)+
                                    |
                              a-D-Manp-(1-6)+
                                    |        |
       a-D-Manp-(1-2)-a-D-Manp-(1-3)+   b-D-Manp-(1-4)-b-D-GlcpNAc-(1-4)-D-GlcNAc
                                             |
a-D-Manp-(1-2)-a-D-Manp-(1-2)-a-D-Manp-(1-3)+
```

**C**

```
       a-D-Manp-(1-2)-a-D-Manp-(1-6)+
                                    |
                              a-D-Manp-(1-6)+
                                    |        |
                      a-D-Manp-(1-3)+   b-D-Manp-(1-4)-b-D-GlcpNAc-(1-4)-D-GlcNAc
                                             |
a-D-Manp-(1-2)-a-D-Manp-(1-2)-a-D-Manp-(1-3)+
```

**Figure 27.**

**D**

```
a-D-Manp-(1-6)+
               |
         b-D-Manp-(1-4)-b-D-GlcpNAc-(1-4)-D-GlcNAc
               |
a-D-Manp-(1-3)+
```

**E**

```
a-D-Manp-(1-6)+
               |
         b-D-Manp-(1-4)-D-GlcNAc
               |
a-D-Manp-(1-3)+
```

**F**

```
               a-D-Manp-(1-6)+
                              |
                        b-D-Manp-(1-4)-D-GlcNAc
                              |
a-D-Manp-(1-2)-a-D-Manp-(1-3)+
```

**G**

```
                              a-D-Manp-(1-6)+
                                             |
                                       b-D-Manp-(1-4)-D-GlcNAc
                                             |
a-D-Manp-(1-2)-a-D-Manp-(1-2)-a-D-Manp-(1-3)+
```

**Figure 28.**

**A**

```
a-D-Neup5Ac-(2-6)-b-D-Galp-(1-4)-b-D-GlcpNAc-(1-2)-a-D-Manp-(1-6)+
                                                                 |
                                                          b-D-Manp-(1-4)-b-D-GlcpNAc-(1-4)-D-GlcNAc
                                                                 |
a-D-Neup5Ac-(2-6)-b-D-Galp-(1-4)-b-D-GlcpNAc-(1-2)-a-D-Manp-(1-3)+
```

**B**

```
                 b-D-Galp-(1-4)-b-D-GlcpNAc-(1-2)-a-D-Manp-(1-6)+
                                                                 |
                                                          b-D-Manp-(1-4)-b-D-GlcpNAc-(1-4)-D-GlcNAc
                                                                 |
a-D-Neup5Ac-(2-3)-b-D-Galp-(1-3)-b-D-GlcpNAc-(1-2)-a-D-Manp-(1-3)+
```

**C**

```
                 b-D-Galp-(1-4)-b-D-GlcpNAc-(1-2)-a-D-Manp-(1-6)+
                                                                 |
                                                          b-D-Manp-(1-4)-b-D-GlcpNAc-(1-4)-D-GlcNAc
                                                                 |
a-D-Neup5Ac-(2-3)-b-D-Galp-(1-4)-b-D-GlcpNAc-(1-2)-a-D-Manp-(1-3)+
```

**D**

```
                 b-D-Galp-(1-4)-b-D-GlcpNAc-(1-2)-a-D-Manp-(1-6)+
                                                                 |
                                                          b-D-Manp-(1-4)-b-D-GlcpNAc-(1-4)-D-GlcNAc
                                                                 |
a-D-Neup5Ac-(2-6)-b-D-Galp-(1-4)-b-D-GlcpNAc-(1-2)-a-D-Manp-(1-3)+
```

**E**

```
                                                                                    a-L-Fucp-(1-6)+
                                                                                                  |
a-D-Neup5Ac-(2-3)-b-D-Galp-(1-4)-b-D-GlcpNAc-(1-2)-a-D-Manp-(1-6)+                        D-GlcNAc
                                                                 |                                |
                                                            b-D-Manp-(1-4)-b-D-GlcpNAc-(1-4)+
                                                                 |
a-D-Neup5Ac-(2-3)-b-D-Galp-(1-4)-b-D-GlcpNAc-(1-2)-a-D-Manp-(1-3)+
```

Figure 29.

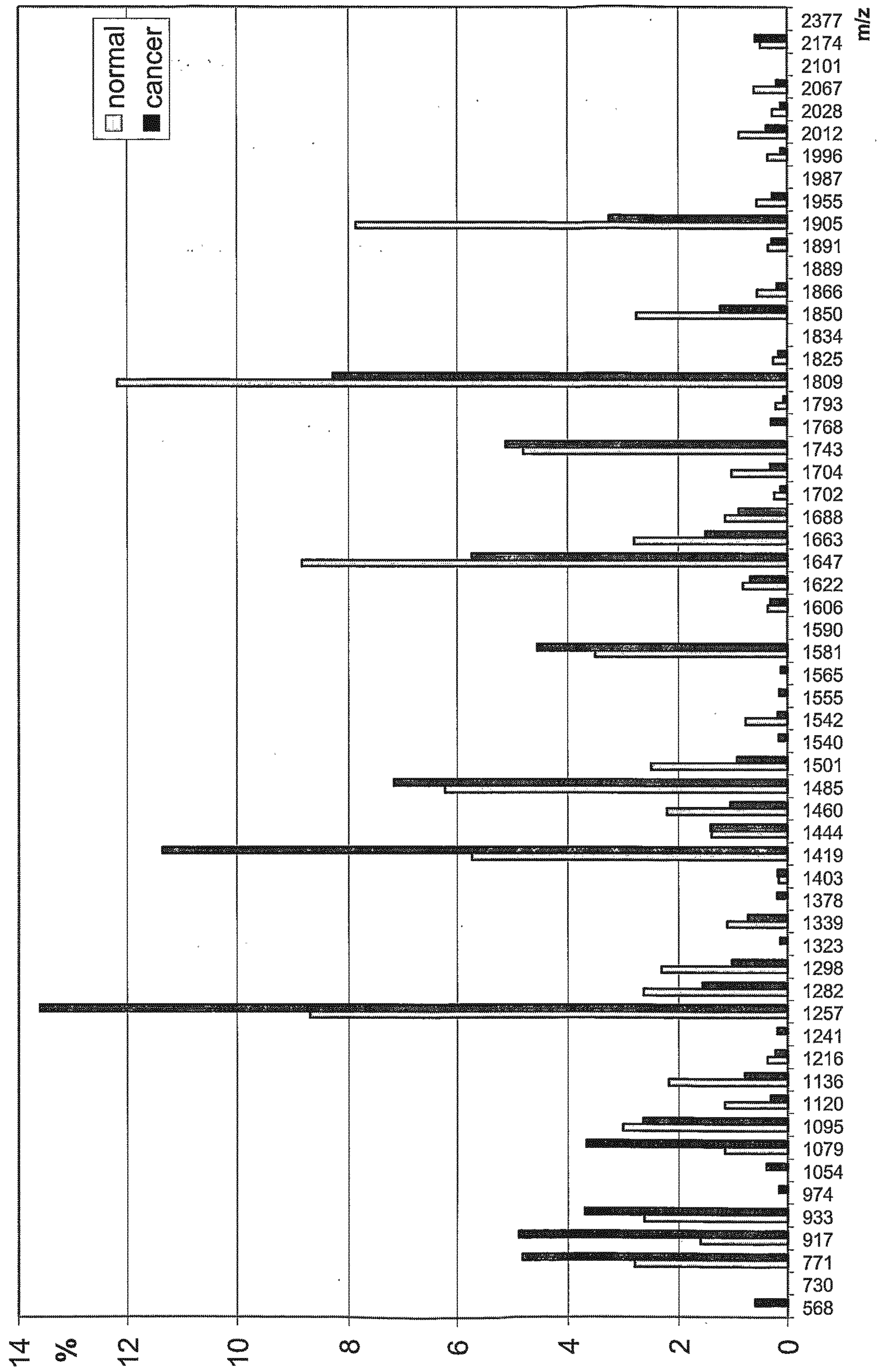
normal
cancer
%
14
12
10
8
6
4
2
0
m/z
2377
2174
2101
2067
2028
2012
1996
1987
1955
1905
1891
1889
1866
1850
1834
1825
1809
1793
1768
1743
1704
1702
1688
1663
1647
1622
1606
1590
1581
1565
1555
1542
1540
1501
1485
1460
1444
1419
1403
1378
1339
1323
1298
1282
1257
1241
1216
1136
1120
1095
1079
1054
974
933
917
771
730
568

**Figure 30.**

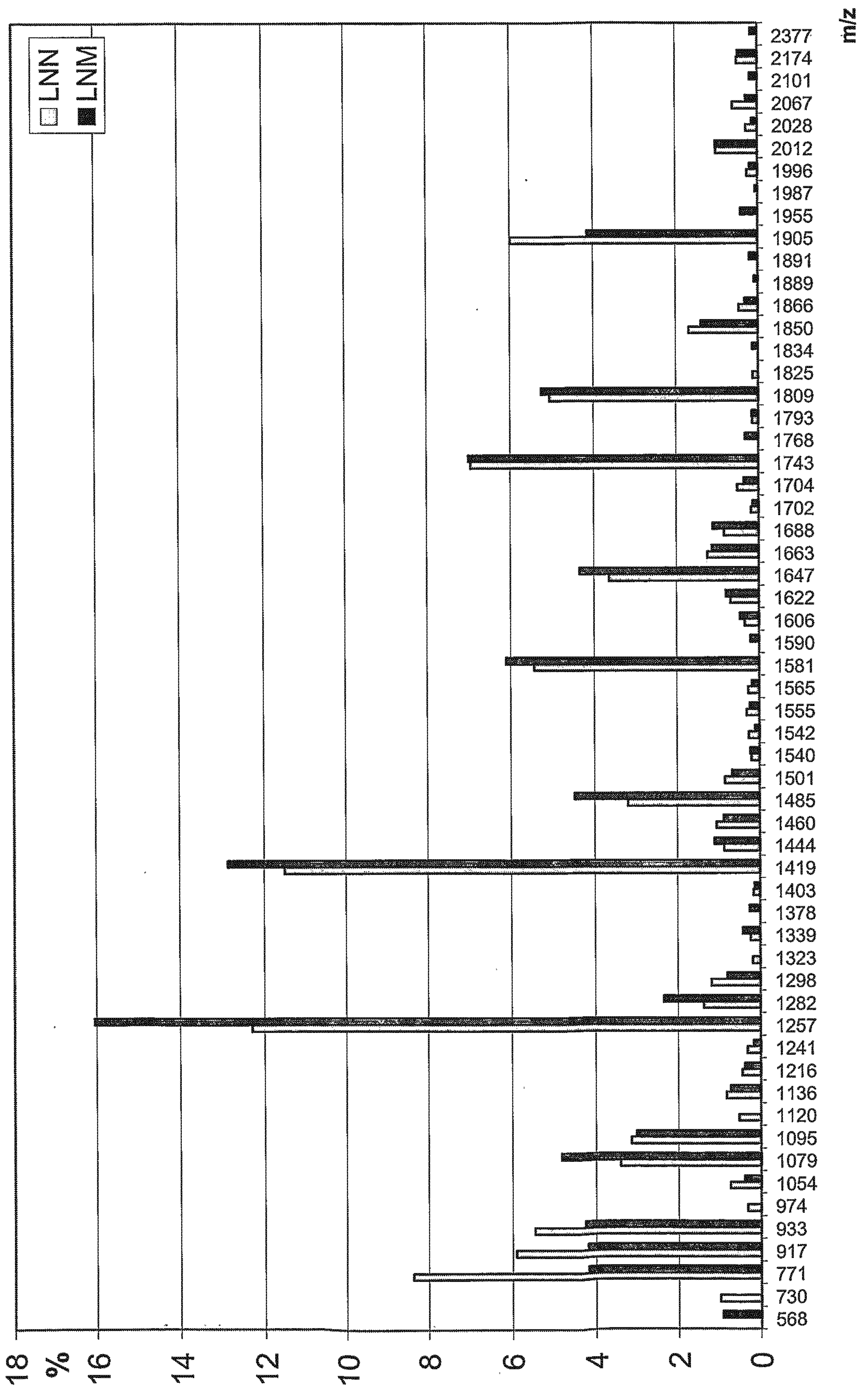
LNN
LNM
m/z
%
0
2
4
6
8
10
12
14
16
18
568
730
771
917
933
974
1054
1079
1095
1120
1136
1216
1241
1257
1282
1298
1323
1339
1378
1403
1419
1444
1460
1485
1501
1540
1542
1555
1565
1581
1590
1606
1622
1647
1663
1688
1702
1704
1743
1768
1793
1809
1825
1834
1850
1866
1889
1891
1905
1955
1987
1996
2012
2028
2067
2101
2174
2377

**Figure 31.**

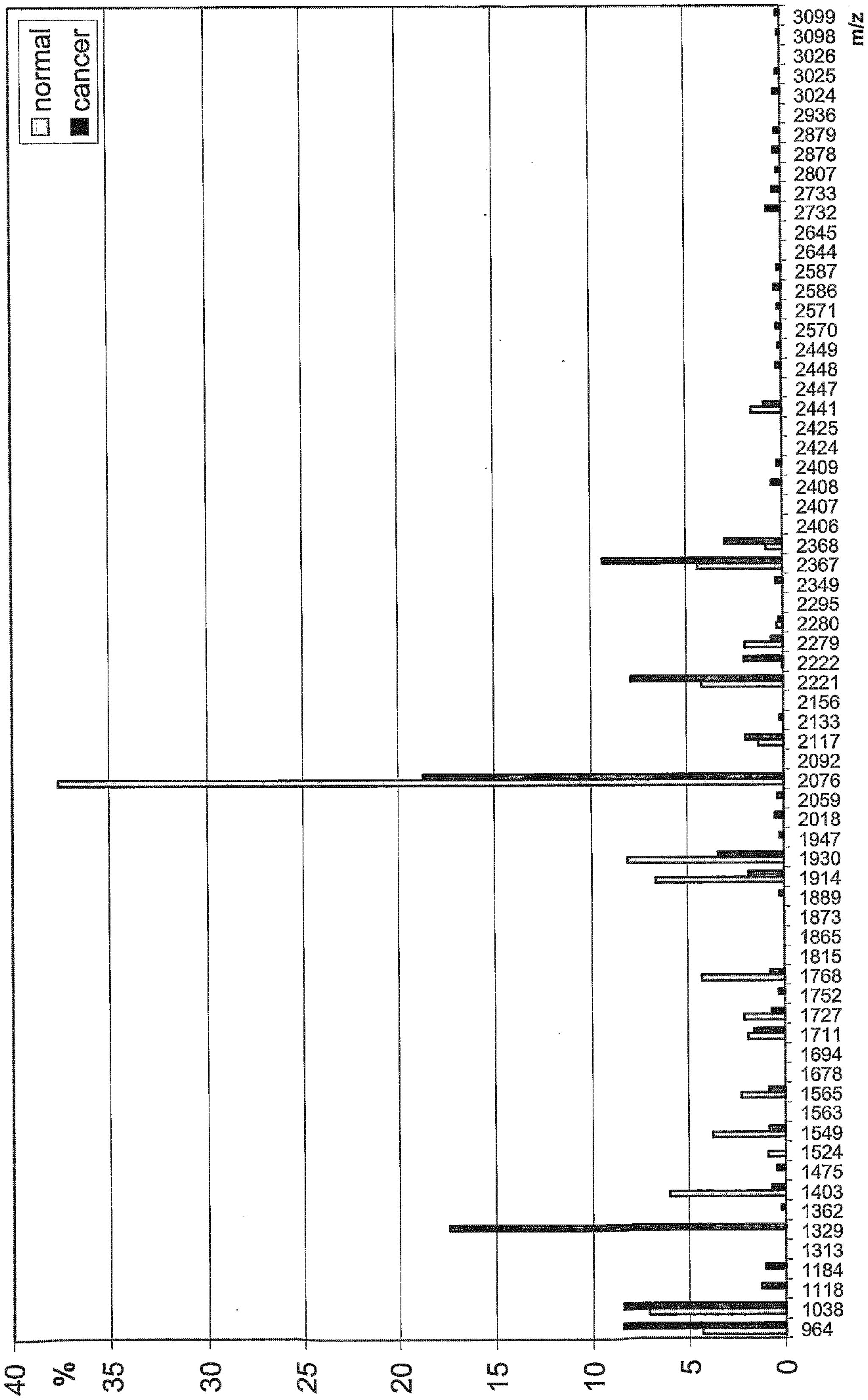
normal
cancer
%
40
35
30
25
20
15
10
5
0
m/z
964
1038
1118
1184
1313
1329
1362
1403
1475
1524
1549
1563
1565
1678
1694
1711
1727
1752
1768
1815
1865
1873
1889
1914
1930
1947
2018
2059
2076
2092
2117
2133
2156
2221
2222
2279
2280
2295
2349
2367
2368
2406
2407
2408
2409
2424
2425
2441
2447
2448
2449
2570
2571
2586
2587
2644
2645
2732
2733
2807
2878
2879
2936
3024
3025
3026
3098
3099

**Figure 32.**

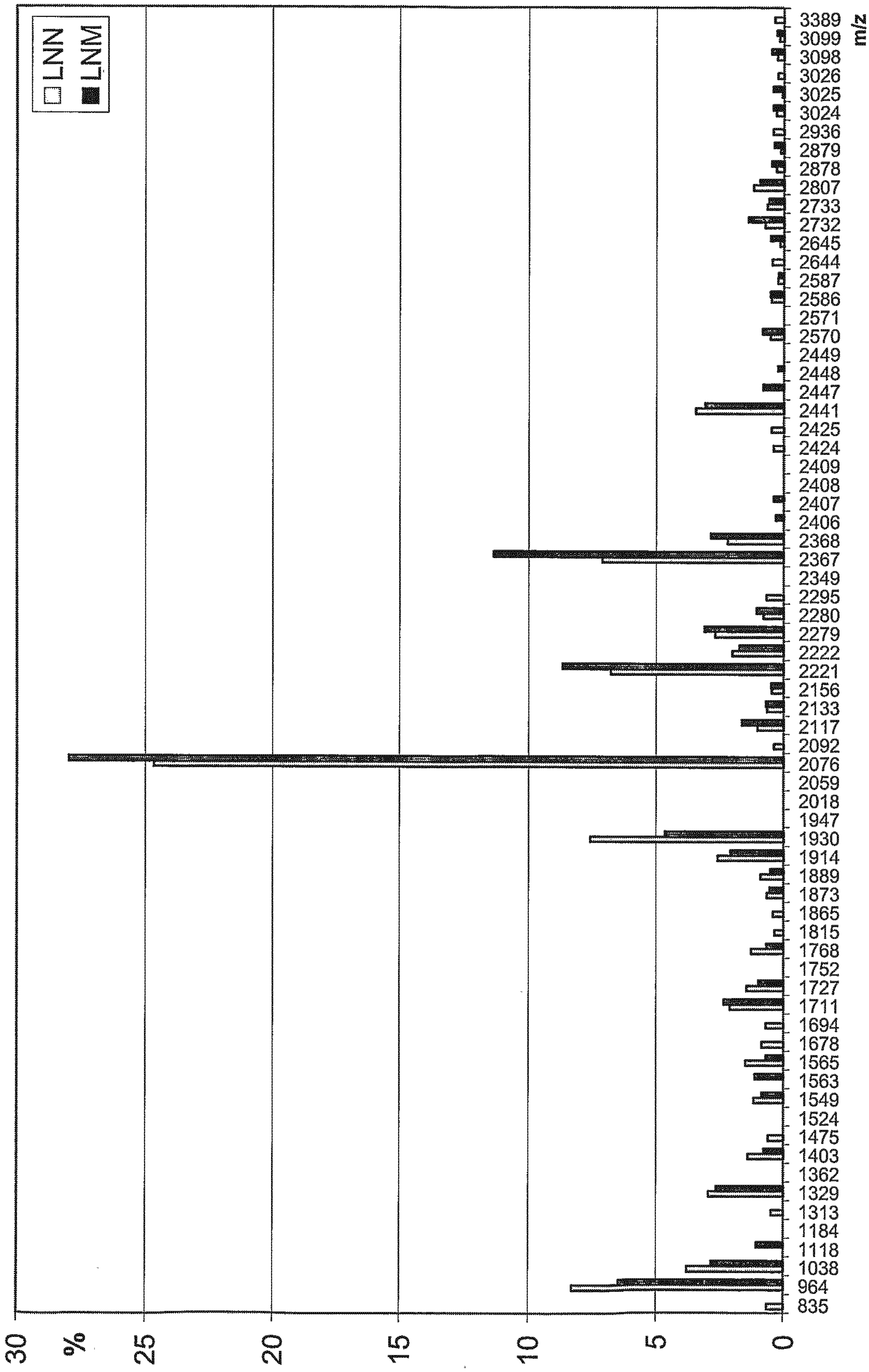
LNN
LNM
%
30
25
20
15
10
5
0
m/z
835
964
1038
1118
1184
1313
1329
1362
1403
1475
1524
1549
1563
1565
1678
1694
1711
1727
1752
1768
1815
1865
1873
1889
1914
1930
1947
2018
2059
2076
2092
2117
2133
2156
2221
2222
2279
2280
2295
2349
2367
2368
2406
2407
2408
2409
2424
2425
2441
2447
2448
2449
2570
2571
2586
2587
2644
2645
2732
2733
2807
2878
2879
2936
3024
3025
3026
3098
3099
3389

**Figure 33.**

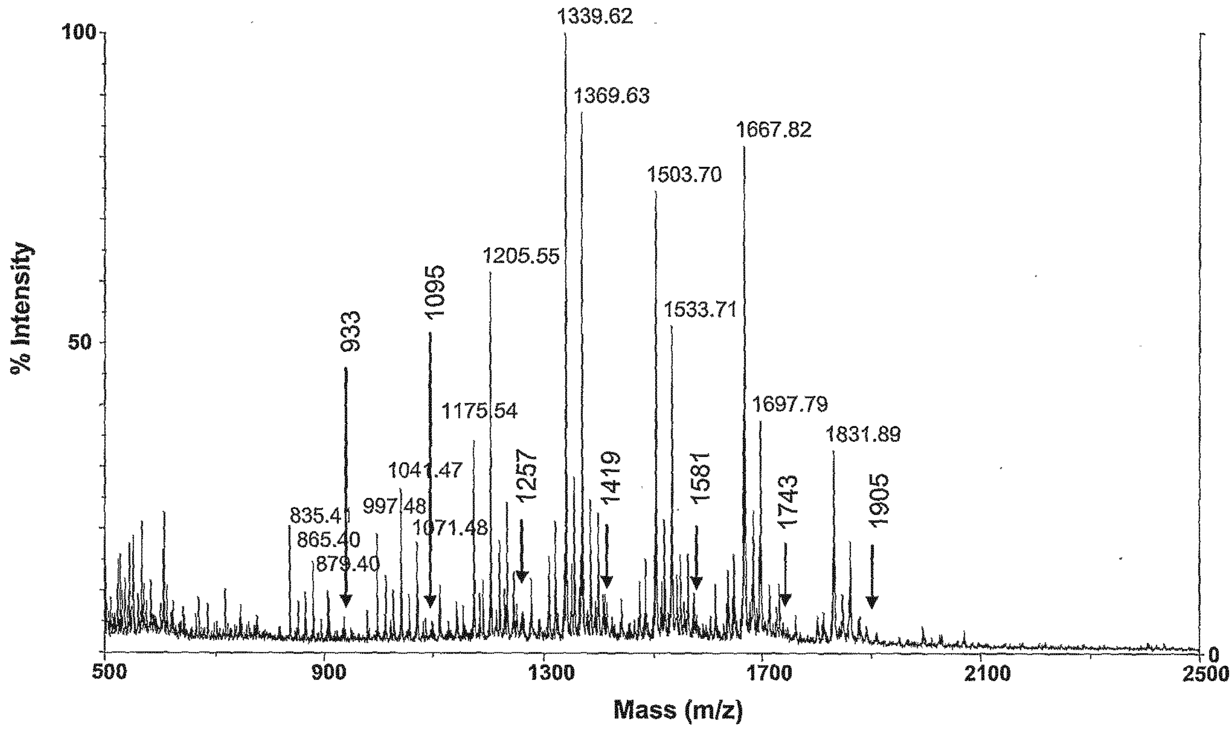
100
50
0
% Intensity
1339.62
1369.63
1667.82
1503.70
1205.55
1095
1533.71
933
1175.54
1697.79
1831.89
1041.47
1257
1419
1581
1743
1905
835.41
997.48
865.40
1071.48
879.40
500
900
1300
1700
2100
2500
Mass (m/z)

Figure 34.

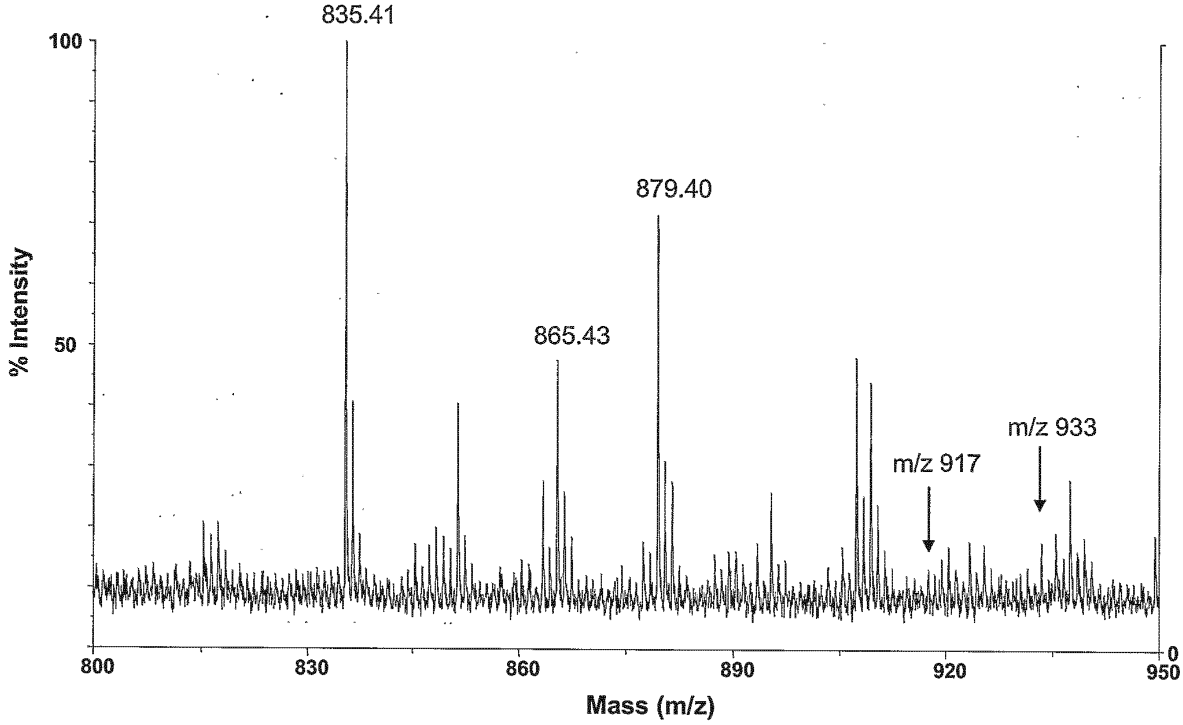


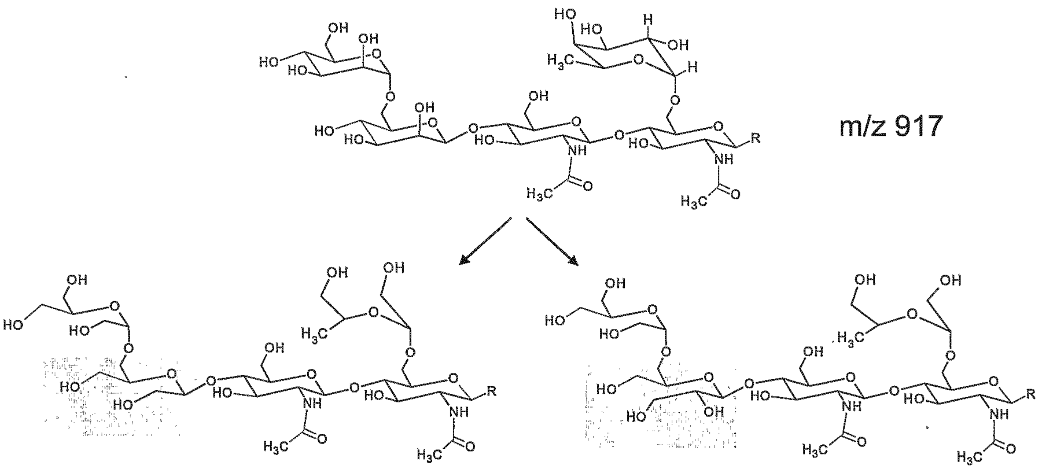

m/z 917

m/z 835

m/z 865

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5902725 A
- US 2004147033 A
- WO 0061636 A
- JP 10084963 B
- JP 6046880 B, Ryuichi Horie
- EP 0601859 A, Chung Y-S
- US 5874060 A
- US 6025481 A
- US 4851511 A
- US 4904596 A
- US 5795961 A
- US 4725 A
- US 557 A
- US 5059520 A
- US 5171667 A
- US 5173292 A
- US 6090789 A
- US 5708163 A
- US 6203999 B
- US 5102663 A
- US 5660834 A
- US 5747048 A
- US 5229289 A
- US 6083929 A


### Non-patent literature cited in the description


- **BROCKHAUSEN et al.** *Biochimica et Biophysica Acta - General Subjects,* 1999, vol. 1473 (1), 67-95
- **MUELLER et al.** *Journal of Biological Chemistry,* 2002, vol. 277 (29), 26103-26112
- **YOUSEFI et al.** *Journal of Biological Chemistry,* 1991, vol. 266 (3), 1772-1782
- **BURCHELL et al.** *Glycobiology,* 1999, vol. 9 (12), 1307-1311
- **DUBE et al.** *Nature Reviews. Drug discovery,* 2005, vol. 4 (6), 477-488
- **KUMAMOTO et al.** *Biochemical and Biophysical Research Communications,* 1998, vol. 247 (2), 514-517
- **GUTIÉRREZ GALLEGO et al.** *Biochimie,* 2003, vol. 85 (3-4), 275-286
- **LLOYD et al.** *Journal of Biological Chemistry,* 1996, vol. 271 (52), 33325-33334
- **MEICHENIN et al.** *Cancer Research,* 2000, vol. 60, 5499-5507
- **PAZYNINA et al.** *Mendeleev Communications,* 2002, vol. 12 (5), 183-184
- **BURCHELL et al.** *Journal of Mammary Gland Biology and Neoplasia,* 2001, vol. 6 (3), 355-364
- **ELLIOT et al.** *Endocrine,* 1997, vol. 7 (1), 15-32
- **MIZUOCHI et al.** *Journal of Biological Chemistry,* 1983, vol. 258 (23), 14126-14129
- **SANDEE D. et al.** *J. Biosci. Bioengin.,* 1993, 266-273
- **WALCHECK B. et al.** *Blood,* 2002, vol. 99, 4063-69
- IUPAC-IUB Commission on Biochemical Nomenclature. Carbohydr. Res. 1998, vol. 322, 167
- *Carbohydr. Res.,* 1997, vol. 297, 1
- *Eur. J. Biochem.,* 1998, vol. 257, 29
- **FU D. ; CHEN L. ; O'NEILL R.A.** *Carbohydr. Res.,* 1994, vol. 261, 173-186
- **HARD K. ; MEKKING A. ; KAMERLING J.P ; DACREMONT G.A.A. ; VLIEGENTHART J.F.G.** *Glycoconjugate J.,* 1991, vol. 8, 17-28
- **HARD K. ; VAN ZADELHOFF G. ; MOONEN P. ; KAMERLING J.P ; VLIEGENTHART J.F.G.** *Eur. J. Biochem.,* 1992, vol. 209, 895-915
- **HELIN J. ; MAAHEIMO H. ; SEPPO A. ; KEANE A. ; RENKONEN O.** *Carbohydr. Res.,* 1995, vol. 266, 191-209
- **ARAP, W. et al.** *Science,* 1998, vol. 279 (5349), 377-80
- **BROCKHAUSEN, I.** *Biochim. Biophys. Acta,* 1999, vol. 1473 (1), 67-95
- **DAVIES et al.** *J. Chromatogr.,* 1992, vol. 609 (1-2), 125-31
- **DOMON, B. ; COSTELLO, C.E.** *Glycoconj. J.,* 1988, vol. 5, 397-409
- **DUBE, D.H. ; BERTOZZI, C.R.** *Nat. Rev. Drug Discov.,* 2005, vol. 4 (6), 477-88
- Carbohydrates in chemistry and biology. Weiley-VHC, 2000
- **FERNANDEZ, L.E. et al.** *Expert Rev. Vaccines,* 2003, vol. 2 (6), 817-23
- **HARVEY, D.J. et al.** *Rapid Commun. Mass Spectrom.,* 1993, vol. 7 (7), 614-9
- **HOLMBERG, L.A. ; SANDMEIER, B.M.** *Expert Opin. Biol. Ther.,* 2001, vol. 1 (5), 881-91

- **HUANG, Y. et al.** *Anal. Chem.,* 2001, vol. 73 (24), 6063-9
- **KUROKAWA, T. et al.** *Eur. J. Biochem.,* 2002, vol. 269, 5459-73
- **NAVEN, T.J. ; HARVEY, D.J.** *Rapid Commun. Mass Spectrom.,* 1996, vol. 10 (11), 1361-6
- **NYMAN, T.A. et al.** *Eur. J. Biochem.,* 1998, vol. 253 (2), 485-93
- **PAPAC, D.I. et al.** *Anal. Chem.,* 1996, vol. 68 (18), 3215-23
- **PACKER, N.H. et al.** *Glycoconj. J.,* 1998, vol. 15 (8), 737-47
- **PLUMMER, T.H. JR. ; TARENTINO, A.L.** *Glycobiology,* 1991, vol. 1 (3), 257-63
- **POWELL, A.K. ; HARVEY, D.J.** *Rapid Commun. Mass Spectrom.,* 1996, vol. 10 (9), 1027-32
- **SAARINEN, J. et al.** *Eur. J. Biochem.,* 1999, vol. 259 (3), 829-40
- **VEROSTEK et al.** *Anal. Biochem.,* 2000, vol. 278 (2), 111-22
- **YLÖNEN, A. et al.** *Glycobiology,* 2001, vol. 11 (7), 523-31